(19) Europäisches Patentamt
European Patent Office
Office européen des brevets

(11) EP 3 243 504 A1

(12) EUROPEAN PATENT APPLICATION

(43) Date of publication:
15.11.2017 Bulletin 2017/46

(51) Int Cl.:
*A61K 9/127* (2006.01) *A61K 48/00* (2006.01)

(21) Application number: 17000214.1

(22) Date of filing: 29.01.2010

(84) Designated Contracting States:
AT BE BG CH CY CZ DE DK EE ES FI FR GB GR HR HU IE IS IT LI LT LU LV MC MK MT NL NO PL PT RO SE SI SK SM TR

(30) Priority: 29.01.2009 US 148366 P
02.03.2009 US 156851 P
10.06.2009 US 185712 P
24.07.2009 US 228373 P
03.09.2009 US 239686 P

(62) Document number(s) of the earlier application(s) in accordance with Art. 76 EPC:
10703734.3 / 2 391 343

(71) Applicant: Arbutus Biopharma Corporation
Burnaby, BC V5J 5J8 (CA)

(72) Inventors:
- Akinc, Akin
Cambridge, MA 02142 (US)
- Querbes, William
Cambridge, MA 02142 (US)
- Wong, Frances
Cambridge, MA 02142 (US)
- Dorkin, Joseph, Robert
Cambridge, MA 02142 (US)
- Qin, Xiaojun
Cambridge, MA 02142 (US)
- Cantley, William
Cambridge, MA 02142 (US)
- Borodovsky, Anna
Cambridge, MA 02142 (US)
- De, Soma
Cambridge, MA 02142 (US)
- Manoharan, Muthiah
Cambridge, MA 02142 (US)
- Jayaraman, Muthusamy
Cambridge, MA 02142 (US)
- Rajeev, Kallanthottathil, G.
Cambridge, MA 02142 (US)

(74) Representative: Müller-Boré & Partner
Patentanwälte PartG mbB
Friedenheimer Brücke 21
80639 München (DE)

Remarks:
•Claims filed after the date of receipt of the application (Rule 68(4) EPC).
•This application was received on 10-02-2017 as a divisional application to the application mentioned under INID code 62.

# (54) IMPROVED LIPID FORMULATION

(57) The invention features an improved lipid formulation comprising a cationic lipid of formula (A), a neutral lipid, a sterol and a PEG or PEG-modified lipid, where $R_1$ and $R_2$ are independently alkyl, alkenyl or alkynyl, each can be optionally substituted, and $R_3$ and $R_4$ are independently lower alkyl or $R_3$ and $R_4$ can be taken together to form an optionally substituted heterocyclic ring. In one embodiment, $R_1$ and $R_2$ are independently selected from oleoyl, pamitoyl, steroyl, linoleyl and $R_3$ and $R_4$ are methyl. Also disclosed are targeting lipids, and specific lipid formulations comprising such targeting lipids.

EP 3 243 504 A1

## Description

### Claim of Priority

This application claims priority from U.S.S.N. 61/148,366, filed January 29, 2009; U.S.S.N. 61/156,851, filed March 2, 2009; U.S.S.N. 61/185,712, filed June 10, 2009; U.S.S.N. 61/228,373, filed July 24, 2009; and U.S.S.N. 61/239,686, filed September 3, 2009, each of which is incorporated by reference in its entirety.

### Technical Field

The invention relates to the field of therapeutic agent delivery using lipid particles. In particular, the invention provides cationic lipids and lipid particles comprising these lipids, which are advantageous for the *in vivo* delivery of nucleic acids, as well as nucleic acid-lipid particle compositions suitable for *in vivo* therapeutic use. Additionally, the invention provides methods of preparing these compositions, as well as methods of introducing nucleic acids into cells using these compositions, e.g., for the treatment of various disease conditions.

### Description of the Related Art

Therapeutic nucleic acids include, e.g., small interfering RNA (siRNA), micro RNA (miRNA), antisense oligonucleotides, ribozymes, plasmids, and immune stimulating nucleic acids. These nucleic acids act via a variety of mechanisms. In the case of siRNA or miRNA, these nucleic acids can down-regulate intracellular levels of specific proteins through a process termed RNA interference (RNAi). Following introduction of siRNA or miRNA into the cell cytoplasm, these double-stranded RNA constructs can bind to a protein termed RISC. The sense strand of the siRNA or miRNA is displaced from the RISC complex providing a template within RISC that can recognize and bind mRNA with a complementary sequence to that of the bound siRNA or miRNA. Having bound the complementary mRNA the RISC complex cleaves the mRNA and releases the cleaved strands. RNAi can provide down-regulation of specific proteins by targeting specific destruction of the corresponding mRNA that encodes for protein synthesis.

The therapeutic applications of RNAi are extremely broad, since siRNA and miRNA constructs can be synthesized with any nucleotide sequence directed against a target protein. To date, siRNA constructs have shown the ability to specifically down-regulate target proteins in both *in vitro* and *in vivo* models. In addition, siRNA constructs are currently being evaluated in clinical studies.

However, two problems currently faced by siRNA or miRNA constructs are, first, their susceptibility to nuclease digestion in plasma and, second, their limited ability to gain access to the intracellular compartment where they can bind RISC when administered systemically as the free siRNA or miRNA. These double-stranded constructs can be stabilized by incorporation of chemically modified nucleotide linkers within the molecule, for example, phosphothioate groups. However, these chemical modifications provide only limited protection from nuclease digestion and may decrease the activity of the construct. Intracellular delivery of siRNA or miRNA can be facilitated by use of carrier systems such as polymers, cationic liposomes or by chemical modification of the construct, for example by the covalent attachment of cholesterol molecules. However, improved delivery systems are required to increase the potency of siRNA and miRNA molecules and reduce or eliminate the requirement for chemical modification.

Antisense oligonucleotides and ribozymes can also inhibit mRNA translation into protein. In the case of antisense constructs, these single stranded deoxynucleic acids have a complementary sequence to that of the target protein mRNA and can bind to the mRNA by Watson-Crick base pairing. This binding either prevents translation of the target mRNA and/or triggers RNase H degradation of the mRNA transcripts. Consequently, antisense oligonucleotides have tremendous potential for specificity of action (*i.e.*, down-regulation of a specific disease-related protein). To date, these compounds have shown promise in several *in vitro* and *in vivo* models, including models of inflammatory disease, cancer, and HIV (reviewed in Agrawal, Trends in Biotech. 14:376-387 (1996)). Antisense can also affect cellular activity by hybridizing specifically with chromosomal DNA. Advanced human clinical assessments of several antisense drugs are currently underway. Targets for these drugs include the bcl2 and apolipoprotein B genes and mRNA products.

One well known problem with the use of therapeutic nucleic acids relates to the stability of the phosphodiester internucleotide linkage and the susceptibility of this linker to nucleases. The presence of exonucleases and endonucleases in serum results in the rapid digestion of nucleic acids possessing phosphodiester linkers and, hence, therapeutic nucleic acids can have very short half-lives in the presence of serum or within cells. (Zelphati, O., et al., Antisense. Res. Dev. 3:323-338 (1993); and Thierry, A.R., et al., pp147-161 in Gene Regulation: Biology of Antisense RNA and DNA (Eds. Erickson, RP and Izant, JG; Raven Press, NY (1992)). Therapeutic nucleic acid being currently being developed do not employ the basic phosphodiester chemistry found in natural nucleic acids, because of these and other known problems.

This problem has been partially overcome by chemical modifications that reduce serum or intracellular degradation. Modifications have been tested at the internucleotide phosphodiester bridge (*e.g.*, using phosphorothioate, methylphosphonate or phosphoramidate linkages), at the nucleotide base (*e.g.,* 5-propynyl-pyrimidines), or at the sugar (*e.g.,* 2'-modified sugars) (Uhlmann E., et al. Antisense: Chemical Modifications. Encyclopedia of Cancer, Vol. X., pp 64-81 Academic Press Inc. (1997)). Others have attempted to improve stability using 2'-5' sugar linkages (*see, e.g.,* US Pat. No. 5,532,130). Other changes have been attempted. However, none of these solutions have proven entirely satisfactory, and *in vivo* free therapeutic nucleic acids still have only limited efficacy.

In addition, as noted above relating to siRNA and miRNA, problems remain with the limited ability of therapeutic nucleic acids to cross cellular membranes (see, Vlassov, et al., Biochim. Biophys. Acta 1197:95-1082 (1994)) and in the problems associated with systemic toxicity, such as complement-mediated anaphylaxis, altered coagulatory properties, and cytopenia (Galbraith, et al., Antisense Nucl. Acid Drug Des. 4:201-206 (1994)).

In spite of recent progress, there remains a need in the art for improved lipid-therapeutic nucleic acid compositions that are suitable for general therapeutic use. Preferably, these compositions would encapsulate nucleic acids with high-efficiency, have high drug:lipid ratios, protect the encapsulated nucleic acid from degradation and clearance in serum, be suitable for systemic delivery, and provide intracellular delivery of the encapsulated nucleic acid. In addition, these lipid-nucleic acid particles should be well-tolerated and provide an adequate therapeutic index, such that patient treatment at an effective dose of the nucleic acid is not associated with significant toxicity and/or risk to the patient. The invention provides such compositions, methods of making the compositions, and methods of using the compositions to introduce nucleic acids into cells, including for the treatment of diseases.

## Summary of Invention

In one aspect, the invention provides improved lipid formulations comprising a cationic lipid of formula A, a neutral lipid, a sterol and a PEG or PEG-modified lipid, wherein formula A is

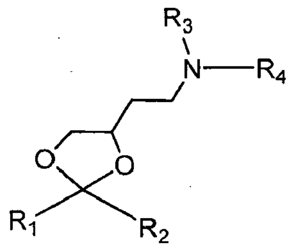

,

where $R_1$ and $R_2$ are independently alkyl, alkenyl or alkynyl, each can be optionally substituted, and $R_3$ and $R_4$ are independently lower alkyl or $R_3$ and $R_4$ can be taken together to form an optionally subsituted heterocyclic ring. In one embodiment, $R_1$ and $R_2$ are independently selected from oleoyl, pamitoyl, steroyl, linoleyl and $R_3$ and $R_4$ are methyl.

In one aspect, the improved lipid formulation also includes a targeting lipid (e.g., a GalNAc and/or folate containing lipid).

In one aspect, the invention provides preparation for the improved lipid formulations via an extrusion or an in-line mixing method.

In one aspect, the invention further provides a method of administering the improved lipid formulations containing RNA-based construct to an animal, and evaluating the expression of the target gene.

In one aspect, a lipid formulation featured in the invention, such as a lipid formulation complexed with an oligonucleotide, such as a double stranded RNA (dsRNA), can be used to modify (e.g., decrease) target gene expression in a tumor cell in vivo or in vitro. In some embodiments, a lipid formulation featured in the invention can be used to modify target gene expression in a tumor cell line, including but not limited to HeLa, HCT116, A375, MCF7, B16F10, Hep3b, HUH7, HepG2, Skov3, U87, and PC3 cell lines.

## Brief Description of the Figures

FIG. 1 is a flow chart of the extrusion method.
FIG. 2 is a flow chart of the in-line mixing method.
FIG. 3 is a schematic of a pump set-up.
FIG. 4 is a graph showing the relative FVII protein with various lipid ratios.
FIG. 5 is a graph showing the effect on body weight change with various lipid ratios.

FIG. 6 is a graph illustrating the relative FVII protein with different amount of cationic lipid A and low PEG lipid.

FIG. 7 is a graph showing the effect on body weight change with different amount of cationic lipid A and low PEG lipid.

FIG. 8 is a graph illustrating the relative FVII protein with different types of phosphatidylcholine.

FIG. 9 is a graph illustrating the relative FVII protein with high mol% of cationic lipid A.

FIG. 10 is a graph illustrating the relative FVII protein with different cholesterol:PEG ratios.

FIG. 11 is a graph illustrating the relative FVII protein at different pH levels.

FIG. 12 is a graph showing the relative FVII protein with various lipid ratios prepared *via* an in-line mixing method.

FIG. 13 is a graph showing the relative FVII protein at different charge ratios.

FIG. 14 is a graph showing the efficacy of various formulations in mouse.

FIGs. 15a and 15b are graphs showing the efficacy of various formulations in rat; (a) formulations preprared via an extrusion process; (b) formulations prepared via an in-line mixing process.

FIGs. 16a-16c compare the effect of ApoE pre-association on (a) LNP01, (b) SNALP, (c) LNP05.

FIG. 17 depicts graphs that show the ApoE dependence of efficacy of formulations comprising LNP08. Wildtype but not ApoE knockout mice showed dose-dependent reduction in FVII protein levels.

FIG. 18 depicts a graph that demonstrates that ApoE dependence of the LNP09 liposomal formulation and the lack of silencing in ApoE KO mice using LNP09 can be effectively rescued by premixing with ApoE.

FIGs. 19a and 19b depict graphs that demonstrate in vivo results of a mouse FVII silencing model, wherein LNP08 formulations also containing varying amounts GalNAc3-DSG or GalNAc3-PEG-DSG are administered to ApoE deficient (KO) mice.

FIG. 20 is a graph showing the efficacy of Lipid A liposomal formulations containing $(GalNAc)_3$-PEG-LCO in ApoE KO mice.

FIG. 21 is a graph showing the efficacy of Lipid A liposomal formulations containing $(GalNAc)_3$-PEG-DSG in ApoE KO mice.

FIG. 22 is a graph showing the effect of precomplexing with varying amounts of ApoE on the uptake of LNP01 and LNP08 formulations in Hep3B cells (4 hours incubation).

FIG. 23 depicts increased uptake of siRNA as well as lipid carrier in the presence of ApoE in Hep3B cells as demonstrated by BODIPY labeling of lipid A.

FIG. 24 depicts the effect of ApoE on silencing in HeLa-GFP cells (20nM with serum). ApoE was pre-complexed with liposomes for 10 minutes at 37°C..

FIG. 25 depicts a graph that demonstrates the effect of ApoE on silencing in HeLa cells (20nM serum-free DMEM). ApoE was pre-complexed with liposomes for 1 hour at 37°C.

FIG. 26 is a graph showing that other ApoE isoforms, ApoE2 or ApoE4, enhance LNP08 silencing comparably to ApoE3 in HeLa cells.

FIG. 27 is a graph showing the uptake of folate liposome in KB cells as demonstrated by FACS.

FIG. 28 is a graph showing the uptake of liposomes containing folate conjugated lipids in KB cells as demonstrated by microscopy.

FIGs. 29a and 29b show silencing of GFP mediated by liposomal formulations containing folate conjugated lipids (a) in the presence of serum or (b) in the absence of serum.

FIG. 30 is a bar graph illustrating the levels of relative serum FVII protein in a dose response study.

FIG. 31 is a bar graph showing the efficacy of Lipid A liposomal formulations containing GalNAc3 in ApoE wildtype mice.

FIG. 32 is a graph showing the time-dependent degradation of Lipid A liposomal formulation in 100 mM NaOAc buffer (pH=5).

FIG. 33 a graph showing the effect of BHT on inhibition of the degration of Lipid A liposomal formulation.

FIG. 34 is a graph showing the effect of vitamine E on inhibition of the degration of Lipid A liposomal formulation.

FIG. 35 is a graph showing the effect of LNP09 on Serum FVII protein levels in wildtype and LDLR KO mice.

FIG. 36 is a graph showing the effect of LNP09 in which 0.5 mol% of the PEG-DMG was replaced with GALNac3-PEG-lipid on Serum FVII protein levels in wildtype and LDLR KO mice.

## Detailed Description

Described herein is an improved lipid formulation, which can be used, for example, as a delivering an agent, e.g., a nucleic acid-based agent, such as an RNA-based construct, to a cell or subject. Also described herein are methods of administering the improved lipid formulations containing an RNA-based construct to an animal, and in some embodiments, evaluating the expression of the target gene. In some embodiments the improved lipid formulation includes a targeting lipid (e.g., a targeting lipid described herein such as a GalNAc or folate containing lipid).

The invention provides improved lipid formulations comprising a cationic lipid of formula A, a neutral lipid, a sterol and a PEG or PEG-modified lipid, wherein formula A is

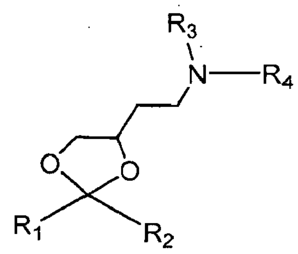

,

where $R_1$ and $R_2$ are independently alkyl, alkenyl or alkynyl, each can be optionally substituted, and $R_3$ and $R_4$ are independently lower alkyl or $R_3$ and $R_4$ can be taken together to form an optionally subsituted heterocyclic ring. In one embodiment, $R_1$ and $R_2$ are independently selected from oleoyl, pamitoyl, steroyl, linoleyl and $R_3$ and $R_4$ are methyl. In one embodiment, $R_1$ and $R_2$ are linoleyl. In one embodiments, $R_1$ and $R_2$ are linoleyl and $R_3$ and $R_4$ are methyl.

In one embodiment, the formulation include from about 25% to about 75% on a molar basis of cationic lipid of formula A e.g., from about 35 to about 65%, from about 45 to about 65%, about 60%, about 57.5%, about 57.1%, about 50% or about 40% on a molar basis. In one embodiment, the cationic lipid of formula A is 2,2-Dilinoleyl-4-dimethylaminoethyl-[1,3]-dioxolane (Lipid A).

In one embodiment, the formuation includes from about 0% to about 15% on a molar basis of the neutral lipid e.g., from about 3 to about 12%, from about 5 to about 10%, about 15%, about 10%, about 7.5%, about 7.1% or about 0% on a molar basis. In one embodiment, the neutral lipid is DPPC. In one embodiment, the neutral lipid is DSPC

In one embodiment, the formulation includes from about 5% to about 50% on a molar basis of the sterol (e.g., about 15 to about 45%, about 20 to about 40%, about 48%, about 40%, about 38.5%, about 35%, about 34.4%, about 31.5% or about 31% on a molar basis. In one embodiment, the sterol is cholesterol.

In one embodiment, the formulation includes from about 0.1% to about 20% on a molar basis of the PEG or PEG-modified lipid (e.g., about 0.5 to about 10%, about 0.5 to about 5%, about 10%, about 5%, about 3.5%, about 1.5%, about 0.5%, or about 0.3% on a molar basis. In one embodiment, the PEG-modified lipid is PEG-DMG. In one embodiment, the PEG-modified lipid is PEG-cDMA.

In one embodiment, the formulations of the inventions include 25-75% of cationic lipid of formula A, 0.5-15% of the neutral lipid, 5-50% of the sterol, and 0.5-20% of the PEG or PEG-modified lipid on a molar basis.

In one embodiment, the formulations of the inventions include 35-65% of cationic lipid of formula A, 3-12% of the neutral lipid, 15-45% of the sterol, and 0.5-10% of the PEG or PEG-modified lipid on a molar basis.

In one embodiment, the formulations of the inventions include 45-65% of cationic lipid of formula A, 5-10% of the neutral lipid, 25-40% of the sterol, and 0.5-5% of the PEG or PEG-modified lipid on a molar basis.

In one embodiment, the formulations of the inventions include about 60% of cationic lipid of formula A, about 7.5% of the neutral lipid, about 31 % of the sterol, and about 1.5% of the PEG or PEG-modified lipid on a molar basis. In one preferred embodiment, the cationic lipid of formula A is 2,2-Dilinoleyl-4-dimethylaminoethyl-[1,3]-dioxolane, the neutral lipid is DSPC, the sterol is cholesterol and the PEG lipid is PEG-DMG. In one embodiment, the PEG or PEG modified lipid comprises a PEG molecule of an average molecular weight of 2,000 Da. In one embodiment, the PEG or PEG modified lipid is a compound of the following formula 1:

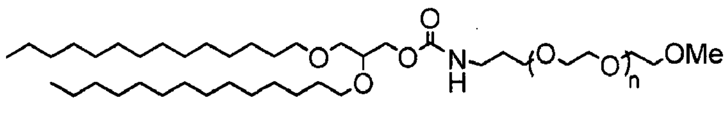

.

In one embodiment, the PEG or PEG modified lipid is PEG-distyryl glycerol (PEG-DSG).

In one embodiment, the PEG or PEG modified lipid is a compound of the formula 1 or PEG-DSG, wherein the PEG molecule has an average molecular weight of 2,000 Da.

In one embodiment, the formulations of the inventions include about 57.5% of cationic lipid of formula A, about 7.5% of the neutral lipid, about 31.5 % of the sterol, and about 3.5% of the PEG or PEG-modified lipid on a molar basis. In one preferred embodiment, the cationic lipid of formula A is 2,2-Dilinoleyl-4-dimethylaminoethyl-[1,3]-dioxolane (Lipid A), the neutral lipid is DSPC, the sterol is cholesterol and the PEG lipid is PEG-DMG (also known as PEG-dimyristoyl glycerol (C14-PEG, or PEG-C14) (PEG with an average mol. Weight of 2000)).

In one embodiment, the formulation of the inventions include about 57.1 % of the cationic lipid of formula A, about 7.1 % of the neutral lipid, about 34.4% of the sterol and about 1.4% of the PEG or PEG-modified lipid on a molar basis. In one preferred embodiment, the cationic lipid of formula A is 2,2-Dilinoleyl-4-dimethylaminoethyl-[1,3]-dioxolane (Lipid A), the neutral lipid is DPPC, the sterol is cholesterol and the PEG lipid is PEG-cDMA (also known as PEG-carbamoyl-1,2-dimyristyloxypropylamine (PEG with an average mol. weight of 2000)).

In one embodiment, the formulation of the inventions include about 60% of the cationic lipid of formula A, about 7.5% of the neutral lipid, about 31% of the sterol and about 1.5% of the PEG or PEG-modified lipid on a molar basis. In one preferred embodiment, the cationic lipid of formula A is 2,2-Dilinoleyl-4-dimethylaminoethyl-[1,3]-dioxolane (Lipid A), the neutral lipid is DSPC, the sterol is cholesterol and the PEG lipid is PEG-DMG (also known as PEG-dimyristoyl glycerol (C14-PEG, or PEG-C14) (PEG with an average mol. Weight of 2000)).

In one embodiment, the formulation of the inventions include about 50% of the cationic lipid of formula A, about 10% of the neutral lipid, about 38.5% of the sterol and about 1.5% of the PEG or PEG-modified lipid on a molar basis. In one preferred embodiment, the cationic lipid of formula A is 2,2-Dilinoleyl-4-dimethylaminoethyl-[1,3]-dioxolane (Lipid A), the neutral lipid is DSPC, the sterol is cholesterol and the PEG lipid is PEG-DMG (also known as PEG-dimyristoyl glycerol (C14-PEG, or PEG-C14) (PEG with an average mol. Weight of 2000)).

In one embodiment, the ratio of lipid:siRNA is at least about 0.5:1, at least about 1:1, at least about 2:1, at least about 3:1, at least about 4:1, at least about 5:1, at least about 6:1, at least about 7:1, at least about 11:1 or at least about 33:1. In one embodiment, the ratio of lipid:siRNA ratio is between about 1:1 to about 35:1, about 3:1 to about 15:1, about 4:1 to about 15:1, about 5:1 to about 13:1. In one embodiment, the ratio of lipid:siRNA ratio is between about 0.5:1 to about 12:1.

In one aspect, the improved lipid formulation also includes a targeting lipid. In some embodiments, the targeting lipid includes a GalNAc moiety (i.e., an N-galactosamine moiety). For example, a targeting lipid including a GalNAc moiety can include those disclosed in USSN 12/328,669, filed 12/4/2008, which is incorporated herein by reference in its entirety. A targeting lipid can also include any other lipid (e.g., targeting lipid) known in the art, for example, as described in USSN 12/328,669 or International Publication No. WO 2008/042973, the contents of each of which are incorporated herein by reference in their entirety. In some embodiments, the targeting lipid includes a plurality of GalNAc moieties, e.g., two or three GalNAc moieties. In some embodiments, the targeting lipid contains a plurality, e.g., two or three N-acetylgalactosamine (GalNAc) moieties. In some embodiments, the lipid in the targeting lipid is 1,2-Di-*O*-hexadecyl-*sn*-glyceride (i.e., DSG). In some embodiments, the targeting lipid includes a PEG moiety (e.g., a PEG moiety having a molecular weight of at least about 500 Da, such as about 1000 Da, 1500 Da, 2000 Da or greater), for example, the targeting moiety is connected to the lipid via a PEG moiety.

In some embodiments, the targeting lipid includes a folate moiety. For example, a targeting lipid including a folate moiety can include those disclosed in USSN 12/328,669, filed 12/4/2008, which is incorporated herein by reference in its entirety. In another embodiment, a targeting lipid including a folate moiety can include the compound of formula 5.

Exemplary targeting lipids are represented by formula L below:

$$(\text{Targeting group})_n\text{-L-Lipid} \qquad \text{formula L}$$

wherein:

Targeting group is any targeting group that known by one skilled in the art and/or described herein (e.g., a cell surface receptor);
n is an integer from 1 to 5, (e.g., 3)
L is a linking group; and
Lipid is a lipid such as a lipid described herein (e.g., a neutral lipid such as DSG).

In some embodiments, the linking group includes a PEG moiety. In another embodiment, the PEG moiety can vary in size from a molecular weight of about 1,000 to about 20,000 daltons (e.g., from about 1,500 to about 5,000 daltons, e.g., about 1000 daltons, about 2000 daltons, about 3400 daltons, or about 5000 daltons.

In some embodiments, the targeting lipid is a compound of formula 2, 3, 4, 5, 6 or 7 as provided below:

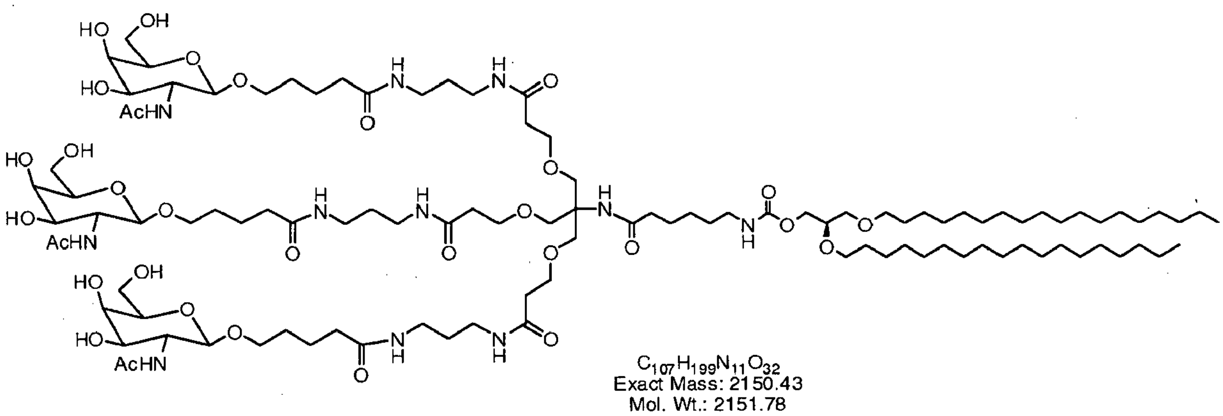

$C_{107}H_{199}N_{11}O_{32}$
Exact Mass: 2150.43
Mol. Wt.: 2151.78

formula **2**

**GalNAc3-PEG-DSG**

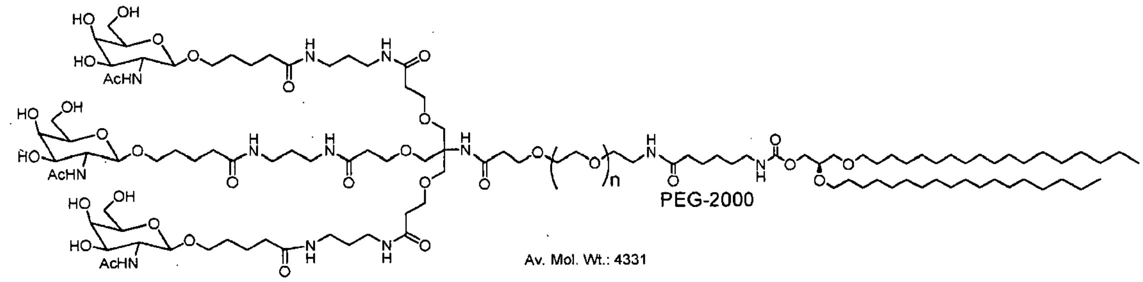

Av. Mol. Wt.: 4331

formula **3**

**GalNAc3-PEG-DSG**

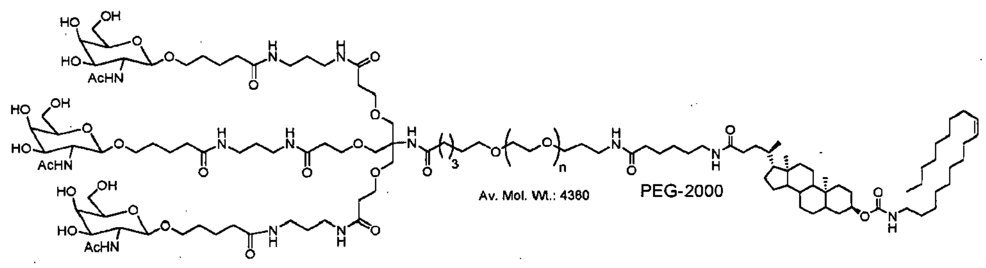

Av. Mol. Wt.: 4360

**(GalNAc)$_3$-PEG-LCO**

formula **4**

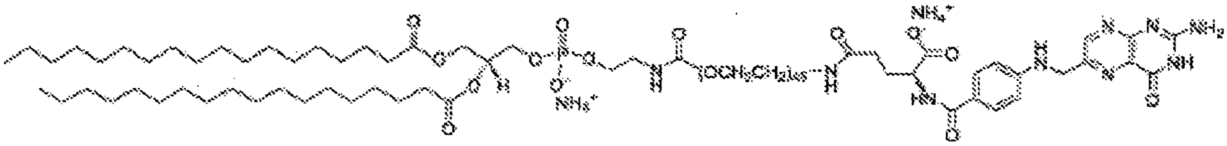

**Folate-PEG-DSPE**

1,2-distearoyl-sn-glycero-3-phosphoethanolamine-N-[folate(polyethylene glycol)-2000] (ammonium salt)

formula **5**

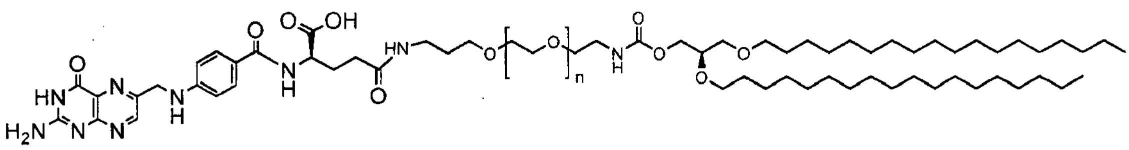

Mol Wt: ~ 3028

**Folate-PEG2000-DSG**

formula **6**

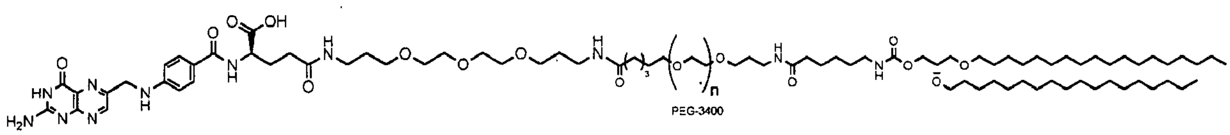

MW: ~ 4761

**Folate-PEG3400-DSG**

Formula 7

In some embodiments, the targeting lipid is present in the formulation in an amount of from about 0.001% to about 5% (e.g., about 0.005%, 0.15%, 0.3%, 0.5%, 1.5%, 2%, 2.5%, 3%, 4%, or 5%) on a molar basis. In some embodiments, the targeting lipid is included in a formulation described herein such as LNP05 or LNP08.

In some embodiments, the lipid formulation also included an antioxidant (e.g., a radical scavenger). The antioxidant can be present in the formulation, for example, at an amound from about 0.01% to about 5%. The antioxidant can be hydrophobic or hydrophilic (e.g., soluble in lipids or soluble in water). In some embodiments, the antioxidant is a phenolic compound, for example, butylhydroxytoluene, resveratrol, coenzyme Q10, or other flavinoids, or a vitamin, for example, vitamin E or vitamin C. Other exemplary antioxidants include lipoic acid, uric acid, a carotene such as beta-carotene or retinol (vitamin A), glutathione, melatonin, selenium, and ubiquinol.

In some embodiments, the receptor for the targeting lipid (e.g., a GalNAc containing lipid) is the asialoglycoprotein receptor (i.e., ASGPR).

In one embodiment, the formulations of the invention are produced *via* an extrusion method or an in-line mixing method.

The extrusion method (also refer to as preformed method or batch process) is a method where the empty liposomes (i.e. no nucleic acid) are prepared first, followed by the the addition of nucleic acid to the empty liposome. Extrusion of liposome compositions through a small-pore polycarbonate membrane or an asymmetric ceramic membrane results in a relatively well-defined size distribution. Typically, the suspension is cycled through the membrane one or more times until the desired liposome complex size distribution is achieved. The liposomes may be extruded through successively smaller-pore membranes, to achieve a gradual reduction in liposome size. In some instances, the lipid-nucleic acid compositions which are formed can be used without any sizing. These methods are disclosed in the US 5,008,050; US 4,927,637; US 4,737,323; Biochim Biophys Acta. 1979 Oct 19;557(1):9-23; Biochim Biophys Acta. 1980 Oct 2;601(3):559-7; Biochim Biophys Acta. 1986 Jun 13;858(1):161-8; and Biochim. Biophys. Acta 1985 812, 55-65, which are hereby incorporated by reference in their entirety.

The in-line mixing method is a method wherein both the lipids and the nucleic acid are added in parallel into a mixing chamber. The mixing chamber can be a simple T-connector or any other mixing chamber that is known to one

skill in the art. These methods are disclosed in US patent nos. 6,534,018 and US 6,855,277; US publication 2007/0042031 and Pharmaceuticals Research, Vol. 22, No. 3, Mar. 2005, p. 362-372, which are hereby incorporated by reference in their entirety.

It is further understood that the formulations of the invention can be prepared by any methods known to one of ordinary skill in the art.

In a further embodiment, representative formulations prepared *via* the extrusion method are delineated in Table 1, wherein Lipid A is a compound of formula A, where $R_1$ and $R_2$ are linoleyl and $R_3$ and $R_4$ are methyl, i.e., 2,2-Dilinoleyl-4-dimethylaminoethyl-[1,3]-dioxolane. References to "Lipid A" throughout the application, for example, in other tables and in the Examples, refer to this same lipid of formula A, where $R_1$ and $R_2$ are linoleyl and $R_3$ and $R_4$ are methyl, unless explicitly otherwise defined.

Table 1

| Composition (mole %) | | | | siRNA | Lipid A/ siRN A | Charge ratio | Total Lipid/ siRNA | Entrapment (%) | Zeta potential | Particle size (nm) | PDI |
|---|---|---|---|---|---|---|---|---|---|---|---|
| Lipid A | DSPC | Chol | PEG | | | | | | | | |
| 20 | 30 | 40 | 10 | 1955 | 2.13 | 1.12 | 12.82 | 39 | -0.265 | 85.3 | 0.109 |
| 20 | 30 | 40 | 10 | 1955 | 2.35 | 1.23 | 14.15 | 53 | -0.951 | 86.8 | 0.081 |
| 20 | 30 | 40 | 10 | 1955 | 2.37 | 1.25 | 14.29 | 70 | 0.374 | 79.1 | 0.201 |
| 20 | 30 | 40 | 10 | 1955 | 3.23 | 1.70 | 19.48 | 77 | 5.89 | 81.4 | 0.099 |
| 20 | 30 | 40 | 10 | 1955 | 3.91 | 2.05 | 23.53 | 85 | 10.7 | 80.3 | 0.105 |
| 30 | 20 | 40 | 10 | 1955 | 2.89 | 1.52 | 11.36 | 44 | -9.24 | 82.7 | 0.142 |
| 30 | 20 | 40 | 10 | 1955 | 3.34 | 1.76 | 13.16 | 57 | -4.32 | 76.3 | 0.083 |
| 30 | 20 | 40 | 10 | 1955 | 3.34 | 1.76 | 13.16 | 76 | -1.75 | 74.8 | 0.067 |
| 30 | 20 | 40 | 10 | 1955 | 4.10 | 2.15 | 16.13 | 93 | 3.6 | 72.8 | 0.082 |
| 30 | 20 | 40 | 10 | 1955 | 5.64 | 2.97 | 22.22 | 90 | 4.89 | 70.8 | 0.202 |
| 40 | 10 | 40 | 10 | 1955 | 3.02 | 1.59 | 8.77 | 57 | -12.3 | 63.3 | 0.146 |
| 40 | 10 | 40 | 10 | 1955 | 3.35 | 1.76 | 9.74 | 77 | 7.73 | 57 | 0.192 |
| 40 | 10 | 40 | 10 | 1955 | 3.74 | 1.97 | 10.87 | 92 | 13.2 | 56.9 | 0.203 |
| 40 | 10 | 40 | 10 | 1955 | 5.80 | 3.05 | 16.85 | 89 | 13.8 | 64 | 0.109 |
| 40 | 10 | 40 | 10 | 1955 | 8.00 | 4.20 | 23.26 | 86 | 14.7 | 65.2 | 0.132 |
| 45 | 5 | 40 | 10 | 1955 | 3.27 | 1.72 | 8.33 | 60 | -10.7 | 56.4 | 0.219 |
| 45 | 5 | 40 | 10 | 1955 | 3.30 | 1.74 | 8.43 | 89 | 12.6 | 40.8 | 0.238 |
| 45 | 5 | 40 | 10 | 1955 | 4.45 | 2.34 | 11.36 | 88 | 12.4 | 51.4 | 0.099 |
| 45 | 5 | 40 | 10 | 1955 | 7.00 | 3.68 | 17.86 | 84 | 13.2 | 78.1 | 0.055 |
| 45 | 5 | 40 | 10 | 1955 | 9.80 | 5.15 | 25.00 | 80 | 13.9 | 64.2 | 0.106 |
| 50 | 0 | 40 | 10 | 1955 | 27.0 3 | 14.21 | 68.97 | 29 | | 42.0 | 0.155 |
| 20 | 35 | 40 | 5 | 1955 | 3.00 | 1.58 | 16.13 | 31 | -8.14 | 76.8 | 0.068 |
| 20 | 35 | 40 | 5 | 1955 | 3.32 | 1.75 | 17.86 | 42 | -4.88 | 79.3 | 0.093 |
| 20 | 35 | 40 | 5 | 1955 | 3.05 | 1.60 | 16.39 | 61 | -4.48 | 64.4 | 0.12 |
| 20 | 35 | 40 | 5 | 1955 | 3.67 | 1.93 | 19.74 | 76 | 3.89 | 72.9 | 0.161 |
| 20 | 35 | 40 | 5 | 1955 | 4.71 | 2.48 | 25.32 | 79 | 10.7 | 76.6 | 0.067 |
| 30 | 25 | 40 | 5 | 1955 | 2.47 | 1.30 | 8.62 | 58 | -2.8 | 79.1 | 0.153 |
| 30 | 25 | 40 | 5 | 1955 | 2.98 | 1.57 | 10.42 | 72 | -2.73 | 74.1 | 0.046 |
| 30 | 25 | 40 | 5 | 1955 | 3.29 | 1.73 | 11.49 | 87 | 13.6 | 72.5 | 0.079 |
| 30 | 25 | 40 | 5 | 1955 | 4.99 | 2.62 | 17.44 | 86 | 14.6 | 72.3 | 0.057 |

(continued)

| Composition (mole %) | | | | | | | | | | | |
|---|---|---|---|---|---|---|---|---|---|---|---|
| Lipid A | DSPC | Chol | PEG | siRNA | Lipid A/ siRN A | Charge ratio | Total Lipid/ siRNA | Entrapment (%) | Zeta potential | Particle size (nm) | PDI |
| 30 | 25 | 40 | 5 | 1955 | 7.15 | 3.76 | 25.00 | 80 | 13.8 | 75.8 | 0.069 |
| 40 | 15 | 40 | 5 | 1955 | 2.79 | 1.46 | 7.14 | 70 | -3.52 | 65.4 | 0.068 |
| 40 | 15 | 40 | 5 | 1955 | 3.29 | 1.73 | 8.43 | 89 | 13.3 | 58.8 | 0.078 |
| 40 | 15 | 40 | 5 | 1955 | 4.33 | 2.28 | 11.11 | 90 | 14.9 | 62.3 | 0.093 |
| 40 | 15 | 40 | 5 | 1955 | 7.05 | 3.70 | 18.07 | 83 | 14.7 | 64.8 | 0.046 |
| 40 | 15 | 40 | 5 | 1955 | 9.63 | 5.06 | 24.69 | 81 | 15.4 | 63.2 | 0.06 |
| 45 | 10 | 40 | 5 | 1955 | 2.44 | 1.28 | 6.25 | 80 | -1.86 | 70.7 | 0.226 |
| 45 | 10 | 40 | 5 | 1955 | 3.21 | 1.69 | 8.24 | 91 | 8.52 | 59.1 | 0.102 |
| 45 | 10 | 40 | 5 | 1955 | 4.29 | 2.25 | 10.99 | 91 | 9.27 | 66.5 | 0.207 |
| 45 | 10 | 40 | 5 | 1955 | 6.50 | 3.42 | 16.67 | 90 | 9.33 | 59.6 | 0.127 |
| 45 | 10 | 40 | 5 | 1955 | 8.67 | 4.56 | 22.22 | 90 | 11.2 | 63.5 | 0.083 |
| 20 | 35 | 40 | 5 | 1661 | 4.10 | 2.16 | 22.06 | 68 | -3.94 (-2.95) | 85.6 | 0.041 |
| 20 | 35 | 40 | 5 | 1661 | 4.83 | 2.54 | 25.97 | 77 | 1.7 (1.73) | 81.5 | 0.096 |
| 30 | 25 | 40 | 5 | 1661 | 3.86 | 2.03 | 13.51 | 74 | 3.63 | 59.9 | 0.139 |
| 30 | 25 | 40 | 5 | 1661 | 5.38 | 2.83 | 18.75 | 80 | 12 | 67.3 | 0.106 |
| 30 | 25 | 40 | 5 | 1661 | 7.07 | 3.72 | 24.69 | 81 | 10.7 | 69.5 | 0.145 |
| 40 | 15 | 40 | 5 | 1661 | 3.85 | 2.02 | 9.87 | 76 | -3.79 | 63 | 0.166 |
| 40 | 15 | 40 | 5 | 1661 | 4.88 | 2.56 | 12.50 | 80 | 1.76 | 64.6 | 0.073 |
| 40 | 15 | 40 | 5 | 1661 | 7.22 | 3.80 | 18.52 | 81 | 5.87 | 69 | 0.094 |
| 40 | 15 | 40 | 5 | 1661 | 9.75 | 5.12 | 25.00 | 80 | 9.25 | 65.5 | 0.177 |
| 45 | 10 | 40 | 5 | 1661 | 2.83 | 1.49 | 7.25 | 69 | -10.2 | 67.8 | 0.036 |
| 45 | 10 | 40 | 5 | 1661 | 3.85 | 2.02 | 9.87 | 76 | 3.53 | 57.1 | 0.058 |
| 45 | 10 | 40 | 5 | 1661 | 4.88 | 2.56 | 12.50 | 80 | 6.22 | 57.9 | 0.096 |
| 45 | 10 | 40 | 5 | 1661 | 7.05 | 3.70 | 18.07 | 83 | 12.8 | 58.2 | 0.108 |
| 45 | 10 | 40 | 5 | 1661 | 9.29 | 4.88 | 23.81 | 84 | 9.89 | 55.6 | 0.067 |
| 45 | 20 | 30 | 5 | 1955 | 4.01 | 2.11 | 9.61 | 71 | 3.99 | 57.6 | 0.249 |
| 45 | 20 | 30 | 5 | 1661 | 3.70 | 1.95 | 8.86 | 77 | 4.33 | 74.4 | 0.224 |
| 50 | 15 | 30 | 5 | 1955 | 4.75 | 2.50 | 10.12 | 60 | 13 | 59.1 | 0.29 |
| 50 | 15 | 30 | 5 | 1661 | 3.80 | 2.00 | 8.09 | 75 | 5.48 | 82.5 | 0.188 |

(continued)

| Composition (mole %) | | | | siRNA | Lipid A/ siRN A | Charge ratio | Total Lipid/ siRNA | Entrapment (%) | Zeta potential | Particle size (nm) | PDI |
|---|---|---|---|---|---|---|---|---|---|---|---|
| Lipid A | DSPC | Chol | PEG | | | | | | | | |
| 55 | 10 | 30 | 5 | 1955 | 3.85 | 2.02 | 7.38 | 74 | 1.83 | 49.9 | 0.152 |
| 55 | 10 | 30 | 5 | 1661 | 4.13 | 2.17 | 7.91 | 69 | -6.76 | 53.9 | 0.13 |
| 60 | 5 | 30 | 5 | 1955 | 5.09 | 2.68 | 8.84 | 56 | -10.8 | 60 | 0.191 |
| 60 | 5 | 30 | 5 | 1661 | 4.67 | 2.46 | 8.11 | 61 | -11.5 | 63.7 | 0.254 |
| 65 | 0 | 30 | 5 | 1955 | 4.75 | 2.50 | 7.53 | 60 | 4.24 | 48.6 | 0.185 |
| 65 | 0 | 30 | 5 | 1661 | 6.06 | 3.19 | 9.62 | 47 | -8.3 | 45.7 | 0.147 |
| 56.5 | 10 | 30 | 3.5 | 1661 | 3.70 | 1.95 | 6.61 | 77 | -0.0189 | 54.3 | 0.096 |
| 56.5 | 10 | 30 | 3.5 | 1955 | 3.56 | 1.87 | 6.36 | 80 | 0.997 | 54.8 | 0.058 |
| 57.5 | 10 | 30 | 2.5 | 1661 | 3.48 | 1.83 | 5.91 | 82 | 2.63 | 70.1 | 0.049 |
| 57.5 | 10 | 30 | 2.5 | 1955 | 3.20 | 1.68 | 5.45 | 89 | 4.3 | 71.4 | 0.046 |
| 58.5 | 10 | 30 | 1.5 | 1661 | 3.24 | 1.70 | 5.26 | 88 | -1.91 | 81.3 | 0.056 |
| 58.5 | 10 | 30 | 1.5 | 1955 | 3.13 | 1.65 | 5.09 | 91 | 1.86 | 85.7 | 0.047 |
| 59.5 | 10 | 30 | 0.5 | 1661 | 3.24 | 1.70 | 5.01 | 88 | -10.7 | 138 | 0.072 |
| 59.5 | 10 | 30 | 0.5 | 1955 | 3.03 | 1.59 | 4.69 | 94 | -0.603 | 155 | 0.012 |
| 45 | 10 | 40 | 5 | 1661 | 7.57 | 3.98 | 17.05 | 88 | 6.7 | 59.8 | 0.196 |
| 45 | 10 | 40 | 5 | 1661 | 7.24 | 3.81 | 16.30 | 92 | 10.6 | 56.2 | 0.096 |
| 45 | 10 | 40 | 5 | 1661 | 7.48 | 3.93 | 16.85 | 89 | 1.2 | 55.3 | 0.151 |
| 45 | 10 | 40 | 5 | 1661 | 7.84 | 4.12 | 17.65 | 85 | 2.2 | 54.7 | 0.105 |
| 65 | 0 | 30 | 5 | 1661 | 4.01 | 2.11 | 6.37 | 71 | 13.2 | 57.3 | 0.071 |
| 60 | 5 | 30 | 5 | 1661 | 3.70 | 1.95 | 6.43 | 77 | 14 | 58.1 | 0.128 |
| 55 | 10 | 30 | 5 | 1661 | 3.65 | 1.92 | 7.00 | 78 | 5.54 | 63.1 | 0.278 |
| 50 | 10 | 35 | 5 | 1661 | 3.43 | 1.80 | 7.10 | 83 | 12.6 | 58.4 | 0.102 |
| 50 | 15 | 30 | 5 | 1661 | 3.80 | 2.00 | 8.09 | 75 | 15.9 (6.17) | 60.3 | 0.11 |
| 45 | 15 | 35 | 5 | 1661 | 3.70 | 1.95 | 8.60 | 77 | 10.7 | 48.5 | 0.327 |
| 45 | 20 | 30 | 5 | 1661 | 3.75 | 1.97 | 8.97 | 76 | 15.5 14.2 | 63.2 | 0.043 |
| 45 | 25 | 25 | 5 | 1661 | 3.85 | 2.02 | 9.49 | 74 | (4.08) | 61.2 | 0.14 |
| 55 | 10 | 32.5 | 2.5 | 1661 | 3.61 | 1.90 | 6.35 | 79 | 0.0665 | 70.6 | 0.091 |
| 60 | 10 | 27.5 | 2.5 | 1661 | 3.65 | 1.92 | 6.03 | 78 | 5.8 | 72.2 | 0.02 |
| 60 | 10 | 25 | 5 | 1661 | 4.07 | 2.14 | 7.29 | 70 | 3.53 | 48.7 | 0.055 |
| 55 | 5 | 38.5 | 1.5 | 1661 | 3.75 | 1.97 | 6.17 | 76 | 4.05 | 87.7 | 0.066 |
| 60 | 10 | 28.5 | 1.5 | 1661 | 3.43 | 1.80 | 5.47 | 83 | 3.47 | 95.9 | 0.024 |

(continued)

| Composition (mole %) | | | | | | | | | | | |
|---|---|---|---|---|---|---|---|---|---|---|---|
| Lipid A | DSPC | Chol | PEG | siRNA | Lipid A/ siRN A | Charge ratio | Total Lipid/ siRNA | Entrapment (%) | Zeta potential | Particle size (nm) | PDI |
| 55 | 10 | 33.5 | 1.5 | 1661 | 3.48 | 1.83 | 5.91 | 82 | 7.58 | 76.6 | 0.09 |
| 60 | 5 | 33.5 | 1.5 | 1661 | 3.43 | 1.80 | 5.29 | 83 | 7.18 | 148 | 0.033 |
| 55 | 5 | 37.5 | 2.5 | 1661 | 3.75 | 1.97 | 6.39 | 76 | 4.32 | 61.9 | 0.065 |
| 60 | 5 | 32.5 | 2.5 | 1661 | 4.52 | 2.38 | 7.22 | 63 | 2.67 | 65.7 | 0.069 |
| 60 | 5 | 32.5 | 2.5 | 1661 | 3.52 | 1.85 | 5.62 | 81 | 4.98 | 73.2 | 0.101 |
| 45 | 15 (DMPC) | 35 | 5 | 1661 | 3.20 | 1.68 | 7.26 | 89 | 5.9 | 53 | 0.079 |
| 45 | 15 (DPPC) | 35 | 5 | 1661 | 3.43 | 1.80 | 7.88 | 83 | 7.5 | 50.6 | 0.119 |
| 45 | 15 (DOPC) | 35 | 5 | 1661 | 4.52 | 2.38 | 10.51 | 63 | 6 | 44.1 | 0.181 |
| 45 | 15 (POPC) | 35 | 5 | 1661 | 3.85 | 2.02 | 8.89 | 74 | 3.8 | 48 | 0.09 |
| 55 | 5 | 37.5 | 2.5 | 1661 | 3.96 | 2.08 | 6.75 | 72 | -11 | 53.9 | 0.157 |
| 55 | 10 | 32.5 | 2.5 | 1661 | 3.56 | 1.87 | 6.28 | 80 | -4.6 | 56.1 | 0.135 |
| 60 | 5 | 32.5 | 2.5 | 1661 | 3.80 | 2.00 | 6.07 | 75 | -5.8 | 82.4 | 0.097 |
| 60 | 10 | 27.5 | 2.5 | 1661 | 3.75 | 1.97 | 6.18 | 76 | -8.4 | 59.7 | 0.099 |
| 60 | 5 | 30 | 5 | 1661 | 4.19 | 2.20 | 7.28 | 68 | -4.8 | 45.8 | 0.235 |
| 60 | 5 | 33.5 | 1.5 | 1661 | 3.48 | 1.83 | 5.35 | 82 | -10.8 | 73.2 | 0.065 |
| 60 | 5 | 33.5 | 1.5 | 1661 | 6.64 | 3.49 | 10.21 | 86 | -1.8 | 77.8 | 0.090 |
| 60 | 5 | 30 | 5 | 1661 | 3.90 | 2.05 | 6.78 | 73 | 10.2 | 60.9 | 0.062 |
| 60 | 5 | 30 | 5 | 1661 | 4.65 | 2.44 | 8.05 | 82 | 12.6 | 65.9 | 0.045 |
| 60 | 5 | 30 | 5 | 1661 | 5.88 | 3.09 | 10.19 | 81 | 11.9 | 60.7 | 0.056 |
| 60 | 5 | 30 | 5 | 1661 | 7.51 | 3.95 | 13.03 | 76 | 9.4 | 59.6 | 0.065 |
| 60 | 5 | 30 | 5 | 1661 | 9.51 11.0 | 5.00 | 16.51 | 80 | 10.3 | 61.4 | 0.021 |
| 60 | 5 | 30 | 5 | 1661 | 6 | 5.81 | 19.20 | 86 | 12.8 | 62.0 | 0.037 |
| 62.5 | 2.5 | 50 | 5 | 1661 | 6.63 | 3.49 | 11.00 | 43 | 4.8 | 62.2 | 0.107 |
| 45 | 15 | 35 | 5 | 1661 | 3.31 | 1.74 | 7.70 | 86 | 8.6 | 63.0 | 0.077 |
| 45 | 15 | 35 | 5 | 1661 | 6.80 | 3.57 | 15.77 | 84 | 14.9 | 60.8 | 0.120 |
| 60 | 5 | 25 | 10 | 1661 | 6.48 | 3.41 | 13.09 | 44 | 5.6 | 40.6 | 0.098 |
| 60 | 5 | 32.5 | 2.5 | 1661 | 3.43 | 1.81 | 5.48 | 83 | 7.3 | 61.5 | 0.099 |
| 60 | 5 | 30 | 5 | 1661 | 3.90 | 2.05 | 6.78 | 73 | 5.6 | 59.7 | 0.090 |
| 60 | 5 | 30 | 5 | 1661 | 7.61 | 4.00 | 13.20 | 75 | 14.9 | 55.9 | 0.104 |
| 45 | 15 | 35 | 5 | 1955 | 3.13 | 1.65 | 7.27 | 91 | 8.5 | 64.1 | 0.091 |
| 45 | 15 | 35 | 5 | 1955 | 6.42 | 3.37 | 14.89 | 89 | 8 | 57.9 | 0.074 |
| 60 | 5 | 25 | 10 | 1955 | 6.48 | 3.41 | 13.09 | 44 | -12.5 | 34.2 | 0.153 |

(continued)

| Composition (mole %) | | | | siRNA | Lipid A/ siRN A | Charge ratio | Total Lipid/ siRNA | Entrapment (%) | Zeta potential | Particle size (nm) | PDI |
|---|---|---|---|---|---|---|---|---|---|---|---|
| Lipid A | DSPC | Chol | PEG | | | | | | | | |
| 60 | 5 | 32.5 | 2.5 | 1955 | 3.03 | 1.60 | 4.84 | 94 | 1.8 | 72.7 | 0.078 |
| 60 | 5 | 30 | 5 | 1955 | 3.43 | 1.81 | 5.96 | 83 | -0.7 | 61.8 | 0.074 |
| 60 | 5 | 30 | 5 | 1955 | 6.72 | 3.53 | 11.65 | 85 | 6.4 | 65.5 | 0.046 |
| 60 | 5 | 30 | 5 | 1661 | 4.13 | 2.17 | 7.17 | 69 | 1.3 | 47.8 | 0.142 |
| 70 | 5 | 20 | 5 | 1661 | 5.48 | 2.88 | 8.48 | 52 | 7.6 | 48.2 | 0.06 |
| 80 | 5 | 10 | 5 | 1661 | 5.94 | 3.13 | 8.33 | 48 | 8.7 | 51.6 | 0.056 |
| 90 | 5 | 0 | 5 | 1661 | 9.50 | 5.00 | 12.27 | 30 | 1.4 | 48.7 | 0.116 |
| 60 | 5 | 30 | 5 C12PEG | 1661 | 3.85 | 2.03 | 6.68 | 74 | 4.3 | 60.1 | 0.18 |
| 60 | 5 | 30 | 5 | 1661 | 3.70 | 1.95 | 6.43 | 77 | 5.1 | 53.7 | 0.212 |
| 60 | 5 | 30 | 5 C16PEG | 1661 | 3.80 | 2.00 | 6.61 | 75 | 4.8 | 49.2 | 0.14 |
| 60 | 5 | 30 | 5 | 1661 | 4.19 | 2.21 | 7.28 | 68 | 14 | 58.3 | 0.095 |
| 60 | 5 | 29 | 5 | 1661 | 4.07 | 2.14 | 7.07 | 70 | 6.3 | 50.6 | 0.119 |
| 60 | 5 | 30 | 5 | 1955 | 3.56 | 1.88 | 6.19 | 80 | | 56.5 | 0.026 |
| 60 | 5 | 30 | 5 | 1955 | 3.39 | 1.79 | 5.89 | 84 | 9.9 | 70.5 | 0.025 |
| 60 | 5 | 30 | 5 | 1661 | 3.96 | 2.08 | 6.88 | 72 | 0.6 | 53.1 | 0.269 |
| 60 | 5 | 30 | 5 | 1661 | 4.01 | 2.11 | 6.97 | 71 | 0.1 | 50.4 | 0.203 |
| 60 | 5 | 30 | 5 | 1661 | 4.07 | 2.14 | 7.07 | 70 | 0.3 | 53.7 | 0.167 |
| 60 | 5 | 30 | 5 | 1661 | 4.25 | 2.24 | 7.39 | 67 | -0.4 | 56.8 | 0.216 |
| 60 | 5 | 30 | 5 | 1661 | 3.80 | 2.00 | 6.60 | 75 | 3.7 | 61.2 | 0.096 |
| 60 | 5 | 30 | 5 | 1661 | 3.31 | 1.74 | 5.76 | 86 | 4.1 | 111 | 0.036 |
| 60 | 5 | 30 | 5 | 1661 | 4.83 | 2.54 | 8.39 | 59 | -7.7 | 51.7 | 0.109 |
| 60 | 5 | 30 | 5 | 1661 | 4.67 | 2.46 | 8.11 | 61 | -4.2 | 46.3 | 0.122 |
| 60 | 5 | 30 | 5 | 1661 | 3.96 | 2.08 | 6.88 | 72 | -8.4 | 68.2 | 0.161 |
| 57.5 | 7.5 | 33.5 | 1.5 | 1661 | 3.39 | 1.79 | 5.49 | 84 | 1.1 | 79.5 | 0.093 |
| 57.5 | 7.5 | 32.5 | 2.5 | 1661 | 3.39 | 1.79 | 5.69 | 84 | 4.4 | 70.1 | 0.081 |
| 57.5 | 7.5 | 31.5 | 3.5 | 1661 | 3.52 | 1.85 | 6.10 | 81 | 6.8 | 59.3 | 0.098 |
| 57.5 | 7.5 | 30 | 5 | 1661 | 4.19 | 2.21 | 7.65 | 68 | 6.1 | 65.2 | 0.202 |
| 60 | 5 | 30 | 5 | 1661 | 3.96 | 2.08 | 6.88 | 72 | -4 | 60.7 | 0.338 |
| 60 | 5 | 30 | 5 | 1661 | 3.96 | 2.08 | 6.88 | 72 | -4.2 | 79.4 | 0.006 |
| 60 | 5 | 30 | 5 | 1661 | 3.56 | 1.88 | 6.19 | 80 | -1.9 | 69.4 | 0.214 |
| 60 | 5 | 33.5 | 1.5 | 1661 | 3.52 | 1.85 | 5.42 | 81 | 6.2 | 70.4 | 0.163 |
| 60 | 5 | 25 | 10 | 1661 | 5.18 | 2.73 | 10.47 | 55 | 0.7 | 43.3 | 0.351 |

(continued)

| Composition (mole %) | | | | siRNA | Lipid A/ siRN A | Charge ratio | Total Lipid/ siRNA | Entrapment (%) | Zeta potential | Particle size (nm) | PDI |
|---|---|---|---|---|---|---|---|---|---|---|---|
| Lipid A | DSPC | Chol | PEG | | | | | | | | |
| 60 | 5 (DPPC) | 30 | 5 | 1661 | 4.25 | 2.24 | 7.36 | 67 | 4.6 | 49.7 | 0.118 |
| 60 | 5 | 32.5 | 2.5 | 1661 | 3.70 | 1.95 | 5.91 | 77 | 9.7 | 88.1 | 0.064 |
| 57.5 | 7.5 | 31.5 | 3.5 | 1661 | 3.06 | 1.61 | 5.32 | 62 | -2.7 | 53.9 | 0.163 |
| 57.5 | 7.5 | 31.5 | 3.5 | 1661 | 3.65 | 1.92 | 6.33 | 78 | 9.1 | 65.9 | 0.104 |
| 57.5 | 7.5 | 31.5 | 3.5 | 1661 | 4.70 | 2.47 | 8.14 | 81 | 9 | 64.4 | 0.06 |
| 57.5 | 7.5 | 31.5 | 3.5 | 1661 | 6.56 | 3.45 | 11.37 | 87 | 10.5 | 68.8 | 0.066 |

In a further embodiment, representative formulations prepared *via* the in-line mixing method are delineated in Table 2, wherein Lipid A is a compound of formula A, where $R_1$ and $R_2$ are linoleyl and $R_3$ and $R_4$ are methyl:

Table 2

| Composition (mole %) | | | | siRNA | Lipid A/ siRNA | Charge ratio | Total Lipid/ siRNA | Entrapment (%) | Zeta potential | Particle Size (nm) | PDI |
|---|---|---|---|---|---|---|---|---|---|---|---|
| Lipid A | DSPC | Chol | PEG | | | | | | | | |
| 55 | 5 | 37.5 | 2.5 | 1661 | 3.96 | 2.08 | 6.75 | 72 | -11 | 53.9 | 0.157 |
| 55 | 10 | 32.5 | 2.5 | 1661 | 3.56 | 1.87 | 6.28 | 80 | -4.6 | 56.1 | 0.135 |
| 60 | 5 | 32.5 | 2.5 | 1661 | 3.80 | 2.00 | 6.07 | 75 | -5.8 | 82.4 | 0.097 |
| 60 | 10 | 27.5 | 2.5 | 1661 | 3.75 | 1.97 | 6.18 | 76 | -8.4 | 59.7 | 0.099 |
| 60 | 5 | 30 | 5 | 1661 | 4.19 | 2.20 | 7.28 | 68 | -4.8 | 45.8 | 0.235 |
| 60 | 5 | 33.5 | 1.5 | 1661 | 3.48 | 1.83 | 5.35 | 82 | -10.8 | 73.2 | 0.065 |
| 60 | 5 | 33.5 | 1.5 | 1661 | 6.64 | 3.49 | 10.21 | 86 | -1.8 | 77.8 | 0.090 |
| 60 | 5 | 25 | 10 | 1661 | 6.79 | 3.57 | 16.10 | 42 | -4.6 | 72.6 | 0.165 |
| 60 | 5 | 32.5 | 2.5 | 1661 | 3.96 | 2.08 | 6.32 | 72 | -3.9 | 57.6 | 0.102 |
| 60 | 5 | 34 | 1 | 1661 | 3.75 | 1.97 | 5.67 | 76 | -16.3 | 83.5 | 0.171 |
| 60 | 5 | 34.5 | 0.5 | 1661 | 3.28 | 1.72 | 4.86 | 87 | -7.3 | 126.0 | 0.08 |
| 50 | 5 | 40 | 5 | 1661 | 3.96 | 2.08 | 7.94 | 72 | 0.2 | 56.9 | 0.1 |
| 60 | 5 | 30 | 5 | 1661 | 4.75 | 2.50 | 8.25 | 60 | -1.8 | 44.3 | 0.296 |
| 70 | 5 | 20 | 5 | 1661 | 5.00 | 2.63 | 7.74 | 57 | -2.9 | 38.9 | 0.223 |
| 80 | 5 | 10 | 5 | 1661 | 5.18 | 2.73 | 7.27 | 55 | -5.1 | 45.3 | 0.170 |
| 60 | 5 | 30 | 5 | 1661 | 13.60 | 7.14 | 23.57 | 42 | 0.3 | 50.2 | 0.186 |
| 60 | 5 | 30 | 5 | 1661 | 14.51 | 7.63 | 25.19 | 59 | 0.5 | 74.6 | 0.156 |
| 60 | 5 | 30 | 5 | 1661 | 6.20 | 3.26 | 10.76 | 46 | -9.8 | 60.6 | 0.153 |
| 60 | 5 | 30 | 5 | 1661 | 4.60 | 2.42 | 7.98 | 62 | 7.7 | 88.7 | 0.177 |
| 60 | 5 | 30 | 5 | 1661 | 6.20 | 3.26 | 10.76 | 46 | -5 | 44.2 | 0.353 |
| 60 | 5 | 30 | 5 | 1661 | 5.82 | 3.06 | 10.10 | 49 | -14.2 | 50.3 | 0.232 |
| 40 | 5 | 54 | 1 | 1661 | 3.39 | 1.79 | 7.02 | 84 | 0.496 | 95.9 | 0.046 |
| 40 | 7.5 | 51.5 | 1 | 1661 | 3.39 | 1.79 | 7.15 | 84 | 3.16 | 81.8 | 0.002 |
| 40 | 10 | 49 | 1 | 1661 | 3.39 | 1.79 | 7.29 | 84 | 0.652 | 85.6 | 0.017 |
| 50 | 5 | 44 | 1 | 1661 | 3.39 | 1.79 | 5.88 | 84 | 9.74 | 94.7 | 0.030 |
| 50 | 7.5 | 41.5 | 1 | 1661 | 3.43 | 1.81 | 6.06 | 83 | 10.7 | 86.7 | 0.033 |
| 50 | 10 | 39 | 1 | 1661 | 3.35 | 1.76 | 6.02 | 85 | 11.9 | 81.1 | 0.069 |
| 60 | 5 | 34 | 1 | 1661 | 3.52 | 1.85 | 5.32 | 81 | -11.7 | 88.1 | 0.010 |
| 60 | 7.5 | 31.5 | 1 | 1661 | 3.56 | 1.88 | 5.475 | 80 | -10.4 | 81.5 | 0.032 |
| 60 | 10 | 29 | 1 | 1661 | 3.80 | 2.00 | 5.946 667 | 75 | -12.6 | 81.8 | 0.021 |
| 70 | 5 | 24 | 1 | 1661 | 3.70 | 1.95 | 5.012 987 | 77 | -9.6 | 103.0 | 0.091 |
| 70 | 7.5 | 21.5 | 1 | 1661 | 4.13 | 2.17 | 5.681 159 | 69 | -12.8 | 90.3 | 0.073 |

(continued)

| Composition (mole %) | | | | siRNA | Lipid A/ siRNA | Charge ratio | Total Lipid/ siRNA | Entrapment (%) | Zeta potential | Particle Size (nm) | PDI |
|---|---|---|---|---|---|---|---|---|---|---|---|
| Lipid A | DSPC | Chol | PEG | | | | | | | | |
| 70 | 10 | 19 | 1 | 1661 | 3.85 | 2.03 | 5.378 378 | 74 | -14 | 87.7 | 0.043 |
| 60 | 5 | 34 | 1 | 1661 | 3.52 | 1.85 | 5.320 988 | 81 | -7 | 81.1 | 0.142 |
| 60 | 5 | 34 | 1 | 1661 | 3.70 | 1.95 | 5.597 403 | 77 | -5 | 94.0 | 0.090 |
| 60 | 5 | 34 | 1 | 1661 | 3.52 | 1.85 | 5.320 988 | 81 | -8.2 | 83.6 | 0.096 |
| 60 | 7.5 | 27.5 | 5 | 1661 | 5.18 | 2.73 | 9.145 455 | 55 | -5.92 | 39.6 | 0.226 |
| 60 | 7.5 | 29 | 3.5 | 1661 | 4.45 | 2.34 | 7.484 375 | 64 | -7.8 | 49.6 | 0.100 |
| 60 | 5 | 31.5 | 3.5 | 1661 | 4.83 | 2.54 | 7.983 051 | 59 | -4.61 | 46.9 | 0.187 |
| 60 | 7.5 | 31 | 1.5 | 1661 | 3.48 | 1.83 | 5.439 024 | 82 | -6.74 | 77.6 | 0.047 |
| 57.5 | 7.5 | 30 | 5 | 1661 | 4.75 | 2.50 | 8.666 667 | 60 | -6.19 | 40.5 | 0.207 |
| 57.5 | 7.5 | 31.5 | 3.5 | 1661 | 4.83 | 2.54 | 8.372 881 | 59 | -4.34 | 50.7 | 0.171 |
| 57.5 | 5 | 34 | 3.5 | 1661 | 4.67 | 2.46 | 7.983 607 | 61 | -6.49 | 45.7 | 0.107 |
| 57.5 | 7.5 | 33.5 | 1.5 | 1661 | 3.43 | 1.81 | 5.554 217 | 83 | -5.46 | 76.6 | 0.069 |
| 55 | 7.5 | 32.5 | 5 | 1661 | 4.38 | 2.31 | 8.276 923 | 65 | -3.01 | 42.4 | 0.132 |
| 55 | 7.5 | 34 | 3.5 | 1661 | 4.13 | 2.17 | 7.420 29 | 69 | -4.57 | 47.3 | 0.137 |
| 55 | 5 | 36.5 | 3.5 | 1661 | 4.38 | 2.31 | 7.753 846 | 65 | -4.73 | 49.5 | 0.116 |
| 55 | 7.5 | 36 | 1.5 | 1661 | 3.35 | 1.76 | 5.611 765 | 85 | -4.45 | 76.2 | 0.048 |

In one embodiment, the formulations of the invention are entrapped by at least 75%, at least 80% or at least 90%.

In one embodiment, the formulations of the invention further comprise an apolipoprotein. As used herein, the term "apolipoprotein" or "lipoprotein" refers to apolipoproteins known to those of skill in the art and variants and fragments thereof and to apolipoprotein agonists, analogues or fragments thereof described below.

Suitable apolipoproteins include, but are not limited to, ApoA-I, ApoA-II, ApoA-IV, ApoA-V and ApoE, and active polymorphic forms, isoforms, variants and mutants as well as fragments or truncated forms thereof. In certain embodiments, the apolipoprotein is a thiol containing apolipoprotein. "Thiol containing apolipoprotein" refers to an apolipoprotein, variant, fragment or isoform that contains at least one cysteine residue. The most common thiol containing apolipoproteins are ApoA-I Milano (ApoA-$I_M$) and ApoA-I Paris (ApoA-$I_P$) which contain one cysteine residue (Jia et al., 2002, Biochem. Biophys. Res. Comm. 297: 206-13; Bielicki and Oda, 2002, Biochemistry 41: 2089-96). ApoA-II, ApoE2 and ApoE3 are also thiol containing apolipoproteins. Isolated ApoE and/or active fragments and polypeptide analogues thereof, including recombinantly produced forms thereof, are described in U.S. Pat. Nos. 5,672,685; 5,525,472; 5,473,039; 5,182,364; 5,177,189; 5,168,045; 5,116,739; the disclosures of which are herein incorporated by reference. ApoE3 is disclosed in Weisgraber, et al., "Human E apoprotein heterogeneity: cysteine-arginine interchanges in the amino acid sequence of the apo-E isoforms," J. Biol. Chem. (1981) 256: 9077-9083; and Rall, et al., "Structural basis for receptor binding heterogeneity of apolipoprotein E from type III hyperlipoproteinemic subjects," Proc. Nat. Acad. Sci. (1982) 79: 4696-4700. See also GenBank accession number K00396.

In certain embodiments, the apolipoprotein can be in its mature form, in its preproapolipoprotein form or in its proapolipoprotein form. Homo- and heterodimers (where feasible) of pro- and mature ApoA-I (Duverger et al., 1996, Arterioscler. Thromb. Vasc. Biol. 16(12):1424-29), ApoA-I Milano (Klon et al., 2000, Biophys. J. 79:(3)1679-87; Franceschini et al., 1985, J. Biol. Chem. 260: 1632-35), ApoA-I Paris (Daum et al., 1999, J. Mol. Med. 77:614-22), ApoA-II (Shelness et al., 1985, J. Biol. Chem. 260(14):8637-46; Shelness et al., 1984, J. Biol. Chem. 259(15):9929-35), ApoA-IV (Duverger et al., 1991, Euro. J. Biochem. 201(2):373-83), and ApoE (McLean et al., 1983, J. Biol. Chem. 258(14):8993-9000) can also be utilized within the scope of the invention.

In certain embodiments, the apolipoprotein can be a fragment, variant or isoform of the apolipoprotein. The term "fragment" refers to any apolipoprotein having an amino acid sequence shorter than that of a native apolipoprotein and which fragment retains the activity of native apolipoprotein, including lipid binding properties. By "variant" is meant substitutions or alterations in the amino acid sequences of the apolipoprotein, which substitutions or alterations, e.g., additions and deletions of amino acid residues, do not abolish the activity of native apolipoprotein, including lipid binding properties. Thus, a variant can comprise a protein or peptide having a substantially identical amino acid sequence to a native apolipoprotein provided herein in which one or more amino acid residues have been conservatively substituted with chemically similar amino acids. Examples of conservative substitutions include the substitution of at least one hydrophobic residue such as isoleucine, valine, leucine or methionine for another. Likewise, the present invention contemplates, for example, the substitution of at least one hydrophilic residue such as, for example, between arginine and lysine, between glutamine and asparagine, and between glycine and serine (see U.S. Pat. Nos. 6,004,925, 6,037,323 and 6,046,166). The term "isoform" refers to a protein having the same, greater or partial function and similar, identical or partial sequence, and may or may not be the product of the same gene and usually tissue specific (see Weisgraber 1990, J. Lipid Res. 31(8):1503-11; Hixson and Powers 1991, J. Lipid Res. 32(9): 1529-35; Lackner et al., 1985, J. Biol. Chem. 260(2):703-6; Hoeg et al., 1986, J. Biol. Chem. 261(9):3911-4; Gordon et al., 1984, J. Biol. Chem. 259(1):468-74; Powell et al., 1987, Cell 50(6):831-40; Aviram et al., 1998, Arterioscler. Thromb. Vase. Biol. 18(10):1617-24; Aviram et al., 1998, J. Clin. Invest. 101(8):1581-90; Billecke et al., 2000, Drug Metab. Dispos. 28(11):1335-42; Draganov et al., 2000, J. Biol. Chem. 275(43):33435-42; Steinmetz and Utermann 1985, J. Biol. Chem. 260(4):2258-64; Widler et al., 1980, J. Biol. Chem. 255(21):10464-71; Dyer et al., 1995, J. Lipid Res. 36(1):80-8; Sacre et al., 2003, FEBS Lett. 540(1-3):181-7; Weers, et al., 2003, Biophys. Chem. 100(1-3):481-92; Gong et al., 2002, J. Biol. Chem. 277(33):29919-26; Ohta et al., 1984, J. Biol. Chem. 259(23):14888-93 and U.S. Pat. No. 6,372,886).

In certain embodiments, the methods and compositions of the present invention include the use of a chimeric construction of an apolipoprotein. For example, a chimeric construction of an apolipoprotein can be comprised of an apolipoprotein domain with high lipid binding capacity associated with an apolipoprotein domain containing ischemia reperfusion protective properties. A chimeric construction of an apolipoprotein can be a construction that includes separate regions within an apolipoprotein (i.e., homologous construction) or a chimeric construction can be a construction that includes separate regions between different apolipoproteins (i.e., heterologous constructions). Compositions comprising a chimeric construction can also include segments that are apolipoprotein variants or segments designed to have a specific character (e.g., lipid binding, receptor binding, enzymatic, enzyme activating, antioxidant or reduction-oxidation property) (see Weisgraber 1990, J. Lipid Res. 31(8):1503-11; Hixson and Powers 1991, J. Lipid Res. 32(9):1529-35; Lackner et al., 1985, J. Biol. Chem. 260(2):703-6; Hoeg et al, 1986, J. Biol. Chem. 261(9):3911-4; Gordon et al., 1984, J. Biol. Chem. 259(1):468-74; Powell et al., 1987, Cell 50(6):831-40; Aviram et al., 1998, Arterioscler. Thromb. Vasc. Biol. 18(10):1617-24; Aviramet al., 1998, J. Clin. Invest. 101(8):1581-90; Billecke et al., 2000, Drug Metab. Dispos. 28(11):1335-42; Draganov et al., 2000, J. Biol. Chem. 275(43):33435-42; Steinmetz and Utermann 1985, J. Biol. Chem.

260(4):2258-64; Widler et al., 1980, J. Biol. Chem. 255(21):10464-71; Dyer et al., 1995, J. Lipid Res. 36(1):80-8; Sorenson et al., 1999, Arterioscler. Thromb. Vasc. Biol. 19(9):2214-25; Palgunachari 1996, Arterioscler. Throb. Vasc. Biol. 16(2):328-38: Thurberg et al., J. Biol. Chem. 271(11):6062-70; Dyer 1991, J. Biol. Chem. 266(23):150009-15; Hill 1998, J. Biol. Chem. 273(47):30979-84).

Apolipoproteins utilized in the invention also include recombinant, synthetic, semi-synthetic or purified apolipoproteins. Methods for obtaining apolipoproteins or equivalents thereof, utilized by the invention are well-known in the art. For example, apolipoproteins can be separated from plasma or natural products by, for example, density gradient centrifugation or immunoaffinity chromatography, or produced synthetically, semi-synthetically or using recombinant DNA techniques known to those of the art (see, e.g., Mulugeta et al., 1998, J. Chromatogr. 798(1-2): 83-90; Chung et al., 1980, J. Lipid Res. 21(3):284-91; Cheung et al., 1987, J. Lipid Res. 28(8):913-29; Persson, et al., 1998, J. Chromatogr. 711:97-109; U.S. Pat. Nos. 5,059,528, 5,834,596, 5,876,968 and 5,721,114; and PCT Publications WO 86/04920 and WO 87/02062).

Apolipoproteins utilized in the invention further include apolipoprotein agonists such as peptides and peptide analogues that mimic the activity of ApoA-I, ApoA-I Milano (ApoA-$I_M$), ApoA-I Paris (ApoA-$I_P$), ApoA-II, ApoA-IV, and ApoE. For example, the apolipoprotein can be any of those described in U.S. Pat. Nos. 6,004,925, 6,037,323, 6,046,166, and 5,840,688, the contents of which are incorporated herein by reference in their entireties.

Apolipoprotein agonist peptides or peptide analogues can be synthesized or manufactured using any technique for peptide synthesis known in the art including, e.g., the techniques described in U.S. Pat. Nos. 6,004,925, 6,037,323 and 6,046,166. For example, the peptides may be prepared using the solid-phase synthetic technique initially described by Merrifield (1963, J. Am. Chem. Soc. 85:2149-2154). Other peptide synthesis techniques may be found in Bodanszky et al., Peptide Synthesis, John Wiley & Sons, 2d Ed., (1976) and other references readily available to those skilled in the art. A summary of polypeptide synthesis techniques can be found in Stuart and Young, Solid Phase Peptide. Synthesis, Pierce Chemical Company, Rockford, Ill., (1984). Peptides may also be synthesized by solution methods as described in The Proteins, Vol. II, 3d Ed., Neurath et. al., Eds., p. 105-237, Academic Press, New York, N.Y. (1976). Appropriate protective groups for use in different peptide syntheses are described in the above-mentioned texts as well as in McOmie, Protective Groups in Organic Chemistry, Plenum Press, New York, N.Y. (1973). The peptides of the present invention might also be prepared by chemical or enzymatic cleavage from larger portions of, for example, apolipoprotein A-I.

In certain embodiments, the apolipoprotein can be a mixture of apolipoproteins. In one embodiment, the apolipoprotein can be a homogeneous mixture, that is, a single type of apolipoprotein. In another embodiment, the apolipoprotein can be a heterogeneous mixture of apolipoproteins, that is, a mixture of two or more different apolipoproteins. Embodiments of heterogenous mixtures of apolipoproteins can comprise, for example, a mixture of an apolipoprotein from an animal source and an apolipoprotein from a semi-synthetic source. In certain embodiments, a heterogenous mixture can comprise, for example, a mixture of ApoA-I and ApoA-I Milano. In certain embodiments, a heterogeneous mixture can comprise, for example, a mixture of ApoA-I Milano and ApoA-I Paris. Suitable mixtures for use in the methods and compositions of the invention will be apparent to one of skill in the art.

If the apolipoprotein is obtained from natural sources, it can be obtained from a plant or animal source. If the apolipoprotein is obtained from an animal source, the apolipoprotein can be from any species. In certain embodiments, the apolipoprotien can be obtained from an animal source. In certain embodiments, the apolipoprotein can be obtained from a human source. In preferred embodiments of the invention, the apolipoprotein is derived from the same species as the individual to which the apolipoprotein is administered.

In one embodiment, the target gene is selected from the group consisting of Factor VII, Eg5, PCSK9, TPX2, apoB, SAA, TTR, RSV, PDGF beta gene, Erb-B gene, Src gene, CRK gene, GRB2 gene, RAS gene, MEKK gene, JNK gene, RAF gene, Erk1/2 gene, PCNA(p21) gene, MYB gene, JUN gene, FOS gene, BCL-2 gene, Cyclin D gene, VEGF gene, EGFR gene, Cyclin A gene, Cyclin E gene, WNT-1 gene, beta-catenin gene, c-MET gene, PKC gene, NFKB gene, STAT3 gene, survivin gene, Her2/Neu gene, topoisomerase I gene, topoisomerase II alpha gene, p73 gene, p21(WAF1/CIP1) gene, p27(KIP1) gene, PPM1D gene, RAS gene, caveolin I gene, MIB I gene, MTAI gene, M68 gene, mutations in tumor suppressor genes, p53 tumor suppressor gene, and combinations thereof. In one embodiment the target gene is a gene expressed in the liver, e.g., the Factor VII (FVII) gene. The effect of the expression of the target gene, e.g., FVII, is evaluated by measuring FVII levels in a biological sample, such as a serum or tissue sample. For example, the level of FVII, e.g., as measured by assay of FVII activity, in blood can be determined. In one embodiment, the level of mRNA in the liver can be evaluated. In another preferred embodiment, at least two types of evaluation are made, e.g., an evaluation of protein level (e.g., in blood), and a measure of mRNA level (e.g., in the liver) are both made.

In one embodiment, the agent is a nucleic acid, such as a double-stranded RNA (dsRNA).

In another embodiment, the nucleic acid agent is a single-stranded DNA or RNA, or double-stranded DNA or RNA, or DNA-RNA hybrid. For example, a double-stranded DNA can be a structural gene, a gene including control and termination regions, or a self-replicating system such as a viral or plasmid DNA. A double-stranded RNA can be, e.g., a dsRNA or another RNA interference reagent. A single-stranded nucleic acid can be, e.g., an antisense oligonucleotide, ribozyme, microRNA, or triplex-forming oligonucleotide.

In yet another embodiment, at various time points after administration of a candidate agent, a biological sample, such as a fluid sample, e.g., blood, plasma, or serum, or a tissue sample, such as a liver sample, is taken from the test subject and tested for an effect of the agent on target protein or mRNA expression levels. In one particularly preferred embodiment, the candidate agent is a dsRNA that targets FVII, and the biological sample is tested for an effect on Factor VII protein or mRNA levels. In one embodiment, plasma levels of FVII protein are assayed, such as by using an immunohistochemistry assay or a chromogenic assay. In another embodiment, levels of FVII mRNA in the liver are tested by an assay, such as a branched DNA assay, or a Northern blot or RT-PCR assay.

In one embodiment, the agent, e.g., a composition including the improved lipid formulation, is evaluated for toxicity. In yet another embodiment, the model subject can be monitored for physical effects, such as by a change in weight or cageside behavior.

In one embodiment, the method further includes subjecting the agent, e.g., a composition comprising the improved lipid formulation, to a further evaluation. The further evaluation can include, for example, (i) a repetition of the evaluation described above, (ii) a repetition of the evaluation described above with a different number of animals or with different doses, or (iii) by a different method, e.g., evaluation in another animal model, e.g., a non-human primate.

In another embodiment, a decision is made regarding whether or not to include the agent and the improved lipid formulation in further studies, such as in a clinical trial, depending on the observed effect of the candidate agent on liver protein or mRNA levels. For example, if a candidate dsRNA is observed to decrease protein or mRNA levels by at least 20%, 30%, 40%, 50%, or more, then the agent can be considered for a clinical trial.

In yet another embodiment, a decision is made regarding whether or not to include the agent and the improved lipid formulation in a pharmaceutical composition, depending on the observed effect of the candidate agent and amino lipid on liver protein or mRNA levels. For example, if a candidate dsRNA is observed to decrease protein or mRNA levels by at least 20%, 30%, 40%, 50%, or more, then the agent can be considered for a clinical trial.

In another aspect, the invention features a method of evaluating the improved lipid formulation for its suitability for delivering an RNA-based construct, e.g., a dsRNA that targets FVII. The method includes providing a composition that includes a dsRNA that targets FVII and a candidate amino lipid, administering the composition to a rodent, e.g., a mouse, evaluating the expression of FVII as a function of at least one of the level of FVII in the blood or the level of FVII mRNA in the liver, thereby evaluating the candidate amino lipid.

Compositions that include lipid containing components, such as a liposome, and these are described in greater detail below. Exemplary nucleic acid-based agents include dsRNAs, antisense oligonucleotides, ribozymes, microRNAs, immunostimulatory oligonucleotides, or triplex-forming oligonucleotides. These agents are also described in greater detail below.

"Alkyl" means a straight chain or branched, noncyclic or cyclic, saturated aliphatic hydrocarbon containing from 1 to 24 carbon atoms. Representative saturated straight chain alkyls include methyl, ethyl, n-propyl, n-butyl, n-pentyl, n-hexyl, and the like; while saturated branched alkyls include isopropyl, sec-butyl, isobutyl, *tert*-butyl, isopentyl, and the like. Representative saturated cyclic alkyls include cyclopropyl, cyclobutyl, cyclopentyl, cyclohexyl, and the like; while unsaturated cyclic alkyls include cyclopentenyl and cyclohexenyl, and the like.

"Alkenyl" means an alkyl, as defined above, containing at least one double bond between adjacent carbon atoms. Alkenyls include both cis and trans isomers. Representative straight chain and branched alkenyls include ethylenyl, propylenyl, 1-butenyl, 2-butenyl, isobutylenyl, 1-pentenyl, 2-pentenyl, 3-methyl-1-butenyl, 2-methyl-2-butenyl, 2,3-dimethyl-2-butenyl, and the like.

"Alkynyl" means any alkyl or alkenyl, as defined above, which additionally contains at least one triple bond between adjacent carbons. Representative straight chain and branched alkynyls include acetylenyl, propynyl, 1-butynyl, 2-butynyl, 1-pentynyl, 2-pentynyl, 3-methyl-1 butynyl, and the like.

"Acyl" means any alkyl, alkenyl, or alkynyl wherein the carbon at the point of attachment is substituted with an oxo group, as defined below. For example, - C(=O)alkyl, -C(=O)alkenyl, and -C(=O)alkynyl are acyl groups.

"Heterocycle" means a 5- to 7-membered monocyclic, or 7- to 10-membered bicyclic, heterocyclic ring which is either saturated, unsaturated, or aromatic, and which contains from 1 or 2 heteroatoms independently selected from nitrogen, oxygen and sulfur, and wherein the nitrogen and sulfur heteroatoms may be optionally oxidized, and the nitrogen heteroatom may be optionally quaternized, including bicyclic rings in which any of the above heterocycles are fused to a benzene ring. The heterocycle may be attached via any heteroatom or carbon atom. Heterocycles include heteroaryls as defined below. Heterocycles include morpholinyl, pyrrolidinonyl, pyrrolidinyl, piperidinyl, piperizynyl, hydantoinyl, valerolactamyl, oxiranyl, oxetanyl, tetrahydrofuranyl, tetrahydropyranyl, tetrahydropyridinyl, tetrahydroprimidinyl, tetrahydrothiophenyl, tetrahydrothiopyranyl, tetrahydropyrimidinyl, tetrahydrothiophenyl, tetrahydrothiopyranyl, and the like.

The terms "optionally substituted alkyl", "optionally substituted alkenyl", "optionally substituted alkynyl", "optionally substituted acyl", and "optionally substituted heterocycle" means that, when substituted, at least one hydrogen atom is replaced with a substituent. In the case of an oxo substituent (=O) two hydrogen atoms are replaced. In this regard, substituents include oxo, halogen, heterocycle, - CN, $-OR^x$, $-NR^xR^y$, $-NR^xC(=O)R^y$ $-NR^xSO_2R^y$, $-C(=O)R^x$, $-C(=O)OR^x$, $-C(=O)NR^xR^y$, $-SO_nR^x$ and $-SO_nNR^xR^y$, wherein n is 0, 1 or 2, $R^x$ and $R^y$ are the same or different and

independently hydrogen, alkyl or heterocycle, and each of said alkyl and heterocycle substituents may be further substituted with one or more of oxo, halogen, -OH, -CN, alkyl, $-OR^x$, heterocycle, $-NR^xR^y$, $-NR^xC(=O)R^y$, $-NR^xSO_2R^y$, $-C(=O)R^x$, $-C(=O)OR^x$, $-C(=O)NR^xR^y$, $-SO_nR^x$ and $-SO_nNR^xR^y$.

"Halogen" means fluoro, chloro, bromo and iodo.

In some embodiments, the methods of the invention may require the use of protecting groups. Protecting group methodology is well known to those skilled in the art (*see*, *for example,* PROTECTIVE GROUPS IN ORGANIC SYNTHESIS, Green, T.W. et. al., Wiley-Interscience, New York City, 1999). Briefly, protecting groups within the context of this invention are any group that reduces or eliminates unwanted reactivity of a functional group. A protecting group can be added to a functional group to mask its reactivity during certain reactions and then removed to reveal the original functional group. In some embodiments an "alcohol protecting group" is used. An "alcohol protecting group" is any group which decreases or eliminates unwanted reactivity of an alcohol functional group. Protecting groups can be added and removed using techniques well known in the art.

**Synthesis**

The compounds of the invention may be prepared by known organic synthesis techniques, including the methods described in more detail in the Examples. In general, the lipid of formula A above may be made by the following Reaction Schemes 1 or 2 while the Folate-PEG2000-DSG and Folate-PEG3400-DSG described herein may be produced as in Reaction Schemes 3 and 4, wherein all substituents are as defined herein unless indicated otherwise.

Scheme 1

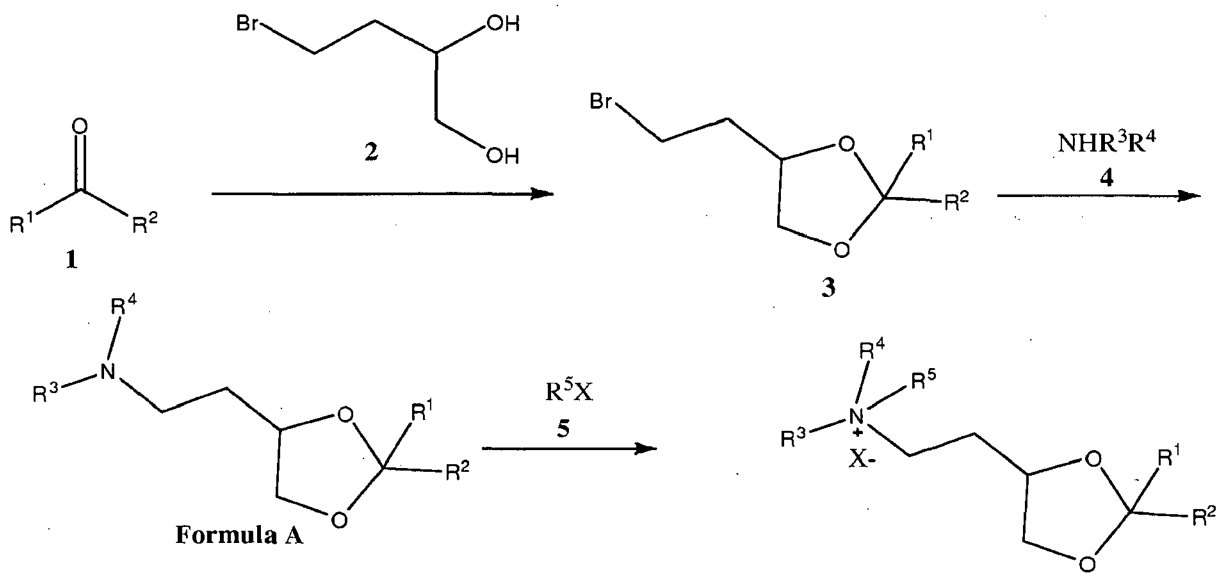


Lipid A, where $R_1$ and $R_2$ are independently alkyl, alkenyl or alkynyl, each can be optionally substituted, and $R_3$ and $R_4$ are independently lower alkyl or $R_3$ and $R_4$ can be taken together to form an optionally subsituted heterocyclic ring, can be prepared according to Scheme 1. Ketone **1** and bromide **2** can be purchased or prepared according to methods known to those of ordinary skill in the art. Reaction of **1** and **2** yields ketal **3**. Treatment of **3** with amine **4** yields lipids of formula A. The lipids of formula A can be converted to the corresponding ammonium salt with an organic salt of formula 5, where X is anion counter ion selected from halogen, hydroxide, phosphate, sulfate, or the like.

Scheme 2

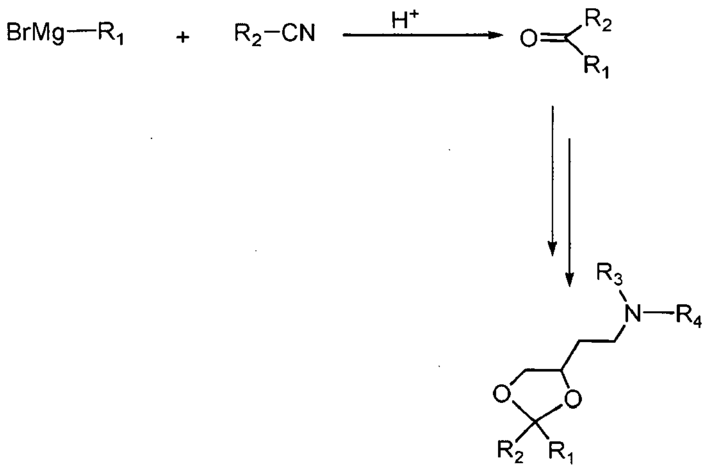

Alternatively, the ketone 1 starting material can be prepared according to Scheme 2. Grignard reagent **6** and cyanide **7** can be purchased or prepared according to methods known to those of ordinary skill in the art. Reaction of **6** and **7** yields ketone **1**. Conversion of ketone 1 to the corresponding lipids of formula A is as described in Scheme 1.

The amino lipids are of the invention are cationic lipids. As used herein, the term "amino lipid" is meant to include those lipids having one or two fatty acid or fatty alkyl chains and an amino head group (including an alkylamino or dialkylamino group) that may be protonated to form a cationic lipid at physiological pH.

Other amino lipids would include those having alternative fatty acid groups and other dialkylamino groups, including those in which the alkyl substituents are different (*e.g.*, N-ethyl-N-methylamino-, N-propyl-N-ethylamino- and the like). For those embodiments in which $R^{11}$ and $R^{12}$ are both long chain alkyl or acyl groups, they can be the same or different. In general, amino lipids having less saturated acyl chains are more easily sized, particularly when the complexes must be sized below about 0.3 microns, for purposes of filter sterilization. Amino lipids containing unsaturated fatty acids with carbon chain lengths in the range of $C_{14}$ to $C_{22}$ are preferred. Other scaffolds can also be used to separate the amino group and the fatty acid or fatty alkyl portion of the amino lipid. Suitable scaffolds are known to those of skill in the art.

In certain embodiments, amino or cationic lipids of the invention have at least one protonatable or deprotonatable group, such that the lipid is positively charged at a pH at or below physiological pH (e.g. pH 7.4), and neutral at a second pH, preferably at or above physiological pH. It will, of course, be understood that the addition or removal of protons as a function of pH is an equilibrium process, and that the reference to a charged or a neutral lipid refers to the nature of the predominant species and does not require that all of the lipid be present in the charged or neutral form. Lipids that have more than one protonatable or deprotonatable group, or which are zwiterrionic, are not excluded from use in the invention.

In certain embodiments, protonatable lipids according to the invention have a pKa of the protonatable group in the range of about 4 to about 11. Most preferred is pKa of about 4 to about 7, because these lipids will be cationic at a lower pH formulation stage, while particles will be largely (though not completely) surface neutralized at physiological pH around pH 7.4. One of the benefits of this pKa is that at least some nucleic acid associated with the outside surface of the particle will lose its electrostatic interaction at physiological pH and be removed by simple dialysis; thus greatly reducing the particle's susceptibility to clearance.

Scheme 3

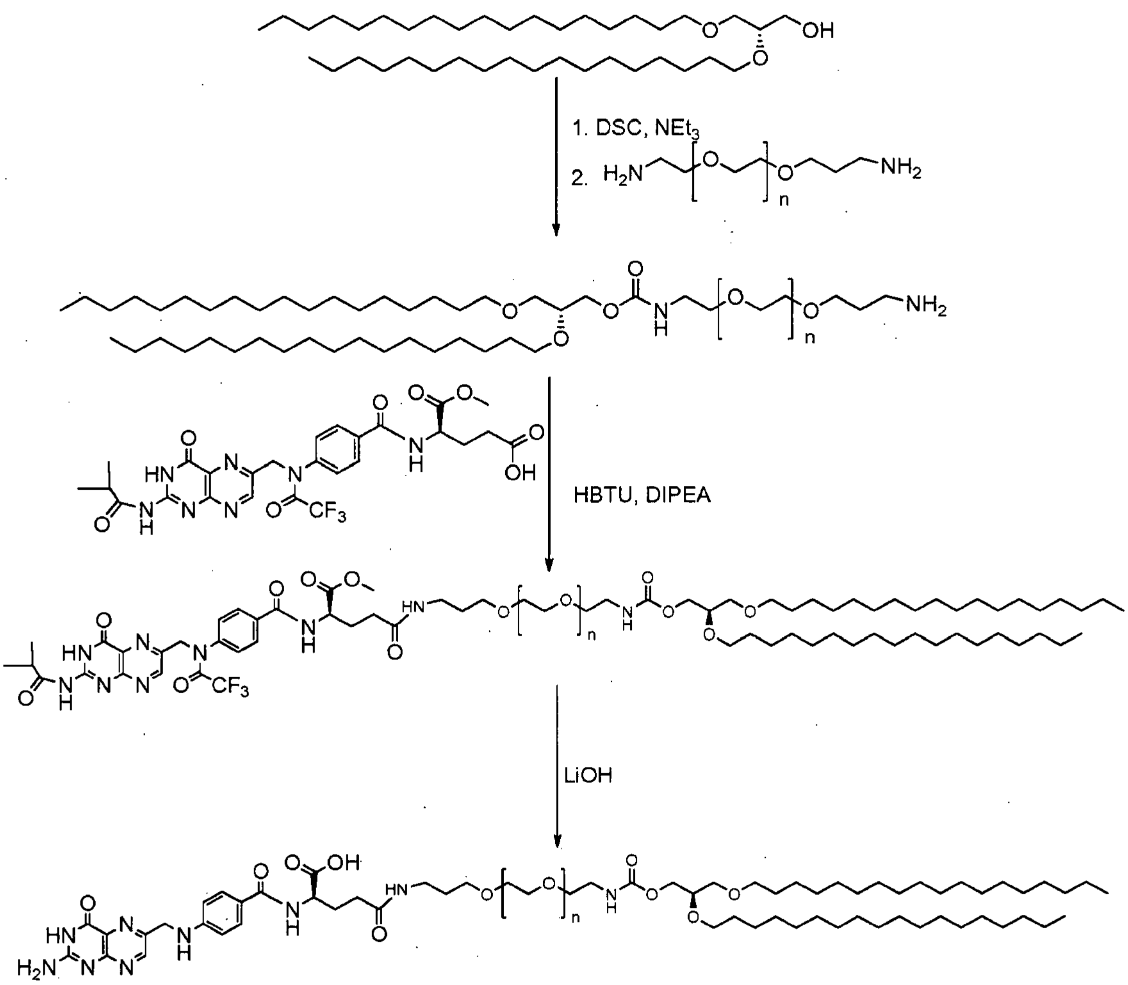


Coupling of the amino PEG derivative with the appropriate alcohol proceeded under standard conditions with disuccinimidyl carbonate (DSC) in the presence of base to produce expected functionalized PEG. Folic acid is subsequently reacted with the other end of the functionalized PEG under standard amide coupling conditions to produce protected Folate-PEG2000-DSG. Removal of each of the protecting groups proceeded in the presence of Lithium Hydroxide to provide the desired Folate-PEG2000-DSG.

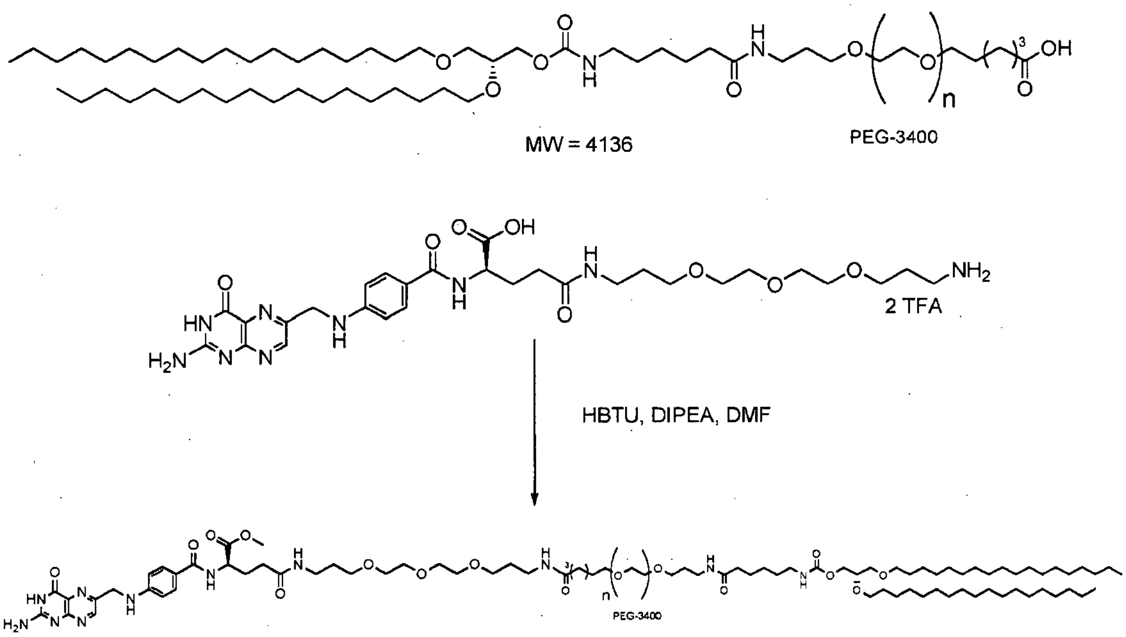


A corresponding functionalized PEG3400 was coupled under standard conditions to provide the desired Folate-PEG3400-DSG.

Lipid Particles

The agents and/or amino lipids for testing in the liver screening model featured herein can be formulated in lipid particles. Lipid particles include, but are not limited to, liposomes. As used herein, a liposome is a structure having lipid-containing membranes enclosing an aqueous interior. Liposomes may have one or more lipid membranes. The invention contemplates both single-layered liposomes, which are referred to as unilamellar, and multi-layered liposomes, which are referred to as multilamellar. When complexed with nucleic acids, lipid particles may also be lipoplexes, which are composed of cationic lipid bilayers sandwiched between DNA layers, as described, *e.g.,* in Felgner, *Scientific American.*

Lipid particles may further include one or more additional lipids and/or other components such as cholesterol. Other lipids may be included in the liposome compositions for a variety of purposes, such as to prevent lipid oxidation or to attach ligands onto the liposome surface. Any of a number of lipids may be present, including amphipathic, neutral, cationic, and anionic lipids. Such lipids can be used alone or in combination. Specific examples of additional lipid components that may be present are described below.

Additional components that may be present in a lipid particle include bilayer stabilizing components such as polyamide oligomers (*see, e.g.,* U.S. Patent No. 6,320,017), peptides, proteins, detergents, lipid-derivatives, such as PEG coupled to phosphatidylethanolamine and PEG conjugated to ceramides (*see*, U.S. Patent No. 5,885,613). In some embodiments, the lipid particle includes a targeting agent such as a targeting lipid described herein.

A lipid particle can include one or more of a second amino lipid or cationic lipid, a neutral lipid, a sterol, and a lipid selected to reduce aggregation of lipid particles during formation, which may result from steric stabilization of particles which prevents charge-induced aggregation during formation.

Examples of lipids suitable for conjugation to nucleic acid agents that can be used in the liver screening model are polyethylene glycol (PEG)-modified lipids, monosialoganglioside Gm1, and polyamide oligomers ("PAO") such as (described in US Pat. No. 6,320,017). Other compounds with uncharged, hydrophilic, steric-barrier moieties, which prevent aggregation during formulation, like PEG, Gm1 or ATTA, can also be coupled to lipids for use as in the methods and compositions of the invention. ATTA-lipids are described, *e.g.*, in U.S. Patent No. 6,320,017, and PEG-lipid conjugates are described, *e.g.*, in U.S. Patent Nos. 5,820,873, 5,534,499 and 5,885,613. Typically, the concentration of the lipid component selected to reduce aggregation is about 1 to 15% (by mole percent of lipids).

Specific examples of PEG-modified lipids (or lipid-polyoxyethylene conjugates) that are useful in the invention can have a variety of "anchoring" lipid portions to secure the PEG portion to the surface of the lipid vesicle. Examples of suitable PEG-modified lipids include PEG-modified phosphatidylethanolamine and phosphatidic acid, PEG-ceramide conjugates (*e.g.*, PEG-CerC14 or PEG-CerC20) which are described in co-pending USSN 08/486,214, incorporated herein by reference, PEG-modified dialkylamines and PEG-modified 1,2-diacyloxypropan-3-amines. Particularly preferred are PEG-modified diacylglycerols and dialkylglycerols. In some embodiments, the total mol% of PEG lipids within a particle is about 1.5 mol%. For example, when the particle includes a plurality of PEG lipids described herein such as a PEG-modified lipid as described above and a targeting lipid containing a PEG, the total amount of the PEG containing lipids when taken together is about 1.5 mol%.

In embodiments where a sterically-large moiety such as PEG or ATTA are conjugated to a lipid anchor, the selection of the lipid anchor depends on what type of association the conjugate is to have with the lipid particle. It is well known that mePEG (mw2000)-diastearoylphosphatidylethanolamine (PEG-DSPE) will remain associated with a liposome until the particle is cleared from the circulation, possibly a matter of days. Other conjugates, such as PEG-CerC20 have similar staying capacity. PEG-CerC14, however, rapidly exchanges out of the formulation upon exposure to serum, with a $T_{1/2}$ less than 60 mins. in some assays. As illustrated in US Pat. Application SN 08/486,214, at least three characteristics influence the rate of exchange: length of acyl chain, saturation of acyl chain, and size of the steric-barrier head group. Compounds having suitable variations of these features may be useful for the invention. For some therapeutic applications it may be preferable for the PEG-modified lipid to be rapidly lost from the nucleic acid-lipid particle *in vivo* and hence the PEG-modified lipid will possess relatively short lipid anchors. In other therapeutic applications it may be preferable for the nucleic acid-lipid particle to exhibit a longer plasma circulation lifetime and hence the PEG-modified lipid will possess relatively longer lipid anchors. Exemplary lipid anchors include those having lengths of from about $C_{14}$ to about $C_{22}$, preferably from about $C_{14}$ to about $C_{16}$. In some embodiments, a PEG moiety, for example an mPEG-$NH_2$, has a size of about 1000, 2000, 5000, 10,000, 15,000 or 20,000 daltons.

It should be noted that aggregation preventing compounds do not necessarily require lipid conjugation to function properly. Free PEG or free ATTA in solution may be sufficient to prevent aggregation. If the particles are stable after formulation, the PEG or ATTA can be dialyzed away before administration to a subject.

Neutral lipids, when present in the lipid particle, can be any of a number of lipid species which exist either in an uncharged or neutral zwitterionic form at physiological pH. Such lipids include, for example diacylphosphatidylcholine, diacylphosphatidylethanolamine, ceramide, sphingomyelin, dihydrosphingomyelin, cephalin, and cerebrosides. The selection of neutral lipids for use in the particles described herein is generally guided by consideration of, *e.g.*, liposome size and stability of the liposomes in the bloodstream. Preferably, the neutral lipid component is a lipid having two acyl groups, (*i.e.*, diacylphosphatidylcholine and diacylphosphatidylethanolamine). Lipids having a variety of acyl chain groups of varying chain length and degree of saturation are available or may be isolated or synthesized by well-known techniques. In one group of embodiments, lipids containing saturated fatty acids with carbon chain lengths in the range of $C_{14}$ to $C_{22}$ are preferred. In another group of embodiments, lipids with mono or diunsaturated fatty acids with carbon chain lengths in the range of $C_{14}$ to $C_{22}$ are used. Additionally, lipids having mixtures of saturated and unsaturated fatty acid chains can be used. Preferably, the neutral lipids used in the invention are DOPE, DSPC, DPPC, POPC, or any related phosphatidylcholine. The neutral lipids useful in the invention may also be composed of sphingomyelin, dihydrosphingomyeline, or phospholipids with other head groups, such as serine and inositol.

The sterol component of the lipid mixture, when present, can be any of those sterols conventionally used in the field of liposome, lipid vesicle or lipid particle preparation. A preferred sterol is cholesterol.

Other cationic lipids, which carry a net positive charge at about physiological pH, in addition to those specifically described above, may also be included in lipid particles of the invention. Such cationic lipids include, but are not limited to, N,N-dioleyl-N,N-dimethylammonium chloride ("DODAC"); N-(2,3-dioleyloxy)propyl-N,N-N-triethylammonium chloride ("DOTMA"); N,N-distearyl-N,N-dimethylammonium bromide ("DDAB"); N-(2,3-dioleoyloxy)propyl)-N,N,N-trimethylammonium chloride ("DOTAP"); 1,2-Dioleyloxy-3-trimethylaminopropane chloride salt ("DOTAP.Cl"); 3β-(N-(N',N'-dimethylaminoethane)-carbamoyl)cholesterol ("DC-Chol"), N-(1-(2,3-dioleyloxy)propyl)-N-2-(sperminecarboxamido)ethyl)-N,N-dimethylammonium trifluoracetate ("DOSPA"), dioctadecylamidoglycyl carboxyspermine ("DOGS"), 1,2-dileoyl-sn-3-phosphoethanolamine ("DOPE"), 1,2-dioleoyl-3-dimethylammonium propane ("DODAP"), N, N-dimethyl-2,3-dioleyloxy)propylamine ("DODMA"), and N-(1,2-dimyristyloxyprop-3-yl)-N,N-dimethyl-N-hydroxyethyl ammonium bromide ("DMRIE"). Additionally, a number of commercial preparations of cationic lipids can be used, such as, *e.g.*, LIPOFECTIN (including DOTMA and DOPE, available from GIBCO/BRL), and LIPOFECTAMINE (comprising DOSPA and DOPE, available from GIBCO/BRL). In particular embodiments, a cationic lipid is an amino lipid.

Anionic lipids suitable for use in lipid particles of the invention include, but are not limited to, phosphatidylglycerol, cardiolipin, diacylphosphatidylserine, diacylphosphatidic acid, N-dodecanoyl phosphatidylethanoloamine, N-succinyl phosphatidylethanolamine, N-glutaryl phosphatidylethanolamine, lysylphosphatidylglycerol, and other anionic modifying groups joined to neutral lipids.

In numerous embodiments, amphipathic lipids are included in lipid particles of the invention. "Amphipathic lipids" refer to any suitable material, wherein the hydrophobic portion of the lipid material orients into a hydrophobic phase, while the hydrophilic portion orients toward the aqueous phase. Such compounds include, but are not limited to, phospholipids, aminolipids, and sphingolipids. Representative phospholipids include sphingomyelin, phosphatidylcholine, phosphatidylethanolamine, phosphatidylserine, phosphatidylinositol, phosphatidic acid, palmitoyloleoyl phosphatdylcholine, lysophosphatidylcholine, lysophosphatidylethanolamine, dipalmitoylphosphatidylcholine (DPPC), dioleoylphosphatidylcholine (DOPC), distearoylphosphatidylcholine (DSPC), or dilinoleylphosphatidylcholine. Other phosphorus-lacking compounds, such as sphingolipids, glycosphingolipid families, diacylglycerols, and β-acyloxyacids, can also be used. Additionally, such amphipathic lipids can be readily mixed with other lipids, such as triglycerides and sterols.

Also suitable for inclusion in the lipid particles of the invention are programmable fusion lipids. Such lipid particles have little tendency to fuse with cell membranes and deliver their payload until a given signal event occurs. This allows the lipid particle to distribute more evenly after injection into an organism or disease site before it starts fusing with cells. The signal event can be, for example, a change in pH, temperature, ionic environment, or time. In the latter case, a fusion delaying or "cloaking" component, such as an ATTA-lipid conjugate or a PEG-lipid conjugate, can simply exchange out of the lipid particle membrane over time. Exemplary lipid anchors include those having lengths of from about $C_{14}$ to about $C_{22}$, preferably from about $C_{14}$ to about $C_{16}$. In some embodiments, a PEG moiety, for example an mPEG-$NH_2$, has a size of about 1000, 2000, 5000, 10,000, 15,000 or 20,000 daltons.

By the time the lipid particle is suitably distributed in the body, it has lost sufficient cloaking agent so as to be fusogenic. With other signal events, it is desirable to choose a signal that is associated with the disease site or target cell, such as increased temperature at a site of inflammation.

A lipid particle conjugated to a nucleic acid agent can also include a targeting moiety, e.g., a targeting moiety that is specific to a cell type or tissue. Targeting of lipid particles using a variety of targeting moieties, such as ligands, cell surface receptors, glycoproteins, vitamins (e.g., riboflavin) and monoclonal antibodies, has been previously described (*see, e.g.,* U.S. Patent Nos. 4,957,773 and 4,603,044). Exexmplary targeting moieties include a targeting lipid such as a targeting lipid described herein. In some embodiments, the targeting lipid is a GalNAc containing targeting lipid such as GalNAc3-DSG and GalNAc3-PEG-DSG as described herein. The targeting moieties can include the entire protein or fragments thereof. Targeting mechanisms generally require that the targeting agents be positioned on the surface of the lipid particle in such a manner that the targeting moiety is available for interaction with the target, for example, a cell surface receptor. A variety of different targeting agents and methods are known and available in the art, including those described, *e.g.,* in Sapra, P. and Allen, TM, Prog. Lipid Res. 42(5):439-62 (2003); and Abra, RM et al., J. Liposome Res. 12:1-3, (2002).

The use of lipid particles, *i.e.,* liposomes, with a surface coating of hydrophilic polymer chains, such as polyethylene glycol (PEG) chains, for targeting has been proposed (Allen, et al., Biochimica et Biophysica Acta 1237: 99-108 (1995); DeFrees, et al., Journal of the American Chemistry Society 118: 6101-6104 (1996); Blume, et al., Biochimica et Biophysica Acta 1149: 180-184 (1993); Klibanov, et al., Journal of Liposome Research 2: 321-334 (1992); U.S. Patent No. 5,013556; Zalipsky, Bioconjugate Chemistry 4: 296-299 (1993); Zalipsky, FEBS Letters 353: 71-74 (1994); Zalipsky, in Stealth Liposomes Chapter 9 (Lasic and Martin, Eds) CRC Press, Boca Raton Fl (1995). In one approach, a ligand, such as an antibody, for targeting the lipid particle is linked to the polar head group of lipids forming the lipid particle. In another approach, the targeting ligand is attached to the distal ends of the PEG chains forming the hydrophilic polymer coating (Klibanov, et al., Journal of Liposome Research 2: 321-334 (1992); Kirpotin et al., FEBS Letters 388: 115-118 (1996)).

Standard methods for coupling the target agents can be used. For example, phosphatidylethanolamine, which can be activated for attachment of target agents, or derivatized lipophilic compounds, such as lipid-derivatized bleomycin, can be used. Antibody-targeted liposomes can be constructed using, for instance, liposomes that incorporate protein A (*see*, Renneisen, et al., J. Bio. Chem., 265:16337-16342 (1990) and Leonetti, et al., Proc. Natl. Acad. Sci. (USA), 87:2448-2451 (1990). Other examples of antibody conjugation are disclosed in U.S. Patent No. 6,027,726, the teachings of which are incorporated herein by reference. Examples of targeting moieties can also include other proteins, specific to cellular components, including antigens associated with neoplasms or tumors. Proteins used as targeting moieties can be attached to the liposomes via covalent bonds (*see*, Heath, Covalent Attachment of Proteins to Liposomes, 149 Methods in Enzymology 111-119 (Academic Press, Inc. 1987)). Other targeting methods include the biotin-avidin system.

## Therapeutic Agent-Lipid Particle Compositions and Formulations

The invention includes compositions comprising a lipid particle of the invention and an active agent, wherein the active agent is associated with the lipid particle. In particular embodiments, the active agent is a therapeutic agent. In particular embodiments, the active agent is encapsulated within an aqueous interior of the lipid particle. In other embodiments, the active agent is present within one or more lipid layers of the lipid particle. In other embodiments, the active agent is bound to the exterior or interior lipid surface of a lipid particle.

"Fully encapsulated" as used herein indicates that the nucleic acid in the particles is not significantly degraded after exposure to serum or a nuclease assay that would significantly degrade free DNA. In a fully encapsulated system, preferably less than 25% of particle nucleic acid is degraded in a treatment that would normally degrade 100% of free nucleic acid, more preferably less than 10% and most preferably less than 5% of the particle nucleic acid is degraded. Alternatively, full encapsulation may be determined by an Oligreen® assay. Oligreen® is an ultra-sensitive fluorescent nucleic acid stain for quantitating oligonucleotides and single-stranded DNA in solution (available from Invitrogen Corporation, Carlsbad, CA). Fully encapsulated also suggests that the particles are serum stable, that is, that they do not rapidly decompose into their component parts upon *in vivo* administration.

Active agents, as used herein, include any molecule or compound capable of exerting a desired effect on a cell, tissue, organ, or subject. Such effects may be biological, physiological, or cosmetic, for example. Active agents may be any type of molecule or compound, including *e.g.*, nucleic acids, peptides and polypeptides, including, *e.g.,* antibodies, such as, *e.g.,* polyclonal antibodies, monoclonal antibodies, antibody fragments; humanized antibodies, recombinant antibodies, recombinant human antibodies, and Primatized™ antibodies, cytokines, growth factors, apoptotic factors, differentiation-inducing factors, cell surface receptors and their ligands; hormones; and small molecules, including small organic molecules or compounds.

In one embodiment, the active agent is a therapeutic agent, or a salt or derivative thereof. Therapeutic agent derivatives may be therapeutically active themselves or they may be prodrugs, which become active upon further modification. Thus, in one embodiment, a therapeutic agent derivative retains some or all of the therapeutic activity as compared to the unmodified agent, while in another embodiment, a therapeutic agent derivative lacks therapeutic activity.

In various embodiments, therapeutic agents include any therapeutically effective agent or drug, such as anti-inflammatory compounds, anti-depressants, stimulants, analgesics, antibiotics, birth control medication, antipyretics, vasodilators, anti-angiogenics, cytovascular agents, signal transduction inhibitors, cardiovascular drugs, *e.g.*, anti-arrhythmic agents, vasoconstrictors, hormones, and steroids.

In certain embodiments, the therapeutic agent is an oncology drug, which may also be referred to as an anti-tumor drug, an anti-cancer drug, a tumor drug, an antineoplastic agent, or the like. Examples of oncology drugs that may be used according to the invention include, but are not limited to, adriamycin, alkeran, allopurinol, altretamine, amifostine, anastrozole, araC, arsenic trioxide, azathioprine, bexarotene, biCNU, bleomycin, busulfan intravenous, busulfan oral, capecitabine (Xeloda), carboplatin, carmustine, CCNU, celecoxib, chlorambucil, cisplatin, cladribine, cyclosporin A, cytarabine, cytosine arabinoside, daunorubicin, cytoxan, daunorubicin, dexamethasone, dexrazoxane, dodetaxel, doxorubicin, doxorubicin, DTIC, epirubicin, estramustine, etoposide phosphate, etoposide and VP-16, exemestane, FK506, fludarabine, fluorouracil, 5-FU, gemcitabine (Gemzar), gemtuzumab-ozogamicin, goserelin acetate, hydrea, hydroxyurea, idarubicin, ifosfamide, imatinib mesylate, interferon, irinotecan (Camptostar, CPT-111), letrozole, leucovorin, leustatin, leuprolide, levamisole, litretinoin, megastrol, melphalan, L-PAM, mesna, methotrexate, methoxsalen, mithramycin, mitomycin, mitoxantrone, nitrogen mustard, paclitaxel, pamidronate, Pegademase, pentostatin, porfirner sodium, prednisone, rituxan, streptozocin, STI-571, tamoxifen, taxotere, temozolamide, teniposide, VM-26, topotecan (Hycamtin), toremifene, tretinoin, ATRA, valrubicin, velban, vinblastine, vincristine, VP16, and vinorelbine. Other examples of oncology drugs that may be used according to the invention are ellipticin and ellipticin analogs or derivatives, epothilones, intracellular kinase inhibitors and camptothecins.

## Nucleic Acid-Lipid Particles

In certain embodiments, lipid particles of the invention are associated with a nucleic acid, resulting in a nucleic acid-lipid particle. In particular embodiments, the nucleic acid is fully encapsulated in the lipid particle. As used herein, the term "nucleic acid" is meant to include any oligonucleotide or polynucleotide. Fragments containing up to 50 nucleotides are generally termed oligonucleotides, and longer fragments are called polynucleotides. In particular embodiments, oligonucletoides of the invention are 20-50 nucleotides in length.

In the context of this invention, the terms "polynucleotide" and "oligonucleotide" refer to a polymer or oligomer of nucleotide or nucleoside monomers consisting of naturally occurring bases, sugars and intersugar (backbone) linkages. The terms "polynucleotide" and "oligonucleotide" also includes polymers or oligomers comprising non-naturally occurring monomers, or portions thereof, which function similarly. Such modified or substituted oligonucleotides are often preferred over native forms because of properties such as, for example, enhanced cellular uptake and increased stability in the presence of nucleases.

Oligonucleotides are classified as deoxyribooligonucleotides or ribooligonucleotides. A deoxyribooligonucleotide consists of a 5-carbon sugar called deoxyribose joined covalently to phosphate at the 5' and 3' carbons of this sugar to form an alternating, unbranched polymer. A ribooligonucleotide consists of a similar repeating structure where the 5-carbon sugar is ribose.

The nucleic acid that is present in a lipid-nucleic acid particle according to this invention includes any form of nucleic acid that is known. The nucleic acids used herein can be single-stranded DNA or RNA, or double-stranded DNA or RNA, or DNA-RNA hybrids. Examples of double-stranded DNA include structural genes, genes including control and termination regions, and self-replicating systems such as viral or plasmid DNA. Examples of double-stranded RNA include siRNA and other RNA interference reagents. Single-stranded nucleic acids include, *e.g.*, antisense oligonucleotides, ribozymes, microRNA, and triplex-forming oligonucleotides.

Nucleic acids of the invention may be of various lengths, generally dependent upon the particular form of nucleic acid. For example, in particular embodiments, plasmids or genes may be from about 1,000 to 100,000 nucleotide residues

in length. In particular embodiments, oligonucleotides may range from about 10 to 100 nucleotides in length. In various related embodiments, oligonucleotides, both single-stranded, double-stranded, and triple-stranded, may range in length from about 10 to about 50 nucleotides, from about 20 o about 50 nucleotides, from about 15 to about 30 nucleotides, from about 20 to about 30 nucleotides in length.

In particular embodiments, an oligonucleotide (or a strand thereof) of the invention specifically hybridizes to or is complementary to a target polynucleotide. "Specifically hybridizable" and "complementary" are terms which are used to indicate a sufficient degree of complementarity such that stable and specific binding occurs between the DNA or RNA target and the oligonucleotide. It is understood that an oligonucleotide need not be 100% complementary to its target nucleic acid sequence to be specifically hybridizable. An oligonucleotide is specifically hybridizable when binding of the oligonucleotide to the target interferes with the normal function of the target molecule to cause a loss of utility or expression therefrom, and there is a sufficient degree of complementarity to avoid non-specific binding of the oligonucleotide to non-target sequences under conditions in which specific binding is desired, *i.e.,* under physiological conditions in the case of in vivo assays or therapeutic treatment, or, in the case of *in vitro* assays, under conditions in which the assays are conducted. Thus, in other embodiments, this oligonucleotide includes 1, 2, or 3 base substitutions as compared to the region of a gene or mRNA sequence that it is targeting or to which it specifically hybridizes.

RNA Interference Nucleic Acids

In particular embodiments, nucleic acid-lipid particles of the invention are associated with RNA interference (RNAi) molecules. RNA interference methods using RNAi molecules may be used to disrupt the expression of a gene or polynucleotide of interest. In the last 5 years small interfering RNA (siRNA) has essentially replaced antisense ODN and ribozymes as the next generation of targeted oligonucleotide drugs under development. SiRNAs are RNA duplexes normally 21-30 nucleotides long that can associate with a cytoplasmic multi-protein complex known as RNAi-induced silencing complex (RISC). RISC loaded with siRNA mediates the degradation of homologous mRNA transcripts, therefore siRNA can be designed to knock down protein expression with high specificity. Unlike other antisense technologies, siRNA function through a natural mechanism evolved to control gene expression through non-coding RNA. This is generally considered to be the reason why their activity is more potent *in vitro* and *in vivo* than either antisense ODN or ribozymes. A variety of RNAi reagents, including siRNAs targeting clinically relevant targets, are currently under pharmaceutical development, as described, *e.g.,* in de Fougerolles, A. et al., Nature Reviews 6:443-453 (2007).

While the first described RNAi molecules were RNA:RNA hybrids comprising both an RNA sense and an RNA antisense strand, it has now been demonstrated that DNA sense:RNA antisense hybrids, RNA sense:DNA antisense hybrids, and DNA:DNA hybrids are capable of mediating RNAi (Lamberton, J.S. and Christian, A.T., (2003) Molecular Biotechnology 24:111-119). Thus, the invention includes the use of RNAi molecules comprising any of these different types of double-stranded molecules. In addition, it is understood that RNAi molecules may be used and introduced to cells in a variety of forms. Accordingly, as used herein, RNAi molecules encompasses any and all molecules capable of inducing an RNAi response in cells, including, but not limited to, double-stranded polynucleotides comprising two separate strands, *i.e.* a sense strand and an antisense strand, *e.g.,* small interfering RNA (siRNA); polynucleotides comprising a hairpin loop of complementary sequences, which forms a double-stranded region, *e.g.*, shRNAi molecules, and expression vectors that express one or more polynucleotides capable of forming a double-stranded polynucleotide alone or in combination with another polynucleotide.

RNA interference (RNAi) may be used to specifically inhibit expression of target polynucleotides. Double-stranded RNA-mediated suppression of gene and nucleic acid expression may be accomplished according to the invention by introducing dsRNA, siRNA or shRNA into cells or organisms. SiRNA may be double-stranded RNA, or a hybrid molecule comprising both RNA and DNA, *e.g.,* one RNA strand and one DNA strand. It has been demonstrated that the direct introduction of siRNAs to a cell can trigger RNAi in mammalian cells (Elshabir, S.M., et al. Nature 411:494-498 (2001)). Furthermore, suppression in mammalian cells occurred at the RNA level and was specific for the targeted genes, with a strong correlation between RNA and protein suppression (Caplen, N. et al., Proc. Natl. Acad. Sci. USA 98:9746-9747 (2001)). In addition, it was shown that a wide variety of cell lines, including HeLa S3, COS7, 293, NIH/3T3, A549, HT-29, CHO-KI and MCF-7 cells, are susceptible to some level of siRNA silencing (Brown, D. et al. TechNotes 9(1):1-7, available on the worldwide web at www.dot.ambion.dot.com/techlib/tn/91/912.html (9/1/02)).

RNAi molecules targeting specific polynucleotides can be readily prepared according to procedures known in the art. Structural characteristics of effective siRNA molecules have been identified. Elshabir, S.M. et al. (2001) Nature 411:494-498 and Elshabir, S.M. et al. (2001), EMBO 20:6877-6888. Accordingly, one of skill in the art would understand that a wide variety of different siRNA molecules may be used to target a specific gene or transcript. In certain embodiments, siRNA molecules according to the invention are double-stranded and 16 - 30 or 18 - 25 nucleotides in length, including each integer in between. In one embodiment, an siRNA is 21 nucleotides in length. In certain embodiments, siRNAs have 0-7 nucleotide 3' overhangs or 0-4 nucleotide 5' overhangs. In one embodiment, an siRNA molecule has a two nucleotide 3' overhang. In one embodiment, an siRNA is 21 nucleotides in length with two nucleotide 3' overhangs (*i.e.*

they contain a 19 nucleotide complementary region between the sense and antisense strands). In certain embodiments, the overhangs are UU or dTdT 3' overhangs.

Generally, siRNA molecules are completely complementary to one strand of a target DNA molecule, since even single base pair mismatches have been shown to reduce silencing. In other embodiments, siRNAs may have a modified backbone composition, such as, for example, 2'-deoxy- or 2'-O-methyl modifications. However, in preferred embodiments, the entire strand of the siRNA is not made with either 2' deoxy or 2'-O-modified bases.

In another embodiment, the invention provides a cell including a vector for inhibiting the expression of a gene in a cell. The vector includes a regulatory sequence operably linked to a nucleotide sequence that encodes at least one strand of one of the dsRNA of the invention.

In one embodiment, siRNA target sites are selected by scanning the target mRNA transcript sequence for the occurrence of AA dinucleotide sequences. Each AA dinucleotide sequence in combination with the 3' adjacent approximately 19 nucleotides are potential siRNA target sites. In one embodiment, siRNA target sites are preferentially not located within the 5' and 3' untranslated regions (UTRs) or regions near the start codon (within approximately 75 bases), since proteins that bind regulatory regions may interfere with the binding of the siRNP endonuclease complex (Elshabir, S. et al. Nature 411:494-498 (2001); Elshabir, S. et al. EMBO J. 20:6877-6888 (2001)). In addition, potential target sites may be compared to an appropriate genome database, such as BLASTN 2.0.5, available on the NCBI server at www.ncbi.nlm, and potential target sequences with significant homology to other coding sequences eliminated.

In particular embodiments, short hairpin RNAs constitute the nucleic acid component of nucleic acid-lipid particles of the invention. Short Hairpin RNA (shRNA) is a form of hairpin RNA capable of sequence-specifically reducing expression of a target gene. Short hairpin RNAs may offer an advantage over siRNAs in suppressing gene expression, as they are generally more stable and less susceptible to degradation in the cellular environment. It has been established that such short hairpin RNA-mediated gene silencing works in a variety of normal and cancer cell lines, and in mammalian cells, including mouse and human cells. Paddison, P. et al., Genes Dev. 16(8):948-58 (2002). Furthermore, transgenic cell lines bearing chromosomal genes that code for engineered shRNAs have been generated. These cells are able to constitutively synthesize shRNAs, thereby facilitating long-lasting or constitutive gene silencing that may be passed on to progeny cells. Paddison, P. et al., Proc. Natl. Acad. Sci. USA 99(3):1443-1448 (2002).

ShRNAs contain a stem loop structure. In certain embodiments, they may contain variable stem lengths, typically from 19 to 29 nucleotides in length, or any number in between. In certain embodiments, hairpins contain 19 to 21 nucleotide stems, while in other embodiments, hairpins contain 27 to 29 nucleotide stems. In certain embodiments, loop size is between 4 to 23 nucleotides in length, although the loop size may be larger than 23 nucleotides without significantly affecting silencing activity. ShRNA molecules may contain mismatches, for example G-U mismatches between the two strands of the shRNA stem without decreasing potency. In fact, in certain embodiments, shRNAs are designed to include one or several G-U pairings in the hairpin stem to stabilize hairpins during propagation in bacteria, for example. However, complementarity between the portion of the stem that binds to the target mRNA (antisense strand) and the mRNA is typically required, and even a single base pair mismatch is this region may abolish silencing. 5' and 3' overhangs are not required, since they do not appear to be critical for shRNA function, although they may be present (Paddison et al. (2002) Genes & Dev. 16(8):948-58).

<u>MicroRNAs</u>

Micro RNAs (miRNAs) are a highly conserved class of small RNA molecules that are transcribed from DNA in the genomes of plants and animals, but are not translated into protein. Processed miRNAs are single stranded ~17-25 nucleotide (nt) RNA molecules that become incorporated into the RNA-induced silencing complex (RISC) and have been identified as key regulators of development, cell proliferation, apoptosis and differentiation. They are believed to play a role in regulation of gene expression by binding to the 3'-untranslated region of specific mRNAs.RISC mediates down-regulation of gene expression through translational inhibition, transcript cleavage, or both. RISC is also implicated in transcriptional silencing in the nucleus of a wide range of eukaryotes.

The number of miRNA sequences identified to date is large and growing, illustrative examples of which can be found, for example, in: "miRBase: microRNA sequences, targets and gene nomenclature" Griffiths-Jones S, Grocock RJ, van Dongen S, Bateman A, Emight AJ. NAR, 2006, 34, Database Issue, D140-D144; "The microRNA Registry" Griffiths-Jones S. NAR, 2004, 32, Database Issue, D109-D111; and also on the worldwide web at microrna.dot.sanger.dot.ac.dot.uk/sequences/.

<u>Antisense Oligonucleotides</u>

In one embodiment, a nucleic acid is an antisense oligonucleotide directed to a target polynucleotide. The term "antisense oligonucleotide" or simply "antisense" is meant to include oligonucleotides that are complementary to a targeted polynucleotide sequence. Antisense oligonucleotides are single strands of DNA or RNA that are complementary

to a chosen sequence. In the case of antisense RNA, they prevent translation of complementary RNA strands by binding to it. Antisense DNA can be used to target a specific, complementary (coding or non-coding) RNA. If binding takes places this DNA/RNA hybrid can be degraded by the enzyme RNase H. In particular embodiment, antisense oligonucleotides contain from about 10 to about 50 nucleotides, more preferably about 15 to about 30 nucleotides. The term also encompasses antisense oligonucleotides that may not be exactly complementary to the desired target gene. Thus, the invention can be utilized in instances where non-target specific-activities are found with antisense, or where an antisense sequence containing one or more mismatches with the target sequence is the most preferred for a particular use.

Antisense oligonucleotides have been demonstrated to be effective and targeted inhibitors of protein synthesis, and, consequently, can be used to specifically inhibit protein synthesis by a targeted gene. The efficacy of antisense oligonucleotides for inhibiting protein synthesis is well established. For example, the synthesis of polygalactauronase and the muscarine type 2 acetylcholine receptor are inhibited by antisense oligonucleotides directed to their respective mRNA sequences (U. S. Patent 5,739,119 and U. S. Patent 5,759,829). Further, examples of antisense inhibition have been demonstrated with the nuclear protein cyclin, the multiple drug resistance gene (MDG1), ICAM-1, E-selectin, STK-1, striatal $GABA_A$ receptor and human EGF (Jaskulski et al., Science. 1988 Jun 10;240(4858):1544-6; Vasanthakumar and Ahmed, Cancer Commun. 1989;1(4):225-32; Peris et al., Brain Res Mol Brain Res. 1998 Jun 15;57(2):310-20; U. S. Patent 5,801,154; U.S. Patent 5,789,573; U. S. Patent 5,718,709 and U.S. Patent 5,610,288). Furthermore, antisense constructs have also been described that inhibit and can be used to treat a variety of abnormal cellular proliferations, *e.g.* cancer (U. S. Patent 5,747,470; U. S. Patent 5,591,317 and U. S. Patent 5,783,683).

Methods of producing antisense oligonucleotides are known in the art and can be readily adapted to produce an antisense oligonucleotide that targets any polynucleotide sequence. Selection of antisense oligonucleotide sequences specific for a given target sequence is based upon analysis of the chosen target sequence and determination of secondary structure, $T_m$, binding energy, and relative stability. Antisense oligonucleotides may be selected based upon their relative inability to form dimers, hairpins, or other secondary structures that would reduce or prohibit specific binding to the target mRNA in a host cell. Highly preferred target regions of the mRNA include those regions at or near the AUG translation initiation codon and those sequences that are substantially complementary to 5' regions of the mRNA. These secondary structure analyses and target site selection considerations can be performed, for example, using v.4 of the OLIGO primer analysis software (Molecular Biology Insights) and/or the BLASTN 2.0.5 algorithm software (Altschul et al., Nucleic Acids Res. 1997, 25(17):3389-402).

Ribozymes

According to another embodiment of the invention, nucleic acid-lipid particles are associated with ribozymes. Ribozymes are RNA-protein complexes having specific catalytic domains that possess endonuclease activity (Kim and Cech, Proc Natl Acad Sci USA. 1987 Dec;84(24):8788-92; Forster and Symons, Cell. 1987 Apr 24;49(2):211-20). For example, a large number of ribozymes accelerate phosphoester transfer reactions with a high degree of specificity, often cleaving only one of several phosphoesters in an oligonucleotide substrate (Cech et al., Cell. 1981 Dec;27(3 Pt 2):487-96; Michel and Westhof, J Mol Biol. 1990 Dec 5;216(3):585-610; Reinhold-Hurek and Shub, Nature. 1992 May 14;357(6374):173-6). This specificity has been attributed to the requirement that the substrate bind via specific base-pairing interactions to the internal guide sequence ("IGS") of the ribozyme prior to chemical reaction.

At least six basic varieties of naturally-occurring enzymatic RNAs are known presently. Each can catalyze the hydrolysis of RNA phosphodiester bonds *in trans* (and thus can cleave other RNA molecules) under physiological conditions. In general, enzymatic nucleic acids act by first binding to a target RNA. Such binding occurs through the target binding portion of a enzymatic nucleic acid which is held in close proximity to an enzymatic portion of the molecule that acts to cleave the target RNA. Thus, the enzymatic nucleic acid first recognizes and then binds a target RNA through complementary base-pairing, and once bound to the correct site, acts enzymatically to cut the target RNA. Strategic cleavage of such a target RNA will destroy its ability to direct synthesis of an encoded protein. After an enzymatic nucleic acid has bound and cleaved its RNA target, it is released from that RNA to search for another target and can repeatedly bind and cleave new targets.

The enzymatic nucleic acid molecule may be formed in a hammerhead, hairpin, a hepatitis $\delta$ virus, group I intron or RNaseP RNA (in association with an RNA guide sequence) or Neurospora VS RNA motif, for example. Specific examples of hammerhead motifs are described by Rossi et al. Nucleic Acids Res. 1992 Sep 11;20(17):4559-65. Examples of hairpin motifs are described by Hampel *et al.* (Eur. Pat. Appl. Publ. No. EP 0360257), Hampel and Tritz, Biochemistry 1989 Jun 13;28(12):4929-33; Hampel et al., Nucleic Acids Res. 1990 Jan 25;18(2):299-304 and U. S. Patent 5,631,359. An example of the hepatitis $\delta$ virus motif is described by Perrotta and Been, Biochemistry. 1992 Dec 1;31(47):11843-52; an example of the RNaseP motif is described by Guerrier-Takada et al., Cell. 1983 Dec;35(3 Pt 2):849-57; Neurospora VS RNA ribozyme motif is described by Collins (Saville and Collins, Cell. 1990 May 18;61(4):685-96; Saville and Collins, Proc Natl Acad Sci USA. 1991 Oct 1;88(19):8826-30; Collins and Olive, Biochemistry. 1993 Mar 23;32(11):2795-9); and an example of the Group I intron is described in U. S. Patent 4,987,071. Important characteristics of enzymatic nucleic

acid molecules used according to the invention are that they have a specific substrate binding site which is complementary to one or more of the target gene DNA or RNA regions, and that they have nucleotide sequences within or surrounding that substrate binding site which impart an RNA cleaving activity to the molecule. Thus the ribozyme constructs need not be limited to specific motifs mentioned herein.

Methods of producing a ribozyme targeted to any polynucleotide sequence are known in the art. Ribozymes may be designed as described in Int. Pat. Appl. Publ. No. WO 93/23569 and Int. Pat. Appl. Publ. No. WO 94/02595, each specifically incorporated herein by reference, and synthesized to be tested *in vitro* and *in vivo,* as described therein.

Ribozyme activity can be optimized by altering the length of the ribozyme binding arms or chemically synthesizing ribozymes with modifications that prevent their degradation by serum ribonucleases (see *e.g.*, Int. Pat. Appl. Publ. No. WO 92/07065; Int. Pat. Appl. Publ. No. WO 93/15187; Int. Pat. Appl. Publ. No. WO 91/03162; Eur. Pat. Appl. Publ. No. 92110298.4; U. S. Patent 5,334,711; and Int. Pat. Appl. Publ. No. WO 94/13688, which describe various chemical modifications that can be made to the sugar moieties of enzymatic RNA molecules), modifications which enhance their efficacy in cells, and removal of stem II bases to shorten RNA synthesis times and reduce chemical requirements.

Additional specific nucleic acid sequences of oligonucleotides (ODNs) suitable for use in the compositions and methods of the invention are described in U.S. Patent Appln. 60/379,343, U.S. patent application Ser. No. 09/649,527, Int. Publ. WO 02/069369, Int. Publ. No. WO 01/15726, U.S. Pat. No. 6,406,705, and Raney et al., Journal of Pharmacology and Experimental Therapeutics, 298:1185-1192 (2001). In certain embodiments, ODNs used in the compositions and methods of the invention have a phosphodiester ("PO") backbone or a phosphorothioate ("PS") backbone, and/or at least one methylated cytosine residue in a CpG motif.

Nucleic Acid Modifications

In the 1990's DNA-based antisense oligodeoxynucleotides (ODN) and ribozymes (RNA) represented an exciting new paradigm for drug design and development, but their application *in vivo* was prevented by endo- and exo- nuclease activity as well as a lack of successful intracellular delivery. The degradation issue was effectively overcome following extensive research into chemical modifications that prevented the oligonucleotide (oligo) drugs from being recognized by nuclease enzymes but did not inhibit their mechanism of action. This research was so successful that antisense ODN drugs in development today remain intact *in vivo* for days compared to minutes for unmodified molecules (Kurreck, J. 2003. Antisense technologies. Improvement through novel chemical modifications. Eur J Biochem 270:1628-44). However, intracellular delivery and mechanism of action issues have so far limited antisense ODN and ribozymes from becoming clinical products.

RNA duplexes are inherently more stable to nucleases than single stranded DNA or RNA, and unlike antisense ODN, unmodified siRNA show good activity once they access the cytoplasm. Even so, the chemical modifications developed to stabilize antisense ODN and ribozymes have also been systematically applied to siRNA to determine how much chemical modification can be tolerated and if pharmacokinetic and pharmacodynamic activity can be enhanced. RNA interference by siRNA duplexes requires an antisense and sense strand, which have different functions. Both are necessary to enable the siRNA to enter RISC, but once loaded the two strands separate and the sense strand is degraded whereas the antisense strand remains to guide RISC to the target mRNA. Entry into RISC is a process that is structurally less stringent than the recognition and cleavage of the target mRNA. Consequently, many different chemical modifications of the sense strand are possible, but only limited changes are tolerated by the antisense strand (Zhang et al., 2006).

As is known in the art, a nucleoside is a base-sugar combination. Nucleotides are nucleosides that further include a phosphate group covalently linked to the sugar portion of the nucleoside. For those nucleosides that include a pentofuranosyl sugar, the phosphate group can be linked to either the 2', 3' or 5' hydroxyl moiety of the sugar. In forming oligonucleotides, the phosphate groups covalently link adjacent nucleosides to one another to form a linear polymeric compound. In turn the respective ends of this linear polymeric structure can be further joined to form a circular structure. Within the oligonucleotide structure, the phosphate groups are commonly referred to as forming the internucleoside backbone of the oligonucleotide. The normal linkage or backbone of RNA and DNA is a 3' to 5' phosphodiester linkage.

The nucleic acid that is used in a lipid-nucleic acid particle according to this invention includes any form of nucleic acid that is known. Thus, the nucleic acid may be a modified nucleic acid of the type used previously to enhance nuclease resistance and serum stability. Surprisingly, however, acceptable therapeutic products can also be prepared using the method of the invention to formulate lipid-nucleic acid particles from nucleic acids that have no modification to the phosphodiester linkages of natural nucleic acid polymers, and the use of unmodified phosphodiester nucleic acids (*i.e.,* nucleic acids in which all of the linkages are phosphodiester linkages) is a preferred embodiment of the invention.

Backbone Modifications

Antisense, siRNA and other oligonucleotides useful in this invention include, but are not limited to, oligonucleotides containing modified backbones or non-natural internucleoside linkages. Oligonucleotides having modified backbones include those that retain a phosphorus atom in the backbone and those that do not have a phosphorus atom in the backbone. Modified oligonucleotides that do not have a phosphorus atom in their internucleoside backbone can also be considered to be oligonucleosides. Modified oligonucleotide backbones include, for example, phosphorothioates, chiral phosphorothioates, phosphorodithioates, phosphotriesters, aminoalkylphosphotri-esters, methyl and other alkyl phosphonates including 3'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, phosphoroselenate, methylphosphonate, or O-alkyl phosphotriester linkages, and boranophosphates having normal 3'-5' linkages, 2'-5' linked analogs of these, and those having inverted polarity wherein the adjacent pairs of nucleoside units are linked 3'-5' to 5'-3' or 2'-5' to 5'-2'. Particular non-limiting examples of particular modifications that may be present in a nucleic acid according to the invention are shown in Table 2.

Various salts, mixed salts and free acid forms are also included. Representative United States patents that teach the preparation of the above linkages include, but are not limited to, U.S. Patent Nos. 3,687,808; 4,469,863; 4,476,301; 5,023,243; 5,177,196; 5,188,897; 5,264,423; 5,276,019; 5,278,302; 5,286,717; 5,321,131; 5,399,676; 5,405,939; 5,453,496; 5,455,233; 5,466,677; 5,476,925; 5,519,126; 5,536,821; 5,541,306; 5,550,111; 5,563,253; 5,571,799; 5,587,361; and 5,625,050.

In certain embodiments, modified oligonucleotide backbones that do not include a phosphorus atom therein have backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages. These include, *e.g.*, those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N, O, S and $CH_2$ component parts. Representative United States patents that describe the above oligonucleosides include, but are not limited to, U.S. Pat. Nos. 5,034,506; 5,166,315; 5,185,444; 5,214,134; 5,216,141; 5,235,033; 5,264,562; 5,264,564; 5,405,938; 5,434,257; 5,466,677; 5,470,967; 5,489,677; 5,541,307; 5,561,225; 5,596,086; 5,602,240; 5,610,289; 5,602,240; 5,608,046; 5,610,289; 5,618,704; 5,623,070; 5,663,312; 5,633,360; 5,677,437; and 5,677,439.

The phosphorothioate backbone modification (Table 3, #1), where a non-bridging oxygen in the phosphodiester bond is replaced by sulfur, is one of the earliest and most common means deployed to stabilize nucleic acid drugs against nuclease degradation. In general, it appears that PS modifications can be made extensively to both siRNA strands without much impact on activity (Kurreck, J., Eur. J. Biochem. 270:1628-44, 2003). However, PS oligos are known to avidly associate non-specifically with proteins resulting in toxicity, especially upon i.v. administration. Therefore, the PS modification is usually restricted to one or two bases at the 3' and 5' ends. The boranophosphate linker (Table 3, #2) is a recent modification that is apparently more stable than PS, enhances siRNA activity and has low toxicity (Hall et al., Nucleic Acids Res. 32:5991-6000, 2004).

Table 3. Chemical Modifications Applied to siRNA and Other Nucleic Acids

| # | Abbreviation | Name | Modification Site | Structure |
|---|---|---|---|---|
| **1** | PS | Phosphorothioate | Backbone | 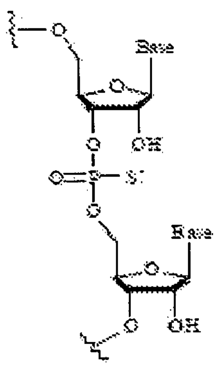 |

(continued)

| # | Abbreviation | Name | Modification Site | Structure |
|---|---|---|---|---|
| **2** | PB | Boranophosphate | Backbone | 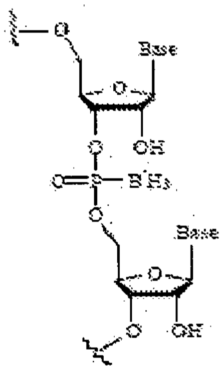 |
| **3** | N3-MU | N3-methyl-uridine | Base | 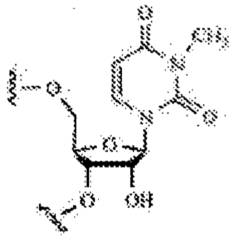 |
| **4** | 5'-BU | 5'-bromo-uracil | Base | 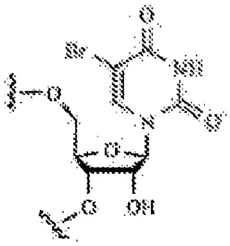 |
| **5** | 5'-IU | 5'-iodo-uracil | Base | 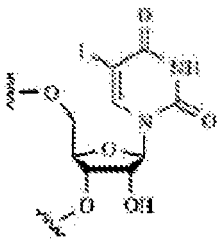 |
| **6** | 2,6-DP | 2,6-diaminopurine | Base | 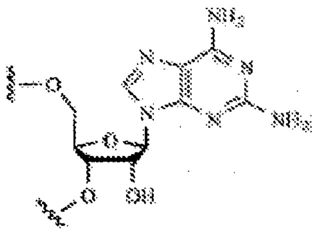 |
| **7** | 2'-F | 2'-Fluoro | Sugar | 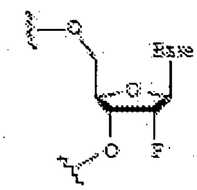 |

(continued)

| # | Abbreviation | Name | Modification Site | Structure |
|---|---|---|---|---|
| **8** | 2'-OME | 2"-O-methyl | Sugar | 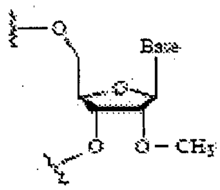 |
| **9** | 2'-O-MOE | 2'-O-(2-methoxylethyl) | Sugar | 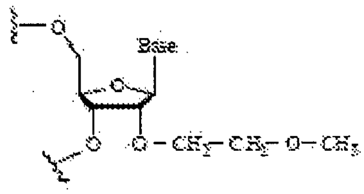 |
| **10** | 2'-DNP | 2'-O-(2,4-dinitrophenyl) | Sugar | 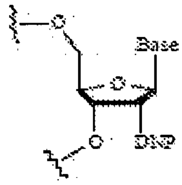 |
| **11** | LNA | Locked Nucleic Acid (methylene bridge connecting the 2'-oxygen with the 4'-carbon of the ribose ring) | Sugar | 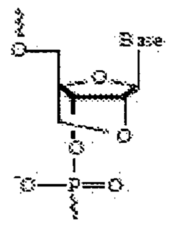 |
| **12** | 2'-Amino | 2'-Amino | Sugar | 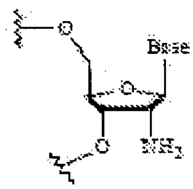 |
| **13** | 2'-Deoxy | 2'-Deoxy | Sugar | 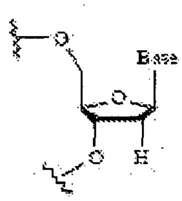 |
| **14** | 4'-thio | 4'-thio-ribonucleotide | Sugar | 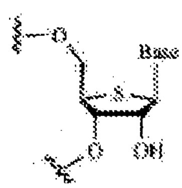 |

Other useful nucleic acids derivatives include those nucleic acids molecules in which the bridging oxygen atoms (those forming the phosphoester linkages) have been replaced with -S-, -NH-, -CH2- and the like. In certain embodiments, the alterations to the antisense, siRNA, or other nucleic acids used will not completely affect the negative charges

associated with the nucleic acids. Thus, the invention contemplates the use of antisense, siRNA, and other nucleic acids in which a portion of the linkages are replaced with, for example, the neutral methyl phosphonate or phosphoramidate linkages. When neutral linkages are used, in certain embodiments, less than 80% of the nucleic acid linkages are so substituted, or less than 50% of the linkages are so substituted.

Base Modifications

Base modifications are less common than those to the backbone and sugar. The modifications shown in 0.3-6 all appear to stabilize siRNA against nucleases and have little effect on activity (Zhang, H.Y., Du, Q., Wahlestedt, C., Liang, Z. 2006. RNA Interference with chemically modified siRNA. Curr Top Med Chem 6:893-900).

Accordingly, oligonucleotides may also include nucleobase (often referred to in the art simply as "base") modifications or substitutions. As used herein, "unmodified" or "natural" nucleobases include the purine bases adenine (A) and guanine (G), and the pyrimidine bases thymine (T), cytosine (C) and uracil (U). Modified nucleobases include other synthetic and natural nucleobases such as 5-methylcytosine (5-me-C or m5c), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl uracil and cytosine, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 8-azaguanine and 8-azaadenine, 7-deazaguanine and 7-deazaadenine and 3-deazaguanine and 3-deazaadenine.

Certain nucleobases are particularly useful for increasing the binding affinity of the oligomeric compounds of the invention, including 5-substituted pyrimidines, 6-azapyrimidines and N-2, N-6 and O-6 substituted purines, including 2-aminopropyladenine, 5-propynyluracil and 5-propynylcytosine. 5-methylcytosine substitutions have been shown to increase nucleic acid duplex stability by 0.6-1.2° C. (Sanghvi, Y. S., Crooke, S. T. and Lebleu, B., eds., Antisense Research and Applications 1993, CRC Press, Boca Raton, pages 276-278). These may be combined, in particular embodiments, with 2'-O-methoxyethyl sugar modifications. United States patents that teach the preparation of certain of these modified nucleobases as well as other modified nucleobases include, but are not limited to, the above noted U.S. Pat. No. 3,687,808, as well as U.S. Pat. Nos. 4,845,205; 5,130,302; 5,134,066; 5,175,273; 5,367,066; 5,432,272; 5,457,187; 5,459,255; 5,484,908; 5,502,177; 5,525,711; 5,552,540; 5,587,469; 5,594,121, 5,596,091; 5,614,617; and 5,681,941.

Sugar Modifications

Most modifications on the sugar group occur at the 2'-OH of the RNA sugar ring, which provides a convenient chemically reactive site (Manoharan, M. 2004. RNA interference and chemically modified small interfering RNAs. Curr Opin Chem Biol 8:570-9; Zhang, H.Y., Du, Q., Wahlestedt, C., Liang, Z. 2006. RNA Interference with chemically modified siRNA. Curr Top Med Chem 6:893-900). The 2'-F and 2'-OME (0.7 and 8) are common and both increase stability, the 2'-OME modification does not reduce activity as long as it is restricted to less than 4 nucleotides per strand (Holen, T., Amarzguioui, M., Babaie, E., Prydz, H. 2003. Similar behaviour of single-strand and double-strand siRNAs suggests they act through a common RNAi pathway. Nucleic Acids Res 31:2401-7). The 2'-O-MOE (0.9) is most effective in siRNA when modified bases are restricted to the middle region of the molecule ( Prakash, T.P., Allerson, C.R., Dande, P., Vickers, T.A., Sioufi, N., Jarres, R., Baker, B.F., Swayze, E.E., Griffey, R.H., Bhat, B. 2005. Positional effect of chemical modifications on short interference RNA activity in mammalian cells. J Med Chem 48:4247-53). Other modifications found to stabilize siRNA without loss of activity are shown in 0.10-14.

Modified oligonucleotides may also contain one or more substituted sugar moieties. For example, the invention includes oligonucleotides that comprise one of the following at the 2' position: OH; F; O-, S-, or N-alkyl, O-alkyl-O-alkyl, O-, S-, or N-alkenyl, or O-, S- or N-alkynyl, wherein the alkyl, alkenyl and alkynyl may be substituted or unsubstituted $C_1$ to $C_{10}$ alkyl or C2 to C10 alkenyl and alkynyl. Particularly preferred are $O[(CH_2)_nO]_mCH_3$, $O(CH_2)_nOCH_3$, $O(CH_2)_2ON(CH_3)_2$, $O(CH_2)_nNH_2$, $O(CH_2)_nCH_3$, $O(CH_2)_nONH_2$, and $O(CH_2)_nON[(CH_2)_nCH_3)]_2$, where n and m are from 1 to about 10. Other preferred oligonucleotides comprise one of the following at the 2' position: $C_1$ to $C_{10}$ lower alkyl, substituted lower alkyl, alkaryl, aralkyl, O-alkaryl or O-aralkyl, SH, $SCH_3$, OCN, Cl, Br, CN, $CF_3$, $OCF_3$, $SOCH_3$, $SO_2CH_3$, $ONO_2$, $NO_2$, $N_3$, $NH_2$, heterocycloalkyl, heterocycloalkaryl, aminoalkylamino, polyalkylamino, substituted silyl, an RNA cleaving group, a reporter group, an intercalator, a group for improving the pharmacokinetic properties of an oligonucleotide, or a group for improving the pharmacodynamic properties of an oligonucleotide, and other substituents having similar properties. One modification includes 2'-methoxyethoxy (2'-O--$CH_2CH_2OCH_3$, also known as 2'-O-(2-methoxyethyl) or 2'-MOE) (Martin et al., Helv. Chim. Acta 1995, 78, 486-504), *i.e.*, an alkoxyalkoxy group. Other modifications include 2'-dimethylaminooxyethoxy, *i.e.*, a $O(CH_2)_2ON(CH_3)_2$ group, also known as 2'-DMAOE, and 2'-dimethylaminoethoxyethoxy (2'-DMAEOE).

Additional modifications include 2'-methoxy (2'-O--$CH_3$), 2'-aminopropoxy (2'-$OCH_2CH_2CH_2NH_2$) and 2'-fluoro (2'-F). Similar modifications may also be made at other positions on the oligonucleotide, particularly the 3' position of the sugar on the 3' terminal nucleotide or in 2'-5' linked oligonucleotides and the 5' position of 5' terminal nucleotide. Oligonucleotides may also have sugar mimetics such as cyclobutyl moieties in place of the pentofuranosyl sugar. Representative United States patents that teach the preparation of such modified sugars structures include, but are not limited to, U.S. Pat. Nos. 4,981,957; 5,118,800; 5,319,080; 5,359,044; 5,393,878; 5,446,137; 5,466,786; 5,514,785; 5,519,134; 5,567,811; 5,576,427; 5,591,722; 5,597,909; 5,610,300; 5,627,053; 5,639,873; 5,646,265; 5,658,873; 5,670,633; and 5,700,920.

In other oligonucleotide mimetics, both the sugar and the internucleoside linkage, *i.e.,* the backbone, of the nucleotide units are replaced with novel groups, although the base units are maintained for hybridization with an appropriate nucleic acid target compound. One such oligomeric compound, an oligonucleotide mimetic that has been shown to have excellent hybridization properties, is referred to as a peptide nucleic acid (PNA). In PNA compounds, the sugar-backbone of an oligonucleotide is replaced with an amide containing backbone, in particular an aminoethylglycine backbone. The nucleobases are retained and are bound directly or indirectly to aza nitrogen atoms of the amide portion of the backbone. Representative United States patents that teach the preparation of PNA compounds include, but are not limited to, U.S. Pat. Nos. 5,539,082; 5,714,331; and 5,719,262. Further teaching of PNA compounds can be found in Nielsen et al. (Science, 1991, 254, 1497-1500).

Particular embodiments of the invention are oligonucleotides with phosphorothioate backbones and oligonucleosides with heteroatom backbones, and in particular --$CH_2$--NH--O--$CH_2$--, --$CH_2$--N($CH_3$) --O--$CH_2$- (referred to as a methylene (methylimino) or MMI backbone) --$CH_2$-O--N($CH_3$) --$CH_2$--, --$CH_2$-N($CH_3$)--N($CH_3$) --$CH_2$-- and --O--N($CH_3$) --$CH_2$--$CH_2$--(wherein the native phosphodiester backbone is represented as --O--P--O--$CH_2$--) of the above referenced U.S. Pat. No. 5,489,677, and the amide backbones of the above referenced U.S. Pat. No. 5,602,240. Also preferred are oligonucleotides having morpholino backbone structures of the above-referenced U.S. Pat. No. 5,034,506.

## Chimeric Oligonucleotides

It is not necessary for all positions in a given compound to be uniformly modified, and in fact more than one of the aforementioned modifications may be incorporated in a single compound or even at a single nucleoside within an oligonucleotide. Certain preferred oligonucleotides of this invention are chimeric oligonucleotides. "Chimeric oligonucleotides" or "chimeras," in the context of this invention, are oligonucleotides that contain two or more chemically distinct regions, each made up of at least one nucleotide. These oligonucleotides typically contain at least one region of modified nucleotides that confers one or more beneficial properties (such as, *e,g.,* increased nuclease resistance, increased uptake into cells, increased binding affinity for the RNA target) and a region that is a substrate for RNase H cleavage.

In one embodiment, a chimeric oligonucleotide comprises at least one region modified to increase target binding affinity. Affinity of an oligonucleotide for its target is routinely determined by measuring the Tm of an oligonucleotide/target pair, which is the temperature at which the oligonucleotide and target dissociate; dissociation is detected spectrophotometrically. The higher the Tm, the greater the affinity of the oligonucleotide for the target. In one embodiment, the region of the oligonucleotide which is modified to increase target mRNA binding affinity comprises at least one nucleotide modified at the 2' position of the sugar, most preferably a 2'-O-alkyl, 2'-O-alkyl-O-alkyl or 2'-fluoro-modified nucleotide. Such modifications are routinely incorporated into oligonucleotides and these oligonucleotides have been shown to have a higher Tm (*i.e.*, higher target binding affinity) than 2'-deoxyoligonucleotides against a given target. The effect of such increased affinity is to greatly enhance oligonucleotide inhibition of target gene expression.

In another embodiment, a chimeric oligonucletoide comprises a region that acts as a substrate for RNAse H. Of course, it is understood that oligonucleotides may include any combination of the various modifications described herein

Another modification of the oligonucleotides of the invention involves chemically linking to the oligonucleotide one or more moieties or conjugates which enhance the activity, cellular distribution or cellular uptake of the oligonucleotide. Such conjugates and methods of preparing the same are known in the art.

Those skilled in the art will realize that for *in vivo* utility, such as therapeutic efficacy, a reasonable rule of thumb is that if a thioated version of the sequence works in the free form, that encapsulated particles of the same sequence, of any chemistry, will also be efficacious. Encapsulated particles may also have a broader range of *in vivo* utilities, showing efficacy in conditions and models not known to be otherwise responsive to antisense therapy. Those skilled in the art know that applying this invention they may find old models which now respond to antisense therapy. Further, they may revisit discarded antisense sequences or chemistries and find efficacy by employing the invention.

The oligonucleotides used in accordance with this invention may be conveniently and routinely made through the well-known technique of solid phase synthesis. Equipment for such synthesis is sold by several vendors including Applied Biosystems. Any other means for such synthesis may also be employed; the actual synthesis of the oligonucleotides is well within the talents of the routineer. It is also well known to use similar techniques to prepare other oligonucleotides such as the phosphorothioates and alkylated derivatives.

Definitions

For convenience, the meaning of certain terms and phrases used in the specification, examples, and appended claims, are provided below. If there is an apparent discrepancy between the usage of a term in other parts of this specification and its definition provided in this section, the definition in this section shall prevail.

"G," "C," "A" and "U" each generally stand for a nucleotide that contains guanine, cytosine, adenine, and uracil as a base, respectively. However, it will be understood that the term "ribonucleotide" or "nucleotide" can also refer to a modified nucleotide, as further detailed below, or a surrogate replacement moiety. The skilled person is well aware that guanine, cytosine, adenine, and uracil may be replaced by other moieties without substantially altering the base pairing properties of an oligonucleotide including a nucleotide bearing such replacement moiety. For example, without limitation, a nucleotide including inosine as its base may base pair with nucleotides containing adenine, cytosine, or uracil. Hence, nucleotides containing uracil, guanine, or adenine may be replaced in the nucleotide sequences of the invention by a nucleotide containing, for example, inosine. Sequences including such replacement moieties are embodiments of the invention.

By "Factor VII" as used herein is meant a Factor VII mRNA, protein, peptide, or polypeptide. The term "Factor VII" is also known in the art as AI132620, Cf7, Coagulation factor VII precursor, coagulation factor VII, FVII, Serum prothrombin conversion accelerator, FVII coagulation protein, and eptacog alfa.

As used herein, "target sequence" refers to a contiguous portion of the nucleotide sequence of an mRNA molecule formed during the transcription of the gene, including mRNA that is a product of RNA processing of a primary transcription product.

As used herein, the term "strand including a sequence" refers to an oligonucleotide including a chain of nucleotides that is described by the sequence referred to using the standard nucleotide nomenclature.

As used herein, and unless otherwise indicated, the term "complementary," when used in the context of a nucleotide pair, means a classic Watson-Crick pair, i.e., GC, AT, or AU. It also extends to classic Watson-Crick pairings where one or both of the nuclotides has been modified as decribed herein, e.g., by a rbose modification or a phosphate backpone modification. It can also include pairing with an inosine or other entity that does not substantially alter the base pairing properties.

As used herein, and unless otherwise indicated, the term "complementary," when used to describe a first nucleotide sequence in relation to a second nucleotide sequence, refers to the ability of an oligonucleotide or polynucleotide including the first nucleotide sequence to hybridize and form a duplex structure under certain conditions with an oligonucleotide or polynucleotide including the second nucleotide sequence, as will be understood by the skilled person. Complementarity can include, full complementarity, substantial complementarity, and sufficient complementarity to allow hybridization under physiological conditions, e.g, under physiologically relevant conditions as may be encountered inside an organism. Full complementarity refers to complementarity, as defined above for an individual pair, at all of the pairs of the first and second sequence. When a sequence is "substantially complementary" with respect to a second sequence herein, the two sequences can be fully complementary, or they may form one or more, but generally not more than 4, 3 or 2 mismatched base pairs upon hybridization, while retaining the ability to hybridize under the conditions most relevant to their ultimate application. Substantial complementarity can also be defined as hybridization under stringent conditions, where stringent conditions may include: 400 mM NaCl, 40 mM PIPES pH 6.4, 1 mM EDTA, 50°C or 70°C for 12-16 hours followed by washing. The skilled person will be able to determine the set of conditions most appropriate for a test of complementarity of two sequences in accordance with the ultimate application of the hybridized nucleotides.

However, where two oligonucleotides are designed to form, upon hybridization, one or more single stranded overhangs, such overhangs shall not be regarded as mismatches with regard to the determination of complementarity. For example, a dsRNA including one oligonucleotide 21 nucleotides in length and another oligonucleotide 23 nucleotides in length, wherein the longer oligonucleotide includes a sequence of 21 nucleotides that is fully complementary to the shorter oligonucleotide, may yet be referred to as "fully complementary" for the purposes of the invention.

"Complementary" sequences, as used herein, may also include, or be formed entirely from, non-Watson-Crick base pairs and/or base pairs formed from non-natural and modified nucleotides, in as far as the above requirements with respect to their ability to hybridize are fulfilled.

The terms "complementary", "fully complementary", "substantially complementary" and sufficient complementarity to allow hybridization under physiological conditions, e.g, under physiologically relevant conditions as may be encountered inside an organism, may be used hereinwith respect to the base matching between the sense strand and the antisense strand of a dsRNA, or between the antisense strand of a dsRNA and a target sequence, as will be understood from the context of their use.

As used herein, a polynucleotide which is "complementary, e.g., substantially complementary to at least part

of' a messenger RNA (mRNA) refers to a polynucleotide which is complementary, e.g., substantially complementary, to a contiguous portion of the mRNA of interest (e.g., encoding Factor VII). For example, a polynucleotide is complementary to at least a part of a Factor VII mRNA if the sequence is substantially complementary to a non-interrupted portion of an mRNA encoding Factor VII.

The term "double-stranded RNA" or "dsRNA", as used herein, refers to a ribonucleic acid molecule, or complex of ribonucleic acid molecules, having a duplex structure including two anti-parallel and substantially complementary, as defined above, nucleic acid strands. The two strands forming the duplex structure may be different portions of one larger RNA molecule, or they may be separate RNA molecules. Where the two strands are part of one larger molecule, and therefore are connected by an uninterrupted chain of nucleotides between the 3'-end of one strand and the 5'end of the respective other strand forming the duplex structure, the connecting RNA chain is referred to as a "hairpin loop". Where the two strands are connected covalently by means other than an uninterrupted chain of nucleotides between the 3'-end of one strand and the 5'end of the respective other strand forming the duplex structure, the connecting structure is referred to as a "linker." The RNA strands may have the same or a different number of nucleotides. The maximum number of base pairs is the number of nucleotides in the shortest strand of the dsRNA. In addition to the duplex structure, a dsRNA may comprise one or more nucleotide overhangs. A dsRNA as used herein is also refered to as a "small inhibitory RNA," "siRNA," "siRNA agent," "iRNA agent" or "RNAi agent."

As used herein, a "nucleotide overhang" refers to the unpaired nucleotide or nucleotides that protrude from the duplex structure of a dsRNA when a 3'-end of one strand of the dsRNA extends beyond the 5'-end of the other strand, or vice versa. "Blunt" or "blunt end" means that there are no unpaired nucleotides at that end of the dsRNA, i.e., no nucleotide overhang. A "blunt ended" dsRNA is a dsRNA that is double-stranded over its entire length, i.e., no nucleotide overhang at either end of the molecule.

The term "antisense strand" refers to the strand of a dsRNA which includes a region that is substantially complementary to a target sequence. As used herein, the term "region of complementarity" refers to the region on the antisense strand that is substantially complementary to a sequence, for example a target sequence, as defined herein. Where the region of complementarity is not fully complementary to the target sequence, the mismatches are most tolerated in the terminal regions and, if present, are generally in a terminal region or regions, e.g., within 6, 5, 4, 3, or 2 nucleotides of the 5' and/or 3' terminus.

The term "sense strand," as used herein, refers to the strand of a dsRNA that includes a region that is substantially complementary to a region of the antisense strand.

The term "identity" is the relationship between two or more polynucleotide sequences, as determined by comparing the sequences. Identity also means the degree of sequence relatedness between polynucleotide sequences, as determined by the match between strings of such sequences. While there exist a number of methods to measure identity between two polynucleotide sequences, the term is well known to skilled artisans (see, e.g., Sequence Analysis in Molecular Biology, von Heinje, G., Academic Press (1987); and Sequence Analysis Primer, Gribskov., M. and Devereux, J., eds., M. Stockton Press, New York (1991)). "Substantially identical," as used herein, means there is a very high degree of homology (preferably 100% sequence identity) between the sense strand of the dsRNA and the corresponding part of the target gene. However, dsRNA having greater than 90%, or 95% sequence identity may be used in the invention, and thus sequence variations that might be expected due to genetic mutation, strain polymorphism, or evolutionary divergence can be tolerated. Although 100% identity is preferred, the dsRNA may contain single or multiple base-pair random mismatches between the RNA and the target gene.

"Introducing into a cell", when referring to a dsRNA, means facilitating uptake or absorption into the cell, as is understood by those skilled in the art. Absorption or uptake of dsRNA can occur through unaided diffusive or active cellular processes, or by auxiliary agents or devices. The meaning of this term is not limited to cells in vitro; a dsRNA may also be "introduced into a cell," wherein the cell is part of a living organism. In such instance, introduction into the cell will include the delivery to the organism. For example, for in vivo delivery, dsRNA can be injected into a tissue site or administered systemically. In vitro introduction into a cell includes methods known in the art such as electroporation and lipofection.

The terms "silence" and "inhibit the expression of," in as far as they refer to the Factor VII gene, herein refer to the at least partial suppression of the expression of the Factor VII gene, as manifested by a reduction of the amount of mRNA from the Factor VII gene which may be isolated from a first cell or group of cells in which the Factor VII gene is transcribed and which has or have been treated such that the expression of the Factor VII gene is inhibited, as compared to a second cell or group of cells substantially identical to the first cell or group of cells but which has or have not been so treated (control cells). The degree of inhibition is usually expressed in terms of

$$\frac{(\text{mRNA in control cells}) - (\text{mRNA in treated cells})}{(\text{mRNA in control cells})} \bullet 100\%$$

Alternatively, the degree of inhibition may be given in terms of a reduction of a parameter that is functionally linked to Factor VII gene transcription, e.g. the amount of protein encoded by the Factor VII gene which is secreted by a cell, or the number of cells displaying a certain phenotype, e.g apoptosis. In principle, Factor VII gene silencing may be determined in any cell expressing the target, either constitutively or by genomic engineering, and by any appropriate assay. However, when a reference is needed in order to determine whether a given siRNA inhibits the expression of the Factor VII gene by a certain degree and therefore is encompassed by the instant invention, the assays provided in the Examples below shall serve as such reference.

For example, in certain instances, expression of the Factor VII gene is suppressed by at least about 20%, 25%, 35%, 40% or 50% by administration of the double-stranded oligonucleotide of the invention. In one embodiment, the Factor VII gene is suppressed by at least about 60%, 70%, or 80% by administration of the double-stranded oligonucleotide of the invention. In a more preferred embodiment, the Factor VII gene is suppressed by at least about 85%, 90%, or 95% by administration of the double-stranded oligonucleotide of the invention.

The terms "treat," "treatment," and the like, refer to relief from or alleviation of a disease or disorder. In the context of the invention insofar as it relates to any of the other conditions recited herein below (*e.g.*, a Factor VII -mediated condition other than a thrombotic disorder), the terms "treat," "treatment," and the like mean to relieve or alleviate at least one symptom associated with such condition, or to slow or reverse the progression of such condition.

A "therapeutically relevant" composition can alleviate a disease or disorder, or a symptom of a disease or disorder when administered at an appropriate dose.

As used herein, the term "Factor VII -mediated condition or disease" and related terms and phrases refer to a condition or disorder characterized by inappropriate, e.g., greater than normal, Factor VII activity. Inappropriate Factor VII functional activity might arise as the result of Factor VII expression in cells which normally do not express Factor VII, or increased Factor VII expression (leading to, e.g., a symptom of a viral hemorrhagic fever, or a thrombus). A Factor VII-mediated condition or disease may be completely or partially mediated by inappropriate Factor VII functional activity. However, a Factor VII-mediated condition or disease is one in which modulation of Factor VII results in some effect on the underlying condition or disorder (*e.g.*, a Factor VII inhibitor results in some improvement in patient well-being in at least some patients).

A "hemorrhagic fever" includes a combination of illnesses caused by a viral infection. Fever and gastrointestinal symptoms are typically followed by capillary hemorrhaging.

A "coagulopathy" is any defect in the blood clotting mechanism of a subject.

As used herein, a "thrombotic disorder" is any disorder, preferably resulting from unwanted FVII expression, including any disorder characterized by unwanted blood coagulation.

As used herein, the phrases "therapeutically effective amount" and "prophylactically effective amount" refer to an amount that provides a therapeutic benefit in the treatment, prevention, or management of a viral hemorrhagic fever, or an overt symptom of such disorder, e.g., hemorraging, fever, weakness, muscle pain, headache, inflammation, or circulatory shock. The specific amount that is therapeutically effective can be readily determined by ordinary medical practitioner, and may vary depending on factors known in the art, such as, e.g. the type of thrombotic disorder, the patient's history and age, the stage of the disease, and the administration of other agents.

As used herein, a "pharmaceutical composition" includes a pharmacologically effective amount of a dsRNA and a pharmaceutically acceptable carrier. As used herein, "pharmacologically effective amount," "therapeutically effective amount" or simply "effective amount" refers to that amount of an RNA effective to produce the intended pharmacological, therapeutic or preventive result. For example, if a given clinical treatment is considered effective when there is at least a 25% reduction in a measurable parameter associated with a disease or disorder, a therapeutically effective amount of a drug for the treatment of that disease or disorder is the amount necessary to effect at least a 25% reduction in that parameter.

The term "pharmaceutically acceptable carrier" refers to a carrier for administration of a therapeutic agent. Such carriers include, but are not limited to, saline, buffered saline, dextrose, water, glycerol, ethanol, and combinations thereof. The term specifically excludes cell culture medium. For drugs administered orally, pharmaceutically acceptable carriers include, but are not limited to pharmaceutically acceptable excipients such as inert diluents, disintegrating agents, binding agents, lubricating agents, sweetening agents, flavoring agents, coloring agents and preservatives. Suitable inert diluents include sodium and calcium carbonate, sodium and calcium phosphate, and lactose, while corn starch and alginic acid are suitable disintegrating agents. Binding agents may include starch and gelatin, while the lubricating agent, if present, will generally be magnesium stearate, stearic acid or talc. If desired, the tablets may be coated with a material such as glyceryl monostearate or glyceryl distearate, to delay absorption in the gastrointestinal tract.

As used herein, a "transformed cell" is a cell into which a vector has been introduced from which a dsRNA molecule may be expressed.

Characteristic of Nucleic Acid-Lipid Particles

In certain embodiments, the invention relates to methods and compositions for producing lipid-encapsulated nucleic acid particles in which nucleic acids are encapsulated within a lipid layer. Such nucleic acid-lipid particles, incorporating siRNA oligonucleotides, are characterized using a variety of biophysical parameters including: (1) drug to lipid ratio; (2) encapsulation efficiency; and (3) particle size. High drug to lipid rations, high encapsulation efficiency, good nuclease resistance and serum stability and controllable particle size, generally less than 200 nm in diameter are desirable. In addition, the nature of the nucleic acid polymer is of significance, since the modification of nucleic acids in an effort to impart nuclease resistance adds to the cost of therapeutics while in many cases providing only limited resistance. Unless stated otherwise, these criteria are calculated in this specification as follows:

Nucleic acid to lipid ratio is the amount of nucleic acid in a defined volume of preparation divided by the amount of lipid in the same volume. This may be on a mole per mole basis or on a weight per weight basis, or on a weight per mole basis. For final, administration-ready formulations, the nucleic acid:lipid ratio is calculated after dialysis, chromatography and/or enzyme (*e.g.,* nuclease) digestion has been employed to remove as much of the external nucleic acid as possible;

Encapsulation efficiency refers to the drug to lipid ratio of the starting mixture divided by the drug to lipid ratio of the final, administration competent formulation. This is a measure of relative efficiency. For a measure of absolute efficiency, the total amount of nucleic acid added to the starting mixture that ends up in the administration competent formulation, can also be calculated. The amount of lipid lost during the formulation process may also be calculated. Efficiency is a measure of the wastage and expense of the formulation; and

Size indicates the size (diameter) of the particles formed. Size distribution may be determined using quasi-elastic light scattering (QELS) on a Nicomp Model 370 sub-micron particle sizer. Particles under 200 nm are preferred for distribution to neo-vascularized (leaky) tissues, such as neoplasms and sites of inflammation.

Methods of preparing lipid particles

The methods and compositions of the invention make use of certain cationic lipids, the synthesis, preparation and characterization of which is described below and in the accompanying Examples. In addition, the present invention provides methods of preparing lipid particles, including those associated with a therapeutic agent, *e.g.*, a nucleic acid. In the methods described herein, a mixture of lipids is combined with a buffered aqueous solution of nucleic acid to produce an intermediate mixture containing nucleic acid encapsulated in lipid particles wherein the encapsulated nucleic acids are present in a nucleic acid/lipid ratio of about 3 wt% to about 25 wt%, preferably 5 to 15 wt%. The intermediate mixture may optionally be sized to obtain lipid-encapsulated nucleic acid particles wherein the lipid portions are unilamellar vesicles, preferably having a diameter of 30 to 150 nm, more preferably about 40 to 90 nm. The pH is then raised to neutralize at least a portion of the surface charges on the lipid-nucleic acid particles, thus providing an at least partially surface-neutralized lipid-encapsulated nucleic acid composition.

As described above, several of these cationic lipids are amino lipids that are charged at a pH below the $pK_a$ of the amino group and substantially neutral at a pH above the $pK_a$. These cationic lipids are termed titratable cationic lipids and can be used in the formulations of the invention using a two-step process. First, lipid vesicles can be formed at the lower pH with titratable cationic lipids and other vesicle components in the presence of nucleic acids. In this manner, the vesicles will encapsulate and entrap the nucleic acids. Second, the surface charge of the newly formed vesicles can be neutralized by increasing the pH of the medium to a level above the $pK_a$ of the titratable cationic lipids present, *i.e.*, to physiological pH or higher. Particularly advantageous aspects of this process include both the facile removal of any surface adsorbed nucleic acid and a resultant nucleic acid delivery vehicle which has a neutral surface. Liposomes or lipid particles having a neutral surface are expected to avoid rapid clearance from circulation and to avoid certain toxicities which are associated with cationic liposome preparations. Additional details concerning these uses of such titratable cationic lipids in the formulation of nucleic acid-lipid particles are provided in US Patent 6,287,591 and US Patent 6,858,225, incorporated herein by reference.

It is further noted that the vesicles formed in this manner provide formulations of uniform vesicle size with high content of nucleic acids. Additionally, the vesicles have a size range of from about 30 to about 150 nm, more preferably about 30 to about 90 nm.

Without intending to be bound by any particular theory, it is believed that the very high efficiency of nucleic acid encapsulation is a result of electrostatic interaction at low pH. At acidic pH (e.g. pH 4.0) the vesicle surface is charged and binds a portion of the nucleic acids through electrostatic interactions. When the external acidic buffer is exchanged for a more neutral buffer (*e.g.*. pH 7.5) the surface of the lipid particle or liposome is neutralized, allowing any external nucleic acid to be removed. More detailed information on the formulation process is provided in various publications (*e.g.*, US Patent 6,287,591 and US Patent 6,858,225).

In view of the above, the present invention provides methods of preparing lipid/nucleic acid formulations. In the methods described herein, a mixture of lipids is combined with a buffered aqueous solution of nucleic acid to produce an intermediate mixture containing nucleic acid encapsulated in lipid particles, *e.g.*, wherein the encapsulated nucleic acids are present in a nucleic acid/lipid ratio of about 10 wt% to about 20 wt%. The intermediate mixture may optionally be sized to obtain lipid-encapsulated nucleic acid particles wherein the lipid portions are unilamellar vesicles, preferably having a diameter of 30 to 150 nm, more preferably about 40 to 90 nm. The pH is then raised to neutralize at least a portion of the surface charges on the lipid-nucleic acid particles, thus providing an at least partially surface-neutralized lipid-encapsulated nucleic acid composition.

In certain embodiments, the mixture of lipids includes at least two lipid components: a first amino lipid component of the present invention that is selected from among lipids which have a pKa such that the lipid is cationic at pH below the pKa and neutral at pH above the pKa, and a second lipid component that is selected from among lipids that prevent particle aggregation during lipid-nucleic acid particle formation. In particular embodiments, the amino lipid is a novel cationic lipid of the present invention.

In preparing the nucleic acid-lipid particles of the invention, the mixture of lipids is typically a solution of lipids in an organic solvent. This mixture of lipids can then be dried to form a thin film or lyophilized to form a powder before being hydrated with an aqueous buffer to form liposomes. Alternatively, in a preferred method, the lipid mixture can be solubilized in a water miscible alcohol, such as ethanol, and this ethanolic solution added to an aqueous buffer resulting in spontaneous liposome formation. In most embodiments, the alcohol is used in the form in which it is commercially available. For example, ethanol can be used as absolute ethanol (100%), or as 95% ethanol, the remainder being water. This method is described in more detail in US Patent 5,976,567).

In accordance with the invention, the lipid mixture is combined with a buffered aqueous solution that may contain the nucleic acids. The buffered aqueous solution of is typically a solution in which the buffer has a pH of less than the $pK_a$ of the protonatable lipid in the lipid mixture. Examples of suitable buffers include citrate, phosphate, acetate, and MES. A particularly preferred buffer is citrate buffer. Preferred buffers will be in the range of 1-1000 mM of the anion, depending on the chemistry of the nucleic acid being encapsulated, and optimization of buffer concentration may be significant to achieving high loading levels (*see, e.g.*, US Patent 6,287,591 and US Patent 6,858,225). Alternatively, pure water acidified to pH 5-6 with chloride, sulfate or the like may be useful. In this case, it may be suitable to add 5% glucose, or another non-ionic solute which will balance the osmotic potential across the particle membrane when the particles are dialyzed to remove ethanol, increase the pH, or mixed with a pharmaceutically acceptable carrier such as normal saline. The amount of nucleic acid in buffer can vary, but will typically be from about 0.01 mg/mL to about 200 mg/mL, more preferably from about 0.5 mg/mL to about 50 mg/mL.

The mixture of lipids and the buffered aqueous solution of therapeutic nucleic acids is combined to provide an intermediate mixture. The intermediate mixture is typically a mixture of lipid particles having encapsulated nucleic acids. Additionally, the intermediate mixture may also contain some portion of nucleic acids which are attached to the surface of the lipid particles (liposomes or lipid vesicles) due to the ionic attraction of the negatively-charged nucleic acids and positively-charged lipids on the lipid particle surface (the amino lipids or other lipid making up the protonatable first lipid component are positively charged in a buffer having a pH of less than the $pK_a$ of the protonatable group on the lipid). In one group of preferred embodiments, the mixture of lipids is an alcohol solution of lipids and the volumes of each of the solutions is adjusted so that upon combination, the resulting alcohol content is from about 20% by volume to about 45% by volume. The method of combining the mixtures can include any of a variety of processes, often depending upon the scale of formulation produced. For example, when the total volume is about 10-20 mL or less, the solutions can be combined in a test tube and stirred together using a vortex mixer. Large-scale processes can be carried out in suitable production scale glassware.

Optionally, the lipid-encapsulated therapeutic agent (e.g., nucleic acid) complexes which are produced by combining the lipid mixture and the buffered aqueous solution of therapeutic agents (nucleic acids) can be sized to achieve a desired size range and relatively narrow distribution of lipid particle sizes. Preferably, the compositions provided herein will be sized to a mean diameter of from about 70 to about 200 nm, more preferably about 90 to about 130 nm. Several techniques are available for sizing liposomes to a desired size. One sizing method is described in U.S. Pat. No. 4,737,323, incorporated herein by reference. Sonicating a liposome suspension either by bath or probe sonication produces a progressive size reduction down to small unilamellar vesicles (SUVs) less than about 0.05 microns in size. Homogenization is another method which relies on shearing energy to fragment large liposomes into smaller ones. In a typical homogenization procedure, multilamellar vesicles are recirculated through a standard emulsion homogenizer until selected liposome sizes, typically between about 0.1 and 0.5 microns, are observed. In both methods, the particle size distribution can be monitored by conventional laser-beam particle size determination. For certain methods herein, extrusion is used to obtain a uniform vesicle size.

Extrusion of liposome compositions through a small-pore polycarbonate membrane or an asymmetric ceramic membrane results in a relatively well-defined size distribution. Typically, the suspension is cycled through the membrane one or more times until the desired liposome complex size distribution is achieved. The liposomes may be extruded

through successively smaller-pore membranes, to achieve a gradual reduction in liposome size. In some instances, the lipid-nucleic acid compositions which are formed can be used without any sizing.

In particular embodiments, methods of the present invention further comprise a step of neutralizing at least some of the surface charges on the lipid portions of the lipid-nucleic acid compositions. By at least partially neutralizing the surface charges, unencapsulated nucleic acid is freed from the lipid particle surface and can be removed from the composition using conventional techniques. Preferably, unencapsulated and surface adsorbed nucleic acids are removed from the resulting compositions through exchange of buffer solutions. For example, replacement of a citrate buffer (pH about 4.0, used for forming the compositions) with a HEPES-buffered saline (HBS pH about 7.5) solution, results in the neutralization of liposome surface and nucleic acid release from the surface. The released nucleic acid can then be removed via chromatography using standard methods, and then switched into a buffer with a pH above the pKa of the lipid used.

Optionally the lipid vesicles (*i.e.,* lipid particles) can be formed by hydration in an aqueous buffer and sized using any of the methods described above prior to addition of the nucleic acid. As described above, the aqueous buffer should be of a pH below the pKa of the amino lipid. A solution of the nucleic acids can then be added to these sized, preformed vesicles. To allow encapsulation of nucleic acids into such "pre-formed" vesicles the mixture should contain an alcohol, such as ethanol. In the case of ethanol, it should be present at a concentration of about 20% (w/w) to about 45% (w/w). In addition, it may be necessary to warm the mixture of pre-formed vesicles and nucleic acid in the aqueous buffer-ethanol mixture to a temperature of about 25° C to about 50° C depending on the composition of the lipid vesicles and the nature of the nucleic acid. It will be apparent to one of ordinary skill in the art that optimization of the encapsulation process to achieve a desired level of nucleic acid in the lipid vesicles will require manipulation of variable such as ethanol concentration and temperature. Examples of suitable conditions for nucleic acid encapsulation are provided in the Examples. Once the nucleic acids are encapsulated within the prefromed vesicles, the external pH can be increased to at least partially neutralize the surface charge. Unencapsulated and surface adsorbed nucleic acids can then be removed as described above.

Method of Use

The lipid particles of the invention may be used to deliver a therapeutic agent to a cell, *in vitro* or *in vivo.* In particular embodiments, the therapeutic agent is a nucleic acid, which is delivered to a cell using a nucleic acid-lipid particles of the invention. While the following description o various methodsof using the lipid particles and related pharmaceutical compositions of the invention are exemplified by description related to nucleic acid-lipid particles, it is understood that these methods and compositions may be readily adapted for the delivery of any therapeutic agent for the treatment of any disease or disorder that would benefit from such treatment.

In certain embodiments, the invention provides methods for introducing a nucleic acid into a cell. Preferred nucleic acids for introduction into cells are siRNA, immune-stimulating oligonucleotides, plasmids, antisense and ribozymes. These methods may be carried out by contacting the particles or compositions of the invention with the cells for a period of time sufficient for intracellular delivery to occur.

The compositions of the invention can be adsorbed to almost any cell type, e.g., tumor cell lines, including but not limited to HeLa, HCT116, A375, MCF7, B16F10, Hep3b, HUH7, HepG2, Skov3, U87, and PC3 cell lines. Once adsorbed, the nucleic acid-lipid particles can either be endocytosed by a portion of the cells, exchange lipids with cell membranes, or fuse with the cells. Transfer or incorporation of the nucleic acid portion of the complex can take place via any one of these pathways. Without intending to be limited with respect to the scope of the invention, it is believed that in the case of particles taken up into the cell by endocytosis the particles then interact with the endosomal membrane, resulting in destabilization of the endosomal membrane, possibly by the formation of non-bilayer phases, resulting in introduction of the encapsulated nucleic acid into the cell cytoplasm. Similarly in the case of direct fusion of the particles with the cell plasma membrane, when fusion takes place, the liposome membrane is integrated into the cell membrane and the contents of the liposome combine with the intracellular fluid. Contact between the cells and the lipid-nucleic acid compositions, when carried out *in vitro,* will take place in a biologically compatible medium. The concentration of compositions can vary widely depending on the particular application, but is generally between about 1 $\mu$mol and about 10 mmol. In certain embodiments, treatment of the cells with the lipid-nucleic acid compositions will generally be carried out at physiological temperatures (about 37°C) for periods of time from about 1 to 24 hours, preferably from about 2 to 8 hours. For *in vitro* applications, the delivery of nucleic acids can be to any cell grown in culture, whether of plant or animal origin, vertebrate or invertebrate, and of any tissue or type. In preferred embodiments, the cells will be animal cells, more preferably mammalian cells, and most preferably human cells.

In one group of embodiments, a lipid-nucleic acid particle suspension is added to 60-80% confluent plated cells having a cell density of from about $10^3$ to about $10^5$ cells/mL, more preferably about $2 \times 10^4$ cells/mL. The concentration of the suspension added to the cells is preferably of from about 0.01 to 20 $\mu$g/mL, more preferably about 1 $\mu$g/mL.

Typical applications include using well known procedures to provide intracellular delivery of siRNA to knock

down or silence specific cellular targets. Alternatively applications include delivery of DNA or mRNA sequences that code for therapeutically useful polypeptides. In this manner, therapy is provided for genetic diseases by supplying deficient or absent gene products (*i.e*., for Duchenne's dystrophy, see Kunkel, et al., Brit. Med. Bull. 45(3):630-643 (1989), and for cystic fibrosis, see Goodfellow, Nature 341:102-103 (1989)). Other uses for the compositions of the invention include introduction of antisense oligonucleotides in cells (see, Bennett, et al., Mol. Pharm. 41:1023-1033 (1992)).

Alternatively, the compositions of the invention can also be used for deliver of nucleic acids to cells *in vivo,* using methods which are known to those of skill in the art. With respect to application of the invention for delivery of DNA or mRNA sequences, Zhu, et al., Science 261:209-211 (1993), incorporated herein by reference, describes the intravenous delivery of cytomegalovirus (CMV)-chloramphenicol acetyltransferase (CAT) expression plasmid using DOTMA-DOPE complexes. Hyde, et al., Nature 362:250-256 (1993), incorporated herein by reference, describes the delivery of the cystic fibrosis transmembrane conductance regulator (CFTR) gene to epithelia of the airway and to alveoli in the lung of mice, using liposomes. Brigham, et al., Am. J. Med. Sci. 298:278-281 (1989), incorporated herein by reference, describes the *in vivo* transfection of lungs of mice with a functioning prokaryotic gene encoding the intracellular enzyme, chloramphenicol acetyltransferase (CAT). Thus, the compositions of the invention can be used in the treatment of infectious diseases.

Therefore, in another aspect, the formulations of the invention can be used to silence or modulate a target gene such as but not limited to FVII, Eg5, PCSK9, TPX2, apoB, SAA, TTR, RSV, PDGF beta gene, Erb-B gene, Src gene, CRK gene, GRB2 gene, RAS gene, MEKK gene, JNK gene, RAF gene, Erk1/2 gene, PCNA(p21) gene, MYB gene, JUN gene, FOS gene, BCL-2 gene, Cyclin D gene, VEGF gene, EGFR gene, Cyclin A gene, Cyclin E gene, WNT-1 gene, beta-catenin gene, c-MET gene, PKC gene, NFKB gene, STAT3 gene, survivin gene, Her2/Neu gene, topoisomerase I gene, topoisomerase II alpha gene, p73 gene, p21(WAF1/CIP1) gene, p27(KIP1) gene, PPM1D gene, RAS gene, caveolin I gene, MIB I gene, MTAI gene, M68 gene, tumor suppressor genes, p53 tumor suppressor gene, p53 family member DN-p63, pRb tumor suppressor gene, APC1 tumor suppressor gene, BRCA1 tumor suppressor gene, PTEN tumor suppressor gene, mLL fusion gene, BCR/ABL fusion gene, TEL/AML1 fusion gene, EWS/FLI1 fusion gene, TLS/FUS1 fusion gene, PAX3/FKHR fusion gene, AML1/ETO fusion gene, alpha v-integrin gene, Flt-1 receptor gene, tubulin gene, Human Papilloma Virus gene, a gene required for Human Papilloma Virus replication, Human Immunodeficiency Virus gene, a gene required for Human Immunodeficiency Virus replication, Hepatitis A Virus gene, a gene required for Hepatitis A Virus replication, Hepatitis B Virus gene, a gene required for Hepatitis B Virus replication, Hepatitis C Virus gene, a gene required for Hepatitis C Virus replication, Hepatitis D Virus gene, a gene required for Hepatitis D Virus replication, Hepatitis E Virus gene, a gene required for Hepatitis E Virus replication, Hepatitis F Virus gene, a gene required for Hepatitis F Virus replication, Hepatitis G Virus gene, a gene required for Hepatitis G Virus replication, Hepatitis H Virus gene, a gene required for Hepatitis H Virus replication, Respiratory Syncytial Virus gene, a gene that is required for Respiratory Syncytial Virus replication, Herpes Simplex Virus gene, a gene that is required for Herpes Simplex Virus replication, herpes Cytomegalovirus gene, a gene that is required for herpes Cytomegalovirus replication, herpes Epstein Barr Virus gene, a gene that is required for herpes Epstein Barr Virus replication, Kaposi's Sarcoma-associated Herpes Virus gene, a gene that is required for Kaposi's Sarcoma-associated Herpes Virus replication, JC Virus gene, human gene that is required for JC Virus replication, myxovirus gene, a gene that is required for myxovirus gene replication, rhinovirus gene, a gene that is required for rhinovirus replication, coronavirus gene, a gene that is required for coronavirus replication, West Nile Virus gene, a gene that is required for West Nile Virus replication, St. Louis Encephalitis gene, a gene that is required for St. Louis Encephalitis replication, Tick-borne encephalitis virus gene, a gene that is required for Tick-borne encephalitis virus replication, Murray Valley encephalitis virus gene, a gene that is required for Murray Valley encephalitis virus replication, dengue virus gene, a gene that is required for dengue virus gene replication, Simian Virus 40 gene, a gene that is required for Simian Virus 40 replication, Human T Cell Lymphotropic Virus gene, a gene that is required for Human T Cell Lymphotropic Virus replication, Moloney-Murine Leukemia Virus gene, a gene that is required for Moloney-Murine Leukemia Virus replication, encephalomyocarditis virus gene, a gene that is required for encephalomyocarditis virus replication, measles virus gene, a gene that is required for measles virus replication, Vericella zoster virus gene, a gene that is required for Vericella zoster virus replication, adenovirus gene, a gene that is required for adenovirus replication, yellow fever virus gene, a gene that is required for yellow fever virus replication, poliovirus gene, a gene that is required for poliovirus replication, poxvirus gene, a gene that is required for poxvirus replication, plasmodium gene, a gene that is required for plasmodium gene replication, Mycobacterium ulcerans gene, a gene that is required for Mycobacterium ulcerans replication, Mycobacterium tuberculosis gene, a gene that is required for Mycobacterium tuberculosis replication, Mycobacterium leprae gene, a gene that is required for Mycobacterium leprae replication, Staphylococcus aureus gene, a gene that is required for Staphylococcus aureus replication, Streptococcus pneumoniae gene, a gene that is required for Streptococcus pneumoniae replication, Streptococcus pyogenes gene, a gene that is required for Streptococcus pyogenes replication, Chlamydia pneumoniae gene, a gene that is required for Chlamydia pneumoniae replication, Mycoplasma pneumoniae gene, a gene that is required for Mycoplasma pneumoniae replication, an integrin gene, a selectin gene, complement system gene, chemokine gene,

chemokine receptor gene, GCSF gene, Gro1 gene, Gro2 gene, Gro3 gene, PF4 gene, MIG gene, Pro-Platelet Basic Protein gene, MIP-1I gene, MIP-1J gene, RANTES gene, MCP-1 gene, MCP-2 gene, MCP-3 gene, CMBKR1 gene, CMBKR2 gene, CMBKR3 gene, CMBKR5v, AIF-1 gene, I-309 gene, a gene to a component of an ion channel, a gene to a neurotransmitter receptor, a gene to a neurotransmitter ligand, amyloid-family gene, presenilin gene, HD gene, DRPLA gene, SCA1 gene, SCA2 gene, MJD1 gene, CACNL1A4 gene, SCA7 gene, SCA8 gene, allele gene found in LOH cells, or one allele gene of a polymorphic gene.

For *in vivo* administration, the pharmaceutical compositions are preferably administered parenterally, *i.e.,* intraarticularly, intravenously, intraperitoneally, subcutaneously, or intramuscularly. In particular embodiments, the pharmaceutical compositions are administered intravenously or intraperitoneally by a bolus injection. For one example, see Stadler, et al., U.S. Patent No. 5,286,634, which is incorporated herein by reference. Intracellular nucleic acid delivery has also been discussed in Straubringer, et al., METHODS IN ENZYMOLOGY, Academic Press, New York. 101:512-527 (1983); Mannino, et al., Biotechniques 6:682-690 (1988); Nicolau, et al., Crit. Rev. Ther. Drug Carrier Syst. 6:239-271 (1989), and Behr, Acc. Chem. Res. 26:274-278 (1993). Still other methods of administering lipid-based therapeutics are described in, for example, Rahman et al., U.S. Patent No. 3,993,754; Sears, U.S. Patent No. 4,145,410; Papahadjopoulos et al., U.S. Patent No. 4,235,871; Schneider, U.S. Patent No. 4,224,179; Lenk et al., U.S. Patent No. 4,522,803; and Fountain et al., U.S. Patent No. 4,588,578.

In other methods, the pharmaceutical preparations may be contacted with the target tissue by direct application of the preparation to the tissue. The application may be made by topical, "open" or "closed" procedures. By "topical," it is meant the direct application of the pharmaceutical preparation to a tissue exposed to the environment, such as the skin, oropharynx, external auditory canal, and the like. "Open" procedures are those procedures which include incising the skin of a patient and directly visualizing the underlying tissue to which the pharmaceutical preparations are applied. This is generally accomplished by a surgical procedure, such as a thoracotomy to access the lungs, abdominal laparotomy to access abdominal viscera, or other direct surgical approach to the target tissue. "Closed" procedures are invasive procedures in which the internal target tissues are not directly visualized, but accessed via inserting instruments through small wounds in the skin. For example, the preparations may be administered to the peritoneum by needle lavage. Likewise, the pharmaceutical preparations may be administered to the meninges or spinal cord by infusion during a lumbar puncture followed by appropriate positioning of the patient as commonly practiced for spinal anesthesia or metrazamide imaging of the spinal cord. Alternatively, the preparations may be administered through endoscopic devices.

The lipid-nucleic acid compositions can also be administered in an aerosol inhaled into the lungs (*see*, Brigham, et al., Am. J. Sci. 298(4):278-281 (1989)) or by direct injection at the site of disease (Culver, Human Gene Therapy, MaryAnn Liebert, Inc., Publishers, New York. pp.70-71 (1994)).

The methods of the invention may be practiced in a variety of hosts. Preferred hosts include mammalian species, such as humans, non-human primates, dogs, cats, cattle, horses, sheep, and the like.

Dosages for the lipid-therapeutic agent particles of the invention will depend on the ratio of therapeutic agent to lipid and the administrating physician's opinion based on age, weight, and condition of the patient.

In one embodiment, the invention provides a method of modulating the expression of a target polynucleotide or polypeptide. These methods generally comprise contacting a cell with a lipid particle of the invention that is associated with a nucleic acid capable of modulating the expression of a target polynucleotide or polypeptide. As used herein, the term "modulating" refers to altering the expression of a target polynucleotide or polypeptide. In different embodiments, modulating can mean increasing or enhancing, or it can mean decreasing or reducing. Methods of measuring the level of expression of a target polynucleotide or polypeptide are known and available in the arts and include, e.g., methods employing reverse transcription-polymerase chain reaction (RT-PCR) and immunohistochemical techniques. In particular embodiments, the level of expression of a target polynucleotide or polypeptide is increased or reduced by at least 10%, 20%, 30%, 40%, 50%, or greater than 50% as compared to an appropriate control value. For example, if increased expression of a polypeptide desired, the nucleic acid may be an expression vector that includes a polynucleotide that encodes the desired polypeptide. On the other hand, if reduced expression of a polynucleotide or polypeptide is desired, then the nucleic acid may be, *e.g*., an antisense oligonucleotide, siRNA, or microRNA that comprises a polynucleotide sequence that specifically hybridizes to a polnucleotide that encodes the target polypeptide, thereby disrupting expression of the target polynucleotide or polypeptide. Alternatively, the nucleic acid may be a plasmid that expresses such an antisense oligonucletoide, siRNA, or microRNA.

In one particular embodiment, the invention provides a method of modulating the expression of a polypeptide by a cell, comprising providing to a cell a lipid particle that consists of or consists essentially of a cationic lipid of formula A, a neutral lipid, a sterol, a PEG or PEG-modified lipid, *e.g*., in a molar ratio of about 35-65% of cationic lipid of formula A, 3-12% of the neutral lipid, 15-45% of the sterol, and 0.5-10% of the PEG or PEG-modified lipid, wherein the lipid particle is associated with a nucleic acid capable of modulating the expression of the polypeptide. In particular embodiments, the molar lipid ratio is approximately 60/7.5/31/1.5, 57.5/7.5/31.5/3.5 or 50/10/38.5/1.5 (mol% LIPID A/DSPC/Chol/PEG-DMG) or 57.1/7.1/34.4/1.4 (mol% LIPID A/DPPC/Chol/PEG-cDMA). In some embodiments, the lipide particle also includes a targeting moiety such as a targeting lipid described herein (e.g., the lipid particle consists essentially of a cationic lipid of formula A, a neutral lipid, a sterol, a PEG or PEG-modified lipid and a targeting moiety). In another group of embodiments, the neutral lipid in these compositions is replaced with DPPC, POPC, DOPE or SM.

In particular embodiments, the therapeutic agent is selected from an siRNA, a microRNA, an antisense oligonucleotide, and a plasmid capable of expressing an siRNA, a microRNA, or an antisense oligonucleotide, and wherein the siRNA, microRNA, or antisense RNA comprises a polynucleotide that specifically binds to a polynucleotide that encodes the polypeptide, or a complement thereof, such that the expression of the polypeptide is reduced.

In other embodiments, the nucleic acid is a plasmid that encodes the polypeptide or a functional variant or fragment thereof, such that expression of the polypeptide or the functional variant or fragment thereof is increased.

In related embodiments, the invention provides a method of treating a disease or disorder characterized by overexpression of a polypeptide in a subject, comprising providing to the subject a pharmaceutical composition of the invention, wherein the therapeutic agent is selected from an siRNA, a microRNA, an antisense oligonucleotide, and a plasmid capable of expressing an siRNA, a microRNA, or an antisense oligonucleotide, and wherein the siRNA, microRNA, or antisense RNA comprises a polynucleotide that specifically binds to a polynucleotide that encodes the polypeptide, or a complement thereof.

In one embodiment, the pharmaceutical composition comprises a lipid particle that consists of or consists essentially of a cationic lipid of formula A, DSPC, Chol and PEG-DMG, PEG-C-DOMG or PEG-cDMA, e.g., in a molar ratio of about 35-65% of cationic lipid of formula A, 3-12% of the neutral lipid, 15-45% of the sterol, and 0.5-10% of the PEG or PEG-modified lipid PEG-DMG, PEG-C-DOMG or PEG-CDMA, wherein the lipid particle is assocated with the therapeutic nucleic acid. In particular embodiments, the molar lipid ratio is approximately 60/7.5/31/1.5, 57.5/7.5/31.5/3.5 or 50/10/38.5/1.5 (mol% LIPID A/DSPC/Chol/PEG-DMG) or 57.1/7.1/34.4/1.4 (mol% LIPID A/DPPC/Chol/PEG-cDMA). In some embodiments, the lipid particle also includes a targeting lipid described herein (e.g., the lipid particle consists essentially of a cationic lipid of formula A, a neutral lipid, a sterol, a PEG or PEG-modified lipid and a targeting moiety). In some embodiments, when the targeting lipid includes a PEG moiety and is added to an existing liposomal formulation, the amount of PEG-modified lipid is reduced such that the total amount of PEG-moidfied lipid (i.e., PEG-modified lipid, for example PEG-DMG, and the PEG-containing targeting lipid) is kept at a constant mol percentage (e.g., 0.3 mol%, 1.4 mol%, 1.5 mol%, or 3.5 mol%). In another group of embodiments, the neutral lipid in these compositions is replaced with DPPC, POPC, DOPE or SM.

In another related embodiment, the invention includes a method of treating a disease or disorder characterized by underexpression of a polypeptide in a subject, comprising providing to the subject a pharmaceutical composition of the invention, wherein the therapeutic agent is a plasmid that encodes the polypeptide or a functional variant or fragment thereof.

The invention further provides a method of inducing an immune response in a subject, comprising providing to the subject the pharmaceutical composition of the invention, wherein the therapeutic agent is an immunostimulatory oligonucleotide. In certain embodiments, the immune response is a humoral or mucosal immune response consists of or consists essentially of a cationic lipid of formula A, DSPC, Chol and PEG-DMG, PEG-C-DOMG or PEG-cDMA, e.g., in a molar ratio of about 35-65% of cationic lipid of formula A, 3-12% of the neutral lipid, 15-45% of the sterol, and 0.5-10% of the PEG or PEG-modified lipid PEG-DMG, PEG-C-DOMG or PEG-cDMA, wherein the lipid particle is assocated with the therapeutic nucleic acid. In particular embodiments, the molar lipid ratio is approximately 60/7.5/31/1.5, 57.5/7.5/31.5/3.5 or 50/10/38.5/1.5 (mol% LIPID A/DSPC/Chol/PEG-DMG) or 57.1/7.1/34.4/1.4 (mol% LIPID A/DPPC/Chol/PEG-cDMA). In some embodiments, the lipid particle also includes a targeting lipid described herein (e.g., the lipid particle consists essentially of a cationic lipid of formula A, a neutral lipid, a sterol, a PEG or PEG-modified lipid and a targeting moiety). In some embodiments, when the targeting lipid includes a PEG moiety and is added to an existing liposomal formulation, the amount of PEG-modified lipid is reduced such that the total amount of PEG-moidfied lipid (i.e., PEG-modified lipid, for example PEG-DMG, and the PEG-containing targeting lipid) is kept at a constant mol percentage (e.g., 0.3 mol%, 1.4 mol%, 1.5 mol%, or 3.5 mol%). In another group of embodiments, the neutral lipid in these compositions is replaced with DPPC, POPC, DOPE or SM.

In further embodiments, the pharmaceutical composition is provided to the subject in combination with a vaccine or antigen. Thus, the invention itself provides vaccines comprising a lipid particle of the invention, which comprises an immunostimulatory oligonucleotide, and is also associated with an antigen to which an immune response is desired. In particular embodiments, the antigen is a tumor antigen or is associated with an infective agent, such as, *e.g.*, a virus, bacteria, or parasiste.

A variety of tumor antigens, infections agent antigens, and antigens associated with other disease are well known in the art and examples of these are described in references cited herein. Examples of antigens suitable for use in the invention include, but are not limited to, polypeptide antigens and DNA antigens. Specific examples of antigens are Hepatitis A, Hepatitis B, small pox, polio, anthrax, influenza, typhus, tetanus, measles, rotavirus, diphtheria, pertussis, tuberculosis, and rubella antigens. In one embodiment, the antigen is a Hepatitis B recombinant antigen. In other aspects, the antigen is a Hepatitis A recombinant antigen. In another aspect, the antigen is a tumor antigen. Examples of such tumor-associated antigens are MUC-1, EBV antigen and antigens associated with Burkitt's lymphoma. In a further aspect,

the antigen is a tyrosinase-related protein tumor antigen recombinant antigen. Those of skill in the art will know of other antigens suitable for use in the invention.

Tumor-associated antigens suitable for use in the subject invention include both mutated and non-mutated molecules that may be indicative of single tumor type, shared among several types of tumors, and/or exclusively expressed or overexpressed in tumor cells in comparison with normal cells. In addition to proteins and glycoproteins, tumor-specific patterns of expression of carbohydrates, gangliosides, glycolipids and mucins have also been documented. Exemplary tumor-associated antigens for use in the subject cancer vaccines include protein products of oncogenes, tumor suppressor genes and other genes with mutations or rearrangements unique to tumor cells, reactivated embryonic gene products, oncofetal antigens, tissue-specific (but not tumor-specific) differentiation antigens, growth factor receptors, cell surface carbohydrate residues, foreign viral proteins and a number of other self proteins.

Specific embodiments of tumor-associated antigens include, *e.g.*, mutated antigens such as the protein products of the Ras p21 protooncogenes, tumor suppressor p53 and BCR-abl oncogenes, as well as CDK4, MUM1, Caspase 8, and Beta catenin; overexpressed antigens such as galectin 4, galectin 9, carbonic anhydrase, Aldolase A, PRAME, Her2/neu, ErbB-2 and KSA, oncofetal antigens such as alpha fetoprotein (AFP), human chorionic gonadotropin (hCG); self antigens such as carcinoembryonic antigen (CEA) and melanocyte differentiation antigens such as Mart 1/Melan A, gp100, gp75, Tyrosinase, TRP1 and TRP2; prostate associated antigens such as PSA, PAP, PSMA, PSM-P1 and PSM-P2; reactivated embryonic gene products such as MAGE 1, MAGE 3, MAGE 4, GAGE 1, GAGE 2, BAGE, RAGE, and other cancer testis antigens such as NY-ESO1, SSX2 and SCP1; mucins such as Muc-1 and Muc-2; gangliosides such as GM2, GD2 and GD3, neutral glycolipids and glycoproteins such as Lewis (y) and globo-H; and glycoproteins such as Tn, Thompson-Freidenreich antigen (TF) and sTn. Also included as tumor-associated antigens herein are whole cell and tumor cell lysates as well as immunogenic portions thereof, as well as immunoglobulin idiotypes expressed on monoclonal proliferations of B lymphocytes for use against B cell lymphomas.

Pathogens include, but are not limited to, infectious agents, *e.g.*, viruses, that infect mammals, and more particularly humans. Examples of infectious virus include, but are not limited to: Retroviridae (*e.g.*, human immunodeficiency viruses, such as HIV-1 (also referred to as HTLV-III, LAV or HTLV-III/LAV, or HIV-III; and other isolates, such as HIV-LP; Picornaviridae (*e.g.,* polio viruses, hepatitis A virus; enteroviruses, human Coxsackie viruses, rhinoviruses, echoviruses); Calciviridae (*e.g.,* strains that cause gastroenteritis); Togaviridae (*e.g.,* equine encephalitis viruses, rubella viruses); Flaviridae (*e.g.,* dengue viruses, encephalitis viruses, yellow fever viruses); Coronoviridae (*e.g.,* coronaviruses); Rhabdoviradae (*e.g.,* vesicular stomatitis viruses, rabies viruses); Coronaviridae (*e.g.,* coronaviruses); Rhabdoviridae (*e.g.,* vesicular stomatitis viruses, rabies viruses); Filoviridae (*e.g.,* ebola viruses); Paramyxoviridae (*e.g.,* parainfluenza viruses, mumps virus, measles virus, respiratory syncytial virus); Oithomyxoviridae (*e.g.*,influenza viruses); Bungaviridae (*e.g.,* Hantaan viruses, bunga viruses, phleboviruses and Nairo viruses); Arena viridae (hemorrhagic fever viruses); Reoviridae (*e.g.,* reoviruses, orbiviurses and rotaviruses); Birnaviridae; Hepadnaviridae (Hepatitis B virus); Parvovirida (parvoviruses); Papovaviridae (papilloma viruses, polyoma viruses); Adenoviridae (most adenoviruses); Herpesviridae herpes simplex virus (HSV) 1 and 2, varicella zoster virus, cytomegalovirus (CMV), herpes virus; Poxviridae (variola viruses, vaccinia viruses, pox viruses); and Iridoviridae (*e.g.,* African swine fever virus); and unclassified viruses (*e.g.,* the etiological agents of Spongiform encephalopathies, the agent of delta hepatitis (thought to be a defective satellite of hepatitis B virus), the agents of non-A, non-B hepatitis (class 1=internally transmitted; class 2=parenterally transmitted (*i.e.,* Hepatitis C); Norwalk and related viruses, and astroviruses).

Also, gram negative and gram positive bacteria serve as antigens in vertebrate animals. Such gram positive bacteria include, but are not limited to Pasteurella species, Staphylococci species, and Streptococcus species. Gram negative bacteria include, but are not limited to, Escherichia coli, Pseudomonas species, and Salmonella species. Specific examples of infectious bacteria include but are not limited to: Helicobacterpyloris, Borelia burgdorferi, Legionella pneumophilia, Mycobacteria sps (*e.g.,* M. tuberculosis, M. avium, M. intracellulare, M. kansaii, M. gordonae), Staphylococcus aureus, Neisseria gonorrhoeae, Neisseria meningitidis, Listeria monocytogenes, Streptococcus pyogenes (Group A Streptococcus), Streptococcus agalactiae (Group B Streptococcus), Streptococcus (viridans group), Streptococcusfaecalis, Streptococcus bovis, Streptococcus (anaerobic sps.), Streptococcus pneumoniae, pathogenic Campylobacter sp., Enterococcus sp., Haemophilus infuenzae, Bacillus antracis, corynebacterium diphtheriae, corynebacterium sp., Erysipelothrix rhusiopathiae, Clostridium perfringers, Clostridium tetani, Enterobacter aerogenes, Klebsiella pneumoniae, Pasturella multocida, Bacteroides sp., Fusobacterium nucleatum, Streptobacillus moniliformis, Treponema pallidium, Treponema pertenue, Leptospira, Rickettsia, and Actinomyces israelli.

Additional examples of pathogens include, but are not limited to, infectious fungi that infect mammals, and more particularly humans. Examples of infectious fingi include, but are not limited to: Cryptococcus neoformans, Histoplasma capsulatum, Coccidioides immitis, Blastomyces dermatitidis, Chlamydia trachomatis, Candida albicans. Examples of infectious parasites include Plasmodium such as Plasmodium falciparum, Plasmodium malariae, Plasmodium ovale, and Plasmodium vivax. Other infectious organisms (*i.e.*, protists) include Toxoplasma gondii.

Pharmaceutical compositions

In one embodiment, the invention provides pharmaceutical compositions comprising a nucleic acid agent identified by the liver screening model described herein. The composition includes the agent, e.g., a dsRNA, and a pharmaceutically acceptable carrier. The pharmaceutical composition is useful for treating a disease or disorder associated with the expression or activity of the gene. Such pharmaceutical compositions are formulated based on the mode of delivery. One example is compositions that are formulated for systemic administration via parenteral delivery.

Pharmaceutical compositions including the identified agent are administered in dosages sufficient to inhibit expression of the target gene, e.g., the Factor VII gene. In general, a suitable dose of dsRNA agent will be in the range of 0.01 to 5.0 milligrams per kilogram body weight of the recipient per day, generally in the range of 1 microgram to 1 mg per kilogram body weight per day. The pharmaceutical composition may be administered once daily, or the dsRNA may be administered as two, three, or more sub-doses at appropriate intervals throughout the day or even using continuous infusion or delivery through a controlled release formulation. In that case, the dsRNA contained in each sub-dose must be correspondingly smaller in order to achieve the total daily dosage. The dosage unit can also be compounded for delivery over several days, e.g., using a conventional sustained release formulation which provides sustained release of the dsRNA over a several day period. Sustained release formulations are well known in the art and are particularly useful for vaginal delivery of agents, such as could be used with the agents of the invention. In this embodiment, the dosage unit contains a corresponding multiple of the daily dose.

The skilled artisan will appreciate that certain factors may influence the dosage and timing required to effectively treat a subject, including but not limited to the severity of the disease or disorder, previous treatments, the general health and/or age of the subject, and other diseases present. Moreover, treatment of a subject with a therapeutically effective amount of a composition can include a single treatment or a series of treatments. Estimates of effective dosages and in vivo half-lives for the individual dsRNAs encompassed by the invention can be made using conventional methodologies or on the basis of in vivo testing using an appropriate animal model, as described elsewhere herein.

In particular embodiments, pharmaceutical compositions comprising the lipid-nucleic acid particles of the invention are prepared according to standard techniques and further comprise a pharmaceutically acceptable carrier. Generally, normal saline will be employed as the pharmaceutically acceptable carrier. Other suitable carriers include, e.g., water, buffered water, 0.9% saline, 0.3% glycine, and the like, including glycoproteins for enhanced stability, such as albumin, lipoprotein, globulin, *etc.* In compositions comprising saline or other salt containing carriers, the carrier is preferably added following lipid particle formation. Thus, after the lipid-nucleic acid compositions are formed, the compositions can be diluted into pharmaceutically acceptable carriers such as normal saline.

The resulting pharmaceutical preparations may be sterilized by conventional, well known sterilization techniques. The aqueous solutions can then be packaged for use or filtered under aseptic conditions and lyophilized, the lyophilized preparation being combined with a sterile aqueous solution prior to administration. The compositions may contain pharmaceutically acceptable auxiliary substances as required to approximate physiological conditions, such as pH adjusting and buffering agents, tonicity adjusting agents and the like, for example, sodium acetate, sodium lactate, sodium chloride, potassium chloride, calcium chloride, *etc.* Additionally, the lipidic suspension may include lipid-protective agents which protect lipids against free-radical and lipid-peroxidative damages on storage. Lipophilic free-radical quenchers, such as $\alpha$-tocopherol and water-soluble iron-specific chelators, such as ferrioxamine, are suitable.

The concentration of lipid particle or lipid-nucleic acid particle in the pharmaceutical formulations can vary widely, *i.e.,* from less than about 0.01%, usually at or at least about 0.05-5% to as much as 10 to 30% by weight and will be selected primarily by fluid volumes, viscosities, *etc.*, in accordance with the particular mode of administration selected. For example, the concentration may be increased to lower the fluid load associated with treatment. This may be particularly desirable in patients having atherosclerosis-associated congestive heart failure or severe hypertension. Alternatively, complexes composed of irritating lipids may be diluted to low concentrations to lessen inflammation at the site of administration. In one group of embodiments, the nucleic acid will have an attached label and will be used for diagnosis (by indicating the presence of complementary nucleic acid). In this instance, the amount of complexes administered will depend upon the particular label used, the disease state being diagnosed and the judgement of the clinician but will generally be between about 0.01 and about 50 mg per kilogram of body weight (e.g., of the nucleic acid agent), preferably between about 0.1 and about 5 mg/kg of body weight. In some embodiments a complex administered includes from about 0.004 and about 50 mg per kilogram of body weight of neucleic acid agent (e.g., from about 0.006 mg/kg to about 0.2 mg/kg).

As noted above, the lipid-therapeutic agent (*e.g.*, nucleic acid) particels of the invention may include polyethylene glycol (PEG)-modified phospholipids, PEG-ceramide, or ganglioside $G_{M1}$-modified lipids or other lipids effective to prevent or limit aggregation. Addition of such components does not merely prevent complex aggregation. Rather, it may also provide a means for increasing circulation lifetime and increasing the delivery of the lipid-nucleic acid composition to the target tissues.

The invention also provides lipid-therapeutic agent compositions in kit form. The kit will typically be comprised

of a container that is compartmentalized for holding the various elements of the kit. The kit will contain the particles or pharmaceutical compositions of the invention, preferably in dehydrated or concentrated form, with instructions for their rehydration or dilution and administration. In certain embodiments, the particles comprise the active agent, while in other embodiments, they do not.

The pharmaceutical compositions containing an agent identified by the liver screening model may be administered in a number of ways depending upon whether local or systemic treatment is desired and upon the area to be treated. Administration may be topical, pulmonary, e.g., by inhalation or insufflation of powders or aerosols, including by nebulizer; intratracheal, intranasal, epidermal and transdermal), oral or parenteral. Admininstration may also be designed to result in preferential localization to particular tissues through local delivery, e.g. by direct intraarticular injection into joints, by rectal administration for direct delivery to the gut and intestines, by intravaginal administration for delivery to the cervix and vagina, by intravitreal administration for delivery to the eye. Parenteral administration includes intravenous, intraarterial, intraarticular, subcutaneous, intraperitoneal or intramuscular injection or infusion; or intracranial, e.g., intrathecal or intraventricular, administration.

Pharmaceutical compositions and formulations for topical administration may include transdermal patches, ointments, lotions, creams, gels, drops, suppositories, sprays, liquids and powders. Conventional pharmaceutical carriers, aqueous, powder or oily bases, thickeners and the like may be necessary or desirable. Coated condoms, gloves and the like may also be useful. Preferred topical formulations include those in which the dsRNAs of the invention are in admixture with a topical delivery component, such as a lipid, liposome, fatty acid, fatty acid ester, steroid, chelating agent or surfactant. Preferred lipids and liposomes include neutral (e.g. dioleoylphosphatidyl DOPE ethanolamine, dimyristoylphosphatidyl choline DMPC, distearolyphosphatidyl choline) negative (e.g. dimyristoylphosphatidyl glycerol DMPG) and cationic (e.g. dioleoyltetramethylaminopropyl DOTAP and dioleoylphosphatidyl ethanolamine DOTMA). DsRNAs of the invention may be encapsulated within liposomes or may form complexes thereto, in particular to cationic liposomes. Alternatively, dsRNAs may be complexed to lipids, in particular to cationic lipids. Preferred fatty acids and esters include but are not limited arachidonic acid, oleic acid, eicosanoic acid, lauric acid, caprylic acid, capric acid, myristic acid, palmitic acid, stearic acid, linoleic acid, linolenic acid, dicaprate, tricaprate, monoolein, dilaurin, glyceryl 1-monocaprate, 1-dodecylazacycloheptan-2-one, an acylcarnitine, an acylcholine, or a $C_{1-10}$ alkyl ester (e.g. isopropylmyristate IPM), monoglyceride, diglyceride or pharmaceutically acceptable salt thereof. Topical formulations are described in detail in U.S. patent application Ser. No. 09/315,298 filed on May 20, 1999 which is incorporated herein by reference in its entirety.

Compositions and formulations for oral administration include powders or granules, microparticulates, nanoparticulates, suspensions or solutions in water or non-aqueous media, capsules, gel capsules, sachets, tablets or minitablets. Thickeners, flavoring agents, diluents, emulsifiers, dispersing aids or binders may be desirable. Preferred oral formulations are those in which dsRNAs of the invention are administered in conjunction with one or more penetration enhancers surfactants and chelators. Preferred surfactants include fatty acids and/or esters or salts thereof, bile acids and/or salts thereof. Preferred bile acids/salts include chenodeoxycholic acid (CDCA) and ursodeoxychenodeoxycholic acid (UDCA), cholic acid, dehydrocholic acid, deoxycholic acid, glucholic acid, glycholic acid, glycodeoxycholic acid, taurocholic acid, taurodeoxycholic acid, sodium tauro-24,25-dihydro-fusidate and sodium glycodihydrofusidate. Preferred fatty acids include arachidonic acid, undecanoic acid, oleic acid, lauric acid, caprylic acid, capric acid, myristic acid, palmitic acid, stearic acid, linoleic acid, linolenic acid, dicaprate, tricaprate, monoolein, dilaurin, glyceryl 1-monocaprate, 1-dodecylazacycloheptan-2-one, an acylcarnitine, an acylcholine, or a monoglyceride, a diglyceride or a pharmaceutically acceptable salt thereof (e.g. sodium). Also preferred are combinations of penetration enhancers, for example, fatty acids/salts in combination with bile acids/salts. A particularly preferred combination is the sodium salt of lauric acid, capric acid and UDCA. Further penetration enhancers include polyoxyethylene-9-lauryl ether, polyoxyethylene-20-cetyl ether. DsRNAs of the invention may be delivered orally, in granular form including sprayed dried particles, or complexed to form micro or nanoparticles. DsRNA complexing agents include poly-amino acids; polyimines; polyacrylates; polyalkylacrylates, polyoxethanes, polyalkylcyanoacrylates; cationized gelatins, albumins, starches, acrylates, polyethyleneglycols (PEG) and starches; polyalkylcyanoacrylates; DEAE-derivatized polyimines, pollulans, celluloses and starches. Particularly preferred complexing agents include chitosan, N-trimethylchitosan, poly-L-lysine, polyhistidine, polyornithine, polyspermines, protamine, polyvinylpyridine, polythiodiethylaminomethylethylene P(TDAE), polyaminostyrene (e.g. p-amino), poly(methylcyanoacrylate), poly(ethylcyanoacrylate), poly(butylcyanoacrylate), poly(isobutylcyanoacrylate), poly(isohexylcynaoacrylate), DEAE-methacrylate, DEAE-hexylacrylate, DEAE-acrylamide, DEAE-albumin and DEAE-dextran, polymethylacrylate, polyhexylacrylate, poly(D,L-lactic acid), poly(DL-lactic-co-glycolic acid (PLGA), alginate, and polyethyleneglycol (PEG). Oral formulations for dsRNAs and their preparation are described in detail in U.S. application. Ser. No. 08/886,829 (filed Jul. 1, 1997), Ser. No. 09/108,673 (filed Jul. 1, 1998), Ser. No. 09/256,515 (filed Feb. 23, 1999), Ser. No. 09/082,624 (filed May 21, 1998) and Ser. No. 09/315,298 (filed May 20, 1999), each of which is incorporated herein by reference in their entirety.

Compositions and formulations for parenteral, intrathecal or intraventricular administration may include sterile aqueous solutions which may also contain buffers, diluents and other suitable additives such as, but not limited to, penetration enhancers, carrier compounds and other pharmaceutically acceptable carriers or excipients.

Pharmaceutical compositions include, but are not limited to, solutions, emulsions, and liposome-containing formulations. These compositions may be generated from a variety of components that include, but are not limited to, preformed liquids, self-emulsifying solids and self-emulsifying semisolids.

The pharmaceutical formulations, which may conveniently be presented in unit dosage form, may be prepared according to conventional techniques well known in the pharmaceutical industry. Such techniques include the step of bringing into association the active ingredients with the pharmaceutical carrier(s) or excipient(s). In general, the formulations are prepared by uniformly and intimately bringing into association the active ingredients with liquid carriers or finely divided solid carriers or both, and then, if necessary, shaping the product.

The compositions may be formulated into any of many possible dosage forms such as, but not limited to, tablets, capsules, gel capsules, liquid syrups, soft gels, suppositories, and enemas. The compositions of the invention may also be formulated as suspensions in aqueous, non-aqueous or mixed media. Aqueous suspensions may further contain substances which increase the viscosity of the suspension including, for example, sodium carboxymethylcellulose, sorbitol and/or dextran. The suspension may also contain stabilizers.

In one embodiment of the invention the pharmaceutical compositions may be formulated and used as foams. Pharmaceutical foams include formulations such as, but not limited to, emulsions, microemulsions, creams, jellies and liposomes. While basically similar in nature these formulations vary in the components and the consistency of the final product. The preparation of such compositions and formulations is generally known to those skilled in the pharmaceutical and formulation arts and may be applied to the formulation of the compositions of the invention

This invention is not limited in its application to the details of construction and the arrangement of components set forth in the following description. The invention is capable of other embodiments and of being practiced or of being carried out in various ways. Also, the phraseology and terminology used herein is for the purpose of description and should not be regarded as limiting. The use of "including," "comprising," or "having," "containing", "involving", and variations thereof herein, is meant to encompass the items listed thereafter and equivalents thereof as well as additional items.

EXAMPLES

The following examples are offered to illustrate, but not to limit the claimed invention.

As used in the Examples provided herein, the term "ApoE" refers to ApoE3 unless otherwise identified.

**Example 1:** Synthesis for precursors of 2,2-Dilinoleyl-4-dimethylaminoethyl-[1,3]-dioxolane.

**Step 1a: Synthesis of methanesulfonic acid octadeca-9, 12-dienyl ester 2**

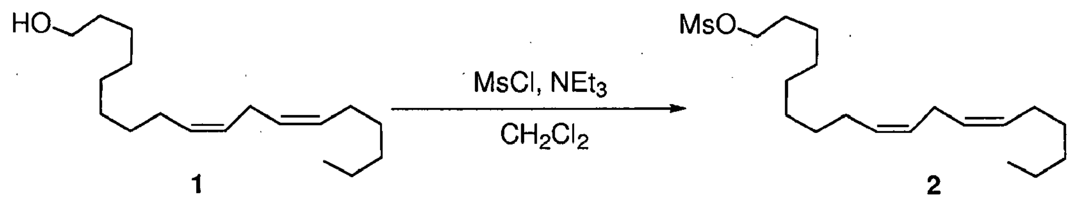


1 2

To a solution of the alcohol **1** (26.6 g, 100 mmol) in dichloromethane (100 mL), triethylamine (13.13 g, 130 mmol) was added and this solution was cooled in ice-bath. To this cold solution, a solution of mesyl chloride (12.6 g, 110 mmol) in dichloromethane (60 mL) was added dropwise and after the completion of the addition, the reaction mixture was allowed to warm to ambient temperature and stirred overnight. The TLC of the reaction mixture showed the completion of the reaction.

The reaction mixture was diluted with dichloromethane (200 mL), washed with water (200 mL), satd. $NaHCO_3$ (200 mL), brine (100 mL) and dried ($NaSO_4$). The organic layer was concentrated to get the crude product which was purified by column chromatography (silica gel) using 0-10% $Et_2O$ in hexanes. The pure product fractions were combined and concentrated to obtain the pure product **2** as colorless oil (30.6 g, 89%). $^1H$ NMR ($CDCl_3$, 400 MHz) $\delta$ = 5.42-5.21 (m, 4H), 4.20 (t, 2H), 3.06 (s, 3H), 2.79 (t, 2H), 2.19-2.00 (m, 4H), 1.90-1.70 (m, 2H), 1.06-1.18 (m, 18H), 0.88 (t, 3H). $^{13}C$ NMR ($CDCl_3$) $\delta$ = 130.76, 130.54, 128.6, 128.4, 70.67, 37.9, 32.05, 30.12, 29.87, 29.85, 29.68, 29.65, 29.53, 27.72, 27.71, 26.15, 25.94, 23.09, 14.60. MS. Molecular weight calculated for $C_{19}H_{36}O_3S$, Cal. 344.53, Found 343.52 (M-H$^-$).

**Step 1b: Synthesis of 18-Bromo-octadeca-6, 9-diene 3**

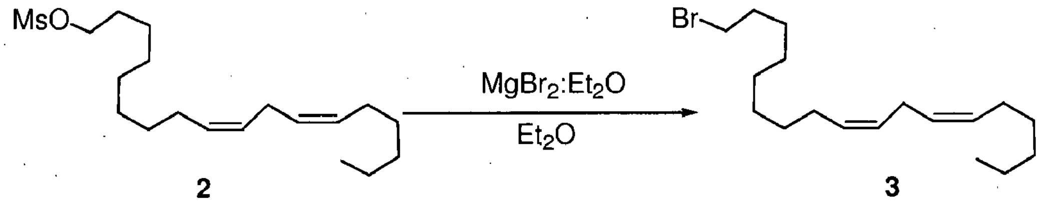


The mesylate (13.44 g, 39 mmol) was dissolved in anhydrous ether (500 mL) and to it the $MgBr.Et_2O$ complex (30.7 g, 118 mmol) was added under argon and the mixture was refluxed under argon for 26 h after which the TLC showed the completion of the reaction. The reaction mixture was diluted with ether (200 mL) and ice-cold water (200 mL) was added to this mixture and the layers were separated. The organic layer was washed with 1% aqueous $K_2CO_3$ (100 mL), brine (100 mL) and dried (Anhyd. $Na_2SO_4$). Concentration of the organic layer provided the crude product which was further purified by column chromatography (silica gel) using 0-1% $Et_2O$ in hexanes to isolate the bromide 3 (12.6 g, 94 %) as a colorless oil. $^1$H NMR ($CDCl_3$, 400 MHz) $\delta$ = 5.41-5.29 (m, 4H), 4.20 (d, 2H), 3.40 (t, *J* = 7 Hz, 2H), 2.77 (t, *J* = 6.6 Hz, 2H), 2.09-2.02 (m, 4H), 1.88-1.00 (m, 2H), 1.46-1.27 (m, 18H), 0.88 (t, *J* = 3.9 Hz, 3H). $^{13}$C NMR ($CDCl_3$) $\delta$ = 130.41, 130.25, 128.26, 128.12, 34.17, 33.05, 31.75, 29.82, 29.57, 29.54, 29.39, 28.95, 28.38, 27.42, 27.40, 25.84, 22.79, 14.28.

**Step 1c: Synthesis of 18-Cyano-octadeca-6,9-diene 4**

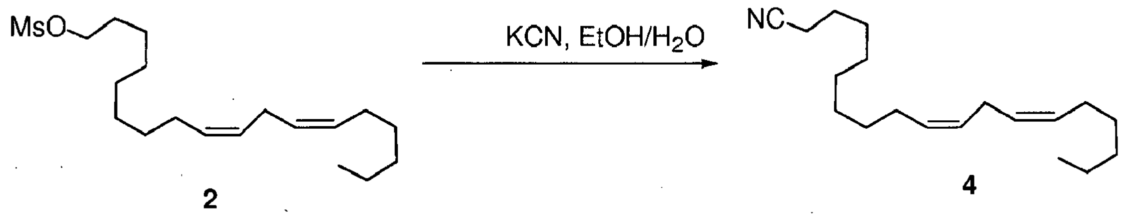


To a solution of the mesylate (3.44 g, 10 mmol) in ethanol (90 mL), a solution of KCN (1.32 g, 20 mmol) in water (10 mL) was added and the mixture was refluxed for 30 min. after which, the TLC of the reaction mixture showed the completion of the reaction after which, ether (200 mL) was added to the reaction mixture followed by the addition of water. The reaction mixture was extracted with ether and the combined organic layers was washed with water (100 mL), brine (200 mL) and dried. Concentration of the organic layer provided the crude which was purified by column chromatography (0-10 % $Et_2O$ in hexanes). The pure product **4** was isolated as colorless oil (2 g, 74%). $^1$H NMR ($CDCl_3$, 400 MHz) $\delta$ = 5.33-5.22 (m, 4H), 2.70 (t, 2H), 2.27-2.23 (m, 2H), 2.00-1.95 (m, 4H), 1.61-1.54 (m, 2H), 1.39-1.20 (m, 18H), 0.82 (t, 3H). $^{13}$C NMR ($CDCl_3$) $\delta$ = 130.20, 129.96, 128.08, 127.87, 119.78, 70.76, 66.02, 32.52, 29.82, 29.57, 29.33, 29.24, 29.19, 29.12, 28.73, 28.65, 27.20, 27.16, 25.62, 25.37, 22.56, 17.10, 14.06. MS. Molecular weight calculated for $C_{19}H_{33}N$, Cal. 275.47, Found 276.6 ($MH^-$).

**Step 1d: Synthesis of Heptatriaconta-6,9,28,31-tetraen-19-one 7**

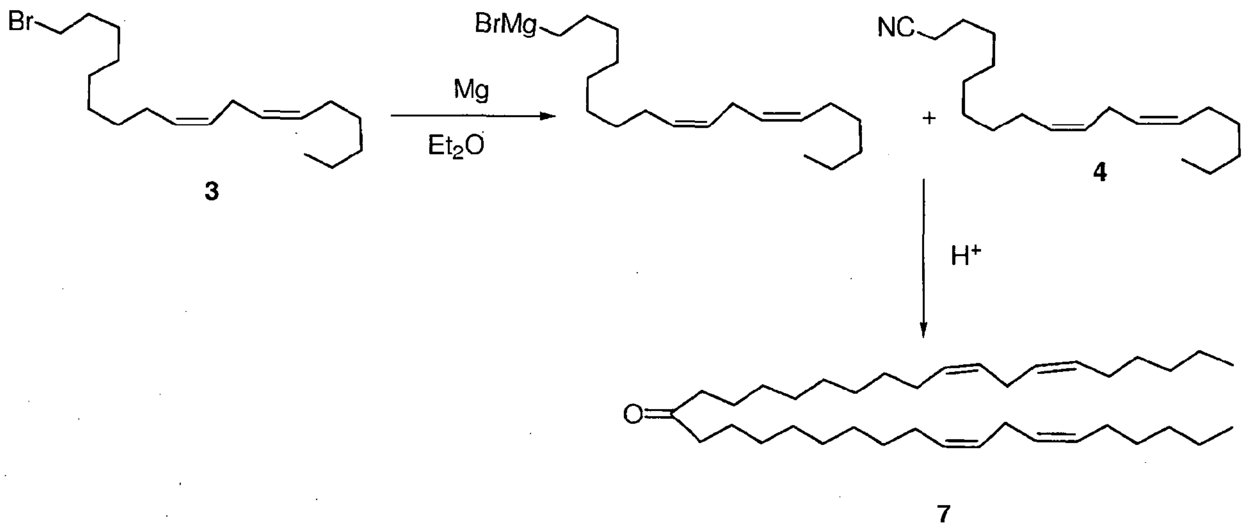


3

4

7

To a flame dried 500 mL 2NRB flask, a freshly activated Mg turnings (0.144 g, 6 mmol) was added and the flask was equipped with a magnetic stir bar and a reflux condenser. This set-up was degassed and flushed with argon and 10 mL of anhydrous ether was added to the flask via syringe. The bromide **3** (26.5 g, 80.47 mmol) was dissolved in anhydrous ether (10 mL) and added dropwise via syringe to the flask. An exothermic reaction was noticed (to confirm/accelerate the Grignard reagent formation, 2 mg of iodine was added and immediate decolorization was observed confirming the formation of the Grignard reagent) and the ether started refluxing. After the completion of the addition the reaction mixture was kept at 35 °C for 1 h and then cooled in ice bath. The cyanide **4** (1.38 g, 5 mmol) was dissolved in anhydrous ether (20 mL) and added dropwise to the reaction mixture with stirring. An exothermic reaction was observed and the reaction mixture was stirred overnight at ambient temperature. The reaction was quenched by adding 10 mL of acetone dropwise followed by ice cold water (60 mL). The reaction mixture was treated with aq. $H_2SO_4$ (10 % by volume, 200 mL) until the solution becomes homogeneous and the layers were separated. The aq. phase was extracted with ether (2x100 mL). The combined ether layers were dried ($Na_2SO_4$) and concentrated to get the crude product which was purified by column (silica gel, 0-10% ether in hexanes) chromatography.

The pure product fractions were evaporated to provide the pure ketone 7 as a colorless oil (2 g, 74%).

In another route, the ketone 7 was synthesized using a two step procedure via the alcohol 6 as follows.

**Step 1a(i): Synthesis of Heptatriaconta-6,9,28,31-tetraen-19-ol 7**

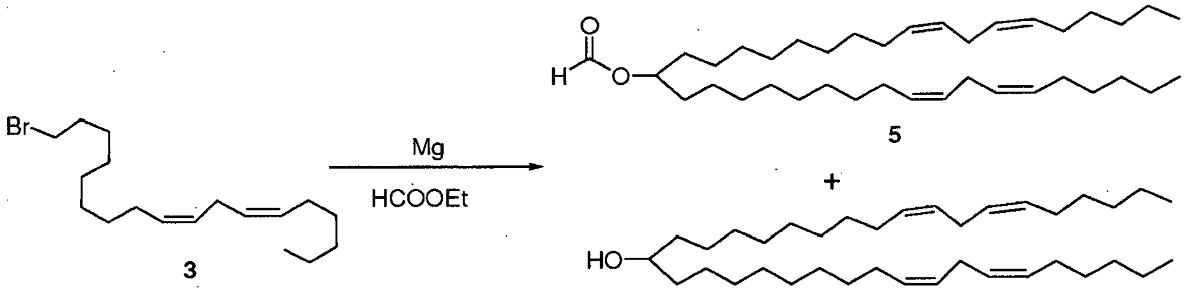


5

3

6

To a flame dried 500 mL RB flask, a freshly activated Mg turnings (2.4 g, 100 mmol) was added and the flask was equipped with a magnetic stir bar, an addition funnel and a reflux condenser. This set-up was degassed and flushed with argon and 10 mL of anhydrous ether was added to the flask via syringe. The bromide **3** (26.5 g, 80.47 mmol) was dissolved in anhydrous ether (50 mL) and added to the addition funnel. About 5 mL of this ether solution was added to the Mg turnings while stirring vigorously. An exothermic reaction was noticed (to confirm/accelerate the Grignard reagent formation, 5 mg of iodine was added and immediate decolorization was observed confirming the formation of the Grignard

reagent) and the ether started refluxing. The rest of the solution of the bromide was added dropwise while keeping the reaction under gentle reflux by cooling the flask in water. After the completion of the addition the reaction mixture was kept at 35 °C for 1 h and then cooled in ice bath. Ethyl formate (2.68 g, 36.2 mmol) was dissolved in anhydrous ether (40 mL) and transferred to the addition funnel and added dropwise to the reaction mixture with stirring. An exothermic reaction was observed and the reaction mixture started refluxing. After the initiation of the reaction the rest of the ethereal solution of formate was quickly added as a stream and the reaction mixture was stirred for a further period of 1 h at ambient temperature. The reaction was quenched by adding 10 mL of acetone dropwise followed by ice cold water (60 mL). The reaction mixture was treated with aq. $H_2SO_4$ (10 % by volume, 300 mL) until the solution becomes homogeneous and the layers were separated. The aq. phase was extracted with ether (2x100 mL). The combined ether layers were dried ($Na_2SO_4$) and concentrated to get the crude product which was purified by column (silica gel, 0-10% ether in hexanes) chromatography. The slightly less polar fractions were concentrated to get the formate **5** (1.9 g) and the pure product fractions were evaporated to provide the pure product **6** as a colorless oil (14.6 g, 78%).

**Step 1a(ii): Synthesis of Heptatriaconta-6,9,28,31-tetraen-19-one 7**

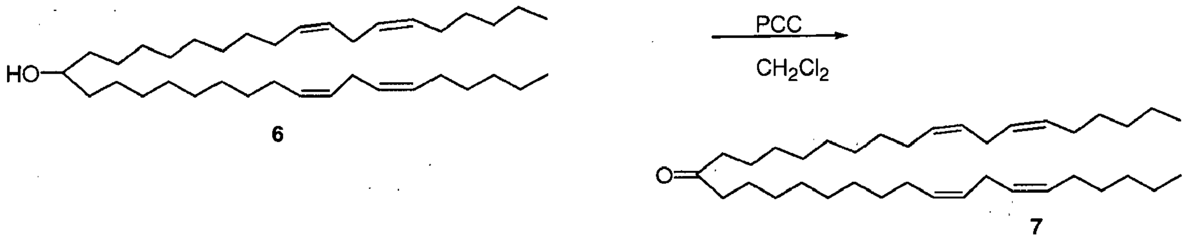

6
7

To a solution of the alcohol **6** (3 g, 5.68 mmol) in $CH_2Cl_2$ (60 mL), a freshly activated 4 A molecular sieves (50 g) was added and to this solution a powdered PCC (4.9 g, 22.7 mmol) was added portionwise over a period of 20 minutes and the mixture was further stirred for 1 hour (**Note:** careful monitoring of the reaction is necessary in order to get good yields since prolonged reaction times leads to lower yields) and the TLC of the reaction mixture was followed every 10 minutes (5% ether in hexanes) and after the completion of the reaction, the reaction mixture was filtered through a pad of silica gel and the residue was washed with $CH_2Cl_2$ (400 mL) and the filtrate was concentrated and the thus obtained crude product was further purified by column chromatography (silica gel, 1% $Et_2O$ in hexanes) to isolate the pure product **7** (2.9 g, 97%) as a colorless oil.

<u>**Example 2**: Process 1 for preparing 2,2-Dilinoleyl-4-dimethylaminoethyl-[1,3]-dioxolane (5a)</u>

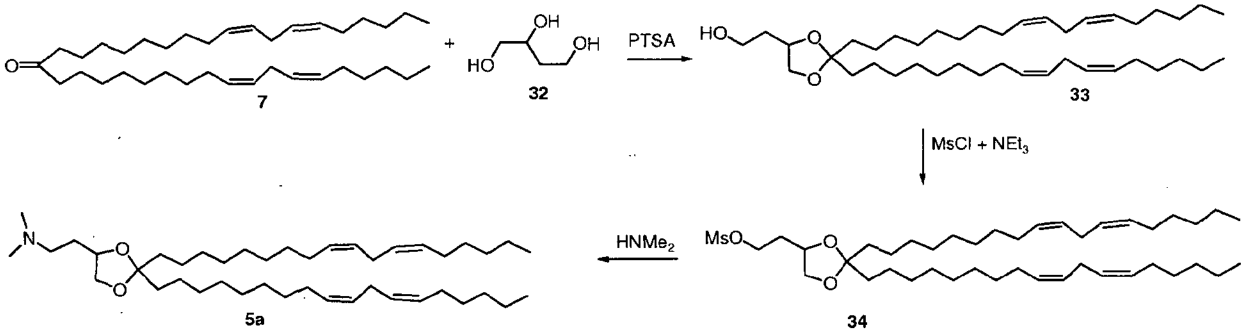

7 32 33
5a 34

**Step 2a: Preparation of Compound 33**

A mixture of compound **32** (10.6 g, 100 mmol), compound **7** (10.54 g, 20 mmol) and PTSA (0.1 eq) was heated under toluene reflux with Soxhlet extractor containing activated 4Å molecular sieves for 3 h. Removal of solvent then column purification (silica gel, 0-30% EtOAc in hexanes) gave compound **33** (11 g, 90 %) as a colorless oil. $^1H$ NMR (400 MHz, $CDCl_3$) $\delta$ 5.45 - 5.24 (m, 8H), 4.30 - 4.17 (m, 1H), 4.08 (dd, $J$ = 7.8, 6.1, 1H), 3.80 (dd, $J$ = 10.6, 5.0, 3H), 3.53 (t, $J$ = 8.0, 1H), 2.77 (t, $J$ = 6.4, 5H), 2.29 - 2.18 (m, 1H), 2.05 (q, $J$ = 6.7, 9H), 1.86 - 1.74 (m, 2H), 1.59 (dd, $J$ =

18.3, 9.7, 5H), 1.42 - 1.18 (m, 43H), 0.89 (t, *J* = 6.8, 6H). $^{13}C$ NMR (101 MHz, $CDCl_3$) δ 130.39, 130.36, 130.35, 128.14, 112.80, 77.54, 77.22, 76.90, 75.74, 70.14, 61.08, 37.97, 37.50, 35.56, 31.74, 30.14, 30.13, 29.88, 29.80, 29.73, 29.57, 29.53, 27.45, 27.41, 25.84, 24.20, 24.00, 22.79, 14.30.

**Step 2b: Preparation of Compound 34**

To an ice-cold solution of compound **33** (10.5 g, 17 mmol) and $NEt_3$ (5 mL) in DCM (100 mL) a solution of MsCl (2.96 g, 20.5 mmol) in DCM (20 mL) was added dropwise with stirring. After 1 h at r.t., aqueous workup gave a pale yellow oil of **34** which was column purified (silica gel, 0-30% EtOAc in hexanes) to provide the pure mesylate (11.1 g, 94%) as a colorless oil. $^{1}H$ NMR (400 MHz, $CDCl_3$) δ 5.44 - 5.26 (m, 8H), 4.37 (m, 2H), 4.26 - 4.13 (m, 1H), 4.10 (m, 1H), 3.53 (m, 1H), 3.02 (s, 3H), 2.76 (d, *J* = 6.4, 4H), 2.05 (d, *J* = 6.9, 10H), 1.55 (s, 4H), 1.29 (d, *J* = 9.8, 34H), 0.88 (t, *J* = 6.9, 6H). Electrospray MS (+ve): Molecular weight for C42H76O5S $(M + H)^+$ Calc. 693.5, Found 693.4.

**Step 2c: Preparation of Compound 5a (2,2-Dilinoleyl-4-dimethylaminoethyl-[1,3]-dioxolane)**

The mesylate **34** (11 g, 15.9 mmol) was dissolved in 400 mL of 2M dimethylamine in THF and the solution was transferred to a Parr pressure reactor and the contents were stirred at 70 °C for 14 h. The reaction mixture was cooled and the TLC of the reaction mixture showed the completion of the reaction. The reaction mixture was concentrated in a rotary evaporator and the thus obtained crude product was purified by column chromatography (silica gel, 0-10% MeOH in dichloromethane) to yield the pure product **5a** (9.4 g, 92%) as a colorless oil. $^{1}H$ NMR (400 MHz, $CDCl_3$) δ 5.45 - 5.24 (m, 8H), 4.07 (dt, *J* = 17.3, 6.4, 2H), 3.48 (t, *J* = 7.3, 1H), 2.77 (t, *J* = 6.4, 4H), 2.47 - 2.25 (m, 2H), 2.24 (d, *J* = 10.5, 6H), 2.04 (q, *J* = 6.6, 8H), 1.73 (ddd, *J* = 22.8, 14.5, 7.9, 2H), 1.59 (dt, *J* = 20.0, 9.9, 4H), 1.43 - 1.18 (m, 34H), 0.89 (t, *J* = 6.8, 6H). $^{13}C$ NMR ($CDCl_3$, 100 MHz) δ = 130.2, 130.1, 128.0, 112.1, 74.8, 70.0, 56.3, 45.5, 37.8, 37.5, 31.8, 31.5, 30.0, 30.0, 29.7, 29.6, 29.6, 29.5, 29.5, 29.3, 29.3, 27.2, 27.2, 25.6, 24.0, 23.7, 22.6, 14.0: Electrospray MS (+ve): Molecular weight for $C_{43}H_{79}NO_2$ $(M + H)^+$ Calc. 642.6, Found 642.6.

__**Example 3:** Process 2 for making 2,2-Dilinoleyl-4-dimethylaminoethyl-[1,3]-dioxolane (5a).__

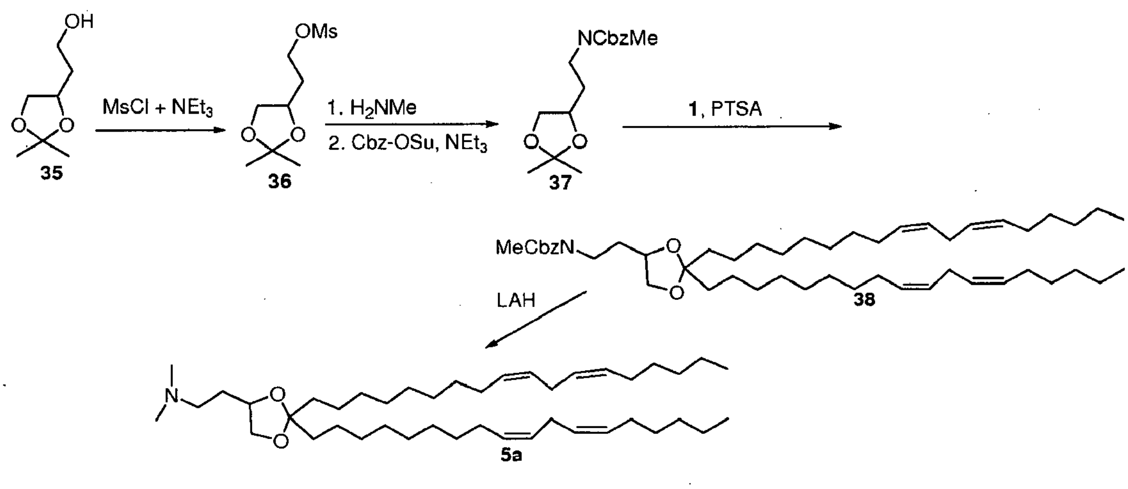


**Step 3a: Preparation of Compound 36**

MsCl (1.1 eq) was added to an ice-cold stirring solution of compound **11** (5 g, 34.2 mmol) and $NEt_3$ (1.2 eq) in DCM (10 mL). After 1 h at r.t., aqueous workup gave a pale yellow oil of **36** (7.7 g, quantitative) which was used without further purification. $^{13}C$ NMR ($CDCl_3$, 100 MHz) δ = 109.2, 72.3, 72.1, 69.1, 67.0, 37.3, 33.4, 26.9, 25.5: Electrospray MS (+ve): Molecular weight for C8H16O5S $(M + H)^+$ Calc. 225.1, Found 225.0.

**Step 3b: Preparation of Compound 37**

Compound **36** (3.9 g, 17.4 mmol) was stirred with ethanolic methylamine (33 %, 100 mL) over 72 h. Removal of solvent gave a residue which was treated with Cbz-OSu (1.2 eq) and $NEt_3$ (3 eq) for 18 h. Aqueous workup then

column chromatography gave compound **37** (5.2 g, 98 %).

Electrospray MS (+ve): Molecular weight for C16H23NO4 $(M + H)^+$ Calc. 294.2, Found 294.0.

**Step 3c: Preparation of Compound 38**

A solution of **7** (1 eq), compound **37** (1 eq), and and *p*-TSA (0.1 eq) is heated under toluene reflux with Dean-Stark apparatus for 18 h. Removal of solvent then column chromatography gives compound **38** as a colorless oil.

**Step 3d: Preparation of 2,2-Dilinoleyl-4-dimethylaminoethyl-[1,3]-dioxolane (Compound 5a)**

An ice-cooled solution of 1 M LAH (2 eq) in THF is treated dropwise over 0.5 h with a solution of compound **38** (1 eq) in hexane. After addition, the solution is warmed to 40°C for 0.5. The mixture is ice-cooled then hydrolyzed with saturated aqueous $Na_2SO_4$. Celite is added (5 g) and the resulting mixture is filtered. The filtrate is reduced. Column chromatography affords compound **5a** as colorless oil.

<u>**Example 4:** Oigonucleotide Synthesis.</u>

<u>**Synthesis**</u>

All oligonucleotides are synthesized on an AKTAoligopilot synthesizer. Commercially available controlled pore glass solid support (dT-CPG, 500Å, Prime Synthesis) and RNA phosphoramidites with standard protecting groups, 5'-*O*-dimethoxytrityl N6-benzoyl-2'-*t*-butyldimethylsilyl-adenosine-3'-*O*-N,N'-diisopropyl-2-cyanoethylphosphoramidite, 5'-O-dimethoxytrityl-N4-acetyl-2'-t-butyldimethylsilyl-cytidine-3'-*O*-N,N'-diisopropyl-2-cyanoethylphosphoramidite, 5'-*O*-dimethoxytrityl-N2--isobutryl-2'-*t*-butyldimethylsilyl-guanosine-3'-*O*-N,N'-diisopropyl-2-cyanoethylphosphoramidite, and 5'-*O*-dimethoxytrityl-2'-*t*-butyldimethylsilyl-uridine-3'-O-N,N'-diisopropyl-2-cyanoethylphosphoramidite (Pierce Nucleic Acids Technologies) were used for the oligonucleotide synthesis. The 2'-F phosphoramidites, 5'-*O*-dimethoxytrityl-N4-acetyl-2'-fluro-cytidine-3'-*O*-N,N'-diisopropyl-2-cyanoethyl-phosphoramidite and 5'-*O*-dimethoxytrityl-2'-fluro-uridine-3'-O-N,N'-diisopropyl-2-cyanoethyl-phosphoramidite are purchased from (Promega). All phosphoramidites are used at a concentration of 0.2M in acetonitrile ($CH_3CN$) except for guanosine which is used at 0.2M concentration in 10% THF/ANC (v/v). Coupling/recycling time of 16 minutes is used. The activator is 5-ethyl thiotetrazole (0.75M, American International Chemicals); for the PO-oxidation iodine/water/pyridine is used and for the PS-oxidation PADS (2%) in 2,6-lutidine/ACN (1:1 v/v) is used.

3'-ligand conjugated strands are synthesized using solid support containing the corresponding ligand. For example, the introduction of cholesterol unit in the sequence is performed from a hydroxyprolinol-cholesterol phosphoramidite. Cholesterol is tethered to *trans*-4-hydroxyprolinol via a 6-aminohexanoate linkage to obtain a hydroxyprolinol-cholesterol moiety. 5'-end Cy-3 and Cy-5.5 (fluorophore) labeled siRNAs are synthesized from the corresponding Quasar-570 (Cy-3) phosphoramidite are purchased from Biosearch Technologies. Conjugation of ligands to 5'-end and or internal position is achieved by using appropriately protected ligand-phosphoramidite building block. An extended 15 min coupling of 0.1 M solution of phosphoramidite in anhydrous $CH_3CN$ in the presence of 5-(ethylthio)-1*H*-tetrazole activator to a solid-support-bound oligonucleotide. Oxidation of the internucleotide phosphite to the phosphate is carried out using standard iodine-water as reported (1) or by treatment with *tert*-butyl hydroperoxide/acetonitrile/water (10: 87: 3) with 10 min oxidation wait time conjugated oligonucleotide. Phosphorothioate is introduced by the oxidation of phosphite to phosphorothioate by using a sulfur transfer reagent such as DDTT (purchased from AM Chemicals), PADS and or Beaucage reagent. The cholesterol phosphoramidite is synthesized in house and used at a concentration of 0.1 M in dichloromethane. Coupling time for the cholesterol phosphoramidite is 16 minutes.

<u>**Deprotection I (Nucleobase Deprotection)**</u>

After completion of synthesis, the support is transferred to a 100 mL glass bottle (VWR). The oligonucleotide is cleaved from the support with simultaneous deprotection of base and phosphate groups with 80 mL of a mixture of ethanolic ammonia [ammonia: ethanol (3:1)] for 6.5 h at 55°C. The bottle is cooled briefly on ice and then the ethanolic ammonia mixture is filtered into a new 250-mL bottle. The CPG is washed with 2 x 40 mL portions of ethanol/water (1:1 v/v). The volume of the mixture is then reduced to ~ 30 mL by roto-vap. The mixture is then frozen on dry ice and dried under vacuum on a speed vac.

<u>**Deprotection II (Removal of 2'-TBDMS group)**</u>

The dried residue is resuspended in 26 mL of triethylamine, triethylamine trihydrofluoride (TEA•3HF) or pyridine-HF and DMSO (3:4:6) and heated at 60°C for 90 minutes to remove the *tert*-butyldimethylsilyl (TBDMS) groups at the 2' position. The reaction is then quenched with 50 mL of 20 mM sodium acetate and the pH is adjusted to 6.5. Oligonucleotide is stored in a freezer until purification.

**Analysis**

The oligonucleotides are analyzed by high-performance liquid chromatography (HPLC) prior to purification and selection of buffer and column depends on nature of the sequence and or conjugated ligand.

**HPLC Purification**

The ligand-conjugated oligonucleotides are purified by reverse-phase preparative HPLC. The unconjugated oligonucleotides are purified by anion-exchange HPLC on a TSK gel column packed in house. The buffers are 20 mM sodium phosphate (pH 8.5) in 10% $CH_3CN$ (buffer A) and 20 mM sodium phosphate (pH 8.5) in 10% $CH_3CN$, 1M NaBr (buffer B). Fractions containing full-length oligonucleotides are pooled, desalted, and lyophilized. Approximately 0.15 OD of desalted oligonucleotidess are diluted in water to 150 μL and then pipetted into special vials for CGE and LC/MS analysis. Compounds are then analyzed by LC-ESMS and CGE.

**siRNA preparation**

For the preparation of siRNA, equimolar amounts of sense and antisense strand ae heated in 1xPBS at 95°C for 5 min and slowly cooled to room temperature. Integrity of the duplex is confirmed by HPLC analysis.

**Table 3. siRNA duplexes for Luc and FVII targeting**

| Duplex | Seq. ID | Sequence 5'-3' | Target |
|---|---|---|---|
| 1000/1001 | 1 | CUU ACG CUG AGU ACU UCG AdTdT | Luc |
| | 2 | UCG AAG UAC UCA GCG UAA GdTdT | |
| AD-1955 | 3 | cuuAcGcuGAGuAcuucGAdTsdT | Luc |
| | 4 | UCGAAGuACUcAGCGuAAGdTsdT | |
| AD-1596 | 5 | GGAUCAUCUCAAGUCUUACdTdT | FVII |
| | 6 | GUAAGACUUGAGAUGAUCCdTdT | |
| AD-1661 | 7 | GGAfUfCAfUfCfUfCAAGfUfCfUfUAfCdTsdT | FVII |
| | 8 | GfUAAGAfCfUfUGAGAfUGAfUfCfCdTsdT | |

Lower case is 2'OMe modification and Nf is a 2'F modified nucleobase, dT is deoxythymidine, s is phosphothioate

**Example 5:** Synthesis of mPEG2000-1,2-Di-*O*-alkyl-*sn*3-carbomoylglyceride.

The PEG-lipids, such as mPEG2000-1,2-Di-*O*-alkyl-*sn*3-carbomoylglyceride were synthesized using the following procedures:

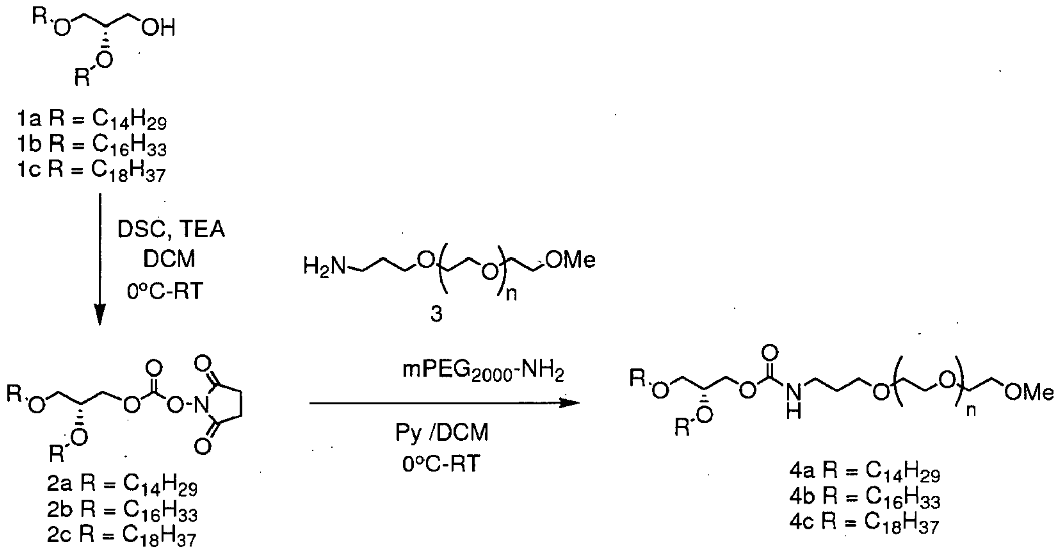


1a R = $C_{14}H_{29}$
1b R = $C_{16}H_{33}$
1c R = $C_{18}H_{37}$

3

2a R = $C_{14}H_{29}$
2b R = $C_{16}H_{33}$
2c R = $C_{18}H_{37}$

4a R = $C_{14}H_{29}$
4b R = $C_{16}H_{33}$
4c R = $C_{18}H_{37}$

mPEG2000-1,2-Di-*O*-alkyl-*sn*3-carbomoylglyceride

**Preparation of compound 4a (PEG-DMG):** 1,2-Di-*O*-tetradecyl-*sn*-glyceride **1a** (30 g, 61.80 mmol) and *N,N'*-succinimidylcarboante (DSC, 23.76 g, 1.5eq) were taken in dichloromethane (DCM, 500 mL) and stirred over an ice water mixture. Triethylamine (25.30 mL, 3eq) was added to stirring solution and subsequently the reaction mixture was allowed to stir overnight at ambient temperature. Progress of the reaction was monitored by TLC. The reaction mixture was diluted with DCM (400 mL) and the organic layer was washed with water (2X500 mL), aqueous $NaHCO_3$ solution (500 mL) followed by standard work-up. Residue obtained was dried at ambient temperature under high vacuum overnight. After drying the crude carbonate **2a** thus obtained was dissolved in dichloromethane (500 mL) and stirred over an ice bath. To the stirring solution n-tPEG$_{2000}$-NH$_2$ (**3**, 103.00 g, 47.20 mmol, purchased from NOF Corporation, Japan) and anhydrous pyridine (80 mL, excess) were added under argon. In some embodiments, the methoxy-(PEG)x-amine has an x= from 45-49, preferably 47-49, and more preferably 49. The reaction mixture was then allowed stir at ambient temperature overnight. Solvents and volatiles were removed under vacuum and the residue was dissolved in DCM (200 mL) and charged on a column of silica gel packed in ethyl acetate. The column was initially eluted with ethyl acetate and subsequently with gradient of 5-10 % methanol in dichloromethane to afford the desired PEG-Lipid **4a** as a white solid (105.30g, 83%). $^1$H NMR ($CDCl_3$, 400 MHz) $\delta$ = 5.20-5.12(m, 1H), 4.18-4.01(m, 2H), 3.80-3.70(m, 2H), 3.70-3.20(m, -O-$CH_2$-$CH_2$-O-, PEG-$CH_2$), 2.10-2.01(m, 2H), 1.70-1.60 (m, 2H), 1.56-1.45(m, 4H), 1.31-1.15(m, 48H), 0.84(t, J= 6.5Hz, 6H). MS range found: 2660-2836.

**Preparation of 4b:** 1,2-Di-*O*-hexadecyl-*sn*-glyceride **1b** (1.00 g, 1.848 mmol) and DSC (0.710 g, 1.5eq) were taken together in dichloromethane (20 mL) and cooled down to 0°C in an ice water mixture. Triethylamine (1.00 mL, 3eq) was added to that and stirred overnight. The reaction was followed by TLC, diluted with DCM, washed with water (2 times), $NaHCO_3$ solution and dried over sodium sulfate. Solvents were removed under reduced pressure and the residue **2b** under high vacuum overnight. This compound was directly used for the next reaction without further purification. MPEG$_{2000}$-NH$_2$ **3** (1.50g, 0.687 mmol, purchased from NOF Corporation, Japan) and compound from previous step **2b** (0.702g, 1.5eq) were dissolved in dichloromethane (20 mL) under argon. The reaction was cooled to 0°C. Pyridine (1 mL, excess) was added to that and stirred overnight. The reaction was monitored by TLC. Solvents and volatiles were removed under vacuum and the residue was purified by chromatography (first Ethyl acetate then 5-10% MeOH/DCM as a gradient elution) to get the required compound **4b** as white solid (1.46 g, 76 %). $^1$H NMR ($CDCl_3$, 400 MHz) $\delta$ = 5.17(t, J= 5.5Hz, 1H), 4.13(dd, J= 4.00Hz, 11.00 Hz, 1H), 4.05(dd, J= 5.00Hz, 11.00 Hz, 1H), 3.82-3.75(m, 2H), 3.70-3.20(m, -O-$CH_2$-$CH_2$-O-, PEG-$CH_2$), 2.05-1.90(m, 2H), 1.80-1.70 (m, 2H), 1.61-1.45(m, 6H), 1.35-1.17(m, 56H), 0.85(t, J= 6.5Hz, 6H). MS range found: 2716-2892.

**Preparation of 4c:** 1,2-Di-*O*-octadecyl-*sn*-glyceride **1c** (4.00 g, 6.70 mmol) and DSC (2.58 g, 1.5eq) were taken together in dichloromethane (60 mL) and cooled down to 0°C in an ice water mixture. Triethylamine (2.75 mL, 3eq) was added to that and stirred overnight. The reaction was followed by TLC, diluted with DCM, washed with water (2 times), $NaHCO_3$ solution and dried over sodium sulfate. Solvents were removed under reduced pressure and the residue under high vacuum overnight. This compound was directly used for the next reaction with further purification. MPEG$_{2000}$-NH$_2$ 3 (1.50g, 0.687 mmol, purchased from NOF Corporation, Japan) and compound from previous step **2c** (0.760g, 1.5eq) were dissolved in dichloromethane (20 mL) under argon. The reaction was cooled to 0°C. Pyridine (1

mL, excess) was added to that and stirred overnight. The reaction was monitored by TLC. Solvents and volatiles were removed under vacuum and the residue was purified by chromatography (first Ethyl acetate then 5-10% MeOH/DCM as a gradient elution) to get the required compound **4c** as white solid (0.92 g, 48 %). $^1$H NMR ($CDCl_3$, 400 MHz) $\delta$ = 5.22-5.15(m, 1H), 4.16(dd, J= 4.00Hz, 11.00 Hz, 1H), 4.06(dd, J= 5.00Hz, 11.00 Hz, 1H), 3.81-3.75(m, 2H), 3.70-3.20(m, $-O-CH_2-CH_2-O-$, PEG-$CH_2$), 1.80-1.70 (m, 2H), 1.60-1.48(m, 4H), 1.31-1.15(m, 64H), 0.85(t, J= 6.5Hz, 6H). MS range found: 2774-2948.

**Example 6**: *In vivo* rodent Factor VII silencing experiments.

C57BL/6 mice (Charles River Labs, MA) and Sprague-Dawley rats (Charles River Labs, MA) received either saline or formulated siRNA via tail vein injection at a volume of 0.01 mL/g. At various time points after administration, serum samples were collected by retroorbital bleed. Serum levels of Factor VII protein were determined in samples using a chromogenic assay (Biophen FVII, Aniara Corporation, OH). To determine liver mRNA levels of Factor VII, animals were sacrificed and livers were harvested and snap frozen in liquid nitrogen. Tissue lysates were prepared from the frozen tissues and liver mRNA levels of Factor VII were quantified using a branched DNA assay (QuantiGene Assay, Panomics, CA).

**Example 7:** Regulation of mammalian gene expression using nucleic acid-lipid particles.

Factor VII (FVII), a prominent protein in the coagulation cascade, is synthesized in the liver (hepatocytes) and secreted into the plasma. FVII levels in plasma can be determined by a simple, plate-based colorimetric assay. As such, FVII represents a convenient model for determining sirna-mediated downregulation of hepatocyte-derived proteins, as well as monitoring plasma concentrations and tissue distribution of the nucleic acid lipid particles and siRNA.

Factor VII Knockdown in Mice

FVII activity was evaluated in FVII siRNA-treated animals at 24 hours after intravenous (bolus) injection in C57BL/6 mice. FVII was measured using a commercially available kit for determining protein levels in serum or tissue, following the manufacturer's instructions at a microplate scale. FVII reduction was determined against untreated control mice, and the results were expressed as % Residual FVII. Four dose levels (2, 5, 12.5, 25 mg/kg FVII siRNA) were used in the initial screen of each novel liposome composition, and this dosing was expanded in subsequent studies based on the results obtained in the initial screen.

Determination of Tolerability

The tolerability of each novel liposomal siRNA formulation was evaluated by monitoring weight change, cageside observations, clinical chemistry and, in some instances, hematology. Animal weights were recorded prior to treatment and at 24 hours after treatment. Data was recorded as % Change in Body Weight. In addition to body weight measurements, a full clinical chemistry panel, including liver function markers, was obtained at each dose level (2, 5, 12.5 and 25 mg/kg siRNA) at 24 hours post-injection using an aliquot of the serum collected for FVII analysis. Samples were sent to the Central Laboratory for Veterinarians (Langley, BC) for analysis. In some instances, additional mice were included in the treatment group to allow collection of whole blood for hematology analysis.

Determination of Therapeutic Index

Therapeutic index (TI) is an arbitrary parameter generated by comparing measures of toxicity and activity. For these studies, TI was determined as:

$$TI = MTD \text{ (maximum tolerated dose)} / ED_{50} \text{ (dose for 50\% FVII knockdown)}$$

The MTD for these studies was set as the lowest dose causing >7% decrease in body weight and a >200-fold increase in alanine aminotransferase (ALT), a clinical chemistry marker with good specificity for liver damage in rodents. The $ED_{50}$ was determined from FVII dose-activity curves.

**Example 8:** General protocol for the extrusion method.

Lipids (Lipid A, DSPC, cholesterol, DMG-PEG) are solubilized and mixed in ethanol according to the desired molar ratio. Liposomes are formed by an ethanol injection method where mixed lipids are added to sodium acetate buffer at pH 5.2. This results in the spontaneous formation of liposomes in 35 % ethanol. The liposomes are extruded through a 0.08 μm polycarbonate membrane at least 2 times. A stock siRNA solution was prepared in sodium acetate and 35% ethanol and was added to the liposome to load. The siRNA-liposome solution was incubated at 37°C for 30 min and, subsequently, diluted. Ethanol was removed and exchanged to PBS buffer by dialysis or tangential flow filtration. A flow chart of this method is shown in FIG. 1.

**Example 9:** General protocol for the in-line mixing method.

Individual and separate stock solutions are prepared - one containing lipid and the other siRNA. Lipid stock containing lipid A, DSPC, cholesterol and PEG lipid is prepared by solubilized in 90% ethanol. The remaining 10% is low pH citrate buffer. The concentration of the lipid stock is 4 mg/mL. The pH of this citrate buffer can range between pH 3-5, depending on the type of fusogenic lipid employed. The siRNA is also solubilized in citrate buffer at a concentration of 4 mg/mL. For small scale, 5 mL of each stock solution is prepared.

Stock solutions are completely clear and lipids must be completely solubilized before combining with siRNA. Therefore stock solutions may be heated to completely solubilize the lipids. The siRNAs used in the process may be unmodified oligonucleotides or modified and may be conjugated with lipophilic moieties such as cholesterol.

The individual stocks are combined by pumping each solution to a T-junction. A dual-head Watson-Marlow pump is used to simultaneously control the start and stop of the two streams. A 1.6 mm polypropylene tubing is further downsized to a 0.8 mm tubing in order to increase the linear flow rate. The polypropylene line (ID = 0.8 mm) are attached to either side of a T-junction. The polypropylene T has a linear edge of 1.6 mm for a resultant volume of 4.1 $mm^3$. Each of the large ends (1.6 mm) of polypropylene line is placed into test tubes containing either solubilized lipid stock or solubilized siRNA. After the T-junction a single tubing is placed where the combined stream will emit. The tubing is then extending into a container with 2x volume of PBS. The PBS is rapidly stirring. The flow rate for the pump is at a setting of 300 rpm or 110 mL/min. Ethanol is removed and exchanged for PBS by dialysis. The lipid formulations are then concentrated using centrifugation or diafiltration to an appropriate working concentration.

FIG. 3 shows a schematic of the in-line mixing method and FIG. 4 is a schematic of pump set-up.

**Example 10:** Efficacy of formulations with various lipid ratios.

**Experimental Plan**
**Animals** C57BL/6
**Total** 39

| Group | Group size | Cone. (mg/mL) | Inj Vol. (uL/g) | Dose* (mg/kg) | Vehicle Lipid A/DSPC/Chol/PEG-DMG (charge ratio) |
|---|---|---|---|---|---|
| 1 | 3 | | 10 | 0.0 | PBS |
| 2 | 3 | 0.10 | 10 | 1.0 | 45/10/40/5 (1.5N/P) |
| 3 | 3 | 0.03 | 10 | 0.3 | 45/10/40/5 (1.5N/P) |
| 4 | 3 | 0.10 | 10 | 1.0 | 50/10/35/5 (1.5N/P) |
| 5 | 3 | 0.03 | 10 | 0.3 | 50/10/35/5 (1.5N/P) |
| 6 | 3 | 0.10 | 10 | 1.0 | 45/15/35/5 (1.5N/P) |
| 7 | 3 | 0.03 | 10 | 0.3 | 45/15/35/5 (1.5N/P) |
| 8 | 3 | 0.10 | 10 | 1.0 | 45/25/25/5 (1.5N/P) |
| 9 | 3 | 0.03 | 10 | 0.3 | 45/25/25/5 (1.5N/P) |
| 10 | 3 | 0.01 | 10 | 0.1 | 57.5/10/30 (2.5N/P) |
| 11 | 3 | 0.003 | 10 | 0.03 | 57.5/10/30 (2.5N/P) |
| 12 | 3 | 0.01 | 10 | 0.1 | 58.5/10/30 (1.5N/P) |
| 13 | 3 | 0.003 | 10 | 0.03 | 58.5/10/30 (1.5N/P) |

According to the results shown FIG. 4, 10 mol% of DSPC and 30 mol% of cholesterol are favorable. No change in body weight of mice was observed with the above formulations as compared with PBS as illustrated in FIG. 5.

**Example 11:** Efficacy of formulations with different amount of cationic lipid A and low PEG lipid.

| **Experimental Plan** | | | | | |
|---|---|---|---|---|---|
| **Animals** | C57BL/6 | | | | |
| **Total** | 39 | | | | |
| **siRNA** | 1661 | | | | |
| **Group** | **Group size** | **Cone. (mg/mL)** | **Inj Vol. (uL/g)** | **Dose* (mg/kg)** | **Vehicle Lipid A/DSPC/Chol/PEG-DMG** |
| 1 | 3 | | 10 | 0.0 | PBS |
| 2 | 3 | 0.01 | 10 | 0.1 | 60/5/30/5 |
| 3 | 3 | 0.003 | 10 | 0.03 | 60/5/30/5 |
| 4 | 3 | 0.01 | 10 | 0.1 | 60/10/25/5 |
| 5 | 3 | 0.003 | 10 | 0.03 | 60/10/25/5 |
| 6 | 3 | 0.01 | 10 | 0.1 | 55/10/30/5 |
| 7 | 3 | 0.003 | 10 | 0.03 | 55/10/30/5 |
| 8 | 3 | 0.01 | 10 | 0.1 | 60/5/32.5/2.5 |
| 9 | 3 | 0.003 | 10 | 0.03 | 60/5/32.5/2.5 |
| 10 | 3 | 0.01 | 10 | 0.1 | 60/5/27.5/2.5 |
| 11 | 3 | 0.003 | 10 | 0.03 | 60/5/27.5/2.5 |
| 12 | 3 | 0.01 | 10 | 0.1 | 55/10/32.5/2.5 |
| 13 | 3 | 0.003 | 10 | 0.03 | 55/10/32.5/2.5 |
| 14 | 3 | 0.01 | 10 | 0.1 | 55/5/37.5/2.5 |
| 15 | 3 | 0.003 | 10 | 0.03 | 55/5/37.5/2.5 |

According to the results shown FIG. 6, about 60 mol% of lipid A is desirable. No change in body weight of mice was observed with the above formulations as compared with PBS as illustrated in FIG. 7.

**Example 12:** Efficacy of formulations with various phosphatidylcholine with lipids ratio of 45/15/35/5 (lipid A/phosphatidylcholine/cholesterol/PEG lipid).

| **Experimental** | **Plan** | | | | |
|---|---|---|---|---|---|
| **Animals** | C57BL/6 | | | | |
| **Total** | 27 | | | | |
| **siRNA** | 1661 | | | | |
| **Group** | **Group size** | **Conc. (mg/mL)** | **Inj Vol. (uL/g)** | **Dose* (mg/kg)** | **Vehicle** |
| 1 | 3 | | 10 | 0.0 | PBS |
| 2 | 3 | 0.10 | 10 | 1.0 | DMPC |
| 3 | 3 | 0.03 | 10 | 0.3 | DMPC |
| 4 | 3 | 0.10 | 10 | 1.0 | DPPC |
| 5 | -3 | 0.03 | 10 | 0.3 | DPPC |
| 6 | 3 | 0.10 | 10 | 1.0 | DOPC |
| 7 | 3 | 0.03 | 10 | 0.3 | DOPC |
| 8 | 3 | 0.10 | 10 | 1.0 | POPC |
| 9 | 3 | 0.03 | 10 | 0.3 | POPC |

FIG. 8 shows that at 45 mol% of lipid A, different phosphatidylcholine do not have an effect on the efficacy.

**Example 13:** Efficacy of formulations with 60-90 mol% of lipid A.

**Experimental Plan**
**Animals** C57BL/6
**Total** 27
**siRNA** 1661

| Group | Group size | Conc. (mg/mL) | Inj Vol. (uL/g) | Dose* (mg/kg) | Vehicle Lipid A/DSPC/Chol/PEG-DMG |
|---|---|---|---|---|---|
| 1 | 3 | | 10 | 0.00 | PBS |
| 2 | 3 | 0.010 | 10 | 0.10 | 60-5-30-5 |
| 3 | 3 | 0.003 | 10 | 0.03 | 60-5-30-5 |
| 4 | 3 | 0.010 | 10 | 0.10 | 70-5-20-5 |
| 5 | 3 | 0.003 | 10 | 0.03 | 70-5-20-5 |
| 6 | 3 | 0.010 | 10 | 0.10 | 80-5-10-5 |
| 7 | 3 | 0.003 | 10 | 0.03 | 80-5-10-5 |
| 8 | 3 | 0.010 | 10 | 0.10 | 90-5-0-5 |
| 9 | 3 | 0.003 | 10 | 0.03 | 90-5-0-5 |

According to the results presented in FIG. 9, high ratio of the cationic lipid A is not favorable.

**Example 14:** Efficacy of formulations with 57.5 mol% of lipid A, mol% of DSPC and with different Chol:PEG ratios.

**Experimental Plan**
**Animals** C57BL/6
**Total** 27
**siRNA** 1661

| Group | Group size | Conc. (mg/mL) Lipid A/DSPC/Chol/PEG-DMG | Inj Vol. (uL/g) | Dose* (mg/kg) | Vehicle |
|---|---|---|---|---|---|
| 1 | 3 | | 10 | 0.00 | PBS |
| 2 | 3 | 0.010 | 10 | 0.10 | 57.5/7.5/30/5 |
| 3 | 3 | 0.003 | 10 | 0.03 | 57.5/7.5/30/5 |
| 4 | 3 | 0.010 | 10 | 0.10 | 57.5/7.5/31.5/3.5 |
| 5 | 3 | 0.003 | 10 | 0.03 | 57.5/7.5/31.5/3.5 |
| 6 | 3 | 0.010 | 10 | 0.10 | 57.5/7.5/32.5/2.5 |
| 7 | 3 | 0.003 | 10 | 0.03 | 57.5/7.5/32.5/2.5 |
| 8 | 3 | 0.010 | 10 | 0.10 | 57.5/7.5/33.5/1.5 |
| 9 | 3 | 0.003 | 10 | 0.03 | 57.5/7.5/33.5/1.5 |

Results are shown in FIG. 10.

**Example 15:** Efficacy of formulations at different pH levels.

**Experimental Plan**
**Animals** C57BL/6
**Total** 21
siRNA 1661

(continued)

| Group | Group size | Conc. (mg/mL) | Inj Vol. (uL/g) | Dose* (mg/kg) | Vehicle |
|---|---|---|---|---|---|
| 1 | 3 | | **10** | **0.0** | PBS |
| 5 | 3 | 0.010 | 10 | 0.10 | PH3 |
| 6 | 3 | 0.003 | 10 | 0.03 | PH3 |
| 8 | 3 | 0.010 | 10 | 0.10 | PH4 |
| 9 | 3 | 0.003 | 10 | 0.03 | PH4 |
| 17 | 3 | 0.010 | 10 | 0.10 | PH5 |
| 18 | 3 | 0.003 | 10 | 0.03 | PH5 |

According to the results shown in FIG. 11, pH level between 3-5 generally do not affect the efficacy.

**Example 16:** Efficacy of formulations mixed via in-line mixing method.

**Experimental Plan**

| | |
|---|---|
| **Animals** | C57BL/6 |
| **Total** | 75 |
| **siRNA** | 1661 |

| Group | Group size | Conc. (mg/mL) | Inj Vol. (uL/g) | Dose* (mg/kg) | Vehicle Lipid A/DSPC/Chol/PEG-DMG |
|---|---|---|---|---|---|
| 1 | 3 | | 10 | 0.00 | PBS |
| 2 | 3 | 0.010 | 10 | 0.10 | 57.5/7.5/31.5/3.5 |
| 3 | 3 | 0.003 | 10 | 0.03 | 57.5/7.5/31.5/3.5 |
| 4 | 3 | 0.010 | 10 | 0.10 | 57.5/5/34/3.5 |
| 5 | 3 | 0.003 | 10 | 0.03 | 57.5/5/34/3.5 |
| 6 | 3 | 0.010 | 10 | 0.10 | 55/7.5/34/3.5 |
| 7 | 3 | 0.003 | 10 | 0.03 | 55/7.5/34/3.5 |
| 8 | 3 | 0.010 | 10 | 0.10 | 55/5/36.5/3.5 |
| 9 | 3 | 0.003 | 10 | 0.03 | 55/5/36.5/3.5 |
| 10 | 3 | 0.010 | 10 | 0.10 | 60/7.5/27.5/5 |
| 11 | 3 | 0.003 | 10 | 0.03 | 60/7.5/27.5/5 |
| 12 | 3 | 0.010 | 10 | 0.10 | 57.5/7.5/30/5 |
| 13 | 3 | 0.003 | 10 | 0.03 | 57.5/7.5/30/5 |
| 14 | 3 | 0.010 | 10 | 0.10 | 55/7.5/32.5/5 |
| 15 | 3 | 0.003 | 10 | 0.03 | 55/7.5/32.5/5 |
| 16 | 3 | 0.010 | 10 | 0.10 | 60/5/31.5/3.5 |
| 17 | 3 | 0.003 | 10 | 0.03 | 60/5/31.5/3.5 |
| 18 | 3 | 0.010 | 10 | 0.10 | 60/7.5/29/3.5 |
| 19 | 3 | 0.003 | 10 | 0.03 | 60/7.5/29/3.5 |
| 20 | 3 | 0.010 | 10 | 0.10 | 60/7.5/31/1.5 |
| 21 | 3 | 0.003 | 10 | 0.03 | 60/7.5/31/1.5 |
| 22 | 3 | 0.010 | 10 | 0.10 | 57.7/7.5/33.5/1.5 |
| 23 | 3 | 0.003 | 10 | 0.03 | 57.7/7.5/33.5/1.5 |
| 24 | 3 | 0.010 | 10 | 0.10 | 55/7.5/36/1.5 |
| 25 | 3 | 0.003 | 10 | 0.03 | 55/7.5/36/1.5 |

Results are shown in FIG. 12.

**Example 17:** Efficacy of formulations mixed via in-line mixing method with lipid ratio of 60/7.5/31/1.5 (Lipid A/DSPC/Chol/PEG) at various charge ratios.

**Experimental Plan**

| | |
|---|---|
| **Animals** | C57BL/6 |
| **Total** | 39 |
| siRNA | 1661 |

| Group | Group size | Conc. (mg/mL) | inj Vol. (uL/g) | Dose* (mg/kg) | Vehicle Lipid A/DSPC/Chol/PEG-DMG Charge ratio (N/P) |
|---|---|---|---|---|---|
| 1 | 3 | | 10 | 0.00 | PBS |
| 2 | 3 | 0.010 | 10 | 0.10 | 60-7.5-31-1.5 N/P1.0 |
| 3 | 3 | 0.003 | 10 | 0.03 | 60-7.5-31-1.5 N/P1.0 |
| 4 | 3 | 0.010 | 10 | 0.10 | 60-7.5-31-1.5 N/P 1.5 |
| 5 | 3 | 0.003 | 10 | 0.03 | 60-7.5-31-1.5 N/P 1.5 |
| 6 | 3 | 0.010 | 10 | 0.10 | 60-7.5-31-1.5 N/P 2.0 |
| 7 | 3 | 0.003 | 10 | 0.03 | 60-7.5-31-1.5 N/P 2.0 |
| 8 | 3 | 0.010 | 10 | 0.10 | 60-7.5-31-1.5 N/P 3.0 |
| 9 | 3 | 0.003 | 10 | 0.03 | 60-7.5-31-1.5 N/P 3.0 |
| 10 | 3 | 0.010 | 10 | 0.10 | 60-7.5-31-1.5 N/P 5.0 |
| 11 | 3 | 0.003 | 10 | 0.03 | 60-7.5-31-1.5 N/P 5.0 |

Results are shown in FIG. 13.

**Example 18:** Efficacy of formulations at various siRNA:Lipid ratios via an extrustion method or an in-line mixing method.

| | Mouse $ED_{50}$ (mg/kg) | | | | Rat $ED_{50}$ (mg/kg) | |
|---|---|---|---|---|---|---|
| Formulation | (Lipid A : DSPC : Cholesterol : PEG-DMG) Lipid:siRNA ratio | Process | Protein | mRNA | Protein | mRNA |
| LNP05 | 57.5/7.5/31.5/3.5, lipid:siRNA ~ 6 | Extrusion | 0.04 | 0.1 | 0.1 | 0.15 |
| LNP06 | 57.5/7.5/31.5/3.5, lipid:siRNA ~ 11 | Extrusion | 0.02 | 0.04 | < 0.05 | 0.1 |
| LNP07 | 60/7.5/31/1.5, lipid:siRNA ~ 6 | In-line mixing | 0.02 | 0.06 | 0.1 | 0.2 |
| LNP08 | 60/7.5/31/1.5, lipid: siRNA ~ 11 | In-line mixing | 0.01 | 0.04 | < 0.05 | < 0.05 |
| LNP09 | 50/10/38.5/1.5 lipid:siRNA ~ 10 | In-line mixing | ~0.02 | | | |
| XTC-SNALP | DLinDMA/DPPC/C holesterol/PEG-cDMA 57.1/7.1/34.4/1.4 Lipid:siRNA ~ 7 | In-line mixing | ~0.01 | ~0.02 | | |

FIG. 14 illustrates the efficacy of various formulations in mouse and FIG. 15 shows the efficacy of various formulations in rat.

**Example 19**: Role of ApoE in the Cellular Uptake of Liposomes in HeLa Cells.

This study compared whether pre-complexation with human recombinant ApoE may increase the uptake of our LNP05 neutral liposome formulation in vitro in HeLa cells. Furthermore we compared the uptake of LNP05 plus or minus ApoE to the uptake of two other liposomes LNP01 (ND98, Cholesterol, and PEG-Ceramide C16) and SNALP (PEG-cDMA;DLinDMA;DPPC;cholesterol) under the same conditions.

Experimental protocol:

HeLa cells were seeded in 96 well plates (Grenier) at 6000 cells per well overnight. Three different liposome formulations of Alexa-fluor 647 labeled GFP siRNA: 1) LNP01, 2) SNALP, 3) LNP05 were diluted in one of 3 media conditions to a 50nM final concentration. Media conditions examined were OptiMem, DMEM with 10% FBS or DMEM with 10% FBS plus 10ug/mL of human recombinant ApoE (Fitzgerald Industries). The indicated liposomes either in media or in media-precomplexed with ApoE for 10 minutes were added to cells for either 4, 6, or 24 hours. Three replicated were performed for each experimental condition. After addition to HeLa cells in plates for indicated time points cells were fixed in 4% paraformaldehyde for 15 minutes then nuclei and cytoplasm stained with DAPI and Syto dye. Images were acquired using an Opera spinning disc automated confocal system from Perkin Elmer. Quantitation of Alexa Fluor 647 siRNA uptake was performed using Acapella software. Four different parameters were quanitifed: 1) Cell number, 2) the number of siRNA positive spots per field, 3) the number of siRNA positive spots per cell and 4) the integrated spot signal or the average number of siRNA spots per cell times the average spot intensity. The average spot signal therefore is a rough estimate of the total amount of siRNA content per cell. Quantitation of Alexa-fluor siRNA uptake was performed only for the 6 hour time point.

FIG. 16 illustrates the effect of ApoE on various liposome formulations. The uptake of more neutral charged liposomes SNALP and LNP05 into cells was enhanced by the pre-complexation with human recombinant ApoE ay 6 hours. The uptake into HeLa cells of the liposome LNP01 which carried a positive charge was unaffected by ApoE presence. The number of spots per field, spots per cell and integrated spot intensity was enhanced roughly 3 fold for SNALP but dramatically enhanced for LNP05 as much as 20 fold. Almost no uptake of LNP05 particles in the absence of ApoE at 6 hours and even at 24 hours. ApoE binding to neutral liposomes particularly LNP05 can dramatically enhance the cellular uptake of these lipid nanoparticles.

**Example 20:** Efficacy of LNP08 liposomes show ApoE dependence of in mice.

To further examine the role of ApoE in efficacy of various liposome formulations, wildtype and ApoE knockout mice were administered LNP08 liposomes containing the AD-1661 siRNA composition, at 0.2, 0.067, and 0.022 mg/kg. Figure 17 shows dose-dependent attenuation of FVII protein levels in wild type but not ApoE deficient knockout mice when administered with LNP08 liposomes, suggesting a role of ApoE in cellular uptake and/or delivery to the liver. Administration of LNP08 or LNP05 liposomes premixed with different ApoE lipoprotein at different concentrations results in reduction of FVII protein levels in the ApoE knockout mice; attenuation of FVII protein levels were also observed in wild type mice using some, but not all, ApoE containing liposomes (data not shown). Further, wildtype and ApoE knockout mice were administered LNP09 liposomes (LNP09 is a lipid A-containing LNP) containing the AD-1661 siRNA composition at 0.2 mg/kg. As shown in FIG. 18, lipid A activity could be rescued in ApoE knockout mice by premixing LNP09 (an lipid A-containing LNP) with ApoE.

**Example 21:** Incorporation of GalNAc lipids into liposome formulations.

To explore potential alternate delivery mechanisms, in vivo experiments were performed using liposome formulations comprising N-acetyl galactosamine (GalNAc) conjugated lipids. GalNAc was chosen as a possible targeting ligand as it is known that the GalNAc receptor is thought to be highly expressed in the liver. A study was therefore performed using C57BL/6 and ApoE knockout mice essentially as described in Example 6 to test the efficacy of the LNP08 formulations futher comprising various concentrations of GalNAc3-DSG and GalNAc3-PEG-DSG lipids. In all experiments, the total amount of PEG-conjugated lipids was kept constant (e.g., where 0.5% mol of GalNAc3-PEG is added, the corresponding amount of PEG-DMG was decreased by 0.5 % mol to keep the total PEG-lipid at 1.5% mol). Three animals were used for each of the nine groups per genotype in the experiment for a total of 54 animals:

| Group | Target | siRNA | Vehicle |
|---|---|---|---|
| 1 | | | PBS |
| 2 | FVII | 1661 | LNP08 with 0.05% mol GALNAc3-DSG |
| 3 | FVII | 1661 | LNP08 with 0.15% mol GALNAc3-DSG |
| 4 | FVII | 1661 | LNP08 with 0.5% mol GALNAc3-DSG |
| 5 | FVII | 1661 | LNP08 with 1.5% mol GALNAc3-DSG |
| 6 | FVII | 1661 | LNP08 with 0.05% mol GALNAc3-PEG-DSG |

(continued)

| Group | Target | siRNA | Vehicle |
|---|---|---|---|
| 7 | FVII | 1661 | LNP08 with 0.15% mol GALNAc3-PEG-DSG |
| 8 | FVII | 1661 | LNP08 with 0.5% mol GALNAc3-PEG-DSG |
| 9 | FVII | 1661 | LNP08 with 1.5% mol GALNAc3-PEG-DSG |

Each animal received 0.2 mg/kg of a saline control (PBS) or an AD-1661 (1661) siRNA composition targeting FVII expression, formulated as described in the above table, via tail vein injection at a volume of approximately 0.01 mL/g. At various time points after administration, serum samples were collected by retroorbital bleed. Serum levels of Factor VII protein were measured as described above.

The in vivo results of the mouse FVII silencing model with GalNAc lipids included in LNP08 are provided in FIGs. 19a and FIG. 19b. The total mol% of PEG lipids (i.e., the amount of GalNAc3 lipid and PEG-lipid) is kept constant at 1.5 mol% relative to lipids. As shown in FIG. 17, both GalNAc3-DSG and GalNAc3-PEG-DSG showed silencing activity in wild type mice. GalNAc3-PEG-DSG rescued silencing activity in ApoE KO mice.

**Example 22:** Efficacy of Lipid A formulations containing $(GalNAc)_3$-PEG-LCO in ApoE KO mice.

**Experimental Plan**

**Animals** ApoE KO mice

**Total** 21

**siRNA** 1661

| Group | Group size | Conc. (mg/mL) | Inj Vol. (uL/g) | Dose (mg/kg) | Vehicle |
|---|---|---|---|---|---|
| 1 | 3 | | 10 | | PBS |
| 2 | 3 | 0.020 | 10 | 0.20 | LNP08 |
| 3 | 3 | 0.020 | 10 | 0.20 | LNP08 w 0.005% |
| 4 | 3 | 0.020 | 10 | 0.20 | LNP08 w 0.05% |
| 5 | 3 | 0.020 | 10 | 0.20 | LNP08 w 0.15% |
| 6 | 3 | 0.020 | 10 | 0.20 | LNP08 w 0.5% |
| 7 | 3 | 0.020 | 10 | 0.20 | LNP08 w 1.5% |

The efficacy of LNP08 liposomal formulations further comprising (GalNAc)3-PEG-LCO of formula 4, another GalNAc-conjugated lipid,was tested essentially as described in Example 21 above. As shown in FIG. 20, FVII silencing is enhanced by (GalNAc)3-PEG-LCO in a dose-dependent manner, reaching maximal silencing at 1.5 mol% of the targeting lipid, the highest concentration tested.

**Example 23:** Efficacy of Lipid A formulations containing $(GalNAc)_3$-PEG-DSG in ApoE KO mice.

**Experimental Plan**

**Animals** ApoE KO mice

**Total** 21

**siRNA** 1661

| Group | Group size | Conc. (mg/mL) | Inj Vol. (uL/g) | Dose (mg/kg) | Vehicle |
|---|---|---|---|---|---|
| 1 | 3 | | 10 | | PBS |
| 2 | 3 | 0.020 | 10 | 0.20 | LNP08 |
| 3 | 3 | 0.020 | 10 | 0.20 | LNP08 w 0.005% |
| 4 | 3 | 0.020 | 10 | 0.20 | LNP08 w 0.015% |
| 5 | 3 | 0.020 | 10 | 0.20 | LNP08 w 0.05% |

(continued)

| Group | Group size | Conc. (mg/mL) | Inj Vol. (uL/g) | Dose (mg/kg) | Vehicle |
|---|---|---|---|---|---|
| 6 | 3 | 0.020 | 10 | 0.20 | LNP08 w 0.15% |
| 7 | 3 | 0.020 | 10 | 0.20 | LNP08 w 0.5% |

LNP08 formulations, further comprising 0.005 mol% - 0.5 mol% of (GalNAc)3-PEG-DSG were tested for efficacy in a mouse FVII model as described above. As shown in FIG. 21, significant enhancement of uptake by the presence of (GalNAc)3-PEG-DSG can be detected even at the lowest concentration tested.

**Example 24:** Uptake of Lipid A Containing Liposomes Pre-complexed with ApoE in HeLa-GFP, Hep3B-GFP or Primary Hepatoctyes.

The goal of these experiments was to examine whether pre-complexing liposomal delivery vehicles with ApoE would increase the amount of siRNA taken up *in vitro* into cell lines or primary hepatocytes.

HeLa-GFP or Hep3B-GFP cells were seeded in 96 well plates (Greiner) overnight at a density ranging from 8000-12,000 cells per well. Primary hepatocytes were seeded at 20,000 cells per well similarly but on collagen coated plates. The following morning LNP08 or LNP01 formulated GFP targeted Alexa-fluor 647 tagged siRNAs were incubated in OptiMem, DMEM or DMEM with 10% FBS media plus or minus concentrations of ApoE3 (Fitzgerald Research) ranging from 0.01 to 20ug/mL final in solution. Incubations were allowed to proceed for 10-60 minutes in a 37 degree incubator. Media was removed from cells cultured overnight and the liposome mixtures +/- ApoE, added to cells for the indicated time periods ranging from 15 minutes up to 6 hours. At that time cells were either fixed for 30 minutes in 4% paraformaldehyde and counterstained with DAPI to visualize nuclei, or incubated for 30 minutes with 2ug/mL of Hoescht dye also to visualize nuclei and imaged live. Images were acquired using an Opera spinning disk confocal imaging system (Perkin Elmer) using a 20X objective. Quantitation of siRNA uptake into cells from the images was performed using Acapella software (Perkin Elmer).

As shown in FIG. 22, pre-complexation of lipid A liposomes dramatically enhanced the uptake of lipid A liposomes into multiple cell types in vitro. Similar results were obtained when uptake of LNP01 vs. LNP08 +/- ApoE was examined in primary hepatocytes.

**Example 25**: Uptake of BODIPY labeled Liposomes Pre-complexed with ApoE in Hep3B GFP Cells.

The goal of these experiments was to investigate whether ApoE pre-complexation to liposomes increased the cellular uptake of the entire LNP particle as compared to just the encapsulated siRNA. To perform these experiments a new version of the lipid A was synthesized with a fluorescent BODIPY group attached. This labeled lipid was incorporated at a 10% ratio of the total amount of unlabeled lipid A in a standard LNP08 formulation.

The uptake of these lipids was performed similarly to the above experiment done with all unlabeled lipid A in the formulation. The difference being that following the uptake of the liposomal formulation with labeled lipid A images in both the AF647 (siRNA) and BODIPY (lipid A) channel were acquired. The amount of both the siRNA and BODIPY labeled lipid in the presence and absence of ApoE after image acquisition on the Opera system was determined again using Acapella software.

As shown in FIG. 23 (data are average of 30 fields, 10 each from 3 replicates wells), experiments with labeled lipid A revealed that pre-complexation of liposomes with ApoE enhanced the uptake of the entire lipid A based LNP containing the siRNA and not just the siRNA itself.

**Example 26:** Effect of ApoE2, ApoE3, or ApoE4 on mRNA Silencing in HeLa-GFP Cells.

The goal of these experiments was to determine if pre-complexation of lipid A containing liposomes with ApoE enhanced not only the uptake of the liposomes but also mRNA target silencing.

HeLa-GFP cells were seeded in 24 well plates overnight at a density of ~50,000 cells per well. The following morning LNP08 formulated GFP targeted Alexa-fluor 647 tagged siRNAs were incubated in DMEM or DMEM with 10% FBS media plus or minus concentrations of ApoE3 (Fitzgerald Research) ranging from 0.01 to 20ug/mL. Incubations were allowed to proceed for 10 or 60 minutes in a 37 degree incubator. Media was removed from cells cultured overnight and the liposome mixtures +/- ApoE3, added to cells. After 24 hours incubation the media was removed and the cells lysed in Lysis buffer (Epicentre) + Proteinase K (50ug/mL). Lysates were incubated for 2 hours at 65 degrees with constant shaking. Lysates were analyzed for GFP mRNA knockdown using the Quantigene branched DNA assay from Panomics. Probes recognizing target GFP and housekeeping Gapdh were run in triplicate for each sample. Data were

expressed as a percentage of untreated control GFP/Gapdh mRNA ratios. In some experiments (only when specifically noted) alternative ApoE isoforms ApoE2 or ApoE4 were used for pre-complexation to liposomes in place of ApoE3.

As shown in FIGs. 24 and 25, pre-complexation with ApoE enhanced silencing with lipid A containing liposomes. FIG. 26 demonstrates that other isoforms ApoE2 or ApoE4 enhanced LNP08 silencing comparably to ApoE3 in HeLa cells. These results suggest that ApoE3 as well as other isoforms of ApoE, either ApoE2 or ApoE4 enhance the mRNA silencing mediated by lipid A containing liposomes likely by mediating more efficient cellular uptake of the particles.

**Example 27:** Folate Liposomes.

• Targeting moiety:

» Folate-PEG2000-DSPE (Avanti) C18 lipid anchor

• Compositions: LNP08 formulations, wherein the amount of PEG-DMG was replaced with varying amounts of the Folate-PEG2000-DSPE:

| • Folate-PEG2000-DSPE content | • Composition (Lipid A: DSPC : Cholesterol : PEG-DMG : Folate-PEG2000:DSPE) in mol % |
|---|---|
| • 0 mol% | • 60 : 7.5 : 31 : 1.5 : 0 |
| • 0.05 mol% | • 60 : 7.5 : 31 : 1.45 : 0.05 |
| • 0.15 mol% | • 60 : 7.5 : 31 : 1.35 : 0.15 |
| • 0.5 mol% | • 60 : 7.5 : 31 : 1.0 : 0.5 |

•

• Size:

» NP08, 0.05 mol%, 0.15 mol% ~80 nm
» 0.5 mol% ~120nm (AD-1955) 180 nm (AD-18747)

• Encapsulation:

» ~90% (for the AD-1955 liposomes)
» Not determined for AD-18747 liposomes since A647 interferes in the assay

• siRNAs: L

» AD-1955 for binding studies
» AD-18747 (active GFP siRNA with Alexa647 on the 3'of the antisense)

AD-18747:

| **SS#** | **Sense seq** | **SS#** | **Antisense seq** |
|---|---|---|---|
| **sense** | | **antisense** | |
| **A-32593** | **AcAuGAAGcAGcACGACuUdTsdT** | **A-32592** | **AAGUCGUGCUGCUUCAUGUdTdTL48** |
| GFP targeted sequence, L48 = Alexa647 | | | |

AD-1955:

| **SS# sense** | **Sense seq** | **SS# antisense** | **Antisense seq** |
|---|---|---|---|
| **A-3372** | **cuuAcGcuGAGuAcuucGAdTsdT** | **A-3374** | **UCGAAGuACUcAGCGuAAGdTsdT** |

Uptake of liposomes by KB cells (FACS):

KB cells were incubated with AD-18747 containing liposomes (AD-18747 (active GFP siRNA with Alexa647 on the 3'of the antisense)). Serum free, folate free media were used. Cells were washed and analyzed by FACS after 1 hr. As shown in FIG. 27, folate-PEG enhanced uptake of liposome in a folate-dependent manner.

Uptake of liposomes by KB cells (microscopy):

KB cells were incubated with 20 nM liposomes containing AD-18747 for 1 or 2 hrs in serum-free media. Cells were fixed, stained with DAPI and imaged on the Opera. Images are not adjusted to the same exposure. As shown in FIG. 28, folate-PEG enhanced uptake of liposome in a folate-dependent manner.

Silencing with folate liposomes (6 hr incubation with liposomes):

AD-18747 containing liposomes were incubated with KB-GFP17 cells for 6 hrs. GFP expression was analyzed after 72 hrs. The results are shown in FIGs. 29a and 29b. Folate lipoosomes showed enhanced silencing. Presence of serum impacted the efficacy.

Having thus described several aspects of at least one embodiment of this invention, it is to be appreciated various alterations, modifications, and improvements will readily occur to those skilled in the art. Such alterations, modifications, and improvements are intended to be part of this disclosure, and are intended to be within the spirit and scope of the invention. Accordingly, the foregoing description is by way of example only. All references cited within this application are incorporated herein in their entirety.

**Example 28**: Dose Response for Lipid A Liposome Formulations Containing GalNAc in ApoE Knockout Mice.

ApoE knockout mice were administered PBS, Lipid A LNP08 without GalNAc, or Lipid A liposome LNP08 in which 0.15 mol% or 0.5 mol% of the PEG-DMG was replaced with the same molar % of GalNAc3 lipid of formula 3, at the following dosages: 0.006 mg/kg, 0.02 mg/kg, 0.06 mg/kg, and 0.2 mg/kg FVII siRNA. The total mol% of PEG lipids (i.e., the amount of GalNAc3 lipid and PEG-lipid) is kept constant at 1.5 mol% relative to lipids. The experiments were performed as described in Example 6.

The in vivo results of the dose response study are provided in FIG. 30. As shown in FIG. 30, dose dependent reduction of FVII level was observed in ApoE mice administered Lipid A formulations containing 0.15 mol% or 0.5 mol% the GalNAc3 lipid of formula 3. There was little or no knockdown of FVII by Lipid A formulation without the GalNAc3 lipid of formula 3 in ApoE knockout mice.

**Example 29:** Efficacy of Lipid A Formulations containing GalNAc in ApoE Wildtype Mice.

C57BL6 mice were administered PBS, Lipid A formulation with lipids ratio (in molar %) of 57.5/7.5/30/5 (lipid A/ distearoylphosphatidylcholine (DSPC) /cholesterol/PEG-distyryl glycerol (PEG-DSG) lipid) without GalNAc3 lipid of formula 3, or the same formulation containing 0.15 mol%, 0.5 mol%, or 1.0 mol% GalNAc3 lipid of formula 3. The total mol% of PEG lipids (i.e., the amount of GalNAc3 lipid and PEG-lipid) is kept constant at 1.5 mol% relative to lipids. Three dose levels (0.1, 0.3, or 1 mg/kg) of FVII siRNA were tested when mice were administered the formulation containing 1.0 mol% GlaNAc3 lipid of formula 3, and all other mice were tested at a dose of 1 mg/kg siRNA. The experiments were otherwise performed essentially as described in Example 6.

The in vivo results of this study are provided in FIG. 31. Previously, efficacy of liposomes containing GalNAc lipids could not easily be shown in ApoE wildtype mice, presumably due to predominance of the ApoE-dependent mechanism driving liver uptake. However, by using 5 mol% C18-PEG (PEG-DSG)-containing formulations, FVII knockdown was observed to be reduced. As shown in FIG. 31, the liver uptake of siRNA was retarded when longer chain (C18) PEG conjugates were used at 5 mol% in the formulation. However, replacement of 0.15 mol% to 1 mol% of the PEG-DSG with the GalNAc3 lipid of formula 3 (also at 0.15 mol% to 1 mol%) in this formulation restored knockdown of FVII in the ApoE wildtype mice, indicating the ability of GalNAc conjugated lipid (e.g., GalNAc3-PEG-lipid) to target the lipid nucleic acid particle to deliver to the liver, further suggesting that other targeting lipids could be used in liposomes containing PEG-DSG to target to other tissues.

**Example 30**: Efficacy of Lipid A Formulations in Various Tumor Cell Lines.

Cells were seeded in 96 well plates at 15-20x$10^3$ per well overnight. On the following day, four different liposome formulations of dsRNAs targeting KSP (AD6248) and Luc (AD1955) were prepared. The formulations were as follows:

1) LNP08- containing C14-PEG (PEG-DMG), 2) LNP08- containing C18-PEG (PEG-DSG), 3) LNP09- containing C14-PEG, and 4) LNP09- containing C18-PEG. The dsRNA-liposome formulations were serially diluted from 500 or 600 nM in serum containing media with or without 3 ug/ml of ApoE. Liposome formulations of Luc targeted siRNA were used for normalization. 1 ug/ml ApoE was also tested for half of the cell lines. Incubations were allowed to proceed for 15 to 25 minutes at 37 °C. Media were removed from cells and 100 μl of the liposome mixtures +/- ApoE were added to the cells for 24 hour incubation. The next day, 100ul of lysis mixture/nuclease free $H_20$ (1:2) and Proteinase K (10ul per ml) were added to the cells and mixed at 65 °C for about 35 minutes. KSP mRNA levels were determined by Quantigene 1.0 in comparison to the levels of GAPDH.

For knockdown of KSP, siRNA duplex (AD6248) having sense strand sequence: AccGAAGuGuuGuuuGuccTsT (SEQ ID NO: ) and antisense strand sequence: GGAcAAAcAAcACUUCGGUTsT (SEQ ID NO: ) was used and described, e.g., in USSN 11/694,215, the contents of which are incorporated herein by reference in its entirety.

IC50 results for eight different tumor cell lines are shown in Table 4.

Table 4

| | | IC50 (nM) | | | |
|---|---|---|---|---|---|
| Cell Line | Cell Type | LNP08-C14 | LNP08-C18 | LNP09-C14 | LNP09-C18 |
| HeLa | Cervical adenocarcinoma | 2.8 | 7.0 | 3.0 | 13.6 |
| Hep3B | Hepatoma | 1.4 | 60.5 | 0.74 | 1.4 |
| A375 | Melanoma | 7.1 | >500 | 2.1 | 28 |
| Hct116 | Colorectal carcinoma | 0.4 | 4.7 | 0.37 | 2.1 |
| MCF7 | Breast adenocarcinoma | 20 | >500 | 0.08 | 0.05 |
| Huh7 | Hepatoma | 14.3 | >>500 | 1.13 | 21.7 |
| GTL16 | Gastric carcinoma | | | 0.83 | 5.3 |

**Example 31:** Lipid A liposomal formulations containing antioxidants.

The stability of lipids in formulation were performed and monitored using the following HPLC and ELSD (Evaporative Light Scattering Detector) conditions.

HPLC:

- Column: Waters Xbridge C18 2.5 μm 2.1x150mm reversed phase column
- Buffer A: 80/20 MeOH/10 mM $NH_4HCO_3$
- Buffer B: 80/20 MeOH/THF
- Gradient: 0~16 min 100% to 20% buffer A

ELSD parameters:

- Model: Polymer lab ELS 2100
- Evaporator temperature: 90
- Nebuliser temperature: 60

Stability assays with Lipid A liposome

Fresh Lipid A liposome samples with loaded siRNA AD1661 were prepared as described herein. These Lipid A liposome samples were incubated in 100 mM NaOAc buffer (pH=5) at 37°C, and were subject to LC-ELSD analysis every other day. Chromatographs at the indicated time points are shown in FIG. 32. The results indicate that Lipid A samples are degraded under mild acidic conditions (pH=5).

In order to examine whether degradation of Lipid A can be prevented by addition of radical scavengers such

as butylated hydroxytoluene (BHT or butylhydroxytoluene), Lipid A-containing liposome formulation LNP08 with lipids molar ratio of 60/7.5/31/1.5 (lipid A/DSPC/Cholesterol/PEG-C14 lipid) loaded with siRNA AD1661 containing BHT was tested in the stability assay as described above. These samples were subject to LC-ELSD analysis at day 5. Chromatographs comparing the stability of Lipid A liposome formulations with or without BHT were shown in FIG. 33. The results indicate that the degradation of Lipid A under mild acidic condition (pH=5) could be inhibited by BHT.

In order to examine whether the degradation of Lipid A can be prevented by antioxidants such as vitamin E, Lipid A-containing liposome formulation LNP09 loaded with siRNA AD1661 containing various amount of vitamin E were tested in the stability assay as described above. These samples were subject to LC-ELSD analysis at day 5. Chromatographs comparing the stability of various Lipid A liposome formulations with or without vitamin E were shown in FIG. 34. The results indicate that the degradation of Lipid A under mild acidic condition (pH=5) could be inhibited by vitamin E. Higher amount of vitamin E caused reverse effect.

**Example 32: Lipid A shows dependence on LDLR for efficacy:**

LNP09 formulations with FVII RNAi were tested in wildtype and LDLR KO mice as shown the table below.

| Group | Mice | Target | siRNA | Inj vol (ul) | Dose (mg/kg) | Vehicle |
|---|---|---|---|---|---|---|
| 1 | Wt | None | | 10 | | PBS |
| 2 | Wt | FVII | AD-1661 | 10 | 0.100 | LNP09 |
| 3 | Wt | FVII | AD-1661 | 10 | 0.030 | LNP09 |
| 4 | Wt | FVII | AD-1661 | 10 | 0.010 | LNP09 |
| 5 | LDLR KO | None | | 10 | | PBS |
| 6 | LDLR KO | FVII | AD-1661 | 10 | 0.100 | LNP09 |
| 7 | LDLR KO | FVII | AD-1661 | 10 | 0.030 | LNP09 |
| 8 | LDLR KO | FVII | AD-1661 | 10 | 0.010 | LNP09 |
| 9 | LDLR KO | FVII | AD-1661 | 10 | 0.010 | LNP09 |

As illustrated in Figure 35, a decrease in efficacy was observed in the LDLR KO mice when compared to the wildtype mice, consistent with the LDL Receptor being a significant receptor for ApoE.

The formulations were further tested wherein 0.5 mol% of the PEG-DMG was replaced with GALNaC3-PEG-lipid (formula 3). The formulations comprising the the GalNac3-PEG-lipids were tested mice (9 groups total, as shown in the table below), and tested for FVII protein level as described above. As shown in Figure 36, the formulations were no longer LDLR dependent, as shown by the equivalent potency of the formulation in wildtype and LDLR knockout mice. Thus, the presence of the targeting lipid such as the GalNAc lipid used here appears to alleviate the LDL Receptor dependence of particles comprising Lipid A.

| Group | Mice | Target | siRNA | Inj vol (ul) | Dose (mg/kg) | Vehicle |
|---|---|---|---|---|---|---|
| 1 | Wt | None | | 10 | | PBS |
| 2 | Wt | FVII | AD-1661 | 10 | 0.100 | LNP09+0.5% GalNAc |
| 3 | Wt | FVII | AD-1661 | 10 | 0.030 | LNP09+0.5% GalNAc |
| 4 | Wt | FVII | AD-1661 | 10 | 0.010 | LNP09+0.5% GalNAc |
| 5 | LDLR KO | None | | 10 | | PBS |
| 6 | LDLR KO | FVII | AD-1661 | 10 | 0.100 | LNP09+0.5% GalNAc |
| 7 | LDLR KO | FVII | AD-1661 | 10 | 0.030 | LNP09+0.5% GalNAc |
| 8 | LDLR KO | FVII | AD-1661 | 10 | 0.010 | LNP09+0.5% GalNAc |
| 9 | LDLR KO | FVII | AD-1661 | 10 | 0.010 | LNP09 |

ITEMS

1. A lipid formulation comprising 45-65% of cationic lipid of formula A, 5-10% of the neutral lipid, 25-40% of the sterol, and 0.5-5% of the PEG or PEG-modified lipid, wherein formula A is

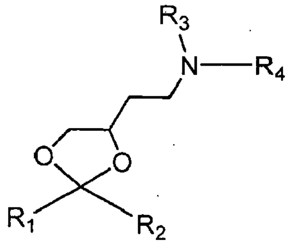

,

where $R_1$ and $R_2$ are independently alkyl, alkenyl or alkynyl, each can be optionally substituted, and $R_3$ and $R_4$ are independently lower alkyl or $R_3$ and $R_4$ can be taken together to form an optionally subsituted heterocyclic ring.

2. The lipid formulation of item 1, wherein the neutral lipid is selected from DSPC, DPPC, DMPC, DPPC, POPC, DOPE and SM.

3. The lipd formulation of item 1, wherein the sterol is cholesterol.

4. The lipid formulation of item 1, wherein the PEG lipid is PEG-$C_{14}$ to PEG-$C_{22}$, PEG-$Cer_{14}$ to PEG-$C_{20}$, or PEG-DSPE.

5. The lipid formulation of item 1, wherein $R_1$ and $R_2$ of formula A are selected from selected from oleoyl, pamitoyl, steroyl, linoleyl and $R_3$ and $R_4$ are methyl.

6. The lipid formulation of item 1, wherein the cationic lipid of formula A is 2,2-Dilinoleyl-4-dimethylaminoethyl-[1,3]-dioxolane, the neutral lipid is DSPC, the sterol is cholesterol and the PEG lipid is PEG-DMG.

7. The lipid formulation of item 6 comprising about 60% of cationic lipid of formula A, about 7.5% of the neutral lipid, about 31 % of the sterol, and about 1.5% of the PEG or PEG-modified lipid.

8. The lipid formulation of item 7, wherein the formulation is prepared by an in-line mixing method.

9. The lipid formulation of item 6 comprising about 57.5% of cationic lipid of formula A, about 7.5% of the neutral lipid, about 31.5 % of the sterol, and about 3.5% of the PEG or PEG-modified lipid.

10. The lipid formulation of item 9, wherein the formulation.is prepared by an extrusion method.

11. The lipid formulation of item 1, further comprising a therapeutic agent.

12. The lipid formulation of item 11, wherein the therapeutic agent comprises a nucleic acid.

13. The lipid formulation of item 11, wherein the nucleic acid is selected from antisense, siRNA, ribozyme and microRNA.

14. The lipid formulation of item 12, wherein the ratio of lipid:nucleic acid is about 3:1 to about 15:1.

15. The lipid formulation of item 14, wherein the ratio of lipid:nucleic acid is about 5:1 to about 13:1.

16. The lipid formulation of item 15, wherein the ratio of lipid nucleic acid is about 7:1 to about 11:1

17. The lipid formulation of item 1, further comprising at least one apolipoprotein.

18. The lipid formulation of item 17, wherein the apolipotprotein is selected from the group consisting of ApoA-I, ApoA-II, ApoA-IV, ApoA-V and ApoE, active polymorphic forms, isoforms, variants and mutants, and fragments or truncated forms thereof.

19. The lipid formulation of item 17, wherein the apolipotprotein is ApoE, active polymorphic forms, isoforms, variants and mutants, and fragments or truncated forms thereof.

20. The lipid formulation of item 1, further comprising a targeting lipid.

21. The lipid formulation of item 1, further comprising a targeting lipid comprising N-acetyl galactosamine as a targeting moiety.

22. The formulation of item 21, wherein the targeting lipid comprises a plurality of N-acetyl galactosamine moieties.

23. The formulation of item 21, wherein said targeting lipid is present in the formulation in a molar amount of from about 0.001% to about 5%.

24. The formulation of item 23, wherein said targeting lipid is present in the formulation in a molar amount of from about 0.005% to about 1.5%.

25. The formulation of item 21, wherein said targeting lipid is the compound selected from the group consisting of formula 2, formula 3, formula 5, formula 6 or formula 7:

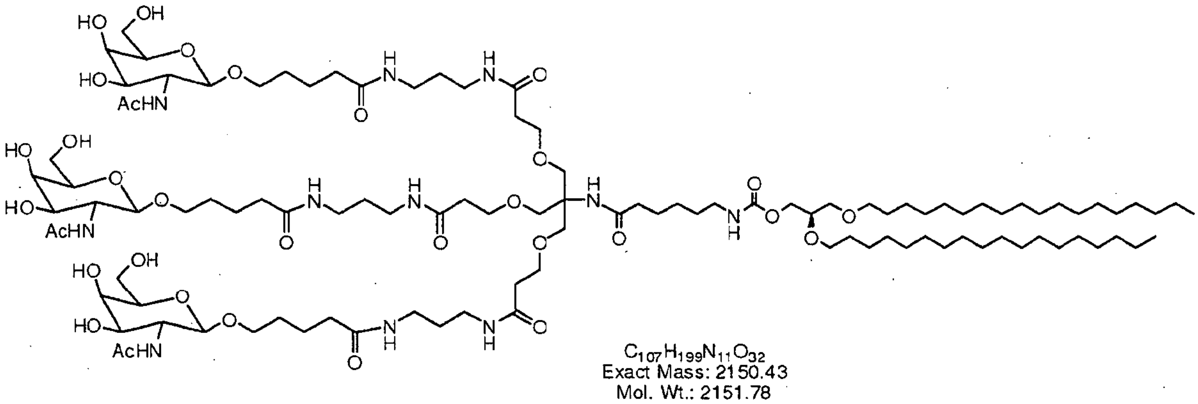

$C_{107}H_{199}N_{11}O_{32}$
Exact Mass: 2150.43
Mol. Wt.: 2151.78

formula 2

**GalNAc3-DSG**

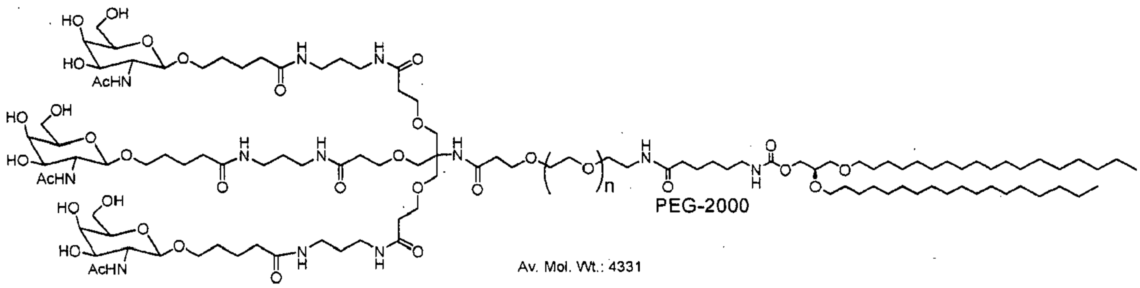

Av. Mol. Wt.: 4331

formula 3

**GalNAc3-PEG-DSG**

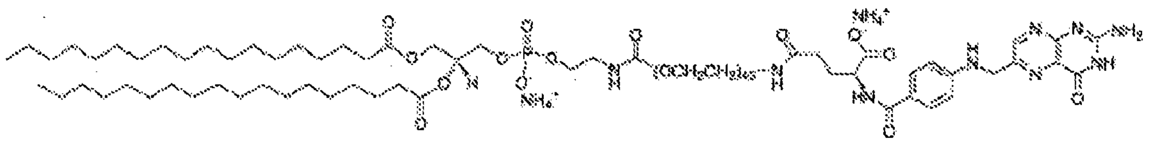

**Folate-PEG-DSPE**

formula 5

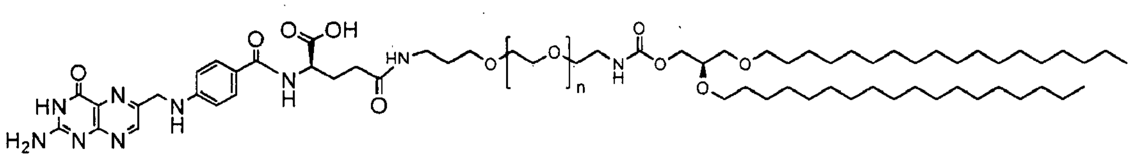

Mol Wt: ~ 3028

**Folate-PEG2000-DSG**

formula 6

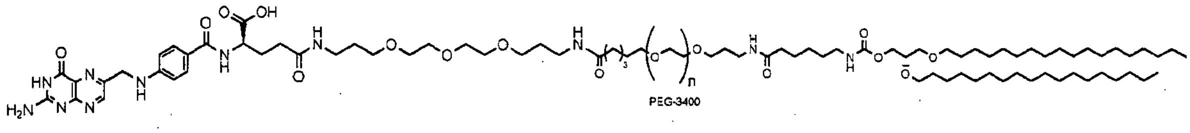

MW: ~ 4761

**Folate-PEG3400-DSG**
Formula 7.

26. The lipid formulation of item 6, comprising about 50% of cationic lipid of formula A, about 10% of the neutral lipid, about 38.5 % of the sterol, and about 1.5% of the PEG or PEG-modified lipid.

27. A method of delivering a therapeutic agent to a target gene comprising administering to a subject the lipid formulation of item 11.

28. The method of item 27, wherein the therapeutic agent is an RNA-based construct.

29. The method of item 28, wherein the RNA-based construct is a dsRNA.

30. The method of item 27, wherein the target gene is selected from the group consisting of Factor VII, Eg5, PCSK9, TPX2, apoB, SAA, TTR, RSV, PDGF beta gene, Erb-B gene, Src gene, CRK gene, GRB2 gene, RAS gene, MEKK gene, JNK gene, RAF gene, Erk1/2 gene, PCNA(p21) gene, MYB gene, JUN gene, FOS gene, BCL-2 gene, Cyclin D gene, VEGF gene, EGFR gene, Cyclin A gene, Cyclin E gene, WNT-1 gene, beta-catenin gene, c-MET gene, PKC gene, NFKB gene, STAT3 gene, survivin gene, Her2/Neu gene, topoisomerase I gene, topoisomerase II alpha gene, mutations in the p73 gene, mutations in the p21(WAF1/CIP1) gene, mutations in the p27(KIP1) gene, mutations in the PPM1D gene, mutations in the RAS gene, mutations in the caveolin I gene, mutations in the MIB I gene, mutations in the MTAI gene, mutations in the M68 gene, mutations in tumor suppressor genes, and mutations in the p53 tumor suppressor gene.

31. The method of item 27, wherein the target gene is Factor VII.

32. The method of item 27, further comprising comparing expression of the target gene with a preselected reference value.

33. The method of item 27, wherein the therapeutic agent is an antisense, siRNA, ribozyme or microRNA.

34. The lipid formulation of item 6 comprising about 57.1% of cationic lipid of formula A, about 7.1% of the neutral lipid, about 34.4 % of the sterol, and about 1.4% of the PEG or PEG-modified lipid.

35. The formulation of item 24, wherein said targeting lipid is present in the formulation in a molar amount of 0.3%.

36. The lipid formulation of item. 1, wherein the concentration of the cationic lipid of Formula A is between 45 and 55%.

37. The lipid formulation of item 36, wherein the concentration of cationic lipid of Formula A is 50%.

## Claims

1. A lipid formulation comprising 45-65% of a cationic lipid of formula A, 5-10% of a neutral lipid, 25-40% of a sterol, and 0.5-5% of a PEG or PEG-modified lipid, wherein formula A is

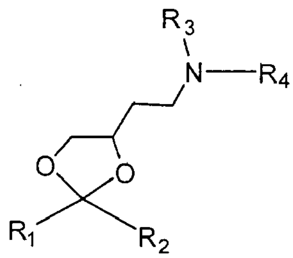

where $R_1$ and $R_2$ are independently alkyl, alkenyl or alkynyl, each can be optionally substituted, and $R_3$ and $R_4$ are independently lower alkyl or $R_3$ and $R_4$ can be taken together to form an optionally substituted heterocyclic ring; and wherein the lipid formulation comprises a targeting lipid, comprising folate as a targeting moiety; or
wherein the lipid formulation comprises a targeting lipid, comprising N-acetyl galactosamine or folate as a targeting moiety, and the lipid formulation comprises a therapeutic agent, wherein the therapeutic agent is mRNA; or
wherein the lipid formulation comprises at least one apolipoprotein, wherein the lipid formulation optionally comprises a targeting lipid comprising N-acetyl galactosamine or folate as a targeting moiety.

2. The lipid formulation of claim 1, wherein the neutral lipid is selected from DSPC, DPPC, DMPC, POPC, DOPE and SM.

3. The lipid formulation of claim 1 or 2, wherein the sterol is cholesterol.

4. The lipid formulation of any one of claims 1-3, wherein the PEG lipid is PEG-$C_{14}$ to PEG-$C_{22}$, PEG-Cer$C_{14}$ to PEG-Cer$C_{20}$ or PEG-DSPE.

5. The lipid formulation of any one of claims 1-4, wherein $R_1$ and $R_2$ of formula A are selected from oleoyl, palmitoyl, steroyl, linoleyl and $R_3$ and $R_4$ are methyl.

6. The lipid formulation of any one of claims 1-5, wherein the cationic lipid of formula A is 2,2-Dilinoleyl-4-dimethylaminoethyl-[1,3]-dioxolane, the neutral lipid is DSPC, the sterol is cholesterol and the PEG lipid is PEG-DMG.

7. The lipid formulation of any one of claims 1-6, comprising about 60% of cationic lipid of formula A, about 7.5% of the neutral lipid, about 31% of the sterol, and about 1.5% of the PEG or PEG-modified lipid, wherein the formulation is prepared by an in-line mixing method.

8. The lipid formulation of any one of claims 1-6, comprising about 57.5% of cationic lipid of formula A, about 7.5% of the neutral lipid, about 31.5% of the sterol, and about 3.5% of the PEG or PEG-modified lipid, wherein the formulation is prepared by an extrusion method.

9. The lipid formulation of any one of claims 1-6, comprising about 50% of cationic lipid of formula A, about 10% of the neutral lipid, about 38.5% of the sterol, and about 1.5% of the PEG or PEG-modified lipid.

10. The lipid formulation of any one of claims 1-6, comprising about 57.1% of cationic lipid of formula A, about 7.1% of the neutral lipid, about 34.4% of the sterol, and about 1.4% of the PEG or PEG-modified lipid.

11. The lipid formulation of any one of claims 1-10, comprising a therapeutic agent.

12. The lipid formulation of claim 1, wherein the ratio of lipid:nucleic acid is about 3:1 to about 15:1, preferably about 5:1 to about 13:1, more preferably about 7:1 to about 11:1.

13. The lipid formulation of any one of claims 1-12, wherein the formulation comprises at least one apolipoprotein, preferably selected from the group consisting of ApoA-I, ApoA-II, ApoA-IV, ApoA-V and ApoE, active polymorphic forms, isoforms, variants and mutants, and fragments or truncated forms thereof.

14. The lipid formulation of claim 1, wherein the concentration of the cationic lipid of Formula A is between 45 and 55%, preferably 50%.

15. The lipid formulation of any one of claims 1-14 for use in delivering a therapeutic agent to a target gene comprising administering said formulation to a subject.

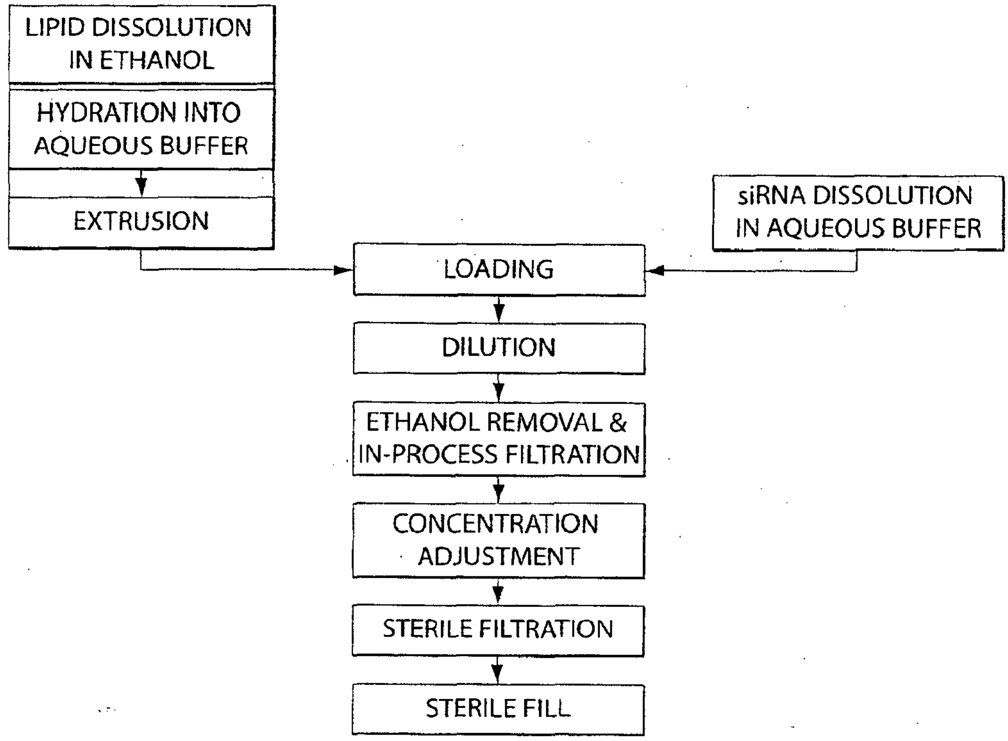
LIPID DISSOLUTION IN ETHANOL
HYDRATION INTO AQUEOUS BUFFER
EXTRUSION
siRNA DISSOLUTION IN AQUEOUS BUFFER
LOADING
DILUTION
ETHANOL REMOVAL & IN-PROCESS FILTRATION
CONCENTRATION ADJUSTMENT
STERILE FILTRATION
STERILE FILL

Fig. 1

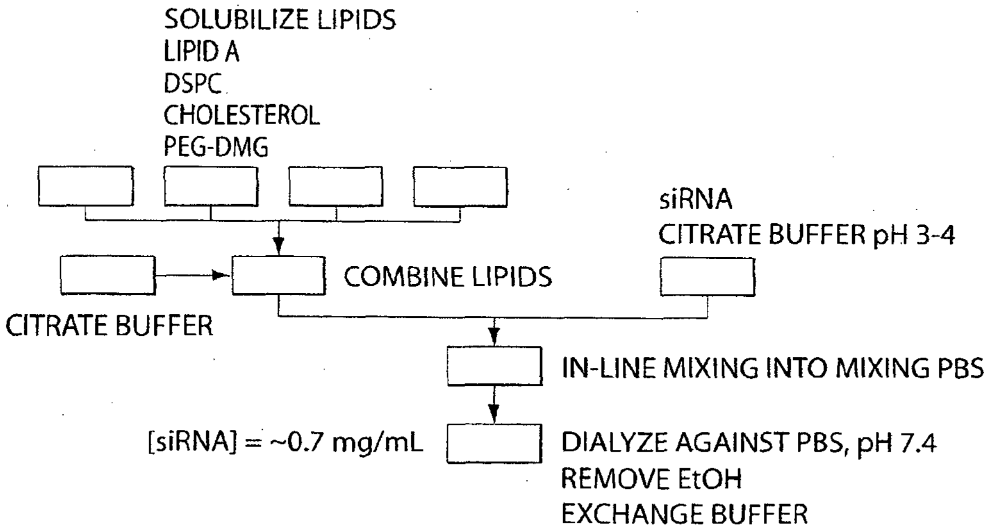
SOLUBILIZE LIPIDS
LIPID A
DSPC
CHOLESTEROL
PEG-DMG
siRNA
CITRATE BUFFER pH 3-4
COMBINE LIPIDS
CITRATE BUFFER
IN-LINE MIXING INTO MIXING PBS
[siRNA] = ~0.7 mg/mL
DIALYZE AGAINST PBS, pH 7.4
REMOVE EtOH
EXCHANGE BUFFER

Fig. 2

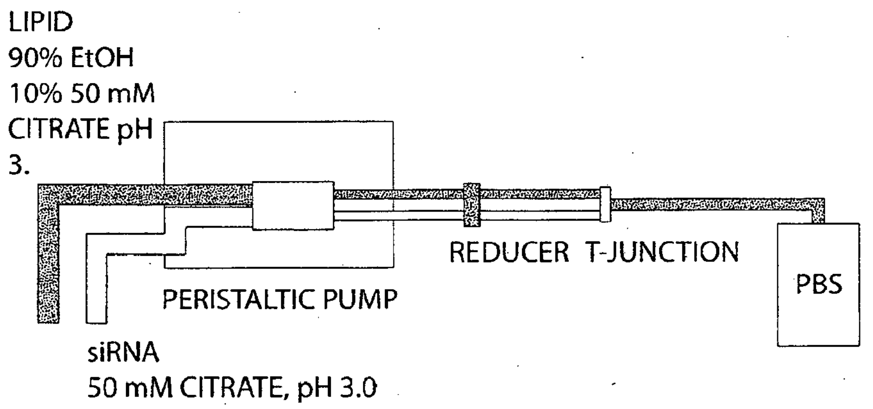
LIPID
90% EtOH
10% 50 mM
CITRATE pH
3.
REDUCER T-JUNCTION
PBS
PERISTALTIC PUMP
siRNA
50 mM CITRATE, pH 3.0

Fig. 3

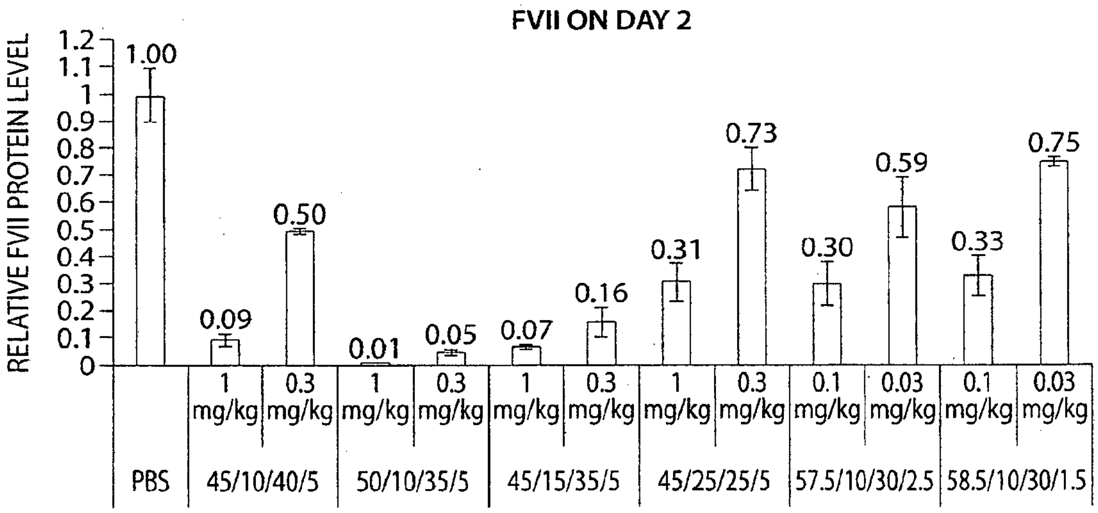
FVII ON DAY 2
RELATIVE FVII PROTEIN LEVEL
1.2
1.1
1
0.9
0.8
0.7
0.6
0.5
0.4
0.3
0.2
0.1
0
1.00
0.09
0.50
0.01
0.05
0.07
0.16
0.31
0.73
0.30
0.59
0.33
0.75
1 mg/kg
0.3 mg/kg
0.1 mg/kg
0.03 mg/kg
PBS
45/10/40/5
50/10/35/5
45/15/35/5
45/25/25/5
57.5/10/30/2.5
58.5/10/30/1.5

Fig. 4

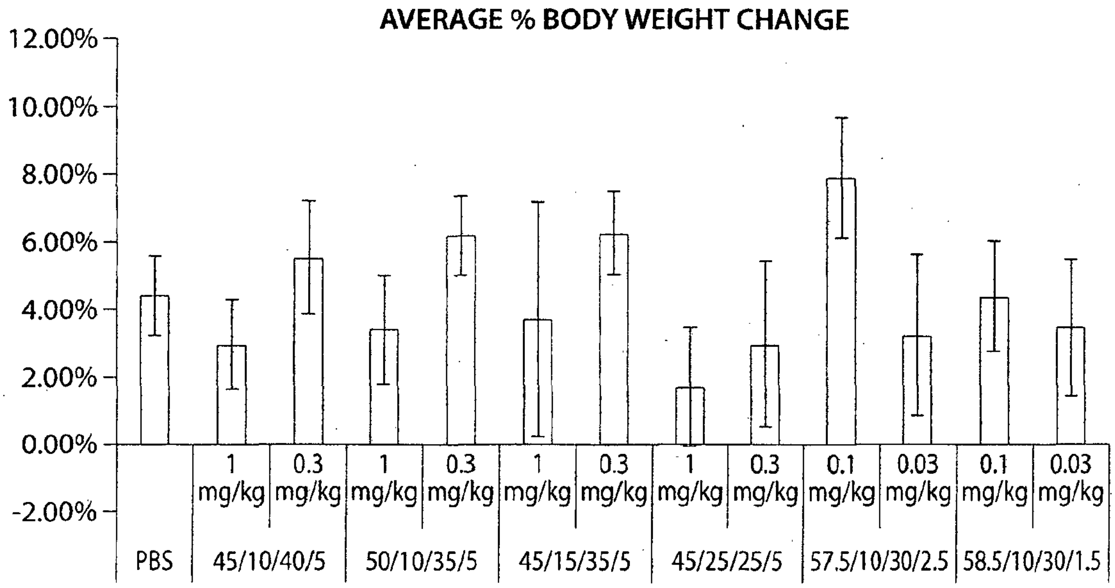
AVERAGE % BODY WEIGHT CHANGE
12.00%
10.00%
8.00%
6.00%
4.00%
2.00%
0.00%
-2.00%
1 mg/kg
0.3 mg/kg
0.1 mg/kg
0.03 mg/kg
PBS
45/10/40/5
50/10/35/5
45/15/35/5
45/25/25/5
57.5/10/30/2.5
58.5/10/30/1.5

Fig. 5

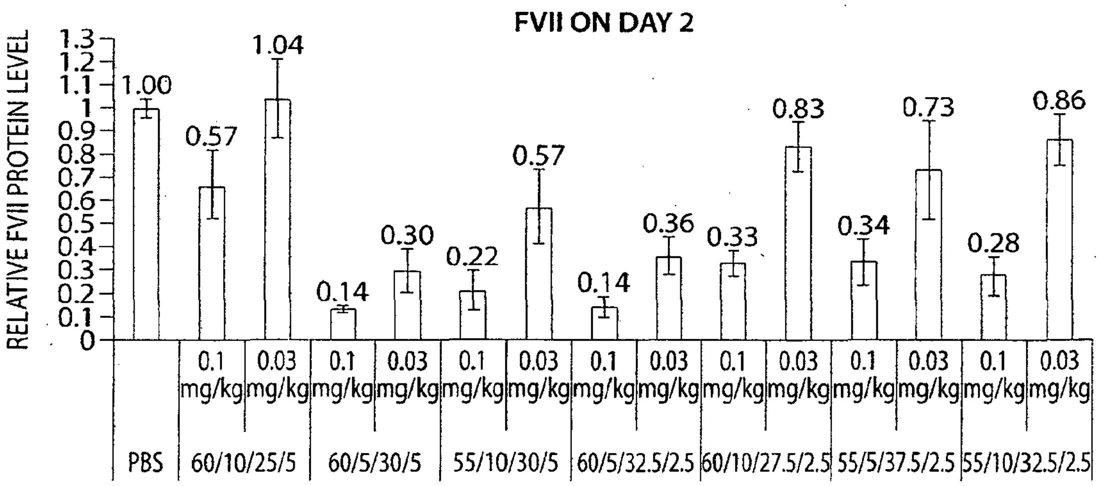
FVII ON DAY 2
RELATIVE FVII PROTEIN LEVEL
1.3
1.2
1.1
1
0.9
0.8
0.7
0.6
0.5
0.4
0.3
0.2
0.1
0
1.00
0.57
1.04
0.14
0.30
0.22
0.57
0.14
0.36
0.33
0.83
0.34
0.73
0.28
0.86
0.1 mg/kg
0.03 mg/kg
PBS
60/10/25/5
60/5/30/5
55/10/30/5
60/5/32.5/2.5
60/10/27.5/2.5
55/5/37.5/2.5
55/10/32.5/2.5

Fig. 6

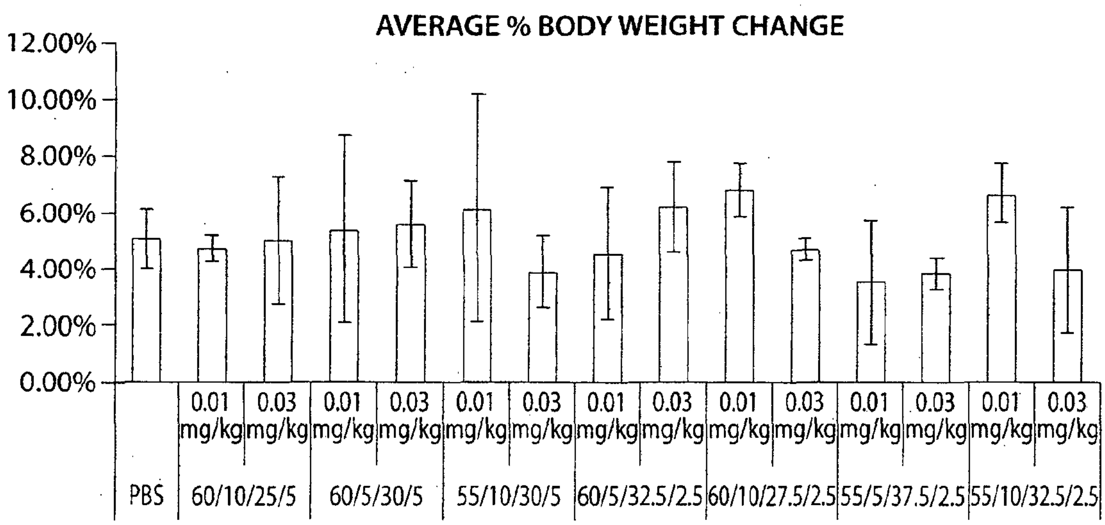
AVERAGE % BODY WEIGHT CHANGE
12.00%
10.00%
8.00%
6.00%
4.00%
2.00%
0.00%
0.01 mg/kg
0.03 mg/kg
PBS
60/10/25/5
60/5/30/5
55/10/30/5
60/5/32.5/2.5
60/10/27.5/2.5
55/5/37.5/2.5
55/10/32.5/2.5

Fig. 7

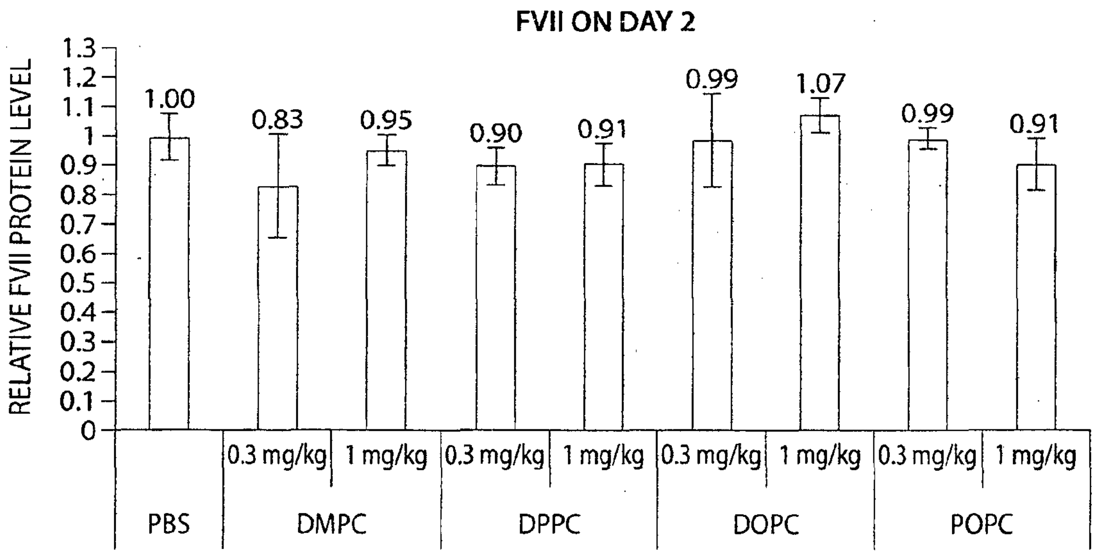
FVII ON DAY 2
RELATIVE FVII PROTEIN LEVEL
1.3
1.2
1.1
1
0.9
0.8
0.7
0.6
0.5
0.4
0.3
0.2
0.1
0
1.00
0.83
0.95
0.90
0.91
0.99
1.07
0.99
0.91
0.3 mg/kg
1 mg/kg
PBS
DMPC
DPPC
DOPC
POPC

Fig. 8

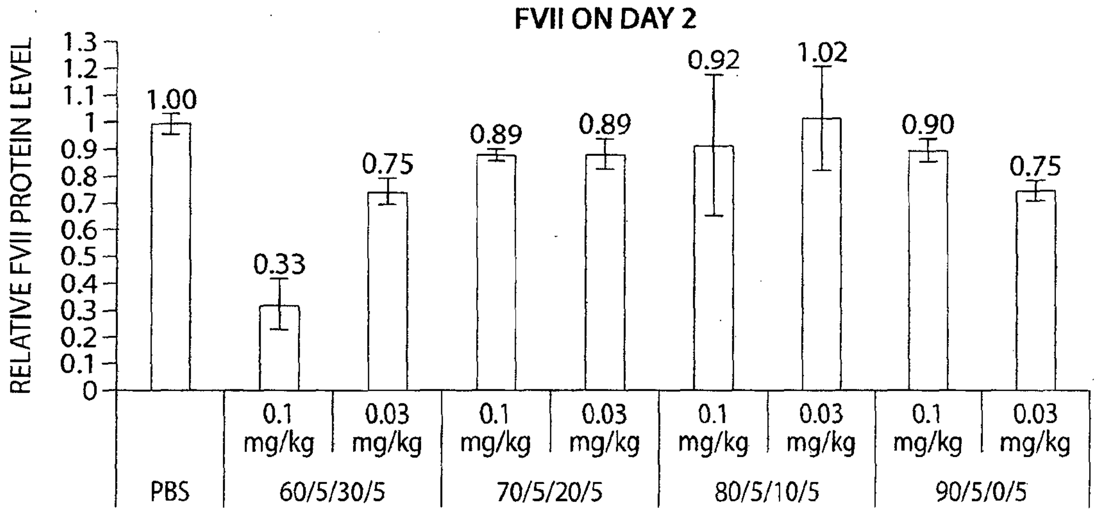
FVII ON DAY 2
RELATIVE FVII PROTEIN LEVEL
1.3
1.2
1.1
1
0.9
0.8
0.7
0.6
0.5
0.4
0.3
0.2
0.1
0
1.00
0.33
0.75
0.89
0.89
0.92
1.02
0.90
0.75
0.1 mg/kg
0.03 mg/kg
PBS
60/5/30/5
70/5/20/5
80/5/10/5
90/5/0/5

Fig. 9

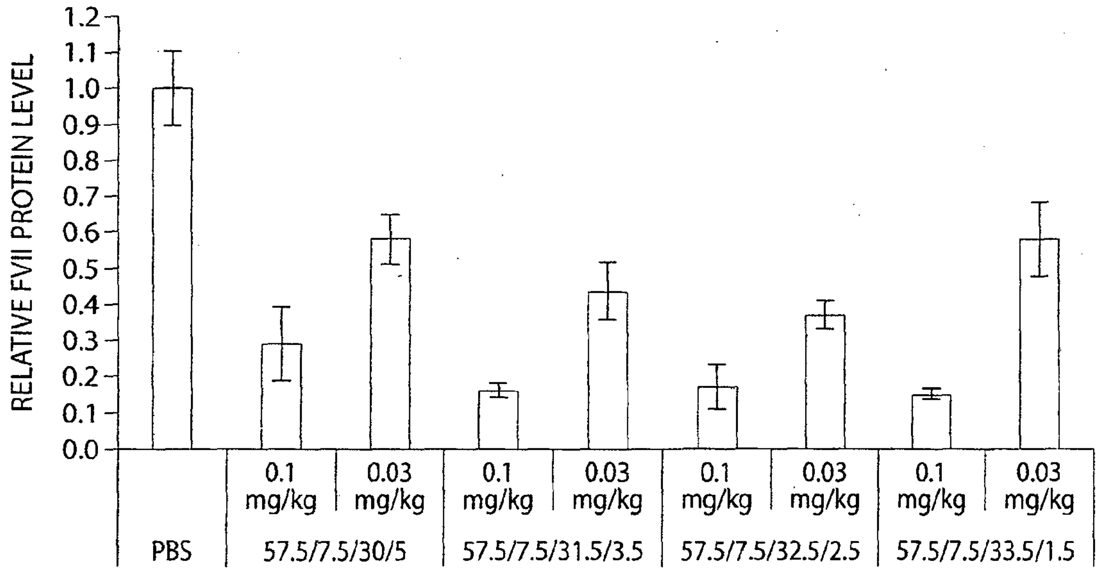
RELATIVE FVII PROTEIN LEVEL
1.2
1.1
1.0
0.9
0.8
0.7
0.6
0.5
0.4
0.3
0.2
0.1
0.0
0.1 mg/kg
0.03 mg/kg
PBS
57.5/7.5/30/5
57.5/7.5/31.5/3.5
57.5/7.5/32.5/2.5
57.5/7.5/33.5/1.5

Fig. 10

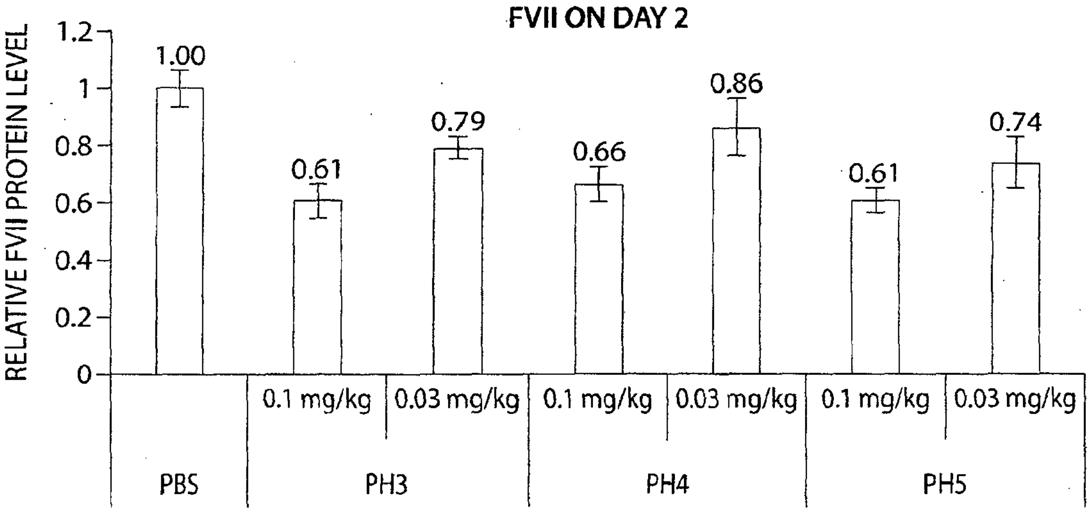
FVII ON DAY 2
RELATIVE FVII PROTEIN LEVEL
1.2
1
0.8
0.6
0.4
0.2
0
1.00
0.61
0.79
0.66
0.86
0.61
0.74
0.1 mg/kg
0.03 mg/kg
0.1 mg/kg
0.03 mg/kg
0.1 mg/kg
0.03 mg/kg
PBS
PH3
PH4
PH5

Fig. 11

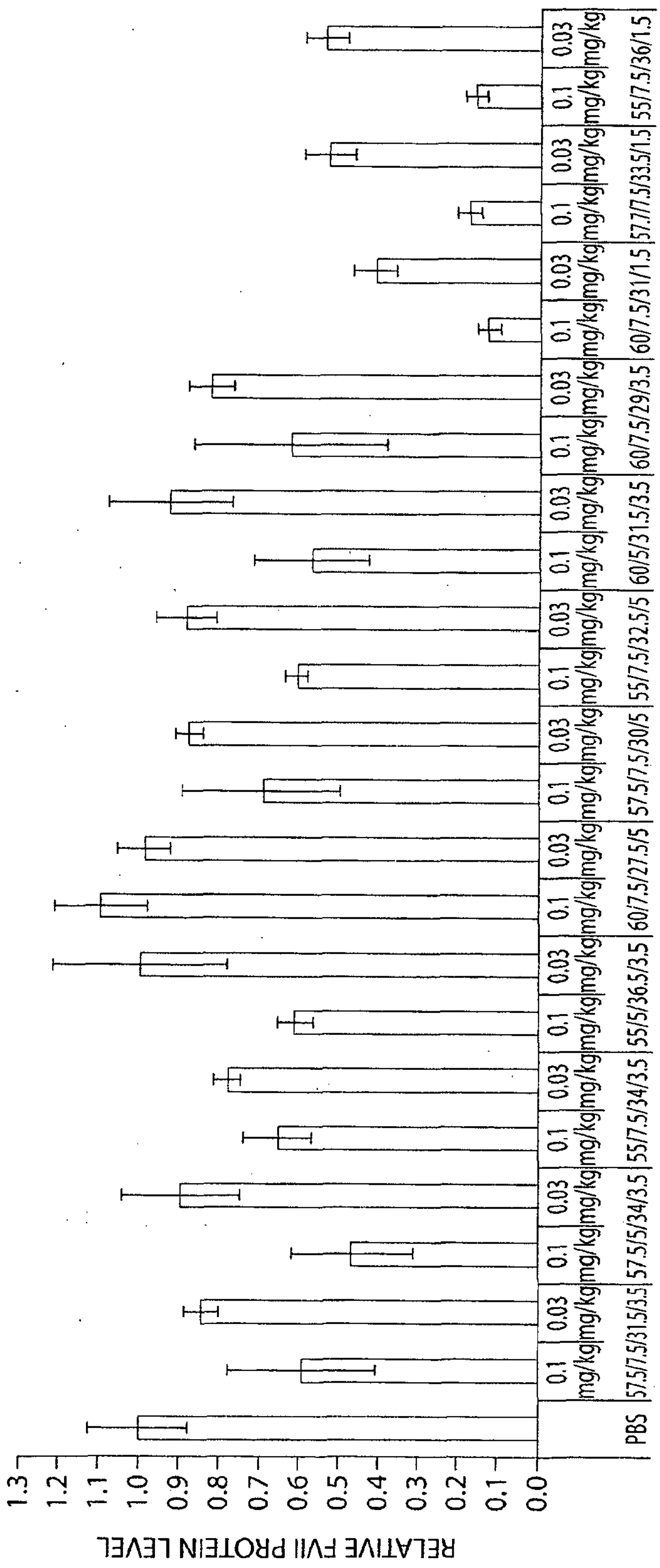
RELATIVE FVII PROTEIN LEVEL
0.0
0.1
0.2
0.3
0.4
0.5
0.6
0.7
0.8
0.9
1.0
1.1
1.2
1.3
PBS
0.1 mg/kg
0.03 mg/kg
57.5/7.5/31.5/3.5
57.5/5/34/3.5
55/7.5/34/3.5
55/5/36.5/3.5
60/7.5/27.5/5
57.5/7.5/30/5
55/7.5/32.5/5
60/5/31.5/3.5
60/7.5/29/3.5
60/7.5/31/1.5
57.7/7.5/33.5/1.5
55/7.5/36/1.5

Fig. 12

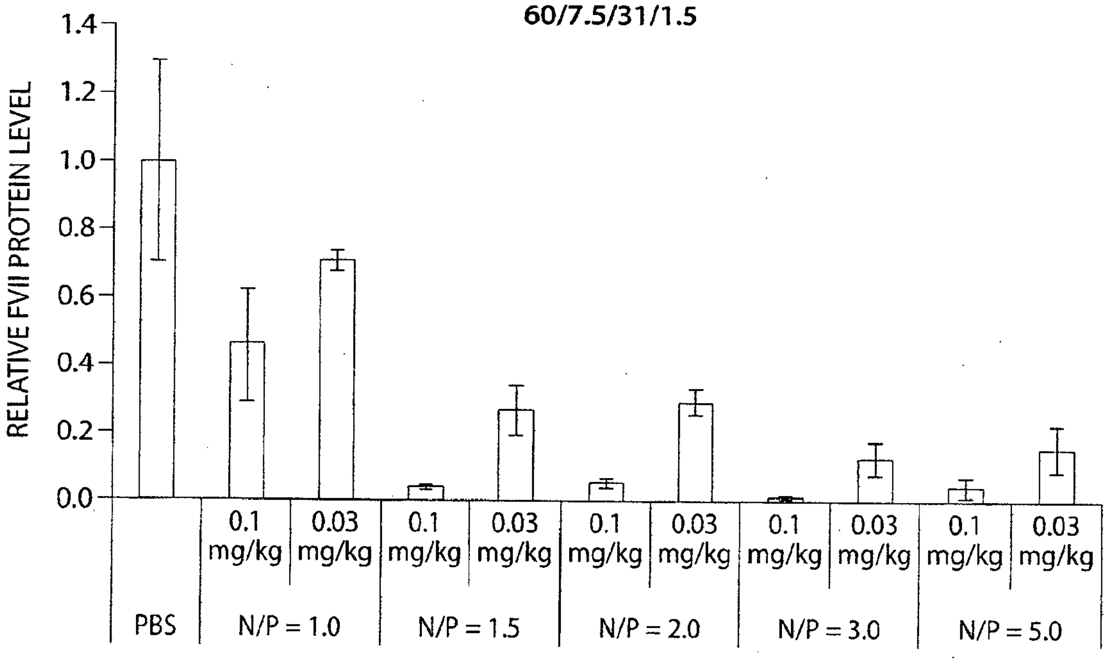
60/7.5/31/1.5
RELATIVE FVII PROTEIN LEVEL
1.4
1.2
1.0
0.8
0.6
0.4
0.2
0.0
0.1 mg/kg
0.03 mg/kg
PBS
N/P = 1.0
N/P = 1.5
N/P = 2.0
N/P = 3.0
N/P = 5.0

Fig. 13

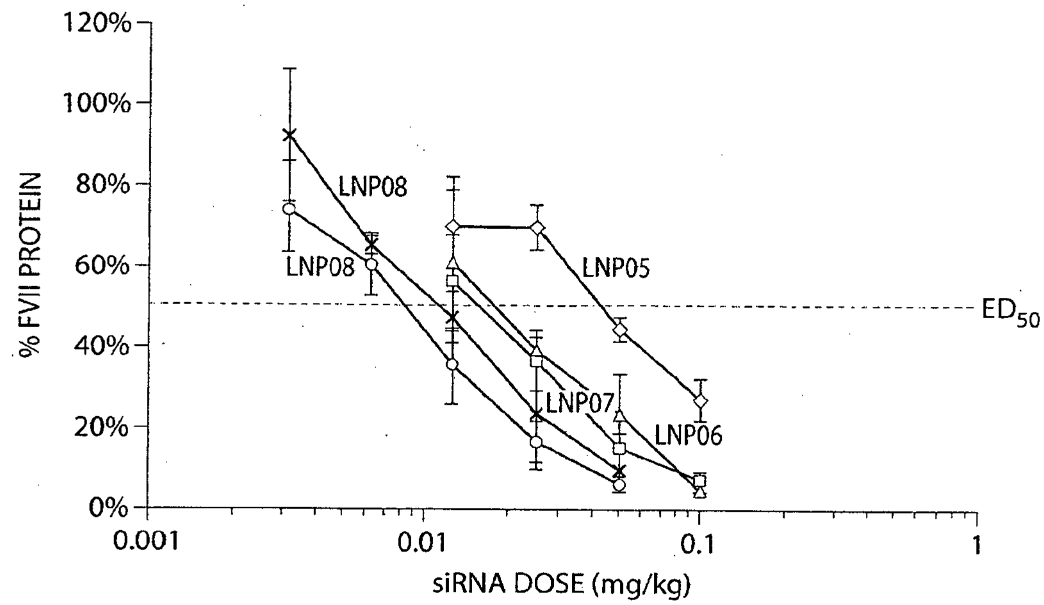
% FVII PROTEIN
120%
100%
80%
60%
40%
20%
0%
0.001
0.01
0.1
1
siRNA DOSE (mg/kg)
LNP08
LNP08
LNP05
LNP07
LNP06
$ED_{50}$

Fig. 14

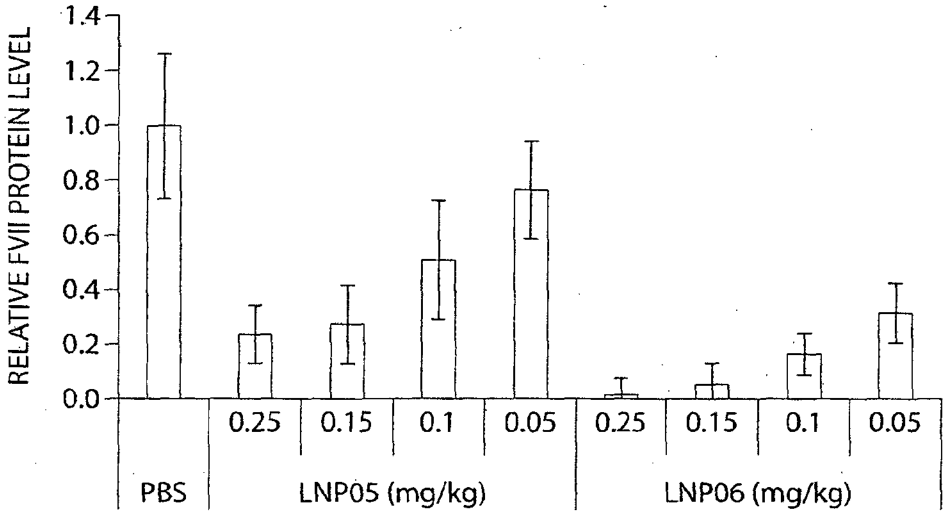


Fig. 15A

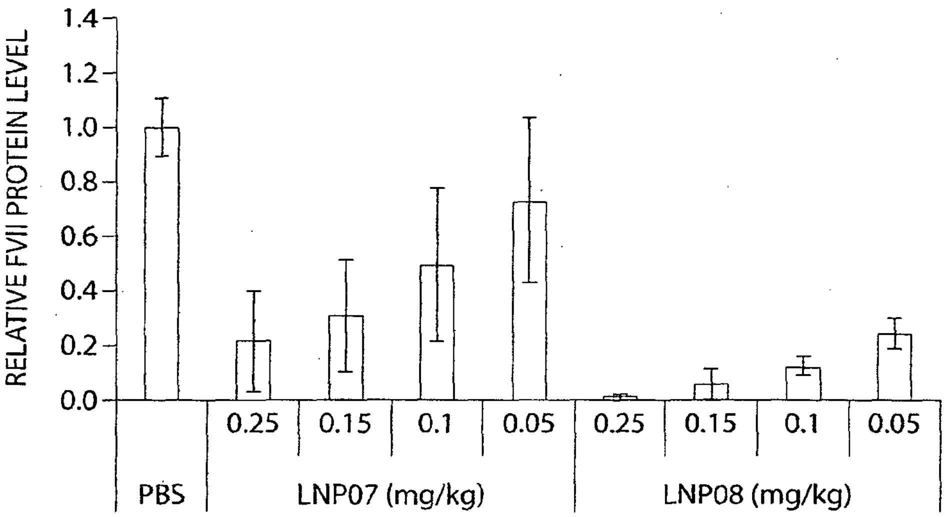


Fig. 15B

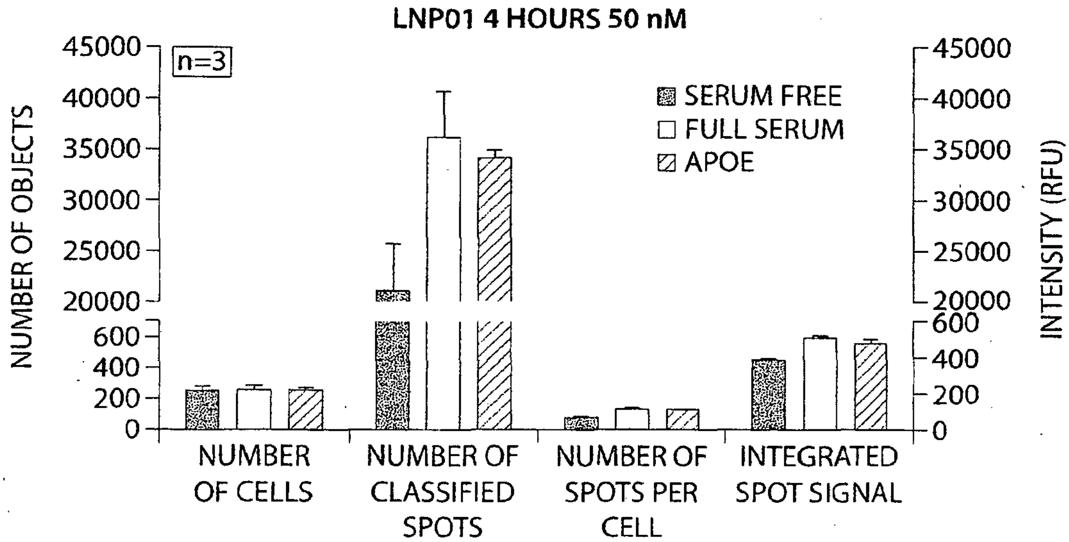


Fig. 16A

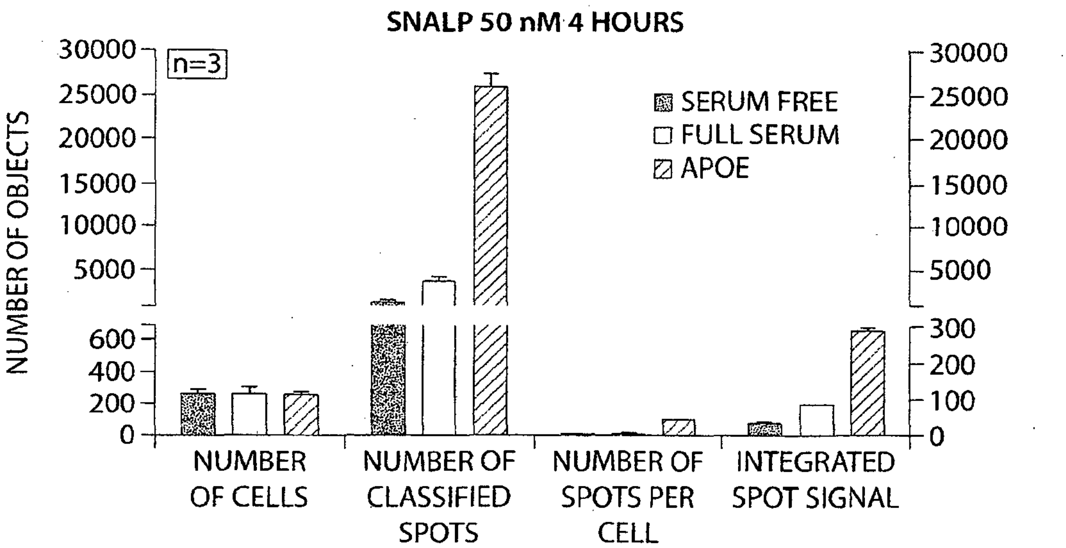


Fig. 16B

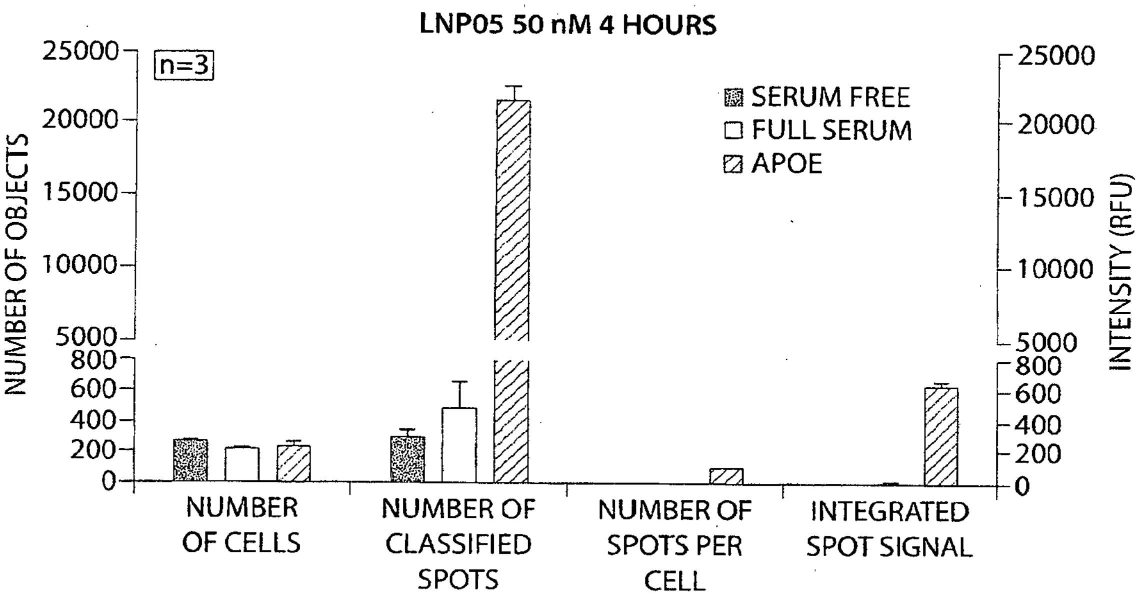
LNP05 50 nM 4 HOURS
n=3
NUMBER OF OBJECTS
INTENSITY (RFU)
25000
20000
15000
10000
5000
800
600
400
200
0
SERUM FREE
FULL SERUM
APOE
NUMBER OF CELLS
NUMBER OF CLASSIFIED SPOTS
NUMBER OF SPOTS PER CELL
INTEGRATED SPOT SIGNAL

Fig. 16C

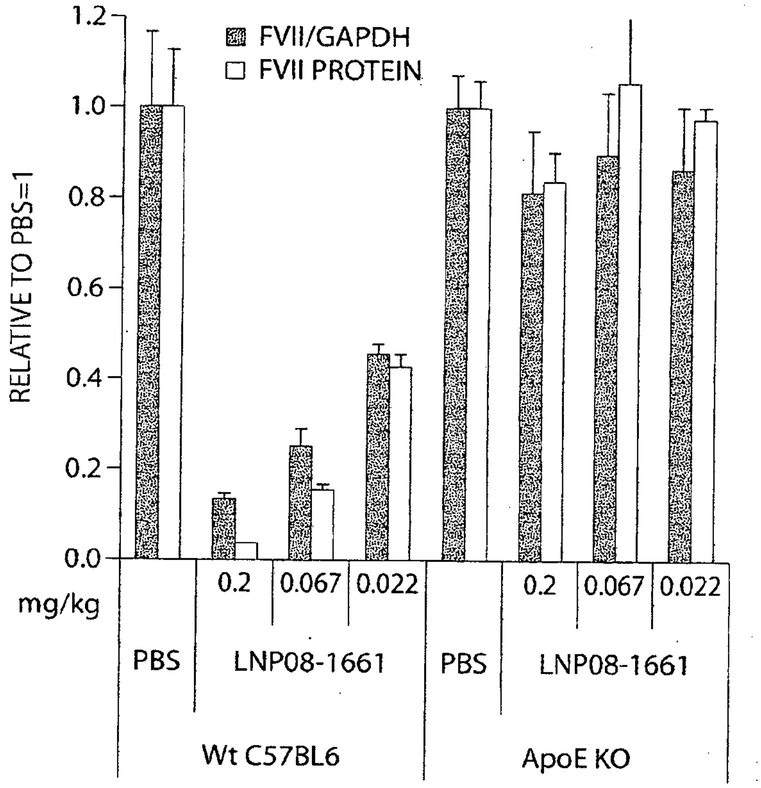
1.2
1.0
0.8
0.6
0.4
0.2
0.0
RELATIVE TO PBS=1
FVII/GAPDH
FVII PROTEIN
mg/kg
0.2
0.067
0.022
0.2
0.067
0.022
PBS
LNP08-1661
PBS
LNP08-1661
Wt C57BL6
ApoE KO

Fig. 17

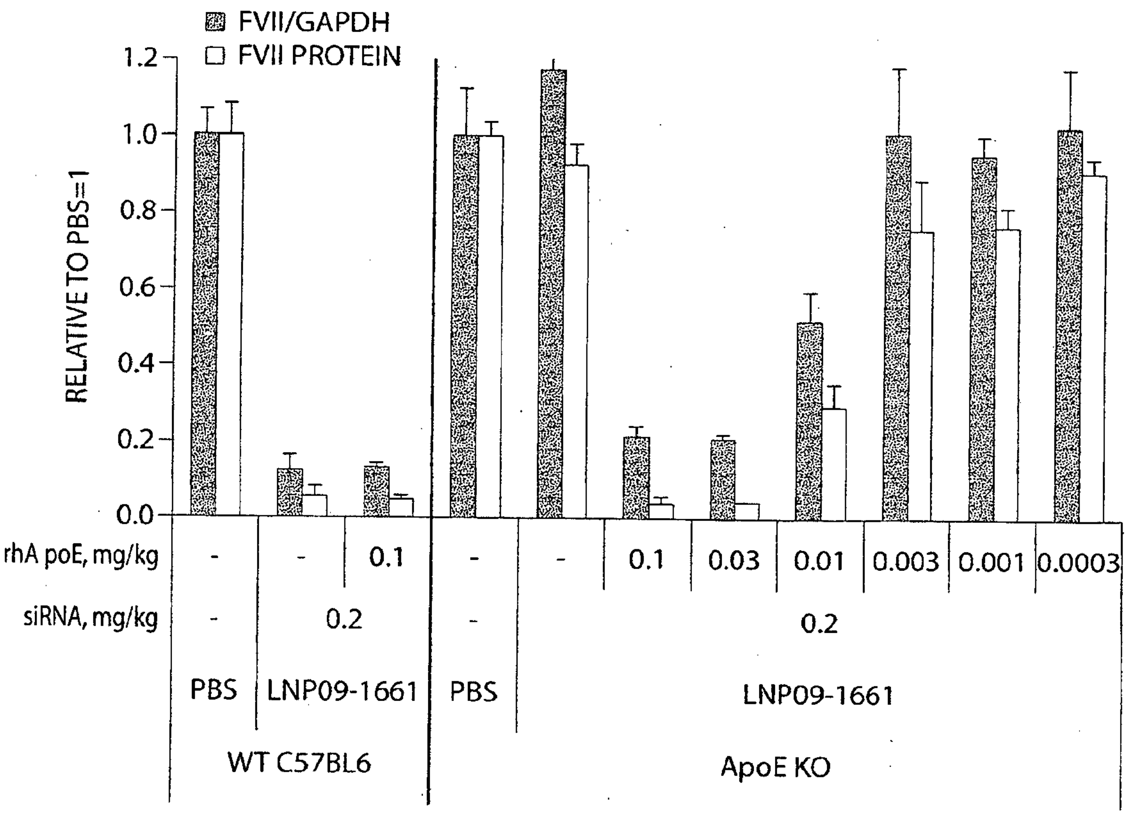
FVII/GAPDH
FVII PROTEIN
RELATIVE TO PBS=1
1.2
1.0
0.8
0.6
0.4
0.2
0.0
rhA poE, mg/kg
siRNA, mg/kg
- - 0.1 - - 0.1 0.03 0.01 0.003 0.001 0.0003
- 0.2 - 0.2
PBS
LNP09-1661
PBS
LNP09-1661
WT C57BL6
ApoE KO

Fig. 18

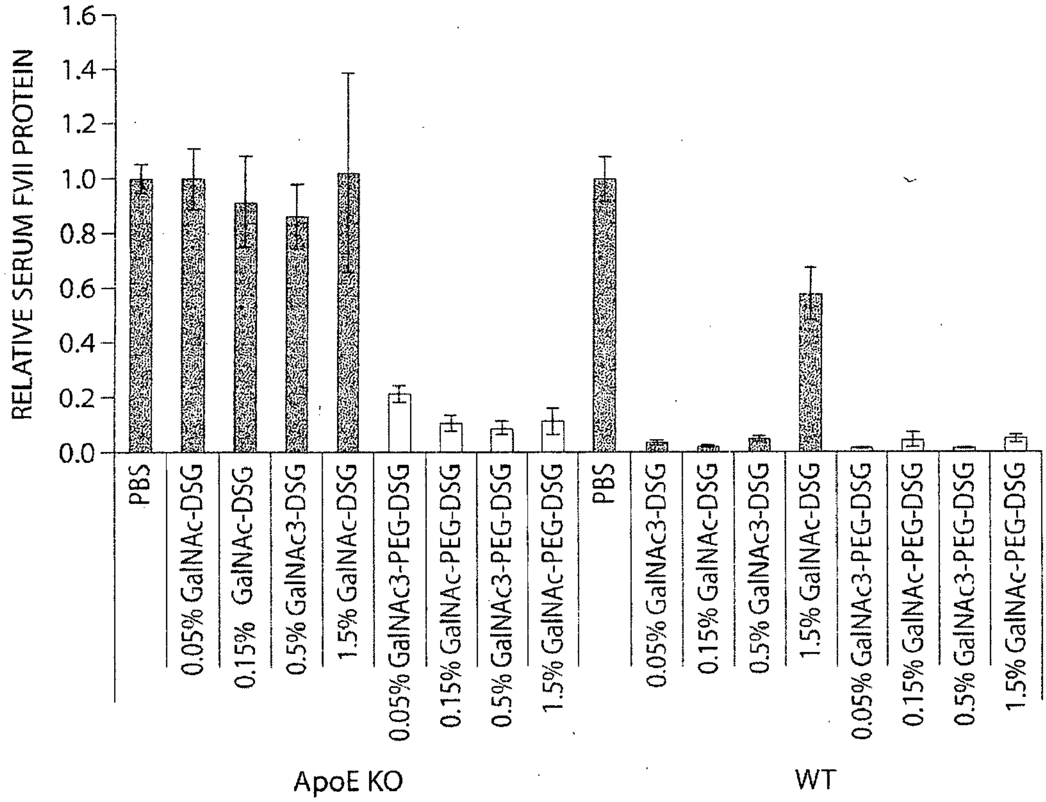

RELATIVE SERUM FVII PROTEIN
1.6
1.4
1.2
1.0
0.8
0.6
0.4
0.2
0.0
PBS
0.05% GalNAc-DSG
0.15% GalNAc-DSG
0.5% GalNAc3-DSG
1.5% GalNAc-DSG
0.05% GalNAc3-PEG-DSG
0.15% GalNAc-PEG-DSG
0.5% GalNAc3-PEG-DSG
1.5% GalNAc-PEG-DSG
PBS
0.05% GalNAc3-DSG
0.15% GalNAc-DSG
0.5% GalNAc3-DSG
1.5% GalNAc-DSG
0.05% GalNAc3-PEG-DSG
0.15% GalNAc-PEG-DSG
0.5% GalNAc3-PEG-DSG
1.5% GalNAc-PEG-DSG
ApoE KO
WT


Fig. 19A

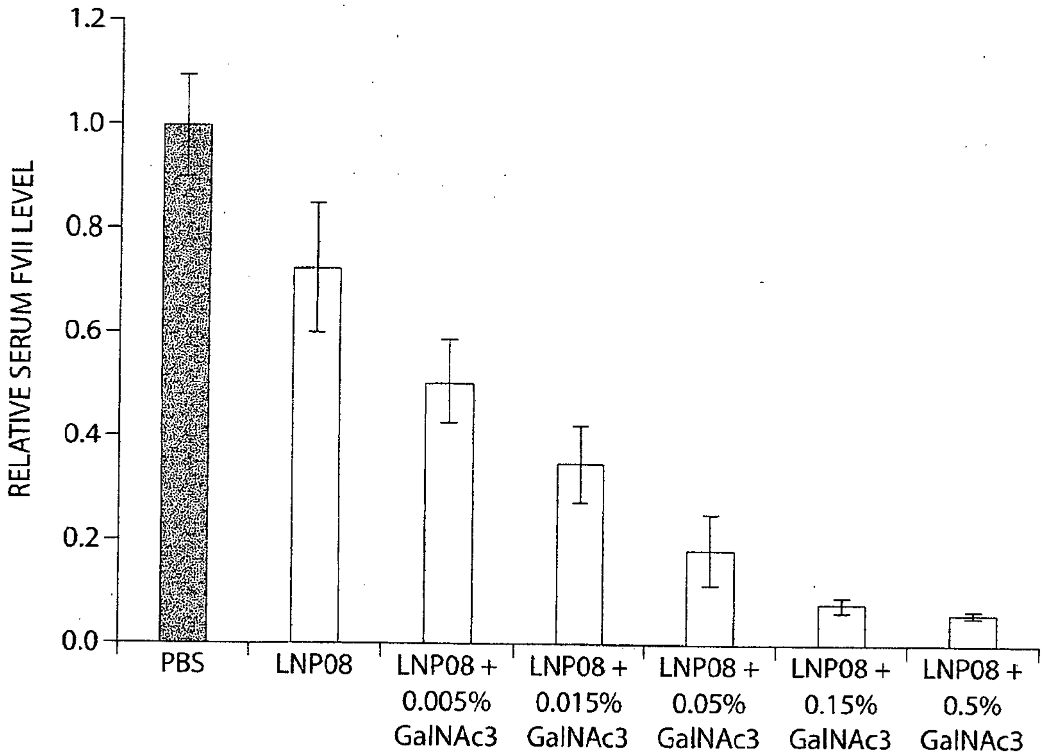
1.2
1.0
0.8
0.6
0.4
0.2
0.0
RELATIVE SERUM FVII LEVEL
PBS
LNP08
LNP08 + 0.005% GalNAc3
LNP08 + 0.015% GalNAc3
LNP08 + 0.05% GalNAc3
LNP08 + 0.15% GalNAc3
LNP08 + 0.5% GalNAc3

Fig. 19B

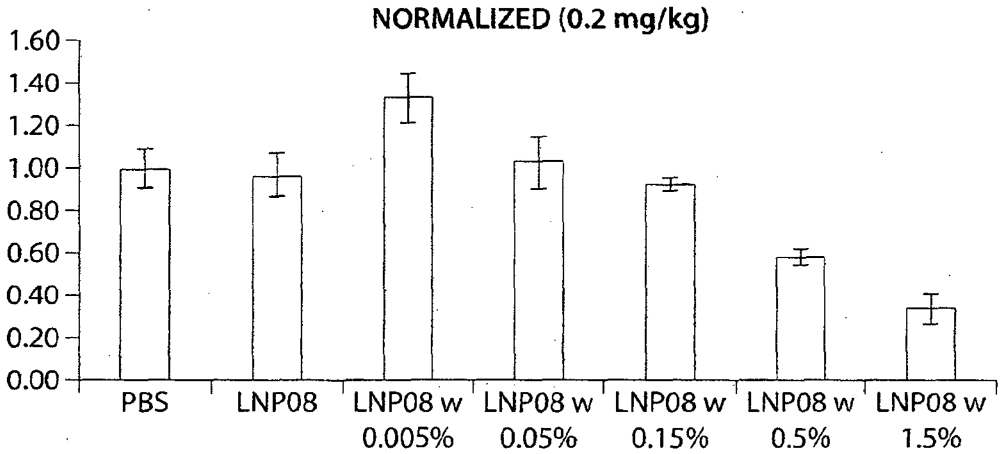
NORMALIZED (0.2 mg/kg)
1.60
1.40
1.20
1.00
0.80
0.60
0.40
0.20
0.00
PBS
LNP08
LNP08 w 0.005%
LNP08 w 0.05%
LNP08 w 0.15%
LNP08 w 0.5%
LNP08 w 1.5%

Fig. 20

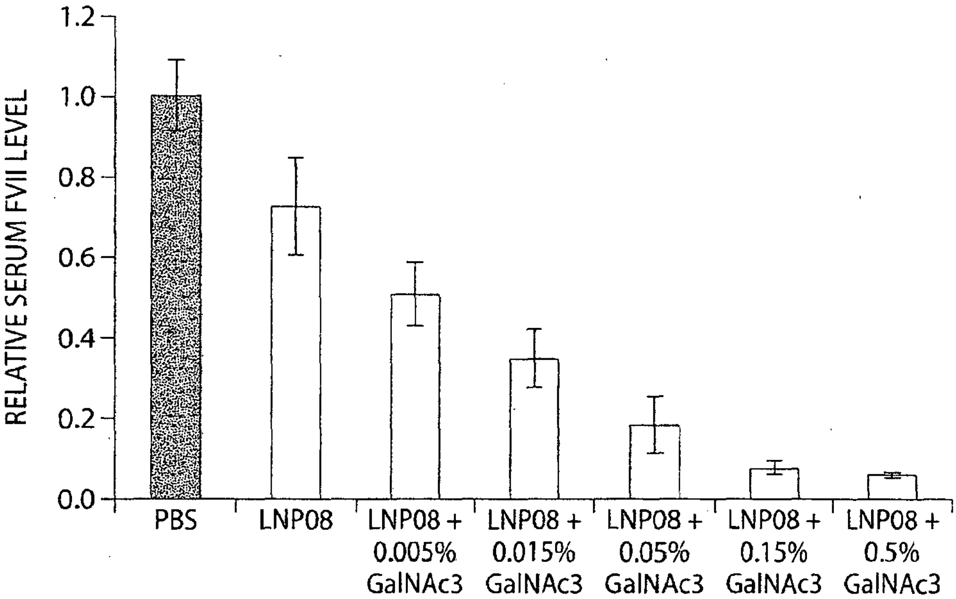
RELATIVE SERUM FVII LEVEL
1.2
1.0
0.8
0.6
0.4
0.2
0.0
PBS
LNP08
LNP08 + 0.005% GalNAc3
LNP08 + 0.015% GalNAc3
LNP08 + 0.05% GalNAc3
LNP08 + 0.15% GalNAc3
LNP08 + 0.5% GalNAc3

Fig. 21

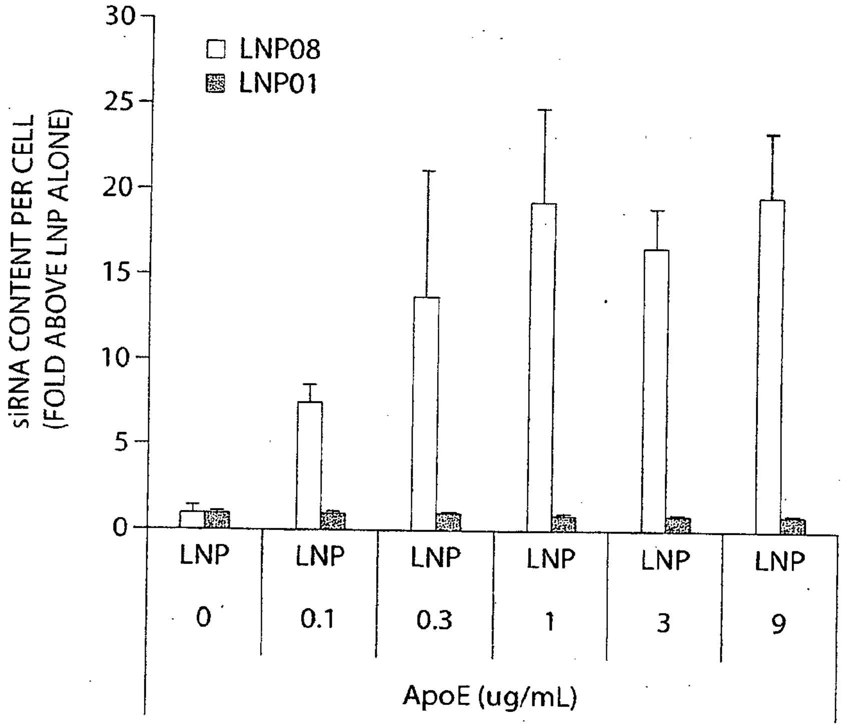
30
25
20
15
10
5
0
siRNA CONTENT PER CELL (FOLD ABOVE LNP ALONE)
LNP08
LNP01
LNP
0
0.1
0.3
1
3
9
ApoE (ug/mL)

Fig. 22

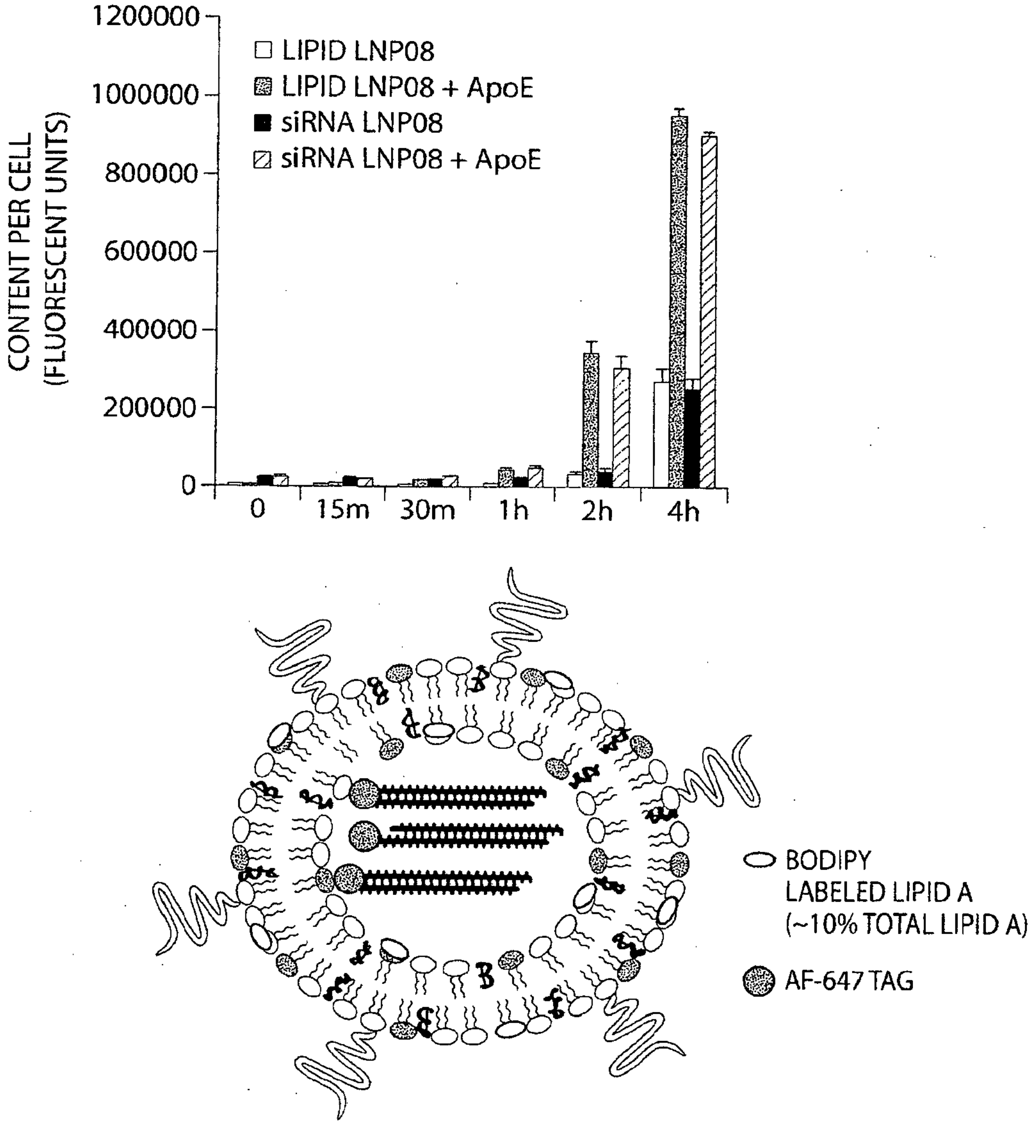


CHEMICAL FORMULA: $C_{64}H_{101}BF_2N_4O_3S$
EXACT MASS: 1054.77
MOLECULAR WEIGHT: 1055.38

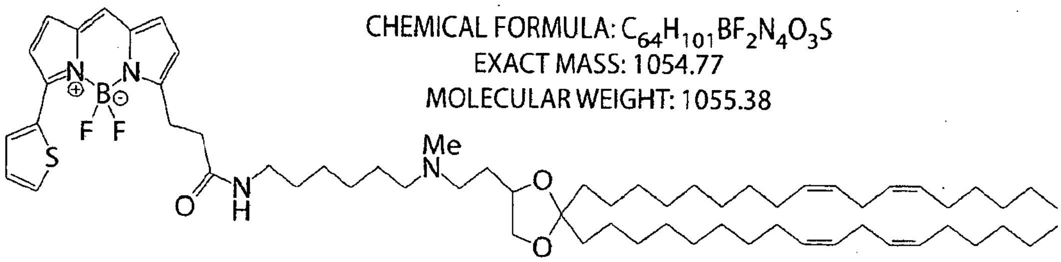

Fig. 23

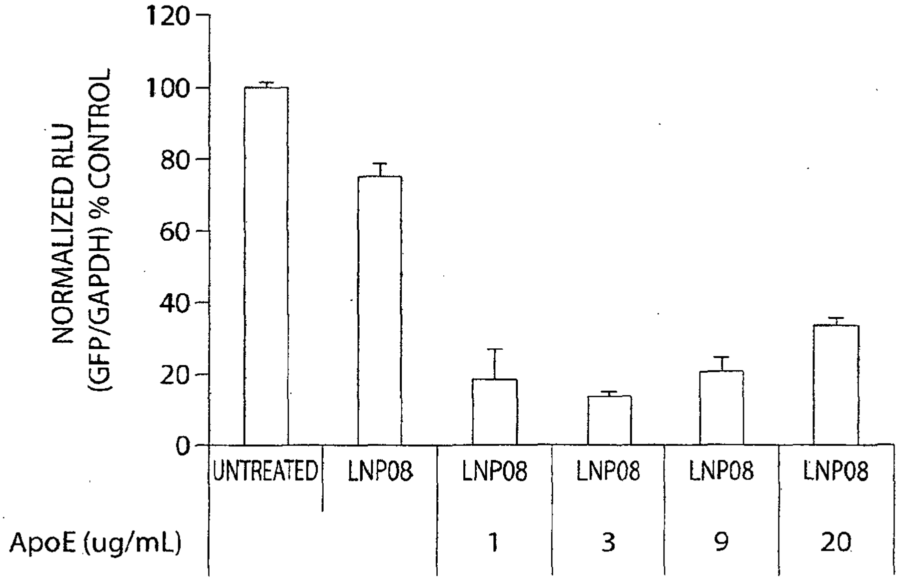
NORMALIZED RLU
(GFP/GAPDH) % CONTROL
120
100
80
60
40
20
0
UNTREATED
LNP08
LNP08
LNP08
LNP08
LNP08
ApoE (ug/mL)
1
3
9
20

Fig. 24

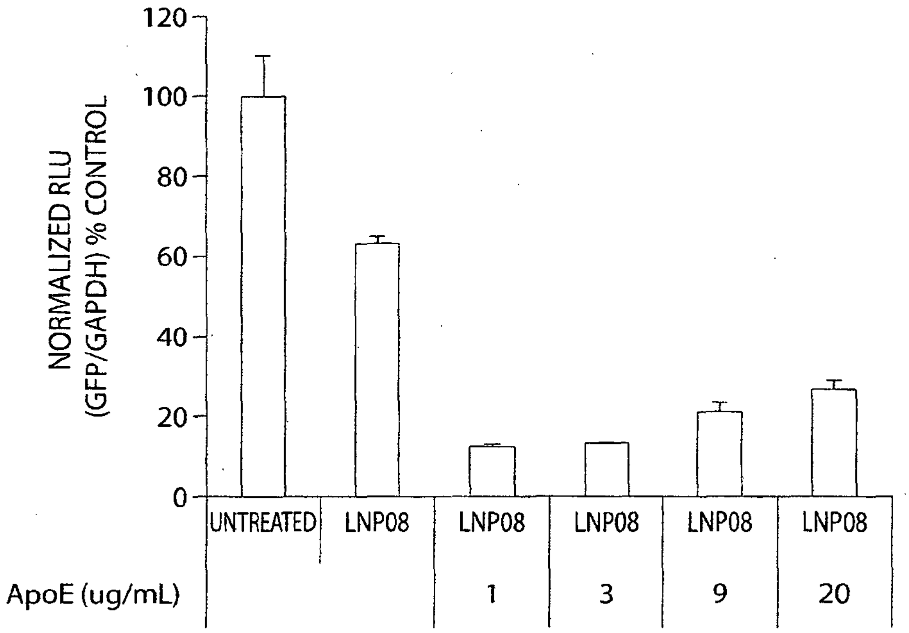
NORMALIZED RLU
(GFP/GAPDH) % CONTROL
120
100
80
60
40
20
0
UNTREATED
LNP08
LNP08
LNP08
LNP08
LNP08
ApoE (ug/mL)
1
3
9
20

Fig. 25

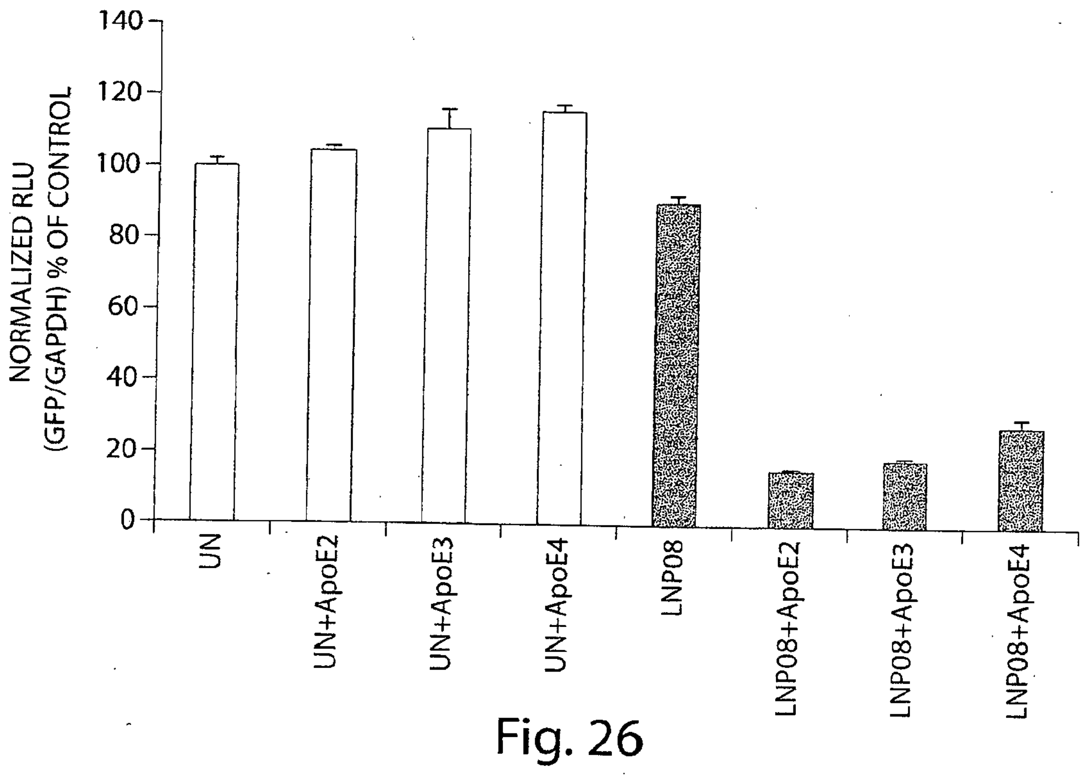
NORMALIZED RLU (GFP/GAPDH) % OF CONTROL
140
120
100
80
60
40
20
0
UN
UN+ApoE2
UN+ApoE3
UN+ApoE4
LNP08
LNP08+ApoE2
LNP08+ApoE3
LNP08+ApoE4

Fig. 26

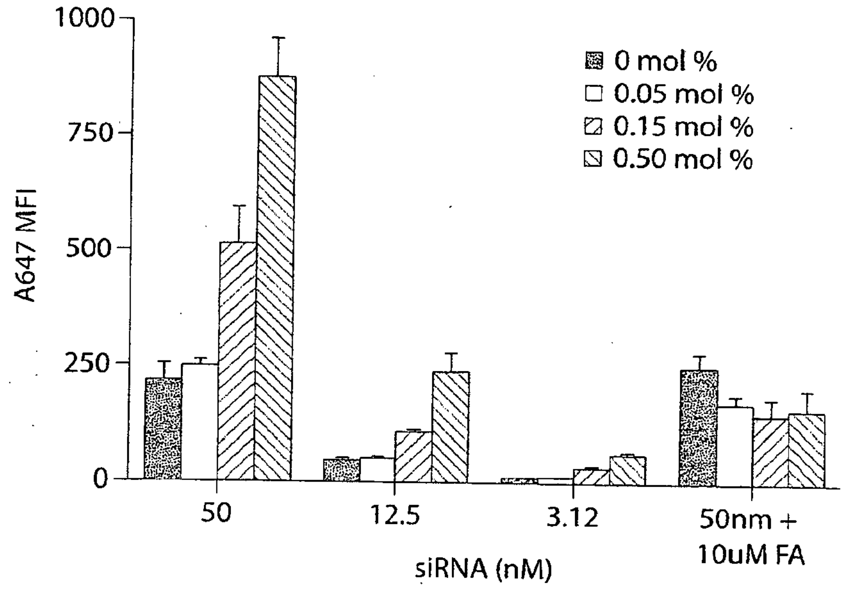
A647 MFI
1000
750
500
250
0
0 mol %
0.05 mol %
0.15 mol %
0.50 mol %
50
12.5
3.12
50nm + 10uM FA
siRNA (nM)

Fig. 27

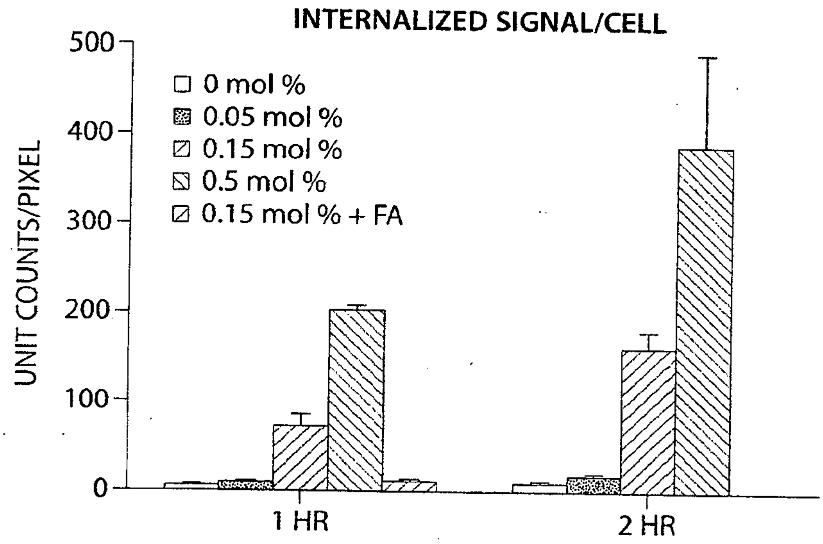

INTERNALIZED SIGNAL/CELL
UNIT COUNTS/PIXEL
500
400
300
200
100
0
0 mol %
0.05 mol %
0.15 mol %
0.5 mol %
0.15 mol % + FA
1 HR
2 HR


Fig. 28

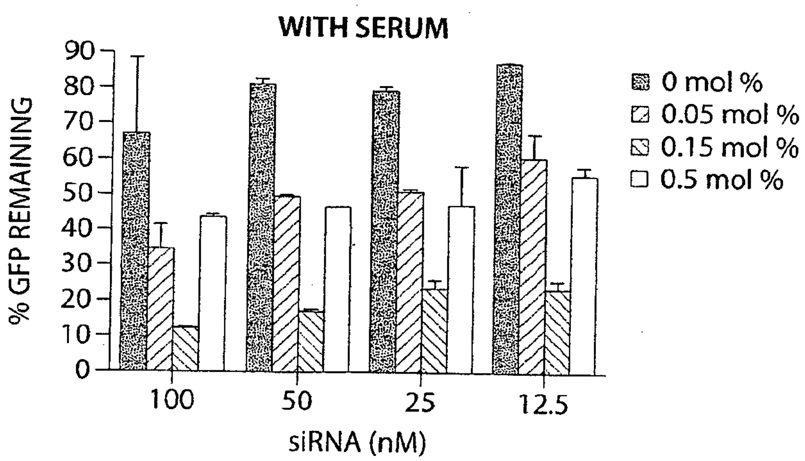


Fig. 29A

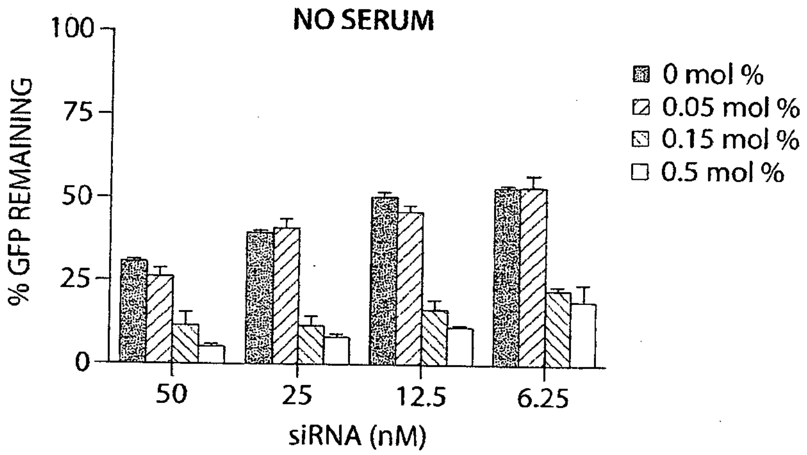


Fig. 29B

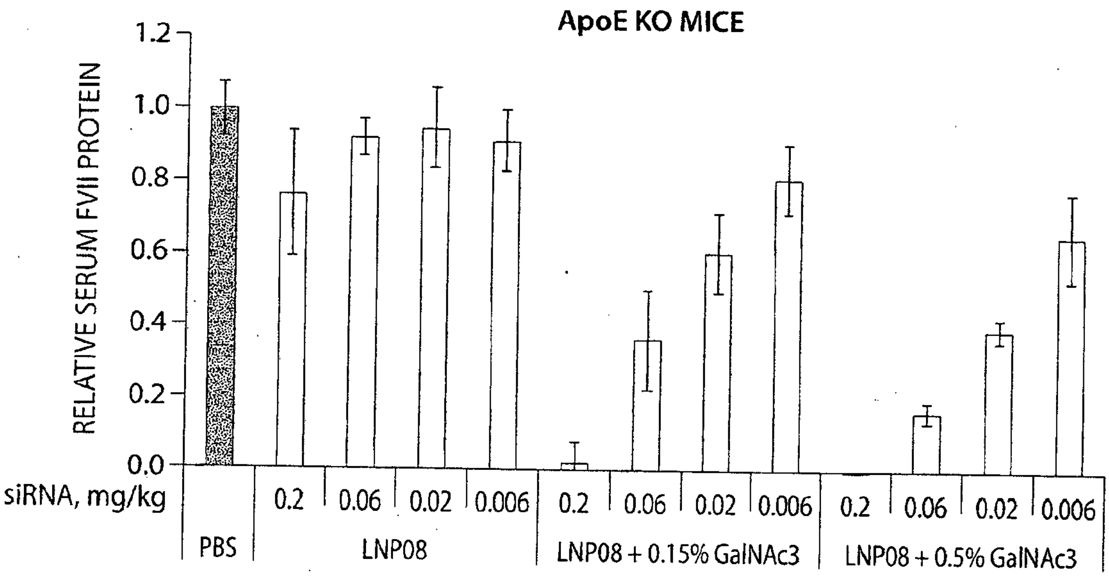
ApoE KO MICE
RELATIVE SERUM FVII PROTEIN
1.2
1.0
0.8
0.6
0.4
0.2
0.0
siRNA, mg/kg
0.2
0.06
0.02
0.006
0.2
0.06
0.02
0.006
0.2
0.06
0.02
0.006
PBS
LNP08
LNP08 + 0.15% GalNAc3
LNP08 + 0.5% GalNAc3

Fig. 30

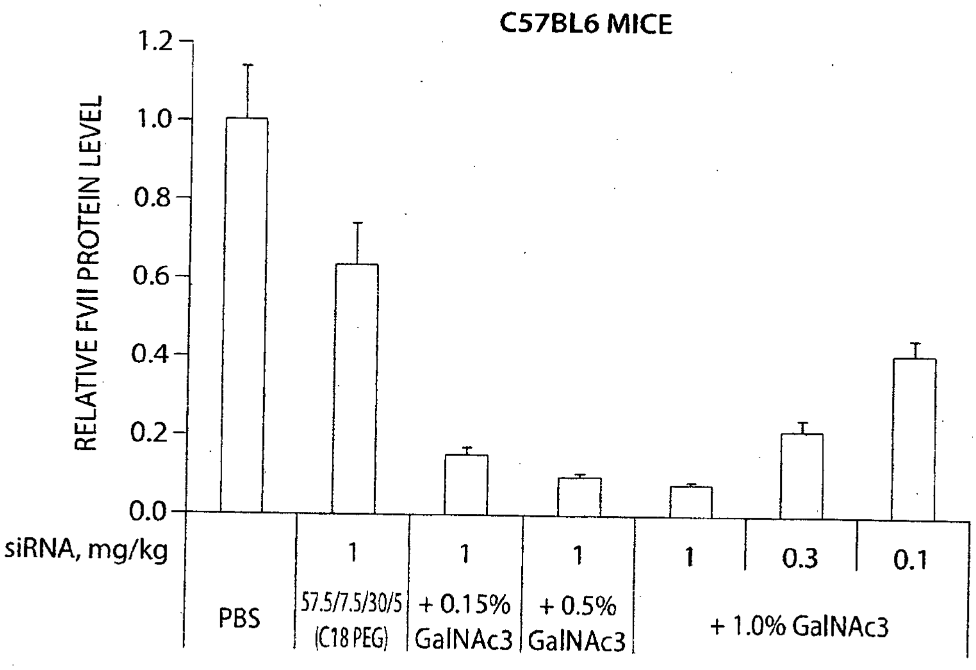
C57BL6 MICE
RELATIVE FVII PROTEIN LEVEL
1.2
1.0
0.8
0.6
0.4
0.2
0.0
siRNA, mg/kg
1
1
1
1
0.3
0.1
PBS
57.5/7.5/30/5 (C18 PEG)
+ 0.15% GalNAc3
+ 0.5% GalNAc3
+ 1.0% GalNAc3

Fig. 31

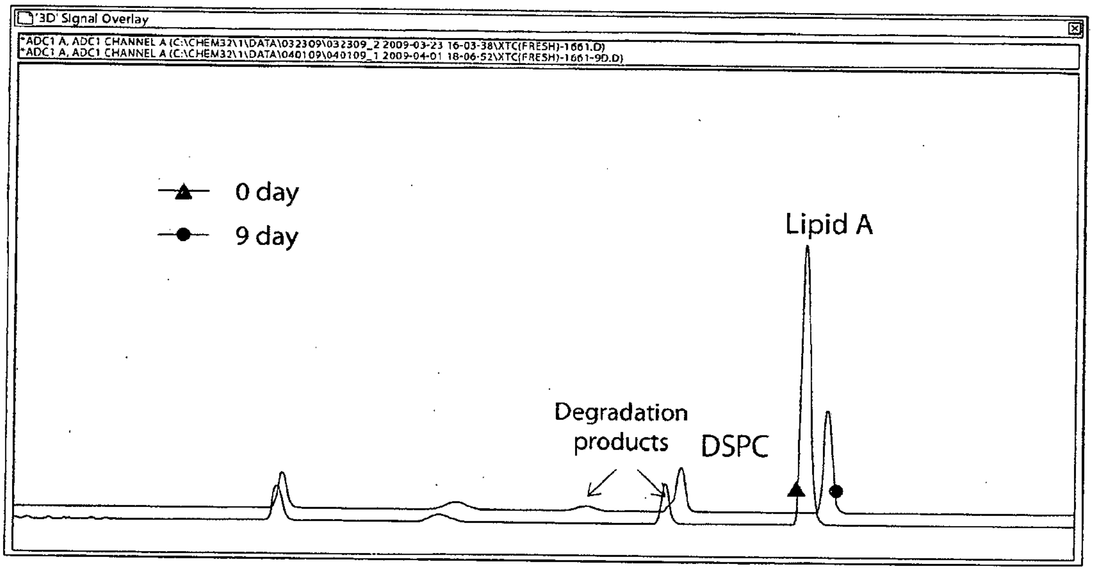
'3D' Signal Overlay
0 day
9 day
Lipid A
Degradation products
DSPC

Fig. 32

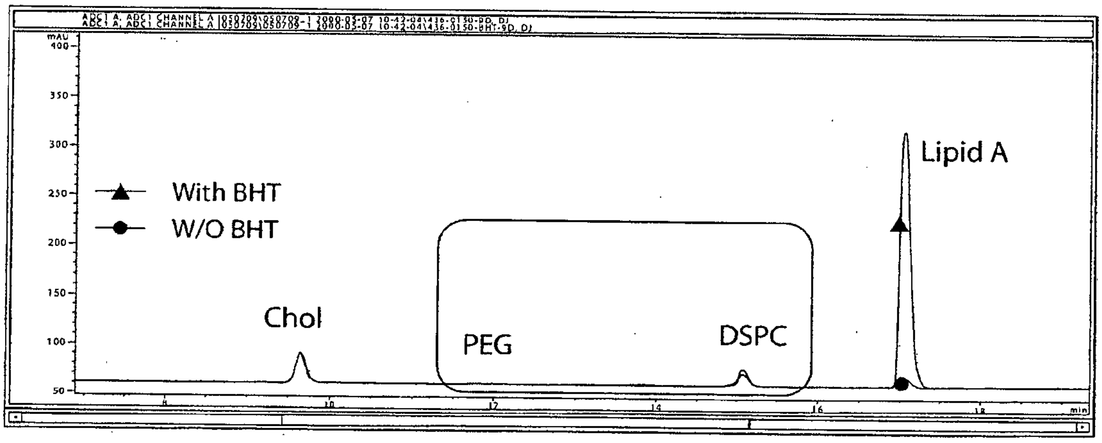
mAU
400
350
300
250
200
150
100
50
With BHT
W/O BHT
Lipid A
Chol
PEG
DSPC
10
12
14
16
18
min

Fig. 33

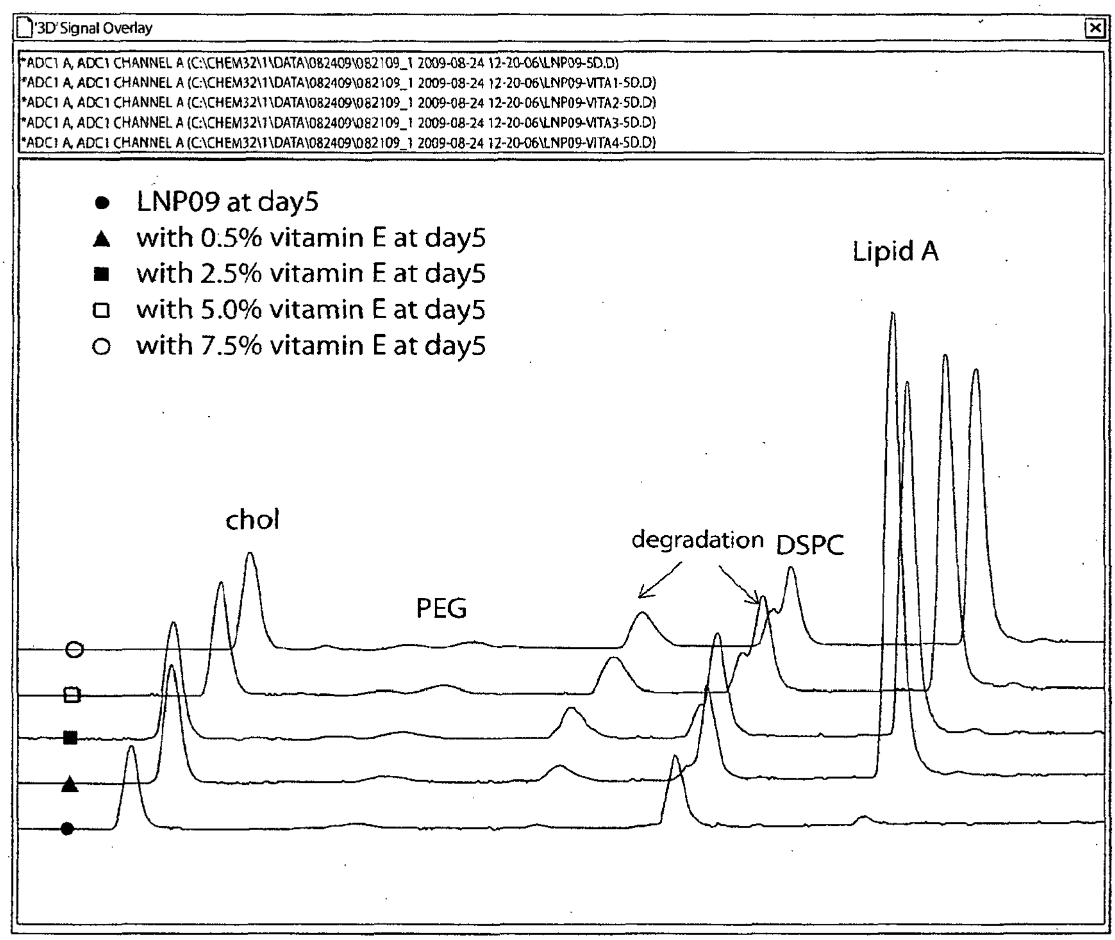
'3D' Signal Overlay
*ADC1 A, ADC1 CHANNEL A (C:\CHEM32\1\DATA\082409\082109_1 2009-08-24 12-20-06\LNP09-5D.D)
*ADC1 A, ADC1 CHANNEL A (C:\CHEM32\1\DATA\082409\082109_1 2009-08-24 12-20-06\LNP09-VITA1-5D.D)
*ADC1 A, ADC1 CHANNEL A (C:\CHEM32\1\DATA\082409\082109_1 2009-08-24 12-20-06\LNP09-VITA2-5D.D)
*ADC1 A, ADC1 CHANNEL A (C:\CHEM32\1\DATA\082409\082109_1 2009-08-24 12-20-06\LNP09-VITA3-5D.D)
*ADC1 A, ADC1 CHANNEL A (C:\CHEM32\1\DATA\082409\082109_1 2009-08-24 12-20-06\LNP09-VITA4-5D.D)
● LNP09 at day5
▲ with 0.5% vitamin E at day5
■ with 2.5% vitamin E at day5
□ with 5.0% vitamin E at day5
○ with 7.5% vitamin E at day5
Lipid A
chol
degradation
DSPC
PEG

Fig. 34

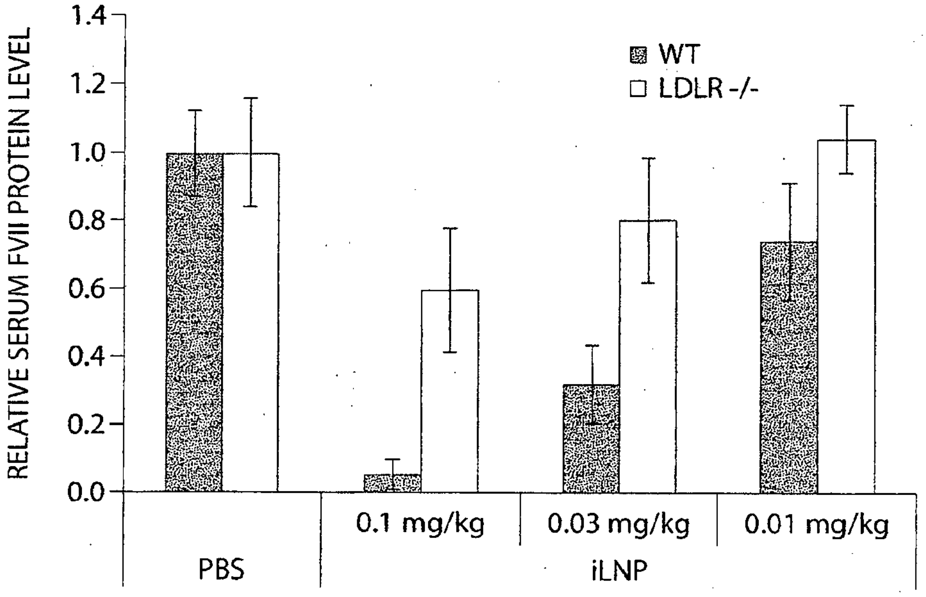
RELATIVE SERUM FVII PROTEIN LEVEL
1.4
1.2
1.0
0.8
0.6
0.4
0.2
0.0
WT
LDLR -/-
0.1 mg/kg
0.03 mg/kg
0.01 mg/kg
PBS
iLNP

Fig. 35

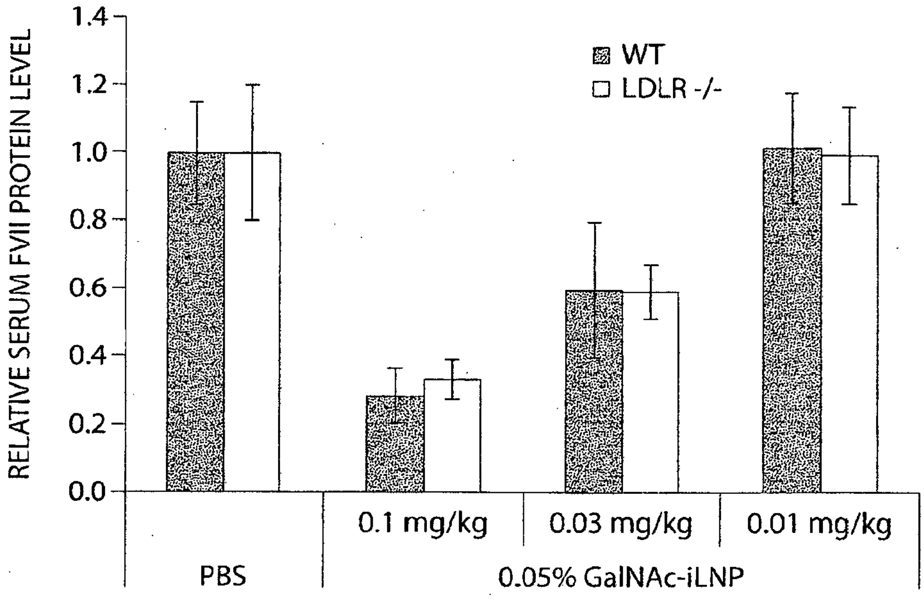
RELATIVE SERUM FVII PROTEIN LEVEL
1.4
1.2
1.0
0.8
0.6
0.4
0.2
0.0
WT
LDLR -/-
0.1 mg/kg
0.03 mg/kg
0.01 mg/kg
PBS
0.05% GalNAc-iLNP

Fig. 36

Europäisches Patentamt
European Patent Office
Office européen des brevets

# EUROPEAN SEARCH REPORT

Application Number
EP 17 00 0214

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | AKINC A ET AL: "A combinatorial library of lipid-like materials for delivery of RNAi therapeutics", NATURE BIOTECHNOLOGY MAY 2008 NATURE PUBLISHING GROUP US, vol. 26, no. 5, May 2008 (2008-05), pages 561-569, XP002527175, DOI: DOI:10.1038/NBT1402 * abstract * * figures 1,3,5 * * page 568, left-hand column, line 20 - right-hand column, line 38 * ----- | 1-15 | INV. A61K9/127 A61K48/00 |
| T | WO 2010/036962 A1 (ALNYLAM PHARMACEUTICALS INC [US]; DE FOUGEROLLES ANTONIN [US]; NOVOBRA) 1 April 2010 (2010-04-01) * page 40, line 28 - page 41, line 32 * * table page 43 * ----- | | |
| A,P | WO 2009/088891 A1 (ALNYLAM PHARMACEUTICALS INC [US]; MADDEN THOMAS D [CA]; CIUFOLINI MARC) 16 July 2009 (2009-07-16) * page 5, formula DLin-K-DMA * * page 29, lines 1-21 * * table 6 * ----- | 1-15 | TECHNICAL FIELDS SEARCHED (IPC) A61K |
| A,P | WO 2009/086558 A1 (TEKMIRA PHARMACEUTICALS CORP [CA]; UNIV BRITISH COLUMBIA [CA]; ALNYLAM) 9 July 2009 (2009-07-09) * page 34, line 21 - page 35, line 10 * * claims; examples * * figure 6 * ----- -/-- | 1-15 | |

The present search report has been drawn up for all claims

2

| Place of search | Date of completion of the search | Examiner |
|---|---|---|
| Munich | 29 September 2017 | Hillers, Nathalie |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

page 1 of 2

Europäisches Patentamt
European Patent Office
Office européen des brevets

# EUROPEAN SEARCH REPORT

Application Number
EP 17 00 0214

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,P | WO 2009/132131 A1 (ALNYLAM PHARMACEUTICALS INC [US]; MANOHARAN MUTHIAH [US]; RAJEEV KALLA) 29 October 2009 (2009-10-29)<br>* abstract *<br>* examples *<br>* page 8, line 18 - page 9, line 28 *<br>* figure 1 *<br>----- | 1-15 | |
| T | WO 2010/048536 A2 (ALNYLAM PHARMACEUTICALS INC [US]; MANOHARAN MUTHIAH [US]; RAJEEV KALLA) 29 April 2010 (2010-04-29)<br>* page 28, line 10 - page 29, line 29; examples *<br>* figures 1,2 *<br>----- | | |

TECHNICAL FIELDS SEARCHED (IPC)

The present search report has been drawn up for all claims

| Place of search | Date of completion of the search | Examiner |
|---|---|---|
| Munich | 29 September 2017 | Hillers, Nathalie |

2

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

## ANNEX TO THE EUROPEAN SEARCH REPORT ON EUROPEAN PATENT APPLICATION NO. EP 17 00 0214

This annex lists the patent family members relating to the patent documents cited in the above-mentioned European search report. The members are as contained in the European Patent Office EDP file on
The European Patent Office is in no way liable for these particulars which are merely given for the purpose of information.

29-09-2017

| Patent document cited in search report | Publication date | Patent family member(s) | Publication date |
|---|---|---|---|
| WO 2010036962 A1 | 01-04-2010 | AU 2009296395 A1 | 01-04-2010 |
| | | CA 2739170 A1 | 01-04-2010 |
| | | EP 2334793 A1 | 22-06-2011 |
| | | EP 3109321 A1 | 28-12-2016 |
| | | HK 1157380 A1 | 28-04-2017 |
| | | JP 5529142 B2 | 25-06-2014 |
| | | JP 2012503493 A | 09-02-2012 |
| | | US 2011263684 A1 | 27-10-2011 |
| | | US 2014179759 A1 | 26-06-2014 |
| | | US 2016194633 A1 | 07-07-2016 |
| | | WO 2010036962 A1 | 01-04-2010 |
| WO 2009088891 A1 | 16-07-2009 | AU 2008347250 A1 | 16-07-2009 |
| | | CA 2711236 A1 | 16-07-2009 |
| | | US 2011097720 A1 | 28-04-2011 |
| | | US 2012225434 A1 | 06-09-2012 |
| | | US 2014295449 A1 | 02-10-2014 |
| | | US 2016274089 A1 | 22-09-2016 |
| | | WO 2009088891 A1 | 16-07-2009 |
| WO 2009086558 A1 | 09-07-2009 | CA 2709875 A1 | 09-07-2009 |
| | | EP 2224912 A1 | 08-09-2010 |
| | | EP 3100718 A1 | 07-12-2016 |
| | | JP 5749494 B2 | 15-07-2015 |
| | | JP 2011509258 A | 24-03-2011 |
| | | JP 2014132014 A | 17-07-2014 |
| | | US 2011117125 A1 | 19-05-2011 |
| | | WO 2009086558 A1 | 09-07-2009 |
| WO 2009132131 A1 | 29-10-2009 | NONE | |
| WO 2010048536 A2 | 29-04-2010 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61148366 A
- US 61156851 B
- US 61185712 B
- US 61228373 B
- US 61239686 B
- US 5532130 A
- US 12328669 B
- US 328669 A
- WO 2008042973 A
- US 5008050 A
- US 4927637 A
- US 4737323 A
- US 6534018 B
- US 6855277 B
- US 20070042031 A
- US 5672685 A
- US 5525472 A
- US 5473039 A
- US 5182364 A
- US 5177189 A
- US 5168045 A
- US 5116739 A
- US 6004925 A
- US 6037323 A
- US 6046166 A
- US 6372886 B
- US 5059528 A
- US 5834596 A
- US 5876968 A
- US 5721114 A
- WO 8604920 A
- WO 8702062 A
- US 5840688 A
- US 6320017 B
- US 5885613 A
- US 5820873 A
- US 5534499 A
- US 08486214 B
- US 486214 A
- US 4957773 A
- US 4603044 A
- US 5013556 A
- US 6027726 A
- US 5739119 A
- US 5759829 A
- US 5801154 A
- US 5789573 A
- US 5718709 A
- US 5610288 A
- US 5747470 A
- US 5591317 A
- US 5783683 A
- EP 0360257 A
- US 5631359 A
- US 4987071 A
- WO 9323569 A
- WO 9402595 A
- WO 9207065 A
- WO 9315187 A
- WO 9103162 A
- EP 92110298 A
- US 5334711 A
- WO 9413688 A
- US 379343 P
- US 649527 A
- WO 02069369 A
- WO 0115726 A
- US 6406705 B
- US 3687808 A
- US 4469863 A
- US 4476301 A
- US 5023243 A
- US 5177196 A
- US 5188897 A
- US 5264423 A
- US 5276019 A
- US 5278302 A
- US 5286717 A
- US 5321131 A
- US 5399676 A
- US 5405939 A
- US 5453496 A
- US 5455233 A
- US 5466677 A
- US 5476925 A
- US 5519126 A
- US 5536821 A
- US 5541306 A
- US 5550111 A
- US 5563253 A
- US 5571799 A
- US 5587361 A
- US 5625050 A
- US 5034506 A
- US 5166315 A
- US 5185444 A
- US 5214134 A
- US 5216141 A

- US 5235033 A
- US 5264562 A
- US 5264564 A
- US 5405938 A
- US 5434257 A
- US 5470967 A
- US 5489677 A
- US 5541307 A
- US 5561225 A
- US 5596086 A
- US 5602240 A
- US 5610289 A
- US 5608046 A
- US 5618704 A
- US 5623070 A
- US 5663312 A
- US 5633360 A
- US 5677437 A
- US 5677439 A
- US 4845205 A
- US 5130302 A
- US 5134066 A
- US 5175273 A
- US 5367066 A
- US 5432272 A
- US 5457187 A
- US 5459255 A
- US 5484908 A
- US 5502177 A
- US 5525711 A
- US 5552540 A
- US 5587469 A
- US 5594121 A
- US 5596091 A
- US 5614617 A
- US 5681941 A
- US 4981957 A
- US 5118800 A
- US 5319080 A
- US 5359044 A
- US 5393878 A
- US 5446137 A
- US 5466786 A
- US 5514785 A
- US 5519134 A
- US 5567811 A
- US 5576427 A
- US 5591722 A
- US 5597909 A
- US 5610300 A
- US 5627053 A
- US 5639873 A
- US 5646265 A
- US 5658873 A
- US 5670633 A
- US 5700920 A
- US 5539082 A
- US 5714331 A
- US 5719262 A
- US 6287591 B
- US 6858225 B
- US 5976567 A
- US 5286634 A, Stadler
- US 3993754 A, Rahman
- US 4145410 A, Sears
- US 4235871 A, Papahadjopoulos
- US 4224179 A, Schneider
- US 4522803 A, Lenk
- US 4588578 A, Fountain
- US 31529899 A
- US 88682997 A
- US 09108673 B
- US 09256515 B
- US 09082624 B
- US 09315298 B
- US 11694215 B

**Non-patent literature cited in the description**

- **AGRAWAL.** *Trends in Biotech.,* 1996, vol. 14, 376-387
- **ZELPHATI, O. et al.** *Antisense. Res. Dev.,* 1993, vol. 3, 323-338
- **THIERRY, A.R. et al.** Gene Regulation: Biology of Antisense RNA and DNA. Raven Press, 1992, 147-161
- **UHLMANN E. et al.** Antisense: Chemical Modifications. Encyclopedia of Cancer. Academic Press Inc, 1997, 64-81
- **VLASSOV et al.** *Biochim. Biophys. Acta,* 1994, vol. 1197, 95-1082
- **GALBRAITH et al.** *Antisense Nucl. Acid Drug Des.,* 1994, vol. 4, 201-206
- *Biochim Biophys Acta,* 19 October 1979, vol. 557 (1), 9-23
- *Biochim Biophys Acta,* 02 October 1980, vol. 601 (3), 559-7
- *Biochim Biophys Acta,* 13 June 1986, vol. 858 (1), 161-8
- *Biochim. Biophys. Acta,* 1985, vol. 812, 55-65
- *Pharmaceuticals Research,* March 2005, vol. 22 (3), 362-372
- **JIA et al.** *Biochem. Biophys. Res. Comm.,* 2002, vol. 297, 206-13
- **BIELICKI ; ODA.** *Biochemistry,* 2002, vol. 41, 2089-96
- **WEISGRABER et al.** Human E apoprotein heterogeneity: cysteine-arginine interchanges in the amino acid sequence of the apo-E isoforms. *J. Biol. Chem.,* 1981, vol. 256, 9077-9083

- **RALL et al.** Structural basis for receptor binding heterogeneity of apolipoprotein E from type III hyperlipoproteinemic subjects. *Proc. Nat. Acad. Sci.,* 1982, vol. 79, 4696-4700
- **DUVERGER et al.** *Arterioscler. Thromb. Vasc. Biol.,* 1996, vol. 16 (12), 1424-29
- **KLON et al.** *Biophys. J.,* 2000, vol. 79 (3), 1679-87
- **FRANCESCHINI et al.** *J. Biol. Chem.,* 1985, vol. 260, 1632-35
- **DAUM et al.** *J. Mol. Med.,* 1999, vol. 77, 614-22
- **SHELNESS et al.** *J. Biol. Chem.,* 1985, vol. 260 (14), 8637-46
- **SHELNESS et al.** *J. Biol. Chem.,* 1984, vol. 259 (15), 9929-35
- **DUVERGER et al.** *Euro. J. Biochem.,* 1991, vol. 201 (2), 373-83
- **APOE ; MCLEAN et al.** *J. Biol. Chem.,* 1983, vol. 258 (14), 8993-9000
- **WEISGRABER.** *J. Lipid Res.,* 1990, vol. 31 (8), 1503-11
- **HIXSON ; POWERS.** *J. Lipid Res.,* 1991, vol. 32 (9), 1529-35
- **LACKNER et al.** *J. Biol. Chem.,* 1985, vol. 260 (2), 703-6
- **HOEG et al.** *J. Biol. Chem.,* 1986, vol. 261 (9), 3911-4
- **GORDON et al.** *J. Biol. Chem.,* 1984, vol. 259 (1), 468-74
- **POWELL et al.** *Cell,* 1987, vol. 50 (6), 831-40
- **AVIRAM et al.** *Arterioscler. Thromb. Vase. Biol.,* 1998, vol. 18 (10), 1617-24
- **AVIRAM et al.** *J. Clin. Invest.,* 1998, vol. 101 (8), 1581-90
- **BILLECKE et al.** *Drug Metab. Dispos.,* 2000, vol. 28 (11), 1335-42
- **DRAGANOV et al.** *J. Biol. Chem.,* 2000, vol. 275 (43), 33435-42
- **STEINMETZ ; UTERMANN.** *J. Biol. Chem.,* 1985, vol. 260 (4), 2258-64
- **WIDLER et al.** *J. Biol. Chem.,* 1980, vol. 255 (21), 10464-71
- **DYER et al.** *J. Lipid Res.,* 1995, vol. 36 (1), 80-8
- **SACRE et al.** *FEBS Lett.,* 2003, vol. 540 (1-3), 181-7
- **WEERS et al.** *Biophys. Chem.,* 2003, vol. 100 (1-3), 481-92
- **GONG et al.** *J. Biol. Chem.,* 2002, vol. 277 (33), 29919-26
- **OHTA et al.** *J. Biol. Chem.,* 1984, vol. 259 (23), 14888-93
- **AVIRAM et al.** *Arterioscler. Thromb. Vasc. Biol.,* 1998, vol. 18 (10), 1617-24
- **SORENSON et al.** *Arterioscler. Thromb. Vasc. Biol.,* 1999, vol. 19 (9), 2214-25
- **PALGUNACHARI.** *Arterioscler. Throb. Vasc. Biol.,* 1996, vol. 16 (2), 328-38
- **THURBERG et al.** *J. Biol. Chem.,* vol. 271, 6062-70
- **DYER.** *J. Biol. Chem.,* 1991, vol. 266 (23), 150009-15
- **HILL.** *J. Biol. Chem.,* 1998, vol. 273 (47), 30979-84
- **MULUGETA et al.** *J. Chromatogr.,* 1998, vol. 798 (1-2), 83-90
- **CHUNG et al.** *J. Lipid Res.,* 1980, vol. 21 (3), 284-91
- **CHEUNG et al.** *J. Lipid Res.,* 1987, vol. 28 (8), 913-29
- **PERSSON et al.** *J. Chromatogr.,* 1998, vol. 711, 97-109
- **MERRIFIELD.** *J. Am. Chem. Soc.,* 1963, vol. 85, 2149-2154
- **BODANSZKY et al.** Peptide Synthesis. John Wiley & Sons, 1976
- Solid Phase Peptide. **STUART ; YOUNG.** Synthesis. Pierce Chemical Company, 1984
- The Proteins. Academic Press, 1976, vol. II, 105-237
- **MCOMIE.** Protective Groups in Organic Chemistry. Plenum Press, 1973
- **GREEN, T.W.** PROTECTIVE GROUPS IN ORGANIC SYNTHESIS. Wiley-Interscience, 1999
- **SAPRA, P. ; ALLEN, TM.** *Prog. Lipid Res.,* 2003, vol. 42 (5), 439-62
- **ABRA, RM et al.** *J. Liposome Res.,* 2002, vol. 12, 1-3
- **ALLEN et al.** *Biochimica et Biophysica Acta,* 1995, vol. 1237, 99-108
- **DEFREES et al.** *Journal of the American Chemistry Society,* 1996, vol. 118, 6101-6104
- **BLUME et al.** *Biochimica et Biophysica Acta,* 1993, vol. 1149, 180-184
- **KLIBANOV et al.** *Journal of Liposome Research,* 1992, vol. 2, 321-334
- **ZALIPSKY.** *Bioconjugate Chemistry,* 1993, vol. 4, 296-299
- **ZALIPSKY.** *FEBS Letters,* 1994, vol. 353, 71-74
- **ZALIPSKY.** Stealth Liposomes. CRC Press, 1995
- **KIRPOTIN et al.** *FEBS Letters,* 1996, vol. 388, 115-118
- **RENNEISEN et al.** *J. Bio. Chem.,* 1990, vol. 265, 16337-16342
- **LEONETTI et al.** *Proc. Natl. Acad. Sci. (USA),* 1990, vol. 87, 2448-2451
- Covalent Attachment of Proteins to Liposomes. **HEATH.** Methods in Enzymology. Academic Press, Inc, 1987, vol. 149, 111-119
- **DE FOUGEROLLES, A. et al.** *Nature Reviews,* 2007, vol. 6, 443-453

- **LAMBERTON, J.S. ; CHRISTIAN, A.T.** *Molecular Biotechnology,* 2003, vol. 24, 111-119
- **ELSHABIR, S.M. et al.** *Nature,* 2001, vol. 411, 494-498
- **CAPLEN, N. et al.** *Proc. Natl. Acad. Sci. USA,* 2001, vol. 98, 9746-9747
- **BROWN, D. et al.** *TechNotes,* vol. 9 (1), 1-7
- **ELSHABIR, S.M. et al.** *EMBO,* 2001, vol. 20, 6877-6888
- **ELSHABIR, S. et al.** *Nature,* 2001, vol. 411, 494-498
- **ELSHABIR, S. et al.** *EMBO J.,* 2001, vol. 20, 6877-6888
- **PADDISON, P. et al.** *Genes Dev.,* 2002, vol. 16 (8), 948-58
- **PADDISON, P. et al.** *Proc. Natl. Acad. Sci. USA,* 2002, vol. 99 (3), 1443-1448
- **PADDISON et al.** *Genes & Dev.,* 2002, vol. 16 (8), 948-58
- **GRIFFITHS-JONES S ; GROCOCK RJ ; VAN DONGEN S ; BATEMAN A ; EMIGHT AJ.** miRBase: microRNA sequences, targets and gene nomenclature. *NAR,* 2006, vol. 34, D140-D144
- **GRIFFITHS-JONES S.** he microRNA Registry. *NAR,* 2004, vol. 32, D109-D111
- **JASKULSKI et al.** *Science,* 10 June 1988, vol. 240 (4858), 1544-6
- **VASANTHAKUMAR ; AHMED.** *Cancer Commun.,* 1989, vol. 1 (4), 225-32
- **PERIS et al.** *Brain Res Mol Brain Res.,* 15 June 1998, vol. 57 (2), 310-20
- **ALTSCHUL et al.** *Nucleic Acids Res.,* 1997, vol. 25 (17), 3389-402
- **KIM ; CECH.** *Proc Natl Acad Sci USA.,* December 1987, vol. 84 (24), 8788-92
- **FORSTER ; SYMONS.** *Cell,* 24 April 1987, vol. 49 (2), 211-20
- **CECH et al.** *Cell,* December 1981, vol. 27 (3), 487-96
- **MICHEL ; WESTHOF.** *J Mol Biol.,* 05 December 1990, vol. 216 (3), 585-610
- **REINHOLD-HUREK ; SHUB.** *Nature,* 14 May 1992, vol. 357 (6374), 173-6
- **ROSSI et al.** *Nucleic Acids Res.,* 11 September 1992, vol. 20 (17), 4559-65
- **HAMPEL ; TRITZ.** *Biochemistry,* 13 June 1989, vol. 28 (12), 4929-33
- **HAMPEL et al.** *Nucleic Acids Res.,* 25 January 1990, vol. 18 (2), 299-304
- **PERROTTA ; BEEN.** *Biochemistry,* 01 December 1992, vol. 31 (47), 11843-52
- **GUERRIER-TAKADA et al.** *Cell,* December 1983, vol. 35 (3), 849-57
- **SAVILLE ; COLLINS.** *Cell,* 18 May 1990, vol. 61 (4), 685-96
- **SAVILLE ; COLLINS.** *Proc Natl Acad Sci USA.,* 01 October 1991, vol. 88 (19), 8826-30
- **COLLINS ; OLIVE.** *Biochemistry,* 23 March 1993, vol. 32 (11), 2795-9
- **RANEY et al.** *Journal of Pharmacology and Experimental Therapeutics,* 2001, vol. 298, 1185-1192
- **KURRECK, J.** Antisense technologies. Improvement through novel chemical modifications. *Eur J Biochem,* 2003, vol. 270, 1628-44
- **KURRECK, J.** *Eur. J. Biochem.,* 2003, vol. 270, 1628-44
- **HALL et al.** *Nucleic Acids Res.,* 2004, vol. 32, 5991-6000
- **ZHANG, H.Y. ; DU, Q. ; WAHLESTEDT, C. ; LIANG, Z.** RNA Interference with chemically modified siRNA. *Curr Top Med Chem,* 2006, vol. 6, 893-900
- Antisense Research and Applications. CRC Press, 1993, 276-278
- **MANOHARAN, M.** RNA interference and chemically modified small interfering RNAs. *Curr Opin Chem Biol,* 2004, vol. 8, 570-9
- **HOLEN, T. ; AMARZGUIOUI, M. ; BABAIE, E. ; PRYDZ, H.** Similar behaviour of single-strand and double-strand siRNAs suggests they act through a common RNAi pathway. *Nucleic Acids Res,* 2003, vol. 31, 2401-7
- **PRAKASH, T.P. ; ALLERSON, C.R. ; DANDE, P. ; VICKERS, T.A. ; SIOUFI, N. ; JARRES, R. ; BAKER, B.F. ; SWAYZE, E.E. ; GRIFFEY, R.H. ; BHAT, B.** Positional effect of chemical modifications on short interference RNA activity in mammalian cells. *J Med Chem,* 2005, vol. 48, 4247-53
- **MARTIN et al.** *Helv. Chim. Acta,* 1995, vol. 78, 486-504
- **NIELSEN et al.** *Science,* 1991, vol. 254, 1497-1500
- **HEINJE, G.** Sequence Analysis in Molecular Biology. Academic Press, 1987
- Sequence Analysis Primer. M. Stockton Press, 1991
- **KUNKEL et al.** *Brit. Med. Bull.,* 1989, vol. 45 (3), 630-643
- **GOODFELLOW.** *Nature,* 1989, vol. 341, 102-103
- **BENNETT et al.** *Mol. Pharm.,* 1992, vol. 41, 1023-1033
- **ZHU et al.** *Science,* 1993, vol. 261, 209-211
- **HYDE et al.** *Nature,* 1993, vol. 362, 250-256
- **BRIGHAM et al.** *Am. J. Med. Sci.,* 1989, vol. 298, 278-281
- **STRAUBRINGER et al.** METHODS IN ENZYMOLOGY. Academic Press, 1983, vol. 101, 512-527
- **MANNINO et al.** *Biotechniques,* 1988, vol. 6, 682-690
- **NICOLAU et al.** *Crit. Rev. Ther. Drug Carrier Syst.,* 1989, vol. 6, 239-271

- **BEHR.** *Acc. Chem. Res.,* 1993, vol. 26, 274-278
- **BRIGHAM et al.** *Am. J. Sci.,* 1989, vol. 298 (4), 278-281
- **CULVER.** Human Gene Therapy. MaryAnn Liebert, Inc., Publishers, 1994, 70-71